(19) Europäisches Patentamt
European Patent Office
Office européen des brevets

(11) EP 3 953 356 B1

(12) **EUROPEAN PATENT SPECIFICATION**

(45) Date of publication and mention of the grant of the patent:
**26.07.2023 Bulletin 2023/30**

(21) Application number: **20717841.9**

(22) Date of filing: **07.04.2020**

(51) International Patent Classification (IPC):
***C07D 487/04*** *(2006.01)* ***C07D 519/00*** *(2006.01)*
***A61P 37/00*** *(2006.01)* ***A61K 31/4985*** *(2006.01)*

(52) Cooperative Patent Classification (CPC):
**C07D 487/04; A61P 37/00; C07D 519/00**

(86) International application number:
**PCT/EP2020/059831**

(87) International publication number:
**WO 2020/207991 (15.10.2020 Gazette 2020/42)**

(54) **HEXAHYDRO-1H-PYRAZINO[1,2-A]PYRAZINE COMPOUNDS FOR THE TREATMENT OF AUTOIMMUNE DISEASE**

HEXAHYDRO-1H-PYRAZINO[1,2-A]PYRAZINVERBINDUNGEN ZUR BEHANDLUNG VON AUTOIMMUNERKRANKUNGEN

COMPOSÉS HEXAHYDRO-1H-PYRAZINO[1,2-A]PYRAZINE POUR LE TRAITEMENT D'UNE MALADIE AUTO-IMMUNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **09.04.2019 PCT/CN2019/081900**
**28.11.2019 PCT/CN2019/121598**
**06.03.2020 PCT/CN2020/078225**

(43) Date of publication of application:
**16.02.2022 Bulletin 2022/07**

(73) Proprietor: **F. Hoffmann-La Roche AG**
**4070 Basel (CH)**

(72) Inventors:
- **ZHU, Wei**
**Shanghai, 201203 (CN)**
- **ZHANG, Zhiwei**
**Shanghai, 201203 (CN)**
- **ZHANG, Zhisen**
**Shanghai, 201203 (CN)**
- **SHEN, Hong**
**Shanghai, 201203 (CN)**
- **LIU, Yongfu**
**Shanghai, 201203 (CN)**
- **LIU, Yafei**
**Shanghai, 201203 (CN)**
- **LIU, Haixia**
**Shanghai, 201203 (CN)**
- **KOU, Buyu**
**Shanghai, 201203 (CN)**
- **DEY, Fabian**
**4070 Basel (CH)**
- **DAI, Lue**
**Shanghai, 201203 (CN)**
- **ZHU, Linuo**
**Shanghai, 201203 (CN)**

(74) Representative: **Sauer, Frank**
**F. Hoffmann-La Roche AG**
**Patent Department**
**Grenzacherstrasse 124**
**4070 Basel (CH)**

(56) References cited:
**WO-A1-2017/106607 WO-A1-2019/238629**
**WO-A1-2020/064792**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 953 356 B1

## Description

The present invention relates to organic compounds useful for therapy and/or prophylaxis in a mammal, and in particular to antagonist of TLR7 and/or TLR8 and/or TLR9 useful for treating systemic lupus erythematosus or lupus nephritis.

## FIELD OF THE INVENTION

Autoimmune connective tissue disease (CTD) include prototypical autoimmune syndromes such as Systemic Lupus Erythematosus (SLE), primary Sjögren's syndrome (pSjS), mixed connective tissue disease (MCTD), Dermatomyositis/Polymyositis (DM/PM), Rheumatoid Arthritis (RA), and systemic sclerosis (SSc). With the exception of RA, no really effective and safe therapies are available to patients. SLE represents the prototypical CTD with a prevalence of 20-150 per 100,000 and causes broad inflammation and tissue damage in distinct organs, from commonly observed symptoms in the skin and joints to renal, lung, or heart failure. Traditionally, SLE has been treated with nonspecific anti-inflammatory or immunosuppressive drugs. However, long term usage of immunosuppressive drug, e.g. corticosteroids is only partially effective, and is associated with undesirable toxicity and side effects. Belimumab is the only FDA-approved drug for lupus in the last 50 years, despite its modest and delayed efficacy in only a fraction of SLE patients (Navarra, S. V. et al Lancet 2011, 377, 721.). Other biologics, such as anti-CD20 mAbs, mAbs against or soluble receptors of specific cytokines, have failed in most clinical studies. Thus, novel therapies are required that provide sustained improvement in a greater proportion of patient groups and are safer for chronic use in many autoimmune as well as autoinflammation diseases.

Toll Like Receptors (TLR) are an important family of pattern recognition receptors (PRR) which can initiate broad immune responses in a wide variety of immune cells. As natural host defense sensors, endosomal TLRs 7, 8 and 9 recognize nucleic acids derived from viruses, bacteria; specifically, TLR7/8 and TLR9 recognize single-stranded RNA (ssRNA) and single-stranded CpG-DNA, respectively. However, aberrant nucleic acid sensing of TRL7,8,9 is considered as a key node in a broad of autoimmune and auto-inflammatory diseases (Krieg, A. M. et al. Immunol. Rev. 2007, 220, 251. Jiménez-Dalmaroni, M. J. et al Autoimmun Rev. 2016, 15, 1. Chen, J. Q., et al. Clinical Reviews in Allergy & Immunology 2016, 50, 1.). Anti-RNA and anti-DNA antibodies are well established diagnostic markers of SLE, and these antibodies can deliver both self-RNA and self-DNA to endosomes. While self-RNA complexes can be recognized by TLR7 and TLR8, self-DNA complexes can trigger TLR9 activation. Indeed, defective clearance of self-RNA and self-DNA from blood and/or tissues is evident in SLE (Systemic Lupus Erythematosus) patients. TLR7 and TLR9 have been reported to be upregulated in SLE tissues, and correlate with chronicity and activity of lupus nephritis, respectively. In B cells of SLE patients, TLR7 expression correlates with anti-RNP antibody production, while TLR9 expression with IL-6 and anti-dsDNA antibody levels. Consistently, in lupus mouse models, TLR7 is required for anti-RNA antibodies, and TLR9 is required for anti-nucleosome antibody. On the other hand, overexpression of TLR7 or human TLR8 in mice promotes autoimmunity and autoinflammation. Moreover, activation of TLR8 specifically contributes to inflammatory cytokine secretion of mDC/macrophages, neutrophil NETosis, induction of Th17 cells, and suppression of Treg cells. In addition to the described role of TLR9 in promoting autoantibody production of B cells, activation of TLR9 by self-DNA in pDC also leads to induction of type I IFNs and other inflammatory cytokines. Given these roles of TLR9 in both pDC and B cells, both as key contributors to the pathogenesis of autoimmune diseases, and the extensive presence of self-DNA complexes that could readily activate TLR9 in many patients with autoimmune diseases, it may have extra benefit to further block self-DNA mediated TLR9 pathways on top of inhibition of TLR7 and TLR8 pathways. Taken together, TLR7, 8, and 9 pathways represent new therapeutic targets for the treatment of autoimmune and auto-inflammatory diseases, for which no effective steroid-free and non-cytotoxic oral drugs exist, and inhibition of all these pathways from the very upstream may deliver satisfying therapeutic effects. As such, we invented oral compounds that target and suppress TLR7, TLR8 and TLR9 for the treatment of autoimmune and auto-inflammatory diseases Closest compounds to the present for the same use are disclosed in WO 2017/106607.

## SUMMARY OF THE INVENTION

The present invention relates to novel compounds of formula (I) or (Ia) or (Ib),

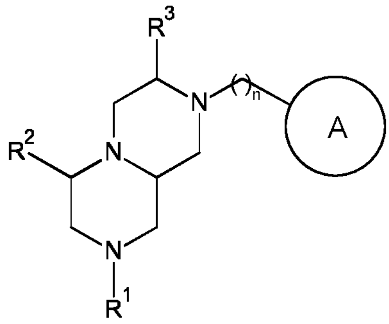

(I) or

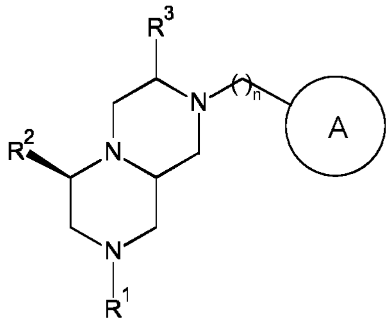

(Ia)

or

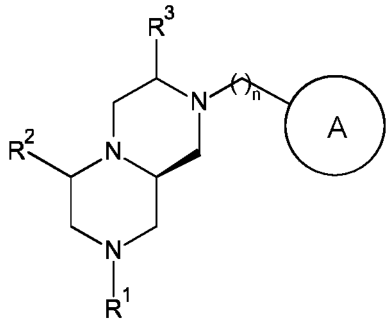

(Ib)

wherein

R1 is

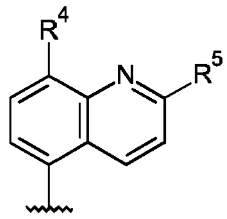
,
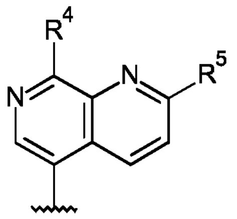
,
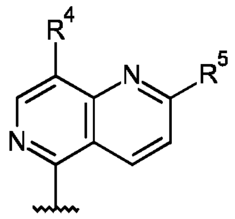
,
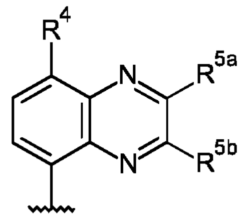
,

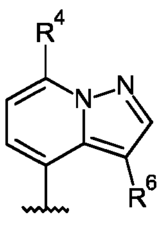
,
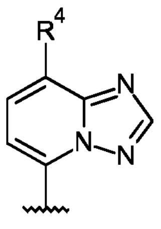
,
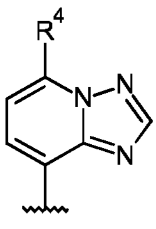
,
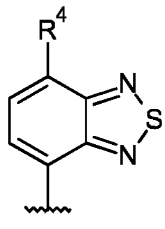
,
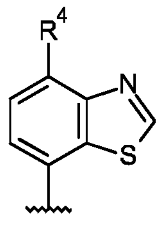
,

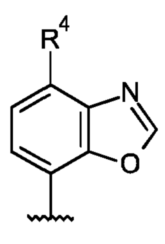
,
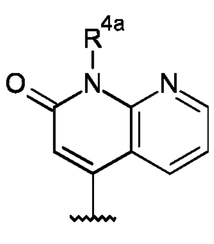
or
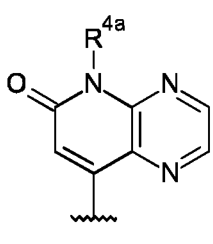
;

wherein $R^4$ is $C_{1-6}$alkyl, $C_{1-6}$alkoxy, halo$C_{1-6}$alkyl, halogen, nitro or cyano; $R^{4a}$ is $C_{1-6}$alkyl or $C_{3-7}$cycloalkyl; $R^5$, $R^{5a}$

and $R^{5b}$ are independently selected from H and deuterium; $R^6$ is H or halogen;
$R^2$ is H or $C_{1-6}$alkyl;
$R^3$ is H;
Ring A is a 5-7 membered monocyclic aryl or heteroaryl; or a 7-12 membered bicyclic heterocyclyl;
or a pharmaceutically acceptable salt thereof.

Another object of the present invention is related to novel compounds of formula (I) or (Ia), their manufacture, medicaments based on a compound in accordance with the invention and their production as well as the use of compounds of formula (I) or (Ia) as TLR7 and/or TLR8 and/or TLR9 antagonist, and for the treatment or prophylaxis of systemic lupus erythematosus or lupus nephritis. The compounds of formula (I) or (Ia) show superior TLR7 and/or TLR8 and/or TLR9 antagonism activity. In addition, the compounds of formula (I) or (Ia) also show good cytotoxicity, phototoxicity, solubility, hPBMC, human microsome stability and SDPK profiles, as well as low CYP inhibition.

## DETAILED DESCRIPTION OF THE INVENTION

### DEFINITIONS

The term "$C_{1-6}$alkyl" denotes a saturated, linear or branched chain alkyl group containing 1 to 6, particularly 1 to 4 carbon atoms, for example methyl, ethyl, *n*-propyl, isopropyl, *n*-butyl, isobutyl, *tert*-butyl and the like. Particular "$C_{1-6}$alkyl" groups are methyl, ethyl and *n*-propyl.

The term "halogen" and "halo" are used interchangeably herein and denote fluoro, chloro, bromo, or iodo.

The term "halo$C_{1-6}$alkyl" denotes a $C_{1-6}$alkyl group wherein at least one of the hydrogen atoms of the $C_{1-6}$alkyl group has been replaced by same or different halogen atoms, particularly fluoro atoms. Examples of halo$C_{1-6}$alkyl include monofluoro-, difluoro- or trifluoro-methyl, - ethyl or -propyl, for example 3,3,3-trifluoropropyl, 2-fluoroethyl, trifluoroethyl, fluoromethyl, difluoromethyl, difluoroethyl or trifluoromethyl.

The term "halopyrrolidinyl" denotes a pyrrolidinyl substituted once, twice or three times by halogen. Examples of halopyrrolidinyl include, but not limited to, difluoropyrrolidinyl and fluoropyrrolidinyl.

The term "$C_{3-7}$cycloalkyl" denotes a saturated carbon ring containing from 3 to 7 carbon atoms, particularly from 3 to 6 carbon atoms, for example, cyclopropyl, cyclobutyl, cyclopentyl, cyclohexyl, cycloheptyl and the like. Particular "$C_{3-7}$cycloalkyl" groups are cyclopropyl, cyclopentyl and cyclohexyl.

The term "aryl" denotes an aromatic hydrocarbon mono- or bicyclic ring system of 5 to 12 ring atoms. Examples of aryl include, but not limited to, phenyl and naphthyl. Aryl can be further substituted by substituents include, but not limited to, $C_{1-6}$alkyl; 3,4,4a,5,7,7a-hexahydro-2H-pyrrolo[3,4-b][1,4]oxazinyl; 1,4-diazepanyl; 2,6-diazaspiro[3.3]heptanyl substituted by $C_{1-6}$alkyl; 5-oxa-2,8-diazaspiro[3.5]nonanyl; amino-1,4-oxazepanyl; azetidinyl substituted by one or two substituents independently selected from amino and $C_{1-6}$alkyl; piperazinyl unsubstituted or substituted by $C_{1-6}$alkyl; and pyrrolidinyl substituted by one or two substituents independently selected from amino, $C_{1-6}$alkoxy and halogen.

The term "heteroaryl" denotes an aromatic heterocyclic mono- or bicyclic ring system of 5 to 12 ring atoms, comprising 1, 2, 3 or 4 heteroatoms selected from N, O and S, the remaining ring atoms being carbon. Examples of heteroaryl moieties include, but not limited to, pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl or quinoxalinyl. Heteroaryl can be further substituted by substituents include, but not limited to, $C_{1-6}$alkyl; 3,4,4a,5,7,7a-hexahydro-2H-pyrrolo[3,4-b][1,4]oxazinyl; 1,4-diazepanyl; 2,6-diazaspiro[3.3]heptanyl substituted by $C_{1-6}$alkyl; 5-oxa-2,8-diazaspiro[3.5]nonanyl; amino-1,4-oxazepanyl; azetidinyl substituted by one or two substituents independently selected from amino and $C_{1-6}$alkyl; piperazinyl unsubstituted or substituted by $C_{1-6}$alkyl; and pyrrolidinyl substituted by one or two substituents independently selected from amino, $C_{1-6}$alkoxy and halogen.

The term "heterocyclyl" or "heterocyclic" denotes a monovalent saturated or partly unsaturated mono or bicyclic ring system of 3 to 12 ring atoms, comprising 1 to 5 ring heteroatoms selected from N, O and S, the remaining ring atoms being carbon. In particular embodiments, heterocyclyl is a monovalent saturated monocyclic ring system of 4 to 7 ring atoms, comprising 1, 2, or 3 ring heteroatoms selected from N, O and S, the remaining ring atoms being carbon. Examples for monocyclic saturated heterocyclyl are aziridinyl, oxiranyl, azetidinyl, oxetanyl, pyrrolidinyl, tetrahydrofuranyl, tetrahydrothienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl. Examples for bicyclic saturated heterocyclic ring are azabicyclo[3.2.1]octyl, quinuclidinyl, oxaazabicyclo[3.2.1]octanyl, azabicyclo[3.3.1]nonanyl, oxaaza-bicyclo[3.3.1]nonanyl, azabicyclo[3.1.0]hexanyl, oxodiazaspiro[3.4]octanyl, acetyloxodiazaspiro[3.4]octanyl, thiaazabicyclo[3.3.1]nonanyl, oxoazaspiro[2.4]heptanyl, oxoazaspiro[3.4]octanyl, oxoazabicyclo[3.1.0]hexanyl and dioxotetrahydropyrrolo[1,2-a]pyrazinyl. Examples for bicyclic heterocyclyl include, but not limited to, ; 2,5-diazabicyclo[2.2.1]heptanyl; 3,4,4a,5,7,7a-hexahydro-2H-pyrrolo[3,4-b][1,4]oxazinyl; 3,6-diazabicyclo[3.1.1]heptanyl; 3,8-diazabicyclo[3.2.1]octanyl; 5-oxa-2,8-diazaspiro[3.5]nonanyl; 9-oxa-3,7-diazabicyclo[3.3.1]nonanyl; 2,6-diazaspiro[3.3]heptanecarbonyl; 1,2,3,4-tetrahydroisoquinolinyl; 5,6,7,8-tetrahydro-1,6-naphthyridinyl; 5,6,7,8-tetrahydro-2,6-naphthyridinyl; 5,6,7,8-tetrahydro-2,7-naphthyridinyl; isoindolinyl.

The term "cis-isomers" and "*trans*-isomers" denote the relative stereochemistry of the molecule or moiety. For example: Intermediate B (

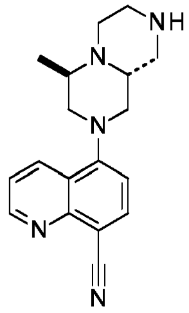

) as the "trans-isomers" refers to a mixture of

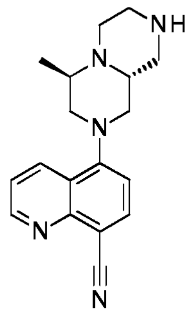

and

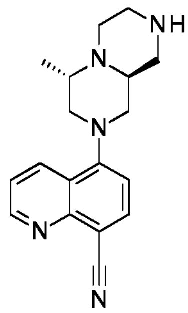

;

similarly, Intermediate A (

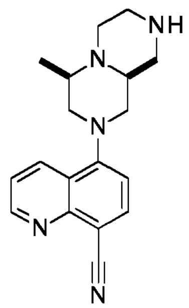

) as the "cis-isomers" refers to a mixture of

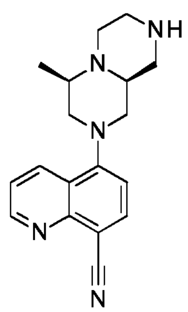

and

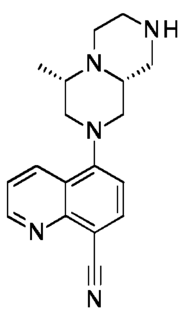

.

The way of showing relative stereochemistry also applies to the final compounds.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "A pharmaceutically active metabolite" denotes a pharmacologically active product produced through metabolism in the body of a specified compound or salt thereof. After entry into the body, most drugs are substrates for chemical reactions that may change their physical properties and biologic effects. These metabolic conversions, which usually affect the polarity of the compounds of the invention, alter the way in which drugs are distributed in and excreted from the body. However, in some cases, metabolism of a drug is required for therapeutic effect.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

**ANTAGONIST OF TLR7 AND/OR TLR8 AND/OR TLR9**

The present invention relates to (i) a compound of formula (I),

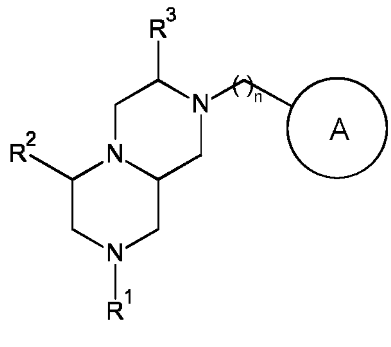

(I),

wherein

$R^1$ is

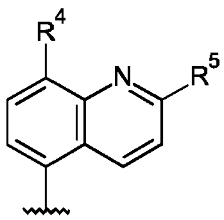 , 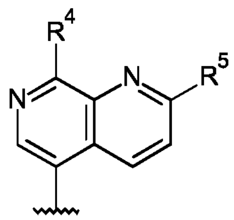 , 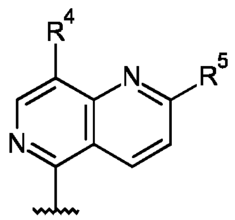 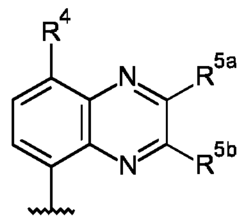 ,

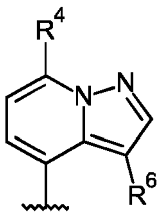 , 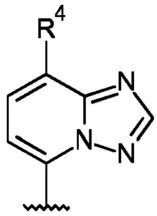 , 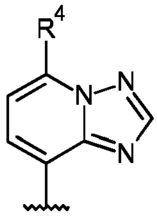 , 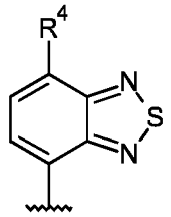 , 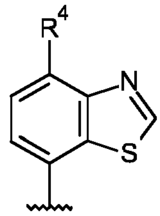 ,

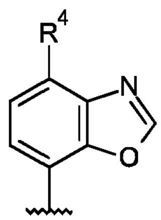 , 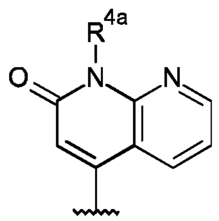 or 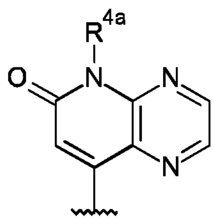 ;

wherein $R^4$ is $C_{1-6}$alkyl, $C_{1-6}$alkoxy, halo$C_{1-6}$alkyl, halogen, nitro or cyano; $R^{4a}$ is $C_{1-6}$alkyl or $C_{3-7}$cycloalkyl; $R^5$, $R^{5a}$ and $R^{5b}$ are independently selected from H and deuterium; $R^6$ is H or halogen;

$R^2$ is H or $C_{1-6}$alkyl;

$R^3$ is H;

Ring A is an unsubstituted or substituted 5-7 membered monocyclic aryl or heteroaryl; or an unsubstituted or substituted 7-12 membered bicyclic heterocyclyl;

n is 0, 1 or 2;

or a pharmaceutically acceptable salt thereof.

A further embodiment of present invention is (ii) a compound of formula (I), wherein Ring A is 1,2,3,4-tetrahydroisoquinolinyl;

5,6,7,8-tetrahydro-1,6-naphthyridinyl;
5,6,7,8-tetrahydro-2,6-naphthyridinyl;
5,6,7,8-tetrahydro-2,7-naphthyridinyl;
isoindolinyl;
phenyl substituted by amino($C_{1-6}$alkoxy)pyrrolidinyl, 5-oxa-2,8-diazaspiro[3.5]nonanyl, morpholinyl or piperazinyl;
pyridinyl substituted once or twice by substituents independently selected from (halopyrrolidinyl)amino; 1,4-diazepanyl; 2,5-diazabicyclo[2.2.1]heptanyl; 3,4,4a,5, 7, 7 a-hexahydro-2H-pyrrolo[3,4-b] [1,4]oxazinyl; 3,6-diazabicyclo[3.1.1]heptanyl; 3,8-diazabicyclo[3.2.1]octanyl; 5-oxa-2,8-diazaspiro[3.5]nonanyl; 9-oxa-3,7-diazabicyclo[3.3.1]nonanyl; amino($C_{1-6}$alkoxy)pyrrolidinyl; amino($C_{1-6}$alkyl)azetidinyl; amino($C_{1-6}$alkyl)pyrrolidinyl; amino-1,4-oxazepanyl; amino-2-azaspiro[3.3]heptanyl; aminoazetidinyl; aminohalopyrrolidinyl; $C_{1-6}$alkyl; $C_{1-6}$alkyl-2,6-diazaspiro[3.3]heptanecarbonyl; $C_{1-6}$alkyl-2,6-diazaspiro[3.3]heptanyl; $C_{1-6}$alkylpiperazinyl and piperazinyl; or
pyrimidinyl substituted once or twice by substituents independently selected from amino($C_{1-6}$alkoxy)pyrrolidinyl, amino($C_{1-6}$alkyl)azetidinyl, aminoazetidinyl, $C_{1-6}$alkyl and piperazinyl.

A further embodiment of present invention is (iii) a compound of formula (Ib),

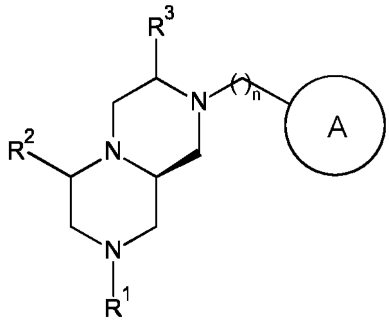

(Ib),

wherein

$R^1$ is

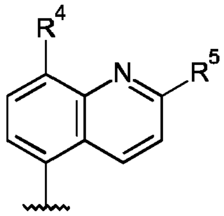
,
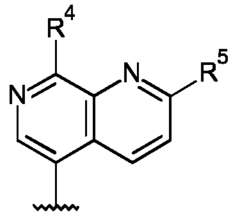
,
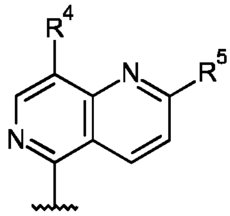
,
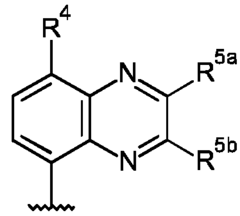
,

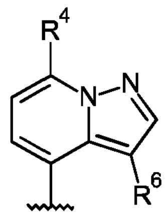
,
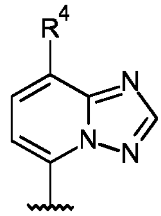
,
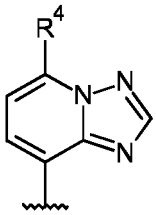
,
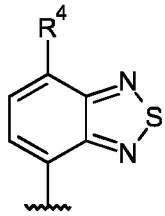
,
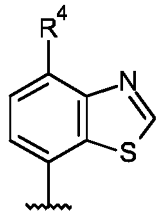
,

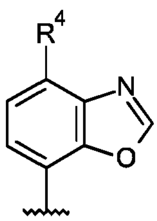
,
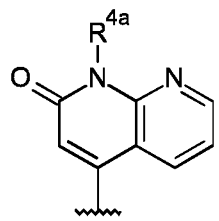
or
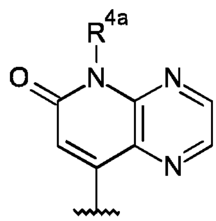
;

wherein $R^4$ is $C_{1-6}$alkyl, $C_{1-6}$alkoxy, halo$C_{1-6}$alkyl, halogen, nitro or cyano; $R^{4a}$ is $C_{1-6}$alkyl or $C_{3-7}$cycloalkyl; $R^5$, $R^{5a}$ and $R^{5b}$ are independently selected from H and deuterium; $R^6$ is H or halogen;
$R^2$ is H or $C_{1-6}$alkyl;
$R^3$ is H;
Ring A is 1,2,3,4-tetrahydroisoquinolinyl;

5,6,7,8-tetrahydro-1,6-naphthyridinyl;
5,6,7,8-tetrahydro-2,6-naphthyridinyl;
5,6,7,8-tetrahydro-2,7-naphthyridinyl;
isoindolinyl;
phenyl substituted by amino($C_{1-6}$alkoxy)pyrrolidinyl, 5-oxa-2,8-diazaspiro[3.5]nonanyl, morpholinyl or piperazinyl;
pyridinyl substituted once or twice by substituents independently selected from (halopyrrolidinyl)amino; 1,4-diazepanyl; 2,5-diazabicyclo[2.2.1]heptanyl; 3,4,4a,5, 7, 7 a-hexahydro-2H-pyrrolo[3,4-b] [1,4]oxazinyl; 3,6-diazabicyclo[3.1.1]heptanyl; 3,8-diazabicyclo[3.2.1]octanyl; 5-oxa-2,8-diazaspiro[3.5]nonanyl; 9-oxa-3,7-diazabicyclo[3.3.1]nonanyl; amino($C_{1-6}$alkoxy)pyrrolidinyl; amino($C_{1-6}$alkyl)azetidinyl; amino($C_{1-6}$alkyl)pyrrolidinyl; amino-1,4-oxazepanyl; amino-2-azaspiro[3.3]heptanyl; aminoazetidinyl; aminohalopyrrolidinyl; $C_{1-6}$alkyl; $C_{1-6}$alkyl-2,6-diazaspiro[3.3]heptanecarbonyl; $C_{1-6}$alkyl-2,6-diazaspiro[3.3]heptanyl; $C_{1-6}$alkylpiperazinyl and piperazinyl; or

pyrimidinyl substituted once or twice by substituents independently selected from amino($C_{1-6}$alkoxy)pyrrolidinyl, amino($C_{1-6}$alkyl)azetidinyl, aminoazetidinyl, $C_{1-6}$alkyl and piperazinyl;

n is 0, 1 or 2;

or a pharmaceutically acceptable salt thereof.

A further embodiment of present invention is (iv) a compound of formula (I) or (Ia) or (Ib), or a pharmaceutically acceptable salt thereof, according to any one of (i) to (iii), wherein $R^1$ is

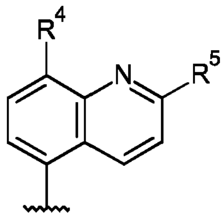 , 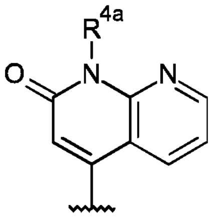 or 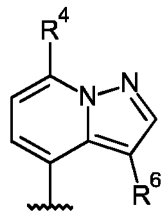 ;

wherein $R^4$ is cyano; $R^{4a}$ is $C_{1-6}$alkyl; $R^5$ is H or deuterium; $R^6$ is H.

A further embodiment of present invention is (v) a compound of formula (I) or (Ia) or (Ib), or a pharmaceutically acceptable salt thereof, according to any one of (i) to (iv), wherein $R^2$ is $C_{1-6}$alkyl.

A further embodiment of present invention is (vi) a compound of formula (I) or (Ia) or (Ib), or a pharmaceutically acceptable salt thereof, according to any one of (i) to (v), wherein

$R^1$ is

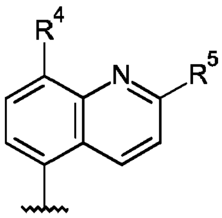 , 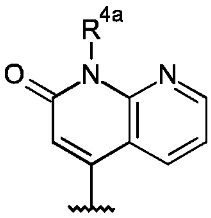 or 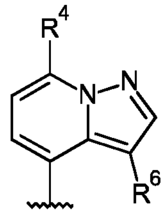 ;

wherein $R^4$ is cyano; $R^{4a}$ is $C_{1-6}$alkyl; $R^5$ is H or deuterium; $R^6$ is H;

$R^2$ is methyl;

$R^3$ is H;

Ring A is 1,2,3,4-tetrahydroisoquinolin-7-yl; 1,2,3,4-tetrahydroisoquinolin-5-yl; 1,2,3,4-tetrahydroisoquinolin-6-yl; 1,2,3,4-tetrahydroisoquinolin-7-yl; 1,2,3,4-tetrahydroisoquinolin-8-yl; 5,6,7,8-tetrahydro-1,6-naphthyridin-2-yl; 5,6,7,8-tetrahydro-1,6-naphthyridin-3-yl; 5,6,7,8-tetrahydro-2,6-naphthyridin-1-yl; 5,6,7,8-tetrahydro-2,7-naphthyridin-4-yl; isoindolin-4-yl; 3-amino-4-methoxy-pyrrolidin-1-ylphenyl; 5-oxa-2,8-diazaspiro[3.5]nonan-2-ylphenyl; morpholin-2-ylphenyl; piperazin-1-ylphenyl; (4-fluoropyrrolidin-3-yl)amino(methyl)pyridinyl; 1,4-diazepan-1-ylpyridinyl; 2,5-diazabicyclo[2.2.1]heptan-2-yl(methyl)pyridinyl; 2,5-diazabicyclo[2.2.1]heptan-2-ylpyridinyl; 2-methylpiperazin-1-yl(methyl)pyridinyl; 3,4,4a,5,7,7a-hexahydro-2H-pyrrolo[3,4-b][1,4]oxazin-6-yl(methyl)pyridinyl; (3,4,4a,5,7,7a-hexahydro-2H-pyrrolo[3,4-b][1,4]oxazin-6-yl)pyridinyl; 3,6-diazabicyclo[3.1.1]heptan-3-yl(methyl)pyridinyl; 3,8-diazabicyclo[3.2.1]octan-3-yl(methyl)pyridinyl; 3,8-diazabicyclo[3.2.1]octan-3-ylpyridinyl; 3-amino-3-methyl-azetidin-1-yl(methyl)pyridinyl; 3-amino-3-methyl-azetidin-1-ylpyridinyl; 3-amino-3-methyl-pyrrolidin-1-yl(methyl)pyridinyl; 3-amino-3-methyl-pyrrolidin-1-ylpyridinyl; 3-amino-4-fluoro-pyrrolidin-1-ylpyridinyl; 3-amino-4-methoxy-pyrrolidin-1-yl(methyl)pyridinyl; 3-amino-4-methoxy-pyrrolidin-1-ylpyridinyl; 3-aminoazetidin-1-ylpyridinyl; 3-methylpiperazin-1-yl(methyl)pyridinyl; 3-methylpiperazin-1-ylpyridinyl; 5-oxa-2,8-diazaspiro[3.5]nonan-2-ylpyridinyl; 6-amino-1,4-oxazepan-4-yl(methyl)pyridinyl; 6-amino-1,4-oxazepan-4-ylpyridinyl; 6-amino-2-azaspiro[3.3]heptan-2-yl(methyl)pyridinyl; 6-methyl-2,6-diazaspiro[3.3]heptan-2-yl(methyl)pyridinyl; 6-methyl-2,6-diazaspiro[3.3]heptan-2-ylpyridinyl; 6-methyl-2,6-diazaspiro[3.3]heptane-2-carbonyl(methyl)pyridinyl; 9-oxa-3,7-diazabicyclo[3.3.1]nonan-3-yl(methyl)pyridinyl; piperazin-1-yl(methyl)pyridinyl; piperazin-1-ylpyridinyl; 3-amino-3-methyl-azetidin-1-yl(methyl)pyrimidinyl; 3-amino-4-methoxy-pyrrolidin-1-yl(methyl)pyrimidinyl; 3-amino-4-methoxy-pyrrolidin-1-ylpyrimidinyl; 3-aminoazetidin-1-ylpyrimidinyl; piperazin-1-yl(methyl)pyrimidinyl or piperazin-1-ylpyrimidinyl;
n is 0, 1 or 2;

or a pharmaceutically acceptable salt thereof.

A further embodiment of present invention is (vii) a compound of formula (I) or (Ia) or (Ib), or a pharmaceutically acceptable salt thereof, according to any one of (i) to (vi), wherein $R^1$ is

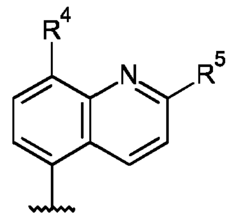

;

wherein $R^4$ is cyano; $R^5$ is H or deuterium.

A further embodiment of present invention is (viii) a compound of formula (I) or (Ia) or (Ib), or a pharmaceutically acceptable salt thereof, according to any one of (i) to (vii), wherein Ring A is 1,2,3,4-tetrahydroisoquinolinyl;

phenyl substituted by morpholinyl;
pyridinyl substituted once or twice by substituents independently selected from 3,4,4a,5, 7, 7 a-hexahydro-2H-pyrrolo[3,4-b] [1,4]oxazinyl; amino($C_{1-6}$alkoxy)pyrrolidinyl; amino($C_{1-6}$alkyl)azetidinyl; aminohalopyrrolidinyl; $C_{1-6}$alkyl; $C_{1-6}$alkyl-2,6-diazaspiro[3.3]heptanyl and piperazinyl;
pyrimidinyl substituted twice by substituents independently selected from amino($C_{1-6}$alkoxy)pyrrolidinyl; amino($C_{1-6}$alkyl)azetidinyl and $C_{1-6}$alkyl.

A further embodiment of present invention is (ix) a compound of formula (I) or (Ia), or a pharmaceutically acceptable salt thereof, according to any one of (i) to (viii), wherein ring A is 1,2,3,4-tetrahydroisoquinolinyl; morpholinylphenyl; piperazinylpyridinyl; (amino($C_{1-6}$alkoxy)pyrrolidinyl)pyridinyl; (aminohalopyrrolidinyl)pyridinyl; piperazinyl($C_{1-6}$alkyl)pyridinyl; (amino($C_{1-6}$alkoxy)pyrrolidinyl)pyridinyl; (aminohalopyrrolidinyl)pyridinyl; ($C_{1-6}$alkyl-2,6-diazaspiro[3.3]heptanyl)pyridinyl; (amino($C_{1-6}$alkyl)azetidinyl)pyridinyl; $C_{1-6}$alkyl(amino($C_{1-6}$alkyl)azetidinyl)pyridinyl; 3,4,4a,5,7,7a-hexahydro-2H-pyrrolo[3,4-b][1,4]oxazinyl($C_{1-6}$alkyl)pyridinyl; (3,4,4a,5,7,7a-hexahydro-2H-pyrrolo[3,4-b][1,4]oxazinyl)pyridinyl; $C_{1-6}$alkyl(amino($C_{1-6}$alkyl)azetidinyl)pyrimidinyl or $C_{1-6}$alkyl(amino($C_{1-6}$alkoxy)pyrrolidinyl)pyrimidinyl.

A further embodiment of present invention is (ix") a compound of formula (I) or (Ia), or a pharmaceutically acceptable salt thereof, according to any one of (i) to (viii), wherein ring A is 1,2,3,4-tetrahydroisoquinolinyl; morpholinylphenyl; piperazinylpyridinyl; (amino($C_{1-6}$alkoxy)pyrrolidinyl)pyridinyl; (aminohalopyrrolidinyl)pyridinyl; piperazinyl($C_{1-6}$alkyl)pyridinyl; (amino($C_{1-6}$alkoxy)pyrrolidinyl)pyridinyl; (aminohalopyrrolidinyl)pyridinyl; ($C_{1-6}$alkyl-2,6-diazaspiro[3.3]heptanyl)pyridinyl; (amino($C_{1-6}$alkyl)azetidinyl)pyridinyl; $C_{1-6}$alkyl(amino($C_{1-6}$alkyl)azetidinyl)pyridinyl; $C_{1-6}$alkyl(3,4,4a,5,7,7a-hexahydro-2H-pyrrolo[3,4-b][1,4]oxazinyl)pyridinyl; $C_{1-6}$alkyl(amino($C_{1-6}$alkyl)azetidinyl)pyrimidinyl or $C_{1-6}$alkyl(amino($C_{1-6}$alkoxy)pyrrolidinyl)pyrimidinyl.

A further embodiment of present invention is (x) a compound of formula (I) or (Ia) or (Ib), or a pharmaceutically acceptable salt thereof, according to any one of (i) to (ix), wherein ring A is 1,2,3,4-tetrahydroisoquinolin-7-yl; morpholin-2-ylphenyl; (3,4,4a,5,7,7a-hexahydro-2H-pyrrolo[3,4-b][1,4]oxazin-6-yl)-2-methyl-3-pyridinyl; (3,4,4a,5,7,7a-hexahydro-2H-pyrrolo[3,4-b] [1,4]oxazin-6-yl)-3-pyridinyl; (3-amino-4-fluoro-pyrrolidin-1-yl)-3-pyridinyl; (3-amino-4-fluoro-pyrrolidin-1-yl)-3-pyridinyl; (3-amino-4-methoxy-pyrrolidin-1-yl)-3-pyridinyl; (3-amino-4-methoxy-pyrrolidin-1-yl)-3-pyridinyl; 2-methyl-6-piperazin-1-yl-3-pyridinyl; 6-(3-amino-3-methyl-azetidin-1-yl)-2-methyl-3-pyridinyl; 6-(3-amino-3-methyl-azetidin-1-yl)-3-pyridinyl; 6-(6-methyl-2,6-diazaspiro[3.3]heptan-2-yl)-3-pyridinyl; 6-piperazin-1-yl-3-pyridinyl; (3-amino-4-methoxy-pyrrolidin-1-yl)-6-methyl-pyrimidin-4-yl or (3-amino-3-methyl-azetidin-1-yl)-6-methyl-pyrimidin-4-yl.

A further embodiment of present invention is (x") a compound of formula (I) or (Ia) or (Ib), or a pharmaceutically acceptable salt thereof, according to any one of (i) to (ix) and (ix"), wherein ring A is 1,2,3,4-tetrahydroisoquinolin-7-yl; morpholin-2-ylphenyl; (3,4,4a,5,7,7a-hexahydro-2H-pyrrolo[3,4-b][1,4]oxazin-6-yl)-2-methyl-3-pyridinyl; (3-amino-4-fluoro-pyrrolidin-1-yl)-3-pyridinyl; (3-amino-4-fluoro-pyrrolidin-1-yl)-3-pyridinyl; (3-amino-4-methoxy-pyrrolidin-1-yl)-3-pyridinyl; (3-amino-4-methoxy-pyrrolidin-1-yl)-3-pyridinyl; 2-methyl-6-piperazin-1-yl-3-pyridinyl; 6-(3-amino-3-methyl-azetidin-1 -yl)-2-methyl-3-pyridinyl; 6-(3-amino-3-methyl-azetidin-1-yl)-3-pyridinyl; 6-(6-methyl-2,6-diazaspiro[3.3]heptan-2-yl)-3-pyridinyl; 6-piperazin-1-yl-3-pyridinyl; (3-amino-4-methoxy-pyrrolidin-1-yl)-6-methyl-pyrimidin-4-yl or (3-amino-3-methyl-azetidin-1-yl)-6-methyl-pyrimidin-4-yl.

A further embodiment of present invention is (xi) a compound of formula (I) or (Ia) or (Ib), or a pharmaceutically acceptable salt thereof, according to any one of (i) to (x), wherein

$R^1$ is

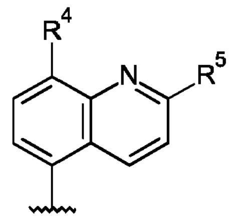

;

wherein $R^4$ is cyano; $R^5$ is H or deuterium;
$R^2$ is $C_{1-6}$alkyl;
$R^3$ is H;
Ring A is 1,2,3,4-tetrahydroisoquinolinyl;

phenyl substituted by morpholinyl;
pyridinyl substituted once or twice by substituents independently selected from 3,4,4a,5, 7, 7 a-hexahydro-2H-pyrrolo[3,4-b] [1,4]oxazinyl; amino($C_{1-6}$alkoxy)pyrrolidinyl; amino($C_{1-6}$alkyl)azetidinyl; aminohalopyrrolidinyl; $C_{1-6}$alkyl; $C_{1-6}$alkyl-2,6-diazaspiro[3.3]heptanyl and piperazinyl;
pyrimidinyl substituted twice by substituents independently selected from amino($C_{1-6}$alkoxy)pyrrolidinyl; amino($C_{1-6}$alkyl)azetidinyl and $C_{1-6}$alkyl;

n is 0, 1 or 2;

or a pharmaceutically acceptable salt thereof.

A further embodiment of present invention is (xii) a compound of formula (I) or (Ia) or (Ib) according to any one of (i) to (xi), wherein

$R^1$ is

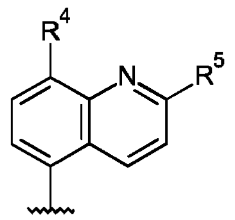

;

wherein $R^4$ is cyano; $R^5$ is H or deuterium;
$R^2$ is $C_{1-6}$alkyl;
$R^3$ is H;
Ring A is 1,2,3,4-tetrahydroisoquinolinyl; morpholinylphenyl; piperazinylpyridinyl; (amino($C_{1-6}$alkoxy)pyrrolidinyl)pyridinyl; (aminohalopyrrolidinyl)pyridinyl; piperazinyl($C_{1-6}$alkyl)pyridinyl; (amino($C_{1-6}$alkoxy)pyrrolidinyl)pyridinyl; (aminohalopyrrolidinyl)pyridinyl; ($C_{1-6}$alkyl-2,6-diazaspiro[3.3]heptanyl)pyridinyl; (amino($C_{1-6}$alkyl)azetidinyl)pyridinyl; $C_{1-6}$alkyl(amino($C_{1-6}$alkyl)azetidinyl)pyridinyl; 3,4,4a,5,7,7a-hexahydro-2H-pyrrolo[3,4-b][1,4]oxazinyl($C_{1-6}$alkyl)pyridinyl; (3,4,4a,5,7,7a-hexahydro-2H-pyrrolo[3,4-b][1,4]oxazinyl)pyridinyl; $C_{1-6}$alkyl(amino($C_{1-6}$alkyl)azetidinyl)pyrimidinyl or $C_{1-6}$alkyl(amino($C_{1-6}$alkoxy)pyrrolidinyl)pyrimidinyl;
n is 0, 1 or 2;

or a pharmaceutically acceptable salt thereof.

A further embodiment of present invention is (xii") a compound of formula (I) or (Ia) or (Ib) according to any

one of (i) to (xi), (ix") and (x"), wherein

$R^1$ is

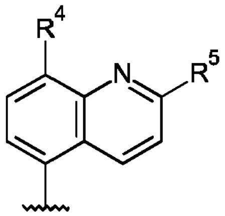

;

wherein $R^4$ is cyano; $R^5$ is H or deuterium;
$R^2$ is $C_{1-6}$alkyl;
$R^3$ is H;
Ring A is 1,2,3,4-tetrahydroisoquinolinyl; morpholinylphenyl; piperazinylpyridinyl; (amino($C_{1-6}$alkoxy)pyrrolidinyl)pyridinyl; (aminohalopyrrolidinyl)pyridinyl; piperazinyl($C_{1-6}$alkyl)pyridinyl; (amino($C_{1-6}$alkoxy)pyrrolidinyl)pyridinyl; (aminohalopyrrolidinyl)pyridinyl; ($C_{1-6}$alkyl-2,6-diazaspiro[3.3]heptanyl)pyridinyl; (amino($C_{1-6}$alkyl)azetidinyl)pyridinyl; $C_{1-6}$alkyl(amino($C_{1-6}$alkyl)azetidinyl)pyridinyl; $C_{1-6}$alkyl(3,4,4a,5,7,7a-hexahydro-2H-pyrrolo[3,4-b][1,4]oxazinyl)pyridinyl; $C_{1-6}$alkyl(amino($C_{1-6}$alkyl)azetidinyl)pyrimidinyl or $C_{1-6}$alkyl(amino($C_{1-6}$alkoxy)pyrrolidinyl)pyrimidinyl;
n is 0, 1 or 2;

or a pharmaceutically acceptable salt thereof.

A further embodiment of present invention is (xiii) a compound of formula (I) or (Ia) or (Ib) according to any one of (i) to (xii), wherein

$R^1$ is

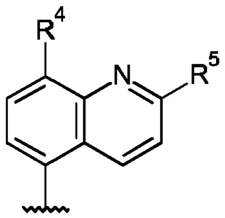

;

wherein $R^4$ is cyano; $R^5$ is H or deuterium;
$R^2$ is methyl;
$R^3$ is H;
Ring A is 1,2,3,4-tetrahydroisoquinolin-7-yl; 4-(morpholin-2-yl)phenyl; (3,4,4a,5,7,7a-hexahydro-2H-pyrrolo[3,4-b][1,4]oxazin-6-yl)-2-methyl-3-pyridinyl; (3,4,4a,5,7,7a-hexahydro-2H-pyrrolo[3,4-b][1,4]oxazin-6-yl)-3-pyridinyl; (3-amino-4-fluoro-pyrrolidin-1-yl)-3-pyridinyl; (3-amino-4-fluoro-pyrrolidin-1-yl)-3-pyridinyl; (3-amino-4-methoxy-pyrrolidin-1-yl)-3-pyridinyl; (3-amino-4-methoxy-pyrrolidin-1-yl)-3-pyridinyl; 2-methyl-6-piperazin-1-yl-3-pyridinyl; 6-(3-amino-3-methyl-azetidin-1-yl)-2-methyl-3-pyridinyl; 6-(3-amino-3-methyl-azetidin-1-yl)-3-pyridinyl; 6-(6-methyl-2,6-diazaspiro[3.3]heptan-2-yl)-3-pyridinyl; 6-piperazin-1-yl-3-pyridinyl; (3-amino-4-methoxy-pyrrolidin-1-yl)-6-methyl-pyrimidin-4-yl or (3-amino-3-methyl-azetidin-1-yl)-6-methyl-pyrimidin-4-yl; n is 0, 1 or 2;

or a pharmaceutically acceptable salt thereof.

A further embodiment of present invention is (xiii") a compound of formula (I) or (Ia) or (Ib) according to any one of (i) to (xii), (ix"), (x") and (xii"), wherein

$R^1$ is

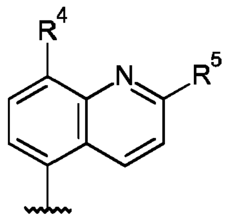

;

wherein $R^4$ is cyano; $R^5$ is H or deuterium;

$R^2$ is methyl;

$R^3$ is H;

Ring A is 1,2,3,4-tetrahydroisoquinolin-7-yl; morpholin-2-ylphenyl; (3,4,4a,5,7,7a-hexahydro-2H-pyrrolo[3,4-b][1,4]oxazin-6-yl)-2-methyl-3-pyridinyl; (3-amino-4-fluoro-pyrrolidin-1-yl)-3-pyridinyl; (3-amino-4-fluoro-pyrrolidin-1-yl)-3-pyridinyl; (3-amino-4-methoxy-pyrrolidin-1-yl)-3-pyridinyl; (3-amino-4-methoxy-pyrrolidin-1-yl)-3-pyridinyl; 2-methyl-6-piperazin-1-yl-3-pyridinyl; 6-(3-amino-3-methyl-azetidin-1-yl)-2-methyl-3-pyridinyl; 6-(3-amino-3-methyl-azetidin-1-yl)-3-pyridinyl; 6-(6-methyl-2,6-diazaspiro[3.3]heptan-2-yl)-3-pyridinyl; 6-piperazin-1-yl-3-pyridinyl; (3-amino-4-methoxy-pyrrolidin-1-yl)-6-methyl-pyrimidin-4-yl or (3-amino-3-methyl-azetidin-1-yl)-6-methyl-pyrimidin-4-yl;

n is 0, 1 or 2;

or a pharmaceutically acceptable salt thereof.

The present invention relates to (i') a compound of formula (I),

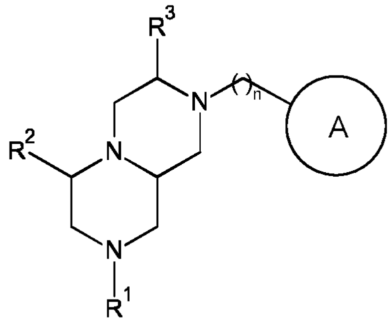

(I),

wherein

$R^1$ is

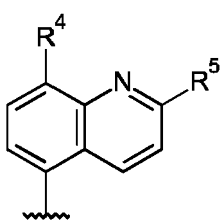

,

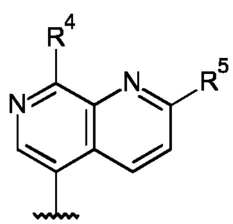

,

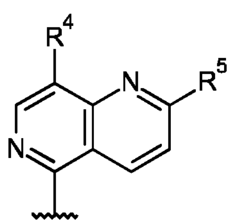

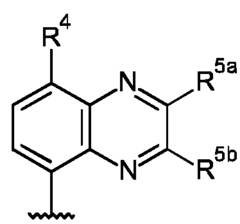

,

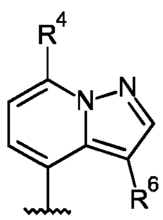

,

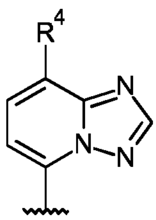

,

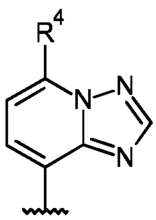

,

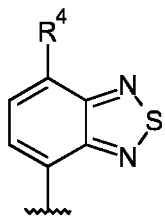

,

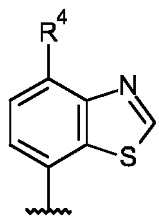

,

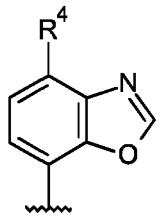 , 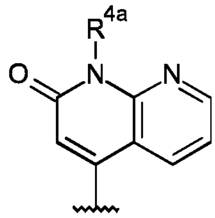 or 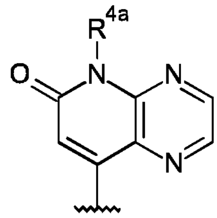 ;

wherein $R^4$ is $C_{1-6}$alkyl, $C_{1-6}$alkoxy, halo$C_{1-6}$alkyl, halogen, nitro or cyano; $R^{4a}$ is $C_{1-6}$alkyl or $C_{3-7}$cycloalkyl; $R^5$, $R^{5a}$ and $R^{5b}$ are independently selected from H and deuterium; $R^6$ is H or halogen;
$R^2$ is H or $C_{1-6}$alkyl;
$R^3$ is H;
Ring A is a 5-7 membered monocyclic aryl or heteroaryl; or a 7-12 membered bicyclic heterocyclyl;
n is 0, 1 or 2;

or a pharmaceutically acceptable salt thereof.

A further embodiment of present invention is (ii') a compound of formula (I), wherein Ring A is 1,2,3,4-tetrahydroisoquinolinyl;

5,6,7,8-tetrahydro-1,6-naphthyridinyl;
5,6,7,8-tetrahydro-2,6-naphthyridinyl;
5,6,7,8-tetrahydro-2,7-naphthyridinyl;
isoindolinyl; or

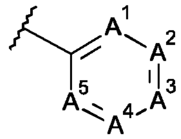 ;

wherein

$A^1$ is N or $CR^a$;
$A^2$ is N or $CR^b$;
$A^3$ is N or $CR^c$;
$A^4$ is N or $CR^d$;
$A^5$ is N or $CR^e$;

wherein $R^a$, $R^b$, $R^c$, $R^d$, $R^e$ are independently selected from H; $C_{1-6}$alkyl; 3,4,4a,5,7,7a-hexahydro-2H-pyrrolo[3,4-b][1,4]oxazinyl; 1,4-diazepanyl; 2,6-diazaspiro[3.3]heptanyl substituted by $C_{1-6}$alkyl; 5-oxa-2,8-diazaspiro[3.5]nonanyl; amino-1,4-oxazepanyl; azetidinyl substituted by one or two substituents independently selected from amino and $C_{1-6}$alkyl; piperazinyl unsubstituted or substituted by $C_{1-6}$alkyl; or pyrrolidinyl substituted by one or two substituents independently selected from amino, $C_{1-6}$alkoxy and halogen.

A further embodiment of present invention is (iii') a compound of formula (Ia), wherein

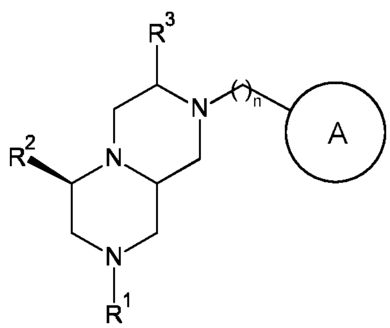

(Ia),

$R^1$ is

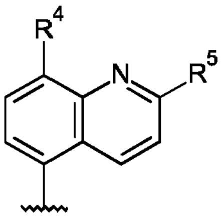,
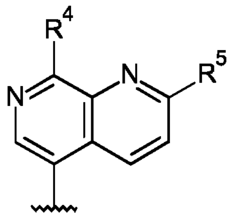,
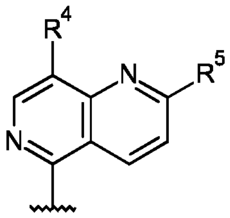,
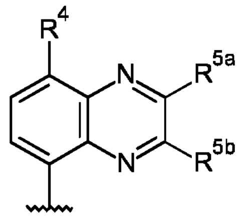,

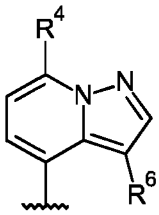,
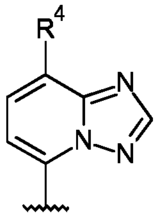,
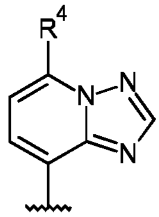,
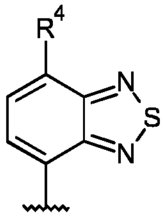,
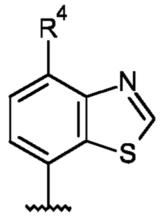,

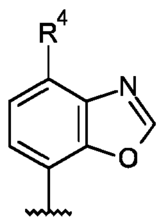,
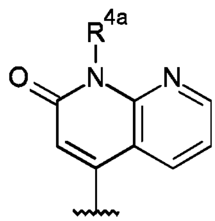
or
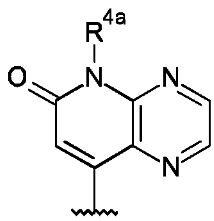;

wherein $R^4$ is $C_{1-6}$alkyl, $C_{1-6}$alkoxy, halo$C_{1-6}$alkyl, halogen, nitro or cyano; $R^{4a}$ is $C_{1-6}$alkyl or $C_{3-7}$cycloalkyl; $R^5$, $R^{5a}$ and $R^{5b}$ are independently selected from H and deuterium; $R^6$ is H or halogen;
$R^2$ is H or $C_{1-6}$alkyl;
$R^3$ is H;
Ring A is 1,2,3,4-tetrahydroisoquinolinyl;

5,6,7,8-tetrahydro-1,6-naphthyridinyl;
5,6,7,8-tetrahydro-2,6-naphthyridinyl;
5,6,7,8-tetrahydro-2,7-naphthyridinyl;
isoindolinyl; or

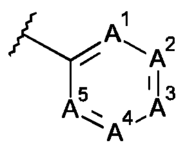;

wherein

$A^1$ is N or $CR^a$;
$A^2$ is N or $CR^b$;
$A^3$ is N or $CR^c$;
$A^4$ is N or $CR^d$;
$A^5$ is N or $CR^e$; wherein $R^a$, $R^b$, $R^c$, $R^d$, $R^e$ are independently selected from H; $C_{1-6}$alkyl; 3,4,4a,5,7,7a-hexahydro-2H-pyrrolo[3,4-b][1,4]oxazinyl; 1,4-diazepanyl; 2,6-diazaspiro[3.3]heptanyl substituted by $C_{1-6}$alkyl; 5-oxa-2,8-diazaspiro[3.5]nonanyl; amino-1,4-oxazepanyl; azetidinyl substituted by one or two substituents independently selected from amino and $C_{1-6}$alkyl; piperazinyl unsubstituted or substituted by $C_{1-6}$alkyl; or pyrrolidinyl substituted by one or two substituents independently selected from amino, $C_{1-6}$alkoxy and halogen;

n is 0, 1 or 2;

or a pharmaceutically acceptable salt thereof.

A further embodiment of present invention is (iv') a compound of formula (I) or (Ia) or (Ib), or a pharmaceutically acceptable salt thereof, according to any one of (i') to (iii'), wherein $R^1$ is

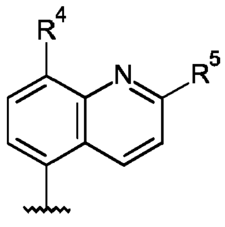
or
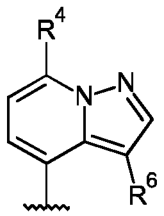
;

wherein $R^4$ is cyano; $R^5$ is H or deuterium; $R^6$ is H or halogen.

A further embodiment of present invention is (v') a compound of formula (I) or (Ia) or (Ib) according to any one of (i') to (iv'), wherein

$R^1$ is

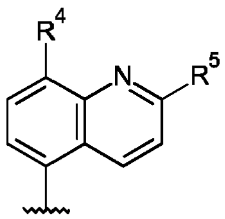
or
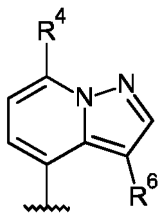
;

wherein $R^4$ is cyano; $R^5$ is H or deuterium; $R^6$ is H or fluoro;
$R^2$ is H or methyl;
$R^3$ is H;
Ring A is 1,2,3,4-tetrahydroisoquinolin-5-yl; 1,2,3,4-tetrahydroisoquinolin-6-yl; 1,2,3,4-tetrahydroisoquinolin-7-yl; 1,2,3,4-tetrahydroisoquinolin-8-yl; 1,4-diazepan-1-ylpyridinyl; 3,4,4a,5,7,7a-hexahydro-2H-pyrrolo[3,4-b][1,4]oxazin-6-ylpyridinyl; 3-amino-3-methyl-azetidin-1-yl(methyl)pyridinyl; 3-amino-3-methyl-azetidin-1-yl(methyl)pyrimidinyl; 3-amino-3-methyl-azetidin-1-ylpyridinyl; 3-amino-4-fluoro-pyrrolidin-1-ylpyridinyl; 3-amino-4-methoxy-pyrrolidin-1-yl(methyl)pyrimidinyl; 3-amino-4-methoxy-pyrrolidin-1-ylphenyl; 3-amino-4-methoxy-pyrrolidin-1-ylpyridinyl; 3-amino-4-methoxy-pyrrolidin-1-ylpyrimidinyl; 3-aminoazetidin-1-ylpyridinyl; 3-aminoazetidin-1-ylpyrimidinyl; 3-methylpiperazin-1-ylpyridinyl; 5,6,7,8-tetrahydro-1,6-naphthyridin-3-yl; 5,6,7,8-tetrahydro-2,6-naphthyridin-1-yl; 5,6,7,8-tetrahydro-2,7-naphthyridin-4-yl; 5-oxa-2,8-diazaspiro[3.5]nonan-2-ylphenyl; 5-oxa-2,8-diazaspiro[3.5]nonan-2-ylpyridinyl; 6-amino-1,4-oxazepan-4-yl(methyl)pyridinyl; 6-amino-1,4-oxazepan-4-ylpyridinyl; 6-methyl-2,6-diazaspiro[3.3]heptan-2-ylpyridinyl; isoindolin-4-yl; piperazin-1-yl(methyl)pyridinyl; piperazin-1-yl(methyl)pyrimidinyl; piperazin-1-ylphenyl; piperazin-1-ylpyridinyl; or piperazin-1-ylpyrimidinyl;
n is 0, 1 or 2;

or a pharmaceutically acceptable salt thereof.

A further embodiment of present invention is (vi') a compound of formula (I) or (Ia) or (Ib), or a pharmaceutically acceptable salt thereof, according to any one of (i') to (v'), wherein $R^1$ is

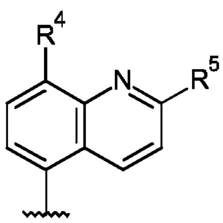
;

wherein $R^4$ is cyano; $R^5$ is H or deuterium.

A further embodiment of present invention is (vii') a compound of formula (I) or (Ia) or (Ib), or a pharmaceutically acceptable salt thereof, according to any one of (i') to (vi'), wherein $R^2$ is $C_{1-6}$alkyl.

A further embodiment of present invention is (viii') a compound of formula (I) or (Ia) or (Ib), or a pharmaceutically acceptable salt thereof, according to any one of (i') to (vii'), wherein $R^2$ is methyl.

A further embodiment of present invention is (ix') a compound of formula (I) or (Ia) or (Ib), or a pharmaceutically acceptable salt thereof, according to any one of (i') to (viii'), wherein

Ring A is 1,2,3,4-tetrahydroisoquinolinyl or

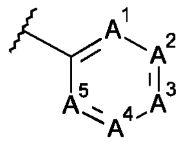
;

wherein
$A^1$ is CH;
$A^2$ is $CR^b$, wherein $R^b$ is H or $C_{1-6}$alkyl;
$A^3$ is N or $CR^c$, wherein $R^c$ is piperazinyl; amino($C_{1-6}$alkoxy)pyrrolidinyl; aminohalopyrrolidinyl; $C_{1-6}$alkyl-2,6-diazaspiro[3.3]heptanyl; or amino($C_{1-6}$alkyl)azetidinyl;
$A^4$ is N or $CR^d$, wherein $R^d$ is amino($C_{1-6}$alkoxy)pyrrolidinyl or amino($C_{1-6}$alkyl)azetidinyl;
$A^5$ is N or $CR^e$, wherein $R^e$ is H or$C_{1-6}$alkyl.

A further embodiment of present invention is (x') a compound of formula (I) or (Ia) or (Ib), or a pharmaceutically acceptable salt thereof, according to any one of (i') to (ix'), wherein

Ring A is 1,2,3,4-tetrahydroisoquinolin-7-yl or

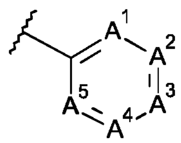
;

wherein
$A^1$ is CH;
$A^2$ is $CR^b$, wherein $R^b$ is H or methyl;
$A^3$ is N or $CR^c$, wherein $R^c$ is piperazin-1-yl; 3-amino-4-methoxy-pyrrolidin-1-yl; 3-amino-4-fluoro-pyrrolidin-1-yl; 6-methyl-2,6-diazaspiro[3.3]heptan-2-yl; or 3-amino-3-methyl-azetidin-1-yl;
$A^4$ is N or $CR^d$, wherein $R^d$ is 3-amino-4-methoxy-pyrrolidin-1-yl or 3-amino-3-methyl-azetidin-1-yl;
$A^5$ is N or $CR^e$, wherein $R^e$ is H or methyl.

A further embodiment of present invention is (xi') a compound of formula (I) or (Ia) or (Ib), or a pharmaceutically acceptable salt thereof, according to any one of (i') to (x'), wherein ring A is 1,2,3,4-tetrahydroisoquinolin-7-yl; 3-amino-3-methyl-azetidin-1-yl(methyl)pyrimidinyl; 3-amino-3-methyl-azetidin-1-ylpyridinyl; 3-amino-4-fluoro-pyrrolidin-1-ylpyridinyl; 3-amino-4-fluoro-pyrrolidin-1-ylpyridinyl; 3-amino-4-methoxy-pyrrolidin-1-yl(methyl)pyrimidinyl; 3-amino-4-methoxy-pyrrolidin-1-ylpyridinyl; 3-amino-4-methoxy-pyrrolidin-1-ylpyridinyl; 6-methyl-2,6-diazaspiro[3.3]heptan-2-ylpyridinyl; piperazin-1-yl(methyl)pyridinyl; or piperazin-1-ylpyridinyl.

A further embodiment of present invention is (xii') a compound of formula (I) or (Ia) or (Ib) according to any one of (i') to (xi'), wherein

$R^1$ is

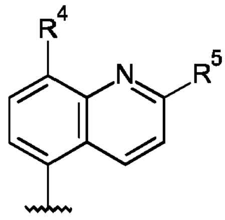

;

wherein $R^4$ is cyano; $R^5$ is H or deuterium;
$R^2$ is $C_{1-6}$alkyl;
$R^3$ is H;
Ring A is 1,2,3,4-tetrahydroisoquinolinyl or

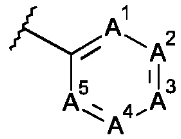

;

wherein

$A^1$ is CH;
$A^2$ is $CR^b$, wherein $R^b$ is H or $C_{1-6}$alkyl;
$A^3$ is N or $CR^c$, wherein $R^c$ is piperazinyl; amino($C_{1-6}$alkoxy)pyrrolidinyl; aminohalopyrrolidinyl; $C_{1-6}$alkyl-2,6-diazaspiro[3.3]heptanyl; or amino($C_{1-6}$alkyl)azetidinyl;
$A^4$ is N or $CR^d$, wherein $R^d$ is amino($C_{1-6}$alkoxy)pyrrolidinyl or amino($C_{1-6}$alkyl)azetidinyl;
$A^5$ is N or $CR^e$, wherein $R^e$ is H or $C_{1-6}$alkyl;

n is 0, 1 or 2;

or a pharmaceutically acceptable salt thereof.

A further embodiment of present invention is (xiii') a compound of formula (I) or (Ia) or (Ib) according to any one of (i') to (xii'), wherein

$R^1$ is

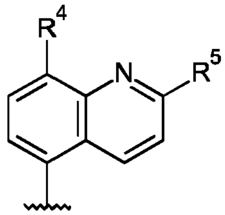

;

wherein $R^4$ is cyano; $R^5$ is H or deuterium;
$R^2$ is methyl;
$R^3$ is H;
Ring A is 1,2,3,4-tetrahydroisoquinolin-7-yl; 3-amino-3-methyl-azetidin-1-yl(methyl)pyrimidinyl; 3-amino-3-methyl-azetidin-1-ylpyridinyl; 3-amino-4-fluoro-pyrrolidin-1-ylpyridinyl; 3-amino-4-fluoro-pyrrolidin-1-ylpyridinyl; 3-amino-4-methoxy-pyrrolidin-1-yl(methyl)pyrimidinyl; 3-amino-4-methoxy-pyrrolidin-1-ylpyndinyl; 3-amino-4-methoxy-pyrrolidin-1-ylpyridinyl; 6-methyl-2,6-diazaspiro[3.3]heptan-2-ylpyridinyl; piperazin-1-yl(methyl)pyridinyl; or piperazin-1-ylpyridinyl;
n is 0, 1 or 2;

or a pharmaceutically acceptable salt thereof.

Another embodiment of present invention is a compound of formula (I) or (Ia) or (Ib) selected from the following:

5-[*cis*-4-methyl-8-(1,2,3,4-tetrahydroisoquinolin-7-ylmethyl)-3,4,6,7,9,9a-hexahydro-*1H*-pyrazino[1,2-a] pyrazin-2-yl] quinoline-8-carbonitrile;
5-[*cis*-4-methyl-8-(1,2,3,4-tetrahydroisoquinolin-5-yl)-3,4,6,7,9,9a-hexahydro-*1H*-pyrazino[1,2-a] pyrazin-2-yl] quinoline-8-carbonitrile;
5-[*cis*-4-methyl-8-[(2-piperazin-1-yl-4-pyridyl)methyl]-3,4,6,7,9,9a-hexahydro-*1H*-pyrazino[1,2-a] pyrazin-2-yl] quinoline-8-carbonitrile;
5-[*cis*-8-isoindolin-4-yl-4-methyl-3,4,6,7,9,9a-hexahydro-*1H*-pyrazino[1,2-a]pyrazin-2-yl]quinoline-8-carbonitrile;
5-[(*4R*,*9aS*)-4-methyl-8-(1,2,3,4-tetrahydroisoquinolin-7-ylmethy1)-3,4,6,7,9,9a-hexahydro-*1H*-pyrazino[1,2-a]pyrazin-2-yl]quinoline-8-carbonitrile;
5-[(*4R*,*9aS*)-4-methyl-8-(5,6,7,8-tetrahydro-1,6-naphthyridin-3-ylmethyl)-3,4,6,7,9,9a-hexahydro- *1H*-pyrazino[1,2-a]pyrazin-2-yl]quinoline-8-carbonitrile;
5-[(*4R*,*9aS*)-4-methyl-8-[2-(4-piperazin-1-ylphenyl)ethyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[(6-piperazin-1-yl-3-pyridyl)methyl]-3,4,6,7,9,9a-hexahydro-*1H*-pyrazino[1,2-a]pyrazin-2-yl]quinoline-8-carbonitrile;
5-[(*4R*,*9aS*)-8-isoindolin-4-yl-4-methyl-3,4,6,7,9,9a-hexahydro-*1H*-pyrazino[1,2-a]pyrazin-2-yl]quinoline-8-carbonitrile;
5-[(*4R*,*9aS*)-4-methyl-8-(1,2,3,4-tetrahydroisoquinolin-6-ylmethyl)-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-(6-piperazin-1-yl-3-pyridyl)ethyl]-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3R*,4R*)*-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[5-[(*3R*,*4R*)-3-amino-4-methoxy-pyrrolidin-1-yl]-2-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[[6-(1,4-diazepan-1-yl)-3-pyridyl]methyl]-4-methyl-3,4,6,7,9,9a-hexahydro-*1H*-pyrazino[1,2-a]pyrazin-2-yl]quinoline-8-carbonitrile;
5-[(*4R*,*9aR*)-4-methyl-8-(5,6,7,8-tetrahydro-2,6-naphthyridin-1-yl)-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3R*,*4S*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3R*,*4R*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[5-[(*3R*,*4S*)-3-amino-4-fluoro-pyrrolidin-1-yl]-2-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[5-[(*3S*)-3-methylpiperazin-1-yl]-2-pyridyl]ethyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[6-[(*3S*)-3-methylpiperazin-1-yl]-2-pyridyl]ethyl]-3,4,6,7,9,9a-hexahydro-1*H*-pyrazino[1,2-a]pyrazin-2-yl]quinoline-8-carbonitrile;
5-[(*4R*,*9aS*)-4-methyl-8-[2-[6-(6-methyl-2,6-diazaspiro[3.3]heptan-2-yl)-3-pyridyl]ethyl]-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3R*,*4S*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3S*,*4S*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[4-[(*3R*,*4R*)-3-amino-4-methoxy-pyrrolidin-1-yl]phenyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-4-methyl-8-[(6-piperazin-1-yl-2-pyridyl)methyl]-3,4,6,7,9,9a-hexahydro-*1H*-pyrazino[1,2-a] pyrazin-2-yl] quinoline-8-carbonitrile;
5-[(*4R*,*9aS*)-4-methyl-8-[2-[4-(5-oxa-2,8-diazaspiro[3.5]nonan-2-yl)phenyl]ethyl]-3,4,6,7,9,9a-hexahydro-*1H*-pyrazino[1,2-a]pyrazin-2-yl]quinoline-8-carbonitrile;
5-[(*4R*,9*a*R)-4-methyl-8-(4-piperazin-1-ylpyrimidin-2-yl)-3,4,6,7,9,9a-hexahydro-*1H*-pyrazino[1,2-a] pyrazin-2-yl] quinoline-8-carbonitrile;
5-[(*4R*,*9aR*)-4-methyl-8-(6-methyl-2-piperazin-1-yl-pyrimidin-4-yl)-3,4,6,7,9,9a-hexahydro-*1H*-pyrazino[1,2-a]pyrazin-2-yl]quinoline-8-carbonitrile;
5-[(*4R*,*9aS*)-4-methyl-8-(2-piperazin-1-yl-4-pyridyl)-3,4,6,7,9,9a-hexahydro-*1H*-pyrazino[1,2-a]pyrazin-2-yl]quinoline-8-carbonitrile;
5-[(*4R*,*9aR*)-4-methyl-8-(2-piperazin-1-ylpyrimidin-4-yl)-3,4,6,7,9,9a-hexahydro-*1H*-pyrazino[1,2-a]pyrazin-2-yl]quinoline-8-carbonitrile;
5-[(*4R*,*9aR*)-4-methyl-8-(4-piperazin-1-yl-2-pyridyl)-3,4,6,7,9,9a-hexahydro-*1H*-pyrazino[1,2-a]pyrazin-2-yl]quinoline-8-carbonitrile;
5-(8-isoindolin-4-yl-3,4,6,7,9,9a-hexahydro-1*H*-pyrazino[1,2-a]pyrazin-2-yl)quinoline-8-carbonitrile;
5-[(*4R*,*9aR*)-8-[2-[(*3R*,*4R*)-3-amino-4-methoxy-pyrrolidin-1-yl]pyrimidin-4-yl]-4-methyl-3,4,6,7,9,9a-hexahydro-1*H*-pyrazino[1,2-a]pyrazin-2-yl]quinoline-8-carbonitrile;
5-[(*4R*,*9aR*)-8-[4-[(*3R*,*4R*)-3-amino-4-methoxy-pyrrolidin-1-yl]pyrimidin-2-yl]-4-methyl-3,4,6,7,9,9a-hexahydro-1*H*-pyrazino[1,2-a]pyrazin-2-yl]quinoline-8-carbonitrile;
5-[2-(4-methyl-6-piperazin-1-yl-3-pyridyl)-3,4,6,7,9,9a-hexahydro-1*H*-pyrazino[1,2-a]pyrazin-8-yl]quinoline-8-carbonitrile;
5-[(*4R*,*9aR*)-8-[4-(3-aminoazetidin-1-yl)pyrimidin-2-yl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[(*3S*,*4R*)-3-amino-4-fluoro-pyrrolidin-1-yl]-4-pyridyl]-4-methyl-3,4,6,7,9,9a-hexahydro-1*H*-pyrazino[1,2-a]pyrazin-2-yl]quinoline-8-carbonitrile;
5-[(*4R*,*9aS*)-8-[2-(3-aminoazetidin-1-yl)-4-pyridyl]-4-methyl-3,4,6,7,9,9a-hexahydro-1*H*-pyrazino[1,2-a] pyrazin-2-yl] quinoline-8-carbonitrile;
5-[(*4R*,*9aS*)-4-methyl-8-(5,6,7,8-tetrahydro-2,7-naphthyridin-4-yl)-3,4,6,7,9,9a-hexahydro-1*H*-pyrazino[1,2-a]pyrazin-2-yl]quinoline-8-carbonitrile;
5-[(*4R*,*9aR*)-4-methyl-8-(5-methyl-4-piperazin-1-yl-pyrimidin-2-yl)-3,4,6,7,9,9a-hexahydro-*1H*-pyrazino[1,2-a]pyrazin-2-yl]quinoline-8-carbonitrile;
5-[(*4R*,*9aR*)-4-methyl-8-(5,6,7,8-tetrahydro-2,7-naphthyridin-4-yl)-3,4,6,7,9,9a-hexahydro-*1H*-pyrazino[1,2-a]pyrazin-2-yl]quinoline-8-carbonitrile;
5-[(*4R*,*9aR*)-8-[2-[(*3R*,*4R*)-3-amino-4-methoxy-pyrrolidin-1-yl]-6-methyl-pyrimidin-4-yl]-4-methyl-3,4,6,7,9,9a-hexahydro-1*H*-pyrazino[1,2-a]pyrazin-2-yl]quinoline-8-carbonitrile;
5-[2-[6-[(6*R*)-6-amino-1,4-oxazepan-4-yl]-4-methyl-3-pyridyl]-3,4,6,7,9,9a-hexahydro-1*H*-pyrazino[1,2-a] pyrazin-8-yl] quinoline-8-carbonitrile;
5-[*trans*-4-methyl-8-(1,2,3,4-tetrahydroisoquinolin-5-yl)-3,4,6,7,9,9a-hexahydro-1*H*-pyrazino[1,2-a] pyrazin-2-yl] quinoline-8-carbonitrile;
5-[(*4R*,*9aS*)-8-[2-[(*6S*)-6-amino-1,4-oxazepan-4-yl]-4-pyridyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[(*6R*)-6-amino-1,4-oxazepan-4-yl]-4-pyridyl]-4-methyl-3,4,6,7,9,9a-hexahydro-*1H*-pyrazino[1,2-a]pyrazin-2-yl]quinoline-8-carbonitrile;
5-[(*4R*,*9aR*)-8-[2-(3-amino-3-methyl-azetidin-1-yl)-6-methyl-pyrimidin-4-yl]-4-methyl-3,4,6,7,9,9a-hexahydro-1*H*-pyrazino[1,2-a]pyrazin-2-yl]quinoline-8-carbonitrile;
5-[(*4R*,*9aS*)-8-[2-(3-amino-3-methyl-azetidin-1-yl)-4-pyridyl]-4-methyl-3,4,6,7,9,9a-hexahydro-*1H*-pyrazino[1,2-a]pyrazin-2-yl]quinoline-8-carbonitrile;
5-[(*4R*,*9aS*)-4-methyl-8-[(5-piperazin-1-yl-3-pyridyl)methyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[6-[(*3R*)-3-methylpiperazin-1-yl]-2-pyridyl]ethyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[6-(5-oxa-2,8-diazaspiro[3.5]nonan-2-yl)-3-pyridyl]ethyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[(2-methyl-6-piperazin-1-yl-3-pyridyl)methyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-(1,2,3,4-tetrahydrolsoquinolin-7-ylmethyl)-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[6-[(*3R*,*4R*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[6-[(*3R*,*4S*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[2-[6-(6-methyl-2,6-diazaspiro[3.3]heptan-2-yl)-3-pyridyl]ethyl]-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[6-(3-amino-3-methyl-azetidin-1-yl)-3-pyridyl]ethyl]-4-methyl-3,4,6,7,9,9a-hexahydro-*1H*-pyrazino[1,2-a]pyrazin-2-yl]quinoline-8-carbonitrile;
4-[(*4R*,*9aS*)-8-[2-[4-[(*3R*,*4R*)-3-amino-4-methoxy-pyrrolidin-1-yl]phenyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile;
4-[(*4R*,*9aS*)-8-[2-[6-[(*3R*,*4R*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile;

4-[(*4R*,*9aS*)-8-[2-[4-[(*3R*,*4R*)-3-amino-4-methoxy-pyrrolidin-1-yl]phenyl]ethyl]-4-methyl-3,4,6,7,9,9a-hexahydro-*1H*-pyrazino[1,2-a]pyrazin-2-yl]pyrazolo[1,5-a]pyridine-7-carbonitrile;
4-[(*4R*,*9aS*)-4-methyl-8-(1,2,3,4-tetrahydrolsoquinolin-7-ylmethyl)-3,4,6,7,9,9a-hexahydro-*1H*-pyrazino[1,2-a]pyrazin-2-yl]-3-fluoro-pyrazolo[1,5-a]pyridine-7-carbonitrile;
5-[(*4R*,*9aS*)-4-methyl-8-(1,2,3,4-tetrahydroisoquinolin-5-ylmethyl)-3,4,6,7,9,9a-hexahydro-*1H*-pyrazino[1,2-a]pyrazin-2-yl]quinoline-8-carbonitrile;
5-[(*4R*,9a*S*)-4-methyl-8-(1,2,3,4-tetrahydrolsoquinolin-8-ylmethyl)-3,4,6,7,9,9a-hexahydro-1H-pyrazino[1,2-a]pyrazin-2-yl]quinoline-8-carbonitrile;
5-[(*4R*,*9aS*)-8-(isoindolin-4-ylmethyl)-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-(3-amino-3-methyl-azetidin-1-yl)-6-methyl-4-pyridyl]-4-methyl-3,4,6,7,9,9a-hexahydro-1*H*-pyrazino[1,2-a]pyrazin-2-yl]quinoline-8-carbonitrile;
4-[(*4R*,*9aS*)-8-[2-[6-(3-amino-3-methyl-azetidin-1-yl)-3-pyridyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile;
4-[(*4R*,*9aS*)-8-[2-[6-[(*3R*,*4S*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile;
5-[(*4R*,*9aS*)-8-[[6-(3-amino-3-methyl-azetidin-1-yl)-2-methyl-3-pyridyl]methyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*4aR*,*7aR*)-3,4,4a,5,7,7a-hexahydro-*2H*-pyrrolo[3,4-b][1,4]oxazin-6-yl]-3-pyridyl]ethyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[(6-piperazin-1-yl-3-pyridyl)methyl]-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-(3-amino-3-methyl-azetidin-1-yl)-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[[2-methyl-6-(6-methyl-2,6-diazaspiro[3.3]heptan-2-yl)-3-pyridyl]methyl]-3,4,6,7,9,9a-hexahydro-1*H*-pyrazino[1,2-a]pyrazin-2-yl]-2-deuterio-quinoline-8-carbonitrile;
5-[(*4R*,*9aS*)-8-[[6-[(*3R*)-3-amino-3-methyl-pyrrolidin-1-yl]-2-methyl-3-pyridyl]methyl]-4-methyl-3,4,6,7,9,9a-hexahydro-1*H*-pyrazino[1,2-a]pyrazin-2-yl]-2-deuterio-quinoline-8-carbonitrile;
5-[(*4R*,*9aS*)-8-[[6-[[(*3S*,4*R*)-4-fluoropyrrolidin-3-yl]amino]-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[[2-methyl-6-(9-oxa-3,7-diazabicyclo[3.3.1]nonan-3-yl)-3-pyridyl]methyl]-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[6-[(*3R*)-3-amino-3-methyl-pyrrolidin-1-yl]-3-pyridyl]ethyl]-4-methyl-3,4,6,7,9,9a-hexahydro-1H-pyrazino[1,2-a]pyrazin-2-yl]-2-deuterio-quinoline-8-carbonitrile;
5-[(*4R*,*9aS*)-4-methyl-8-[[2-methyl-6-[(*2S*)-2-methylpiperazin-1-yl]-3-pyridyl]methyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[[2-methyl-6-[(*3R*)-3-methylpiperazin-1-yl]-3-pyridyl]methyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[(4-methyl-6-piperazin-1-yl-3-pyridyl)methyl]-3,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[(3-methyl-6-piperazin-1-yl-2-pyridyl)methyl]-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[2-[(*3R*,*4S*)-3-amino-4-fluoro-pyrrolidin-1-yl]-4-pyridyl]ethyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-(3,6-diazabicyclo[3.1.1]heptan-3-yl)-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-(3,8-diazabicyclo[3.2.1]octan-3-yl)-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-[(7S, *4S*)-2,5-diazabicyclo[2.2.1]heptan-2-yl]-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile;
4-[(*4R*,*9aR*)-4-methyl-8-(3-methyl-5-piperazin-1-yl-2-pyridyl)-3,4,6,7,9,9a-hexahydro-*1H*-pyrazino[1,2-a]pyrazin-2-yl]-1-methyl-1,8-naphthyridin-2-one;
5-[(*4R*,*9aS*)-8-[[6-[(*1S*,*4S*)-2,5-diazabicyclo[2.2.1]heptan-2-yl]-4-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-[*1S*,*4S*)-2,5-diazabicyclo[2.2.1]heptan-2-yl]-3-methyl-2-pyridyl]-4-methyl-3,4,6,7,9,9a-hexahydro-1H-pyrazino[1,2-a]pyrazin-2-yl]-2-deuterio-quinoline-8-carbonitrile;
5-[(*4R*,*9aR*)-8-[5-[(*3R*,*4S*)-3-amino-4-methoxy-pyrrolidin-1-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;

5-[(*4R*,*9aR*)-8-[5-[(*3R*)-3-amino-3-methyl-pyrrolidin-1-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-[(*4aR*,*7aR*)-3,4,4a,5,7,7a-hexahydro-*2H*-pyrrolo[3,4-b][1,4]oxazin-6-yl]-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-[(*3R*,*4R*)-3-amino-4-methoxy-pyrrolidin-1-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-[(*4aR*,*7aR*)-3,4,4a,5,7,7a-hexahydro-*2H*-pyrrolo[3,4-b][1,4]oxazin-6-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-(6-amino-2-azaspiro[3.3]heptan-2-yl)-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-4-methyl-8-[3-methyl-5-(6-methyl-2,6-diazaspiro[3.3]heptane-2-carbonyl)-2-pyridyl]-3,4,6,7,9,9a-hexahydro-*1H*-pyrazino[1,2-a]pyrazin-2-yl]-2-deuterio-quinoline-8-carbonitrile;
5-[(*4S*,*9aR*)-4-methyl-8-[3-methyl-5-(6-methyl-2,6-diazaspiro[3.3]heptane-2-carbonyl)-2-pyridyl]-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[(*3R*)-3-amino-3-methyl-pyrrolidin-1-yl]-4-pyridyl]-4-methyl-3,4,6,7,9,9a-hexahydro-*1H*-pyrazino[1,2-a]pyrazin-2-yl]quinoline-8-carbonitrile;
5-[(*4S*,*9aS*)-4-methyl-8-[4-[(*2R*)-morpholin-2-yl]phenyl]-3,4,6,7,9,9a-hexahydro-*1H*-pyrazino[1,2-a]pyrazin-2-yl]-2-deuterio-quinoline-8-carbonitrile;
5-[(*4S*,*9aS*)-4-methyl-8-[4-[(*2S*)-morpholin-2-yl]phenyl]-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[5-(3,6-diazabicyclo[3.1.1]heptan-3-yl)-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-[2-[(*3S*,*4S*)-3-amino-4-methoxy-pyrrolidin-1-yl]ethyl]-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[5-[(*6R*)-6-amino-1,4-oxazepan-4-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[5-[(*1S*,*4S*)-2,5-diazabicyclo[2.2.1]heptan-2-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[6-(3,8-diazabicyclo[3.2.1]octan-3-yl)-3-pyridyl]-4-methyl-3,4,6,7,9,9a-hexahydro-*1H*-pyrazino[1,2-a]pyrazin-2-yl]-2-deuterio-quinoline-8-carbonitrile;
4-[(*4S*,*9aR*)-4-methyl-8-[3-methyl-5-(6-methyl-2,6-diazaspiro[3.3]heptan-2-yl)-2-pyridyl]-3,4,6,7,9,9a-hexahydro-*1H*-pyrazino[1,2-a]pyrazin-2-yl]-1-methyl-1,8-naphthyridin-2-one;
5-[(*4S*,*9aR*)-8-[5-[(*4aR*,*7aR*)-3,4,4a,5,7,7a-hexahydro-*2H*-pyrrolo[3,4-b][1,4]oxazin-6-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[6-[(*3R*,*4R*)-3-amino-4-methoxy-pyrrolidin-1-yl]-3-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[6-[2-[(*3R*,*4S*)-3-amino-4-fluoro-pyrrolidin-1-yl]ethyl]-3-pyridyl]-4-methyl-3,4,6,7,9,9a-hexahydro-1*H*-pyrazino[1,2-a]pyrazin-2-yl]-2-deuterio-quinoline-8-carbonitrile;
5-[(*4S*,*9aR*)-8-[5-(3-amino-3-methyl-azetidin-1-yl)-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(4*S*,*9aR*)-4-methyl-8-(5,6,7,8-tetrahydro-1,6-naphthyridin-2-yl)-3,4,6,7,9,9a-hexahydro-1*H*-pyrazino[1,2-a]pyrazin-2-yl]-2-deuterio-quinoline-8-carbonitrile;
5-[(4*S*,9*aR*)-8-[5-[2-[(3*S*,4*S*)-3-amino-4-methoxy-pyrrolidin-1-yl]ethyl]-2-pyridyl]-4-methyl-3,4,6,7,9,9a-hexahydro-1*H*-pyrazino[1,2-a]pyrazin-2-yl]-2-deuterio-quinoline-8-carbonitrile;
5-[(4*S*,9*aR*)-8-[5-[[(3*R*,4*R*)-3-amino-4-methoxy-pyrrolidin-1-yl]methyl]-6-methyl-2-pyridyl]-4-methyl-3,4,6,7,9,9a-hexahydro-1*H*-pyrazino[1,2-a]pyrazin-2-yl]-2-deuterio-quinoline-8-carbonitrile;
5-[(*4R*,*9aS*)-8-[2-[6-[(*4aR*,*7aR*)-3,4,4a,5,7,7a-hexahydro-2*H*-pyrrolo[3,4-b][1,4]oxazin-6-yl]-3-pyridyl]ethyl]-4-methyl-3,4,6,7,9,9a-hexahydro-1*H*-pyrazino[1,2-a]pyrazin-2-yl]quinoline-8-carbonitrile; and
5-[(*4S*,*9aS*)-4-methyl-8-[4-[(*2R*)-morpholin-2-yl]phenyl]-3,4,6,7,9,9a-hexahydro-*1H*-pyrazino[1,2-a] pyrazin-2-yl] quinoline-8-carbonitrile;
or a pharmaceutically acceptable salt thereof.

**SYNTHESIS**

The compounds of the present invention can be prepared by any conventional means. Suitable processes for synthesizing these compounds as well as their starting materials are provided in the schemes below and in the examples. All substituents, in particular, $R^1$ to $R^5$ are as defined above unless otherwise indicated. Furthermore, and unless explicitly otherwise stated, all reactions, reaction conditions, abbreviations and symbols have the meanings well known to a person

of ordinary skill in organic chemistry.

General synthetic routes for preparing the compound of formula(I), (VII) and (XI) are shown below.

## Scheme 1

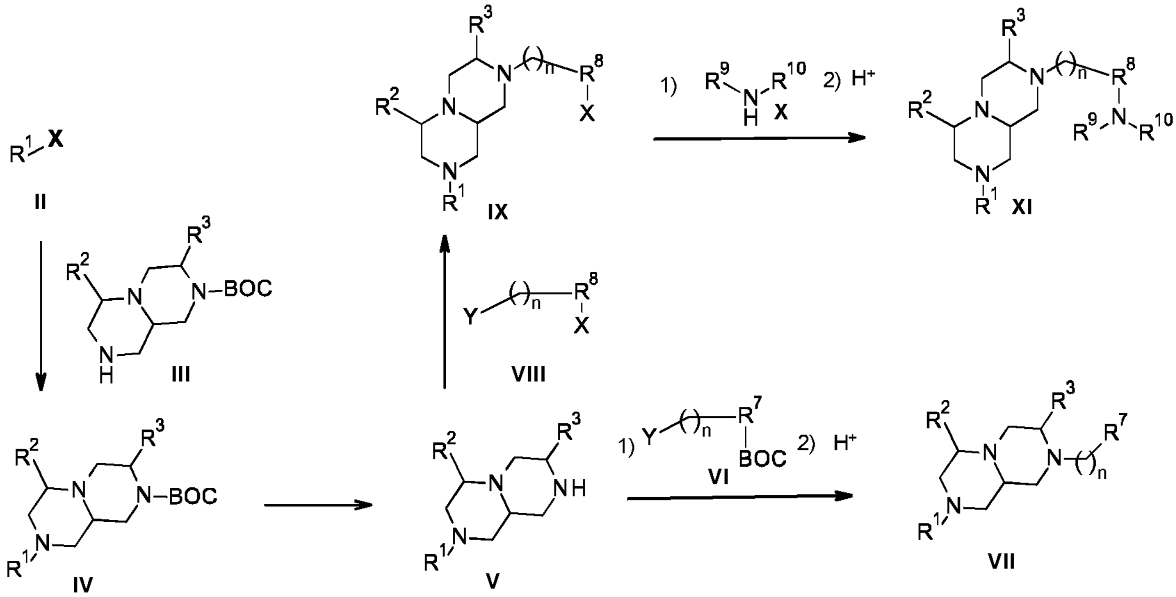


Wherein n is 0, 1 or 2; X is halogen; Y is halogen or methanesulfonate; $R^7$ and $R^8$ is aryl or heteroaryl; $R^9$ and $R^{10}$ together with the nitrogen atom they are attached to form a heterocyclyl.

The synthesis of compounds of the present invention started from halide **II.** Buchwald-Hartwig amination reaction between halide **II** and compound of formula **III** with a catalyst, such as Ruphos Pd-G2, and a base, such as $Cs_2CO_3$ provides compound of formula **IV** (ref: Acc. Chem. Res. 1998, 31, 805-818; Chem. Rev. 2016, 116, 12564-12649; Topics in Current Chemistry, 2002, 219, 131-209; and references cited therein). Alternatively, compound of formula **IV** can also be obtained via nucleophilic substitution between halide **II** and compound of formula **III** in the presence of a base, such as DIPEA, $NaHCO_3$ and $K_2CO_3$. Boc deprotection of compound of formula **IV** in acidic condition (such as HCl in EtOAc and TFA in DCM) gives compound **V,** which can be transformed into compound of formula **VII** via either nucleophilic substitution with compound of formula **VI** in the presence of a base, such as DIPEA $NaHCO_3$ and $K_2CO_3$, or Buchwald-Hartwig amination reaction with compound of formula **VI** followed by appropriate deprotection. Meanwhile, compound of formula **V** can react with compound of formula **VIII** via nucleophilic substitution to give compound of formula **IX.** Buchwald-Hartwig amination reaction or nucleophilic substitution between compound of formula **IX** and amine **X,** followed by appropriate deprotection can provide compound of formula **XI** .

Compounds of formula (Ia) can be synthesized according to Scheme 1 using chiral starting materials.

Compounds of this invention can be obtained as mixtures of diastereomers or enantiomers, which can be separated by methods well known in the art, e.g. (chiral) HPLC or SFC.

This invention also relates to a process for the preparation of a compound of formula (I) or (Ia) comprising any one of the following steps:

a) Buchwald-Hartwig amination reaction or nucleophilic substitution between compound of formula (IX),

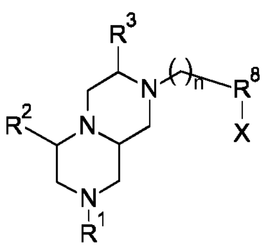

(IX),

and amine (X),

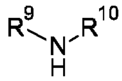 (X);

b) Buchwald-Hartwig amination reaction or nucleophilic substitution between compound of formula (V),

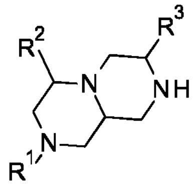 (V),

and compound of formula (VI),

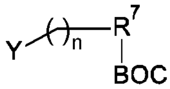 (VI);

wherein n is 0, 1 or 2; X is halogen; Y is halogen or methanesulfonate; $R^7$ and $R^8$ is aryl or heteroaryl; $R^9$ and $R^{10}$ together with the nitrogen atom they are attached to form a heterocyclyl.

A compound of formula (I) or (Ia) when manufactured according to the above process is also an object of the invention.

## INDICATIONS AND METHODS OF TREATMENT

The present invention provides compounds that can be used as TLR7 and/or TLR8 and/or TLR9 antagonist, which inhibits pathway activation through TLR7 and/or TLR8 and/or TLR9 as well as respective downstream biological events including, but not limited to, innate and adaptive immune responses mediated through the production of all types of cytokines and all forms of auto-antibodies. Accordingly, the compounds of the invention are useful for blocking TLR7 and/or TLR8 and/or TLR9 in all types of cells that express such receptor(s) including, but not limited to, plasmacytoid dendritic cell, B cell, T cell, macrophage, monocyte, neutrophil, keratinocyte, epithelial cell. As such, the compounds can be used as a therapeutic or prophylactic agent for systemic lupus erythematosus and lupus nephritis.

The present invention provides compounds for use in methods for treatment or prophylaxis of systemic lupus erythematosus and lupus nephritis in a patient in need thereof.

Another embodiment includes a compound for use in a method of treating or preventing systemic lupus erythematosus and lupus nephritis in a mammal in need of such treatment, wherein the method comprises administering to said mammal a therapeutically effective amount of a compound of formula (I), a stereoisomer, tautomer, or pharmaceutically acceptable salt thereof

## DESCRIPTION OF THE FIGURES

Figure 1. X-ray structure of **compound N**

Figure 2. X-ray structure of **compound P**

Figure 3A. Levels of IP-10 in mouse serum were measured after 1-week treatment of Example 106 or a control immunosuppressant MMF

Figure 3B. Levels of anti-dsDNA antibody in mouse serum were measured after 2-week treatment of Example 106 or a control immunosuppressant MMF

Figure 3C. Urine samples were collected after 5-week treatment of Example 106 or a control immunosuppressant MMF. The levels of urinary albumin (UALB), urinary creatinine (UCR) were measured to calculate the ratio of urinary albumin versus creatinine (UACR)

Figure 3D. Spleens were collected and weighed after 6-week treatment of Example 106 or a control immunosuppressant MMF

## EXAMPLES

The invention will be more fully understood by reference to the following examples. They should not, however, be construed as limiting the scope of the invention.

## ABBREVIATIONS

The invention will be more fully understood by reference to the following examples. They should not, however, be construed as limiting the scope of the invention.

Abbreviations used herein are as follows:

| | |
|---|---|
| ACN: | acetonitrile |
| $Boc_2O$: | di-*tert* butyl dicarbonate |
| BINAP: | 2,2'-Bis(diphenylphosphino)-1,1'-dinaphthalene |
| DCM: | dichloromethane |
| DCE: | dichloroethane |
| DIPEA or DIEA: | *N*,*N*-diisopropylethylamine |
| DIBAL-H: | Diisobutylaluminium hydride |
| DIAD: | diisopropyl azodicarboxylate |
| DMA: | *N,N*-Dimethylacetylamine |
| DMAP: | 4-dimethylaminopyridine |
| DMF: | *N,N*-Dimethylformamide |
| DPPP: | 1,3-Bis(diphenylphosphino)propane |
| EA or EtOAc: | ethyl acetate |
| FA: | formic acid |
| HATU: | 1-[Bis(dimethylamino)methylene]-1H-1,2,3-triazolo[4,5-b]pyridinium 3-oxid hexafluorophosphate |
| $IC_{50}$: | half inhibition concentration |
| IPA: | isopropanol |
| LCMS | liquid chromatography-mass spectrometry |
| MS: | mass spectrometry |
| NBS: | *N*-bromosuccinimide |
| PE: | petroleum ether |
| prep-HPLC: | preparative high performance liquid chromatography |
| prep-TLC: | preparative thin layer chromatography |
| $PPh_3$: | triphenylphosphine |
| $Pd_2(dba)_3$: | tris(dibenzylideneacetone)dipalladium(0) |
| Rf: | retention factor |
| rt: | room temperature |
| RT: | retention time |
| RuPhos Pd G2: | chloro(2-dicyclohexylphosphino-2',6'-dilsopropoxy-1,1'-biphenyl)[2-(2'-amino-1,1'-biphenyl)]palladium(II) 2nd generation |
| Selectfluor | 1-chloromethyl-4-fluoro-1,4-diazoniabicyclo[2.2.2] octane bis(tetrafluoroborate) |
| SFC: | supercritical fluid chromatography |
| tBuXPhos Pd G3: | Methanesulfonato(2-di-t-butylphosphino-2',4',6'-tri-i-propyl-1,1'-biphenyl)(2'-amino-1,1'-biphenyl-2-yl)palladium(II) |
| TEA: | trimethylamine |
| TFA: | trifluoroacetic acid |
| THF: | tetrahydrofuran |
| TLC: | thin layer chromatography |
| XantPhos: | 4,5-bis(diphenylphosphino)-9,9-dimethylxanthene |
| XPhos: | 2-dicyclohexylphosphino-2',4',6'-triisopropylbiphenyl |
| XPhos Pd G2: | chloro(2-dicyclohexylphosphino-2',4',6'-triisopropyl-1,1'-biphenyl)[2-(2'-amino-1,1'-biphenyl)]palladium(II) |
| v/v | volume ratio |
| LYSA | lyophilisation solubility assay |

## GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 μm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge™ Prep-C18 (5 μm, OBDTM 30 × 100 mm) column, SunFire™ Prep-C18 (5 μm, OBD™ 30 × 100 mm) column, Phenomenex Synergi-C18 (10 μm, 25 × 150 mm) or Phenomenex Gemini-C18 (10 μm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

For SFC chiral separation, intermediates were separated by chiral column (Daicel chiralpak IC, 5 μm, 30 × 250 mm), AS (10 μm, 30 × 250 mm) or AD (10 μm, 30 × 250 mm) using Mettler Toledo Multigram III system SFC, Waters 80Q preparative SFC or Thar 80 preparative SFC, solvent system: $CO_2$ and IPA (0.5% TEA in IPA) or $CO_2$ and MeOH (0.1% $NH_3{\cdot}H_2O$ in MeOH), back pressure 100bar, detection UV@ 254 or 220 nm.

LC/MS spectra of compounds were obtained using a LC/MS (Waters™ Alliance 2795-Micromass ZQ, Shimadzu Alliance 2020-Micromass ZQ or Agilent Alliance 6110-Micromass ZQ), LC/MS conditions were as follows (running time 3 or 1.5 mins):

Acidic condition I: A: 0.1% TFA in $H_2O$; B: 0.1% TFA in acetonitrile;
Acidic condition II: A: 0.0375% TFA in $H_2O$; B: 0.01875% TFA in acetonitrile;
Basic condition I: A: 0.1% $NH_3{\cdot}H_2O$ in $H_2O$; B: acetonitrile;
Basic condition II: A: 0.025% $NH_3{\cdot}H_2O$ in $H_2O$; B: acetonitrile;
Neutral condition: A: $H_2O$; B: acetonitrile.
Mass spectra (MS): generally only ions which indicate the parent mass are reported, and unless otherwise stated the mass ion quoted is the positive mass ion $(MH)^+$.
NMR Spectra were obtained using Bruker Avance 400 MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

## PREPARATIVE EXAMPLES

The following examples are intended to illustrate the meaning of the present invention but should by no means represent a limitation within the meaning of the present invention:

### Intermediate A

**5-[*cis*-4-methyl-1,3,4,6,7,8,9,9a-octahydropyrazino[1,2-a]pyrazin-2-yl]quinoline-8-carbonitrile**

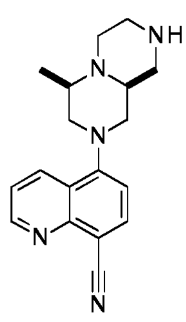

The title compound was prepared according to the following scheme:

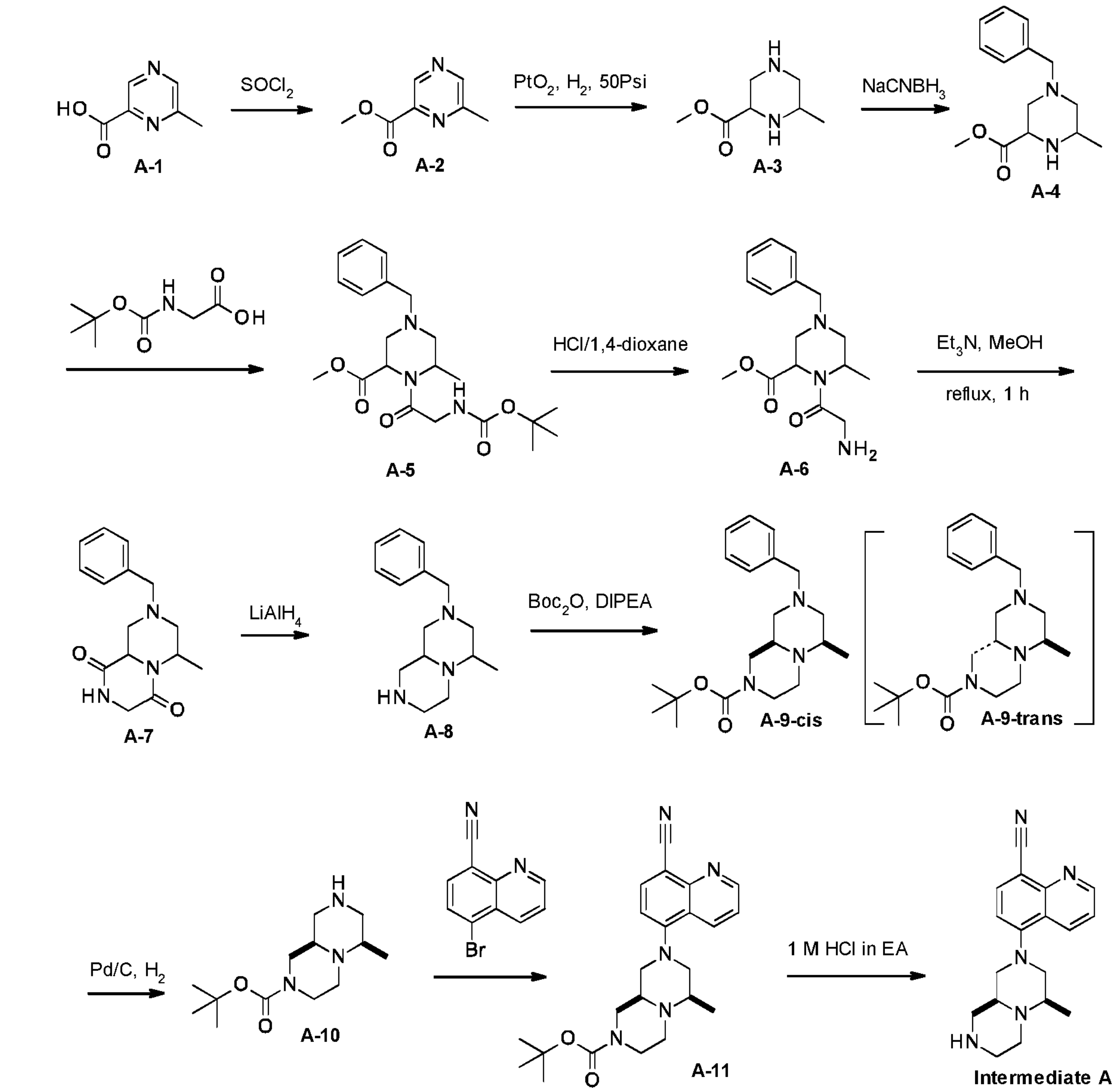


**Step 1: Preparation of methyl 6-methylpyrazine-2-carboxylate (compound A-2)**

To a solution of 6-methylpyrazine-2-carboxylic acid (compound **A-1,** 150 g, 1086 mmol) in Methanol (1600 mL) was added thionyl chloride (388 g, 3258 mmol) dropwise at 0°C, then the reaction was stirred at 25°C for 16 hours. The reaction mixture was concentrated and the residue was dissolved in ice water (1 L). The mixture was basified to pH = 8, extracted with DCM (500 mL) twice. The combined organic layer was dried over $Na_2SO_4$ and concentrated to give compound **A-2** (161 g) as a yellow solid. LCMS $(M+H)^+$: 153.

**Step 2: Preparation of methyl 6-methylpiperazine-2-carboxylate (compound A-3)**

To a solution of methyl 6-methylpyrazine-2-carboxylate (compound **A-2,** 40 g, 262 mmol) in methanol (900 mL) was added acetic acid (32 g, 526 mmol) and platinum(IV) oxide (4 g, 18 mmol). The reaction mixture was stirred at 50°C for 48 hours under hydrogen atmosphere (50 psi). A total of 4 batches of the same scale reaction were combined and filtered. The filtrate was concentrated under vacuum to give compound **A-3** (165 g) as a brown oil. LCMS $(M+H)^+$: 159.

**Step 3: Preparation of methyl-4-benzyl-6-methyl-piperazine-2-carboxylate (compound A-4)**

To a solution of methyl 6-methylpiperazine-2-carboxylate (compound **A-3,** 240 g, 863 mmol) and benzaldehyde (92 g, 863 mmol) in Methanol (2500 mL) was added sodium cyanoborohydride (163 g, 2590 mmol) in batches slowly at

0 °C in 30 minutes. After the reaction mixture was stirred at 20 °C for 12 hours, water (500 mL) was added. The mixture was extracted with EA (1500 mL), and the combined organic layer was dried and concentrated. The residue was purified by silica gel column to give compound **A-4** (225 g, crude) as a yellow oil. LCMS $(M+H)^+$: 249.

**Step 4: Preparation of methyl 4-benzyl-1-[2-(*tert*-butoxycarbonylamino)acetyl]-6-methyl-piperazine-2-carboxylate (compound A-5)**

A solution of Boc-glycine (193 g, 1099 mmol), HATU (418 g, 1099 mmol) and DIPEA (451 mL, 2537 mmol) in DMF (500 mL) was stirred at 20 °C for 30 minutes. Then methyl 4-benzyl-6-methyl-piperazine-2-carboxylate (compound **A-4,** 210 g, 846 mmol) in DMF (100 mL) was added, and the reaction mixture was stirred at 20 °C for 15 hours under nitrogen atmosphere. The reaction mixture was diluted with water and extracted with EtOAc. The organic layer was dried and concentrated. The residue was purified by column chromatography to give compound **A-5** (260 g,) as a yellow oil. LCMS $(M+H)^+$: 406.

**Step 5: Preparation of methyl 1-(2-aminoacetyl)-4-benzyl-6-methyl-piperazine-2-carboxylate (compound A-6)**

To a solution of methyl 4-benzyl-1-[2-(*tert*-butoxycarbonylamino)acetyl]-6-methyl-piperazine-2-carboxylate (compound **A-5,** 230 g, 567 mmol) in methanol (2300 mL) was added HCl in methanol (1150 mL , 4M) slowly at 0 °C. Then the mixture was stirred at 20 °C for 16 hours. The reaction mixture was concentrated *in vacuo* to give compound **A-6** (150 g, crude) as a light yellow solid. LCMS $(M+H)^+$: 306.

**Step 6: Preparation of 2-benzyl-4-methyl-1,3,4,7,8,9a-hexahydropyrazino[1,2-a]pyrazine-6,9-dione(compound A-7)**

A mixture of methyl 1-(2-aminoacetyl)-4-benzyl-6-methyl-piperazine-2-carboxylate hydrochloride (compound **A-6,** 150 g) and triethylamine (306 mL, 2194 mmol) in methanol (400 mL) was stirred at 70 °C for 2 hours. The reaction mixture was concentrated and the residue was purified by flash column chromatography to give compound **A-7** (55 g) as a yellow solid. LCMS $(M+H)^+$: 274.

**Step 7: Preparation of 2-benzyl-4-methyl-1,3,4,6,7,8,9,9a-octahydropyrazino[1,2-a]pyrazine (compound A-8)**

To a solution of 2-benzyl-4-methyl-1,3,4,7,8,9a-hexahydropyrazino[1,2-a]pyrazine-6,9-dione(compound **A-7,** 27 g, 100 mmol) in THF (700 mL) was added lithium aluminum hydride powder (38 g, 1006 mmol) slowly at 0 °C in 30 minutes. The reaction mixture was stirred at 0 °C for 6 hours, then diluted with THF (1000 mL), quenched successively with $H_2O$ (60 mL), 15% aq. NaOH(38 mL) and $H_2O$(67 mL) under ice water bath. The reaction mixture was filtered and the filtrate was dried over $Na_2SO_4$ and concentrated to give **compound A-8** (23 g) as a yellow oil. LCMS $(M+H)^+$: 246.

**Step 8: Preparation of *tert*-butyl *cis*-2-benzyl-4-methyl-3,4,6,7,9,9a-hexahydro-1H-pyrazino[1,2-a]pyrazine-8-carboxylate (compound A-9-cis)**

A mixture of (2-benzyl-4-methyl-1,3,4,6,7,8,9,9a-octahydropyrazino[1,2-a]pyrazine (compound **A-8,** 45.0 g, 117.4 mmol), DIPEA (51.1 mL, 293.5 mmol) and $Boc_2O$ (30.7 g, 140.8 mmol) in DCM (450 mL) was stirred at 20 °C for 16 hours. The reaction mixture was concentrated and the residue was purified by silica gel and prep-HPLC to give compound **A-9-**cis (26.0 g) as a colorless oil and compound **A-9-trans** (17.0 g) as a yellow oil. LCMS $(M+H)^+$: 346.

Compound **A-9-cis,** $^1$H NMR (400MHz, CHLOROFORM-d) δ ppm: 7.36-7.30 (m, 4H), 7.28-7.26 (m, 1H), 4.10 - 3.94 (m, 1H), 3.86-3.70 (m, 1H), 3.57 - 3.40 (m, 2H), 3.16 - 2.85 (m, 2H), 2.82 - 2.67 (m, 2H), 2.58 (br s, 1H), 2.42 - 2.17 (m, 2H), 2.04 - 1.74 (m, 3H), 1.46 (s, 9H), 1.05 (br d, *J* = 5.6 Hz, 3H).

Compound **A-9-trans,** $^1$H NMR (400MHz, CHLOROFORM-d) δ ppm: 7.35 - 7.29 (m, 4H), 7.28 - 7.23 (m, 1H), 4.09 - 3.70 (m, 2H), 3.55 - 3.36 (m, 2H), 3.05 (br s, 2H), 2.71 (br d, *J*= 10.3 Hz, 2H), 2.62 (br d, *J*= 9.5 Hz, 4H), 2.44 (br s, 1H), 2.02 - 1.76 (m, 1H), 1.45 (s, 9H), 1.16 (br d, *J* = 6.4 Hz, 3H).

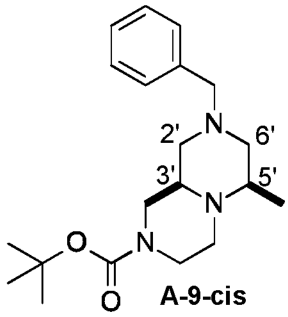
A-9-cis

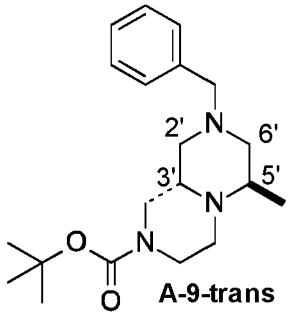
A-9-trans

For compound **A-9-cis,** the NOESY correlation of C3'-H and C5'-H was observed. For compound **A-9-trans,** the NOESY correlation of C3'-H and C5'-H was not observed.

**Step 9: Preparation of *tert*-butyl *cis*-6-methyl-1,3,4,6,7,8,9,9a-octahydropyrazino[1,2-a]pyrazine-2-carboxylate (compound A-10)**

To a solution of tert-butyl cis-2-benzyl-4-methyl-3,4,6,7,9,9a-hexahydro-1H-pyrazino[1,2-a]pyrazine-8-carboxylate (compound A-9-cis, 26.0 g, 75.3 mmol) in THF (500 mL) was added wet Pd/C (5.0 g), the mixture was stirred at 50 °C for 3 hours under hydrogen atmosphere. The reaction mixture was filtered and the filtrate was concentrated to give **compound A-10** (16.5 g) as a yellow oil. LCMS $(M+H)^+$: 256.

**Step 10: Preparation of *tert*-butyl *cis*-2-(8-cyano-5-quinolyl)-4-methyl-3,4,6,7,9,9a-hexahydro-1H-pyrazino[1,2-a]pyrazine-8-carboxylate (compound A-11)**

A mixture of 5-bromoquinoline-8-carbonitrile (1.3 g, 5.6 mmol), tert-butyl cis-2-benzyl-4-methyl-3,4,6,7,9,9a-hexahydro-1H-pyrazino[1,2-a]pyrazine-8-carboxylate (compound **A-9-cis,** 1.6 g, 6.2 mmol), $Cs_2CO_3$ (5.5 g, 16.9 mmol) and RuPhos Pd G2 (875.0 mg, 1.1 mmol) in dioxane (20 mL) was stirred at 90 °C for 16 hours. Then the reaction mixture was filtered and concentrated. The residue was purified by silica gel column to give compound **A-11** (1.5 g) as a yellow foam, LCMS $(M+H)^+$: 408.

**Step 11: Preparation of 5-[*cis*-4-methyl-1,3,4,6,7,8,9,9a-octahydropyrazino[1,2-a]pyrazin-2-yl]quinoline-8-carbonitrile (Intermediate A)**

A mixture of tert-butyl cis-2-(8-cyano-5-quinolyl)-4-methyl-3,4,6,7,9,9a-hexahydro-1H-pyrazino[1,2-a]pyrazine-8-carboxylate (compound **A-11,** 1.6 g, 3.9 mmol) in 1 M HCl in EA (100 mL) was stirred at rt for 16 hours, then the reaction was concentrated. The residue was dissolved in NaOH (2 N, 100 mL) and extracted with EA (100 mL) twice. The organic layer was dried and concentrated to give **Intermediate A** (900 mg) as a light brown foam, LCMS $(M+H)^+$: 308.

**Intermediate B**

**5-[*trans*-4-methyl-1,3,4,6,7,8,9,9a-octahydropyrazino[1,2-a]pyrazin-2-yl]quinoline-8-carbonitrile**

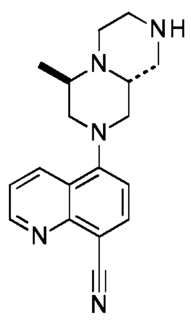

The preparation of **Intermediate B** was the same as **Intermediate A** by using compound **A-9-trans** instead

of compound **A-9-cis** in step 10. **Intermediate B** (900 mg) was obtained as a light brown foam, LCMS $(M+H)^+$: 308.

**Intermediate C**

**5-[(*4R*,*9aS*)-4-methyl-1,3,4,6,7,8,9,9a-octahydropyrazino[1,2-a]pyrazin-2-yl]quinoline-8-carbonitrile**

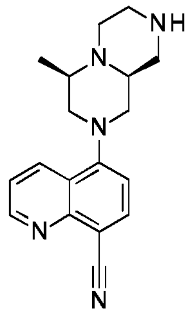

The title compound was prepared according to the following scheme:

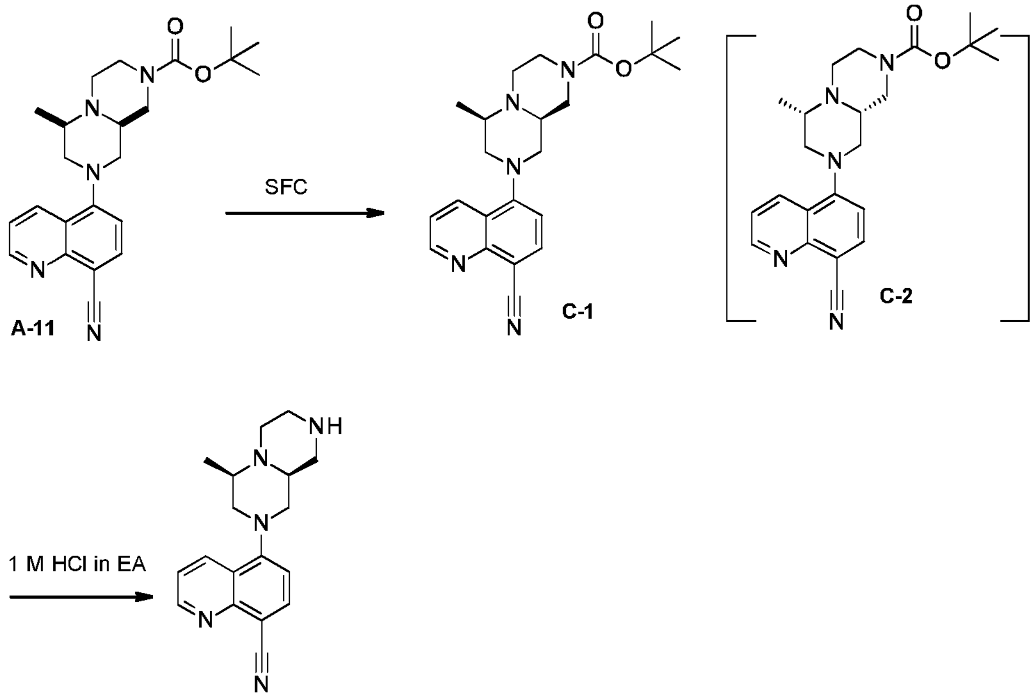


A-11

C-1

C-2

**Intermediate C**

SFC (Gradient: 30% in EtOH (0.1% $NH_3H_2O$) in $CO_2$. Column: Daicel ChiralPak AD, 250×50 mm, 10 μm) separation of Compound **A-11** (8.0 g) gave compound **C-1** (second peak, 3.5 g) and compound **C-2** (first peak, 3.0 g) as yellow solids. A mixture of tert-butyl (*4R,9aR*)-2-(8-cyano-5-quinolyl)-4-methyl-3,4,6,7,9,9a-hexahydro-*1H*-pyrazino[1,2-a]pyrazine-8-carboxylate (compound **C-1,** 3.5 g, 8.6 mmol) in 1 M HCl in EA (100 mL) was stirred at rt for 16 hours, then the reaction was concentrated. The residue was dissolved in NaOH (2 N, 100 mL), extracted with EA (100 mLx2). The organic layer was dried and concentrated to give **Intermediate** C (2.5 g) as a light brown foam, LCMS $(M+H)^+$: 308.

**Intermediate D**

**5-[(*4R,9aR*)-4-methyl-1,3,4,6,7,8,9,9a-octahydropyrazino[1,2-a]pyrazin-2-yl]quinoline-8-carbonitrile**

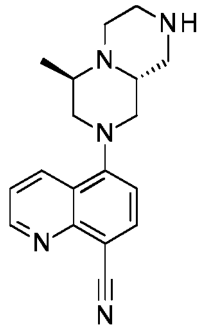

The title compound was prepared according to the following scheme:

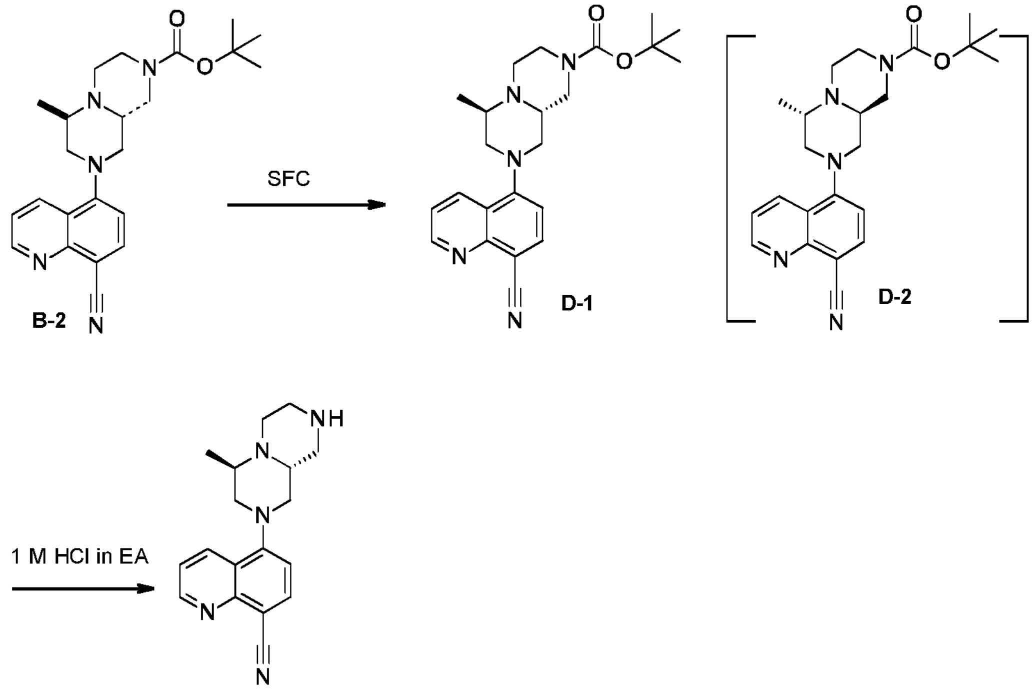

B-2 D-1 D-2

**Intermediate D**

SFC (Gradient: 30% in EtOH (0.1% $NH_3.H_2O$) in $CO_2$. Column: Phenomenex cellulose-2, 250×30 mm, 10 μm) separation of Compound **B-2** (2.4 g) gave compound **D-1** (second peak, 0.9 g) and compound **D-2** (first peak, 0.9 g) as yellow solids. A mixture of tert-butyl (*4R,9aS*)-2-(8-cyano-5-quinolyl)-4-methyl-3,4,6,7,9,9a-hexahydro-*1H*-pyrazino[1,2-a]pyrazine-8-carboxylate (compound **D-1,** 0.9 g, 2.1 mmol) in 1 M HCl in EA (20 mL) was stirred at rt for 16 hours, then the reaction was concentrated. The residue was dissolved in NaOH (2 N, 20 mL), and then extracted with EA (20 mL) twice. The organic layer was dried and concentrated to give **Intermediate D** (550.0 mg) as a light brown foam, LCMS $(M+H)^+$: 308.

**Intermediate E**

**4-[(*4R*,*9aS*)-4-methyl-1,3,4,6,7,8,9,9a-octahydropyrazino[1,2-a]pyrazin-2-yl]pyrazolo[1,5-a]pyridine-7-carbonitrile**

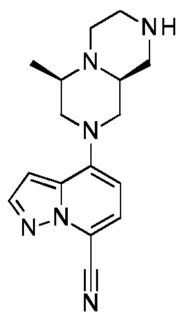

The title compound was prepared according to the following scheme:

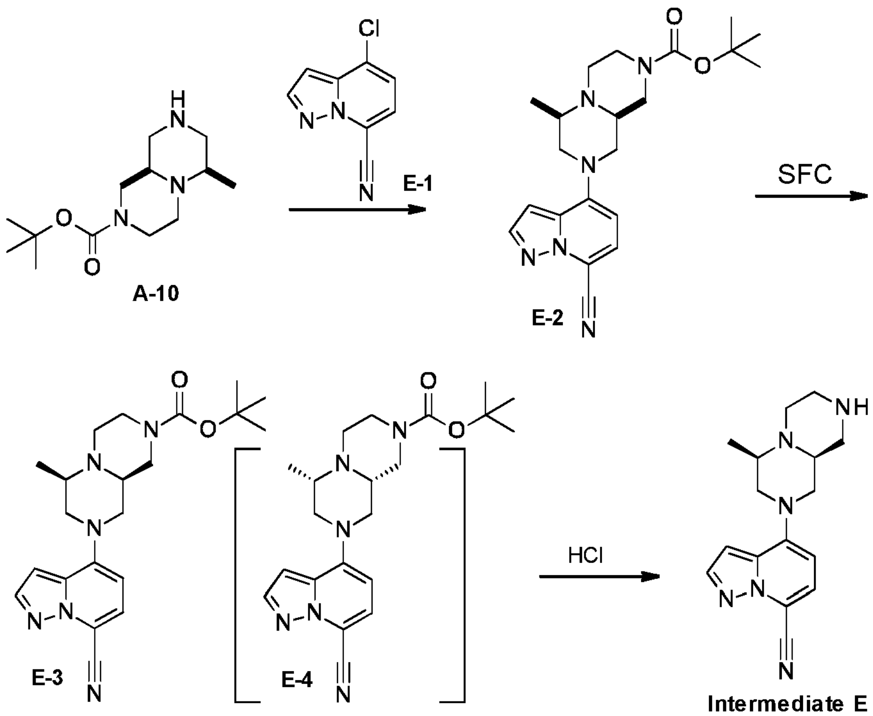

A-10
E-2
E-3
E-4
Intermediate E

**Step** 1: **Preparation of tert-butyl cis-2-(7-cyanopyrazolo[1,5-a]pyridin-4-yl)-4-methyl-3,4,6,7,9,9a-hexahydro-1H-pyrazino[1,2-a]pyrazine-8-carboxylate (compound E-2)**

A mixture of 4-chloropyrazolo[1,5-a]pyridine-7-carbonitrile (compound **E-1,** 1.0 g, 5.6 mmol), *tert*-butyl cis-6-methyl-1,3,4,6,7,8,9,9a-octahydropyrazino[1,2-a]pyrazine-2-carboxylate (compound **A-10,** 1.6 g, 6.2 mmol), $Cs_2CO_3$ (5.5 g, 16.9 mmol) and Ruphos Pd G2 (875.0 mg, 1.1 mmol) in Dioxane (20 mL) was stirred at 90 °C for 16 hours. Then the reaction was concentrated and the residue was purified by silica gel to give compound **E-2** (2.0 g) as a light yellow solid. LCMS $(M+H)^+$: 397.

**Step 2: Preparation of 4-[(*4R*,*9aS*)-4-methyl-1,3,4,6,7,8,9,9a-octahydropyrazino[1,2-a]pyrazin-2-yl]pyrazolo[1,5-a]pyridine-7-carbonitrile (Intermediate E)**

SFC (Gradient: 35% in IPA (0.1% $NH_3H_2O$) in $CO_2$. Column: Daicel ChiralPak AD, 250×20 mm, 5 μm) separation of Compound **E-2** (2.0 g) gave compound **E-3** (second peak, 0.9 g) and compound **E-4** (first peak, 0.9 g) as light

yellow solids. A solution of *tert*-butyl (*4R,9aR*)-2-(7-cyanopyrazolo[1,5-a]pyridin-4-yl)-4-methyl-3,4,6,7,9,9a-hexahydro-*1H*-pyrazino[1,2-a]pyrazine-8-carboxylate (compound **E-3,** 0.9 g, 2.3 mmol) in 1 M HCl in EA (30 mL) was stirred at rt for 16 hours, then the reaction was concentrated to give **Intermediate E** (0.7 g) as a light yellow solid. LCMS $(M+H)^+$: 297.

**Intermediate F**

**4-[(*4R,9aS*)-4-methyl-1,3,4,6,7,8,9,9a-octahydropyrazino[1,2-a]pyrazin-2-yl]-3-fluoro-pyrazolo [1,5-a] pyridine-7-carbonitrile**

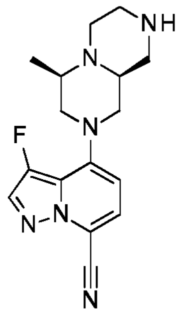

The title compound was prepared according to the following scheme:

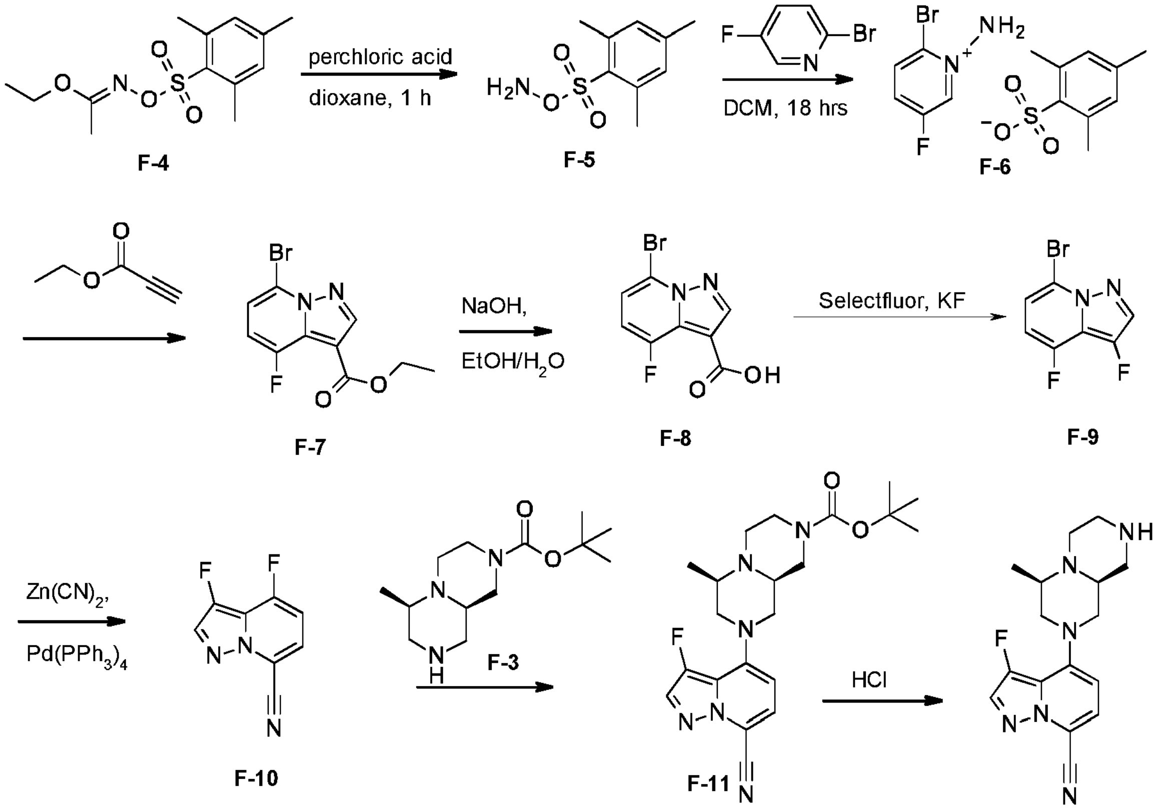


F-4 F-5 F-6 F-7 F-8 F-9 F-10 F-3 F-11

Intermediate F

**Step** 1: **Preparation of amino 2,4,6-trimethylbenzenesulfonate (compound F-5)**

A solution of ethyl (1*E*)-*N*-(2,4,6-trimethylphenyl)sulfonyloxyethanimidate (compound **F-4,** 200 g, 700 mmol) in 1,4-dioxane (500 mL) was added perchloric acid (110 mL) dropwise in 0.5 hours and stirred for 1 hour at 0°C. 1000 mL ice-water was added and the mixture was filtered. The filter cake was dissolved in 1.5 L EtOAc, dried over NaaSOa and stirred for 30 minutes. The organic layer was concentrated (keep the temperature below 25 °C) to give crude product.

The crude product was recrystallized (petroleum/EtOAc=10/1) to give compound **F-5** (110 g) as a white solid. LCMS $(M+H)^+$: 216.

**Step 2: Preparation of 2-bromo-5-fluoro-pyridin-1-ium-1-amine 2,4,6-trimethylbenzenesulfonate (compound F-6)**

A solution of amino 2,4,6-trimethylbenzenesulfonate (compound F-5, 110 g, 511 mmol) and 2-bromo-5-fluoropyridine (60 g, 341 mmol) in DCM (1800 mL) was stirred at 10°C for 18 hours. The mixture was concentrated and the residue was recrystallized in EtOAc to give compound **F-6** (90 g) as a white solid. LCMS $(M+H)^+$: 191.

**Step 3: Preparation of ethyl 7-bromo-4-fluoro-pyrazolo[1,5-a]pyridine-3-carboxylate (compound F-7)**

A solution of 2-bromo-5-fluoro-pyridin-1-ium-1-amine; 2,4,6-trimethylbenzenesulfonate (compound **F-6,** 90 g, 230 mmol); $K_2CO_3$ (64 g, 460 mmol) and ethyl propiolate (28 mL, 276 mmol) in DMF (1300 mL) was stirred at 10°C for 18 hours. The reaction was diluted with water, extracted with EtOAc. The organic layer was dried over $Na_2SO_4$ and concentrated, and the residue was purified by chromatography to give compound **F-7** (11 g,) as a yellow solid. LCMS $(M+H)^+$: 287.

**Step 4: Preparation of 7-bromo-4-fluoro-pyrazolo[1,5-a]pyridine-3-carboxylic acid (compound F-8)**

The mixture of ethyl 7-bromo-4-fluoro-pyrazolo[1,5-a]pyridine-3-carboxylate (compound **F-7,** 5.2 g, 18.1 mmol), NaOH (2.1 g, 54.3 mmol) in EtOH (90.0 mL) and water (70.0 mL) was stirred at 60°C for 2 hours. The reaction mixture was concentrated and then diluted with water. The pH of the reaction suspension was adjusted to 4 with 1 M HCl, a grey solid precipitated, which was collected by filtration to give compound **F-8** (4.0 g) as a grey solid. LCMS $(M+H)^+$: 259.

**Step 5: Preparation of 7-bromo-3,4-difluoro-pyrazolo[1,5-a]pyridine (compound F-9)**

To a solution of 7-bromo-4-fluoro-pyrazolo[1,5-a]pyridine-3-carboxylic acid (compound **F-8,** 4.0 g, 15.4 mmol) and KF (3.6 g, 61.8 mmol) in 1,2-dichloroethane (60.0 mL) and water (50.0 mL) was added Selectfluor (10.9 g, 30.9 mmol). The reaction was stirred at 70°C for 18 hours, then quenched with water, extracted with DCM twice. The combined organic layer was dried over $Na_2SO_4$ and concentrated to give crude compound **F-9** (2.8 g) as a grey solid. LCMS $(M+H)^+$: 233.

**Step 6: Preparation of 3,4-difluoropyrazolo[1,5-a]pyridine-7-carbonitrile (compound F-10)**

A solution of 7-bromo-3,4-difluoro-pyrazolo[1,5-a]pyridine (compound **F-9,** 2.8 g, 12.0 mmol) and zinc cyanide (5.6 g, 48.0 mmol) in DMF (70.0 mL) was added tetrakis(triphenylphosphine)palladium (1.4 g, 1.2 mmol) and stirred at 120°C for 18 hours under $N_2$ atmosphere. The mixture was quenched with water and extracted with EtOAc twice. The combined organic layer was dried and concentrated, and the residue was purified by column chromatography to give compound **F-10** (810.0 mg) as a white solid. LCMS $(M+H)^+$: 180.

$^1H$ NMR (400MHz, CHLOROFORM-d) $\delta$ ppm: 8.00 (d, *J*= 3.6 Hz, 1H), 7.31 (dd, *J*= 4.7, 8.0 Hz, 1H), 6.83 (t, *J*= 8.4 Hz, 1H).

**Step 7: Preparation of tert-butyl (*4R,9aR*)-2-(7-cyanopyrazolo[1,5-a]pyridin-4-yl)-4-methyl-3,4,6,7,9,9a-hexahydro-*1H*-pyrazino[1,2-a]pyrazine-8-carboxylate (compound F-11)**

A mixture of 3,4-difluoropyrazolo[1,5-a]pyridine-7-carbonitrile (compound **F-10,** 250 mg, 1.4 mmol), *tert*-butyl (*6R,9aR*)-6-methyloctahydro-*2H*-pyrazino[1,2-a]pyrazine-2-carboxylate (compound **F-3,** 392 mg, 1.5 mmol) and DIPEA (731 $\mu$L, 4.2 mmol) in DMSO (10.0 mL) was stirred at 120 °C for 16 hours. Then the reaction was diluted with EA, washed with water and brine. The organic layer was dried and concentrated to give compound **F-11(0.5** g) as a yellow oil. LCMS $(M+H)^+$: 415.

**Step 8: Preparation of 4-[(*4R,9aS*)-4-methyl-1,3,4,6,7,8,9,9a-octahydropyrazino[1,2-a]pyrazin-2-yl]-3-fluoro-pyrazolo[1,5-a]pyridine-7-carbonitrile (Intermediate F)**

A mixture of tert-butyl (*4R,9aR*)-2-(7-cyanopyrazolo[1,5-a]pyridin-4-yl)-4-methyl-3,4,6,7,9,9a-hexahydro-*1H*-pyrazino[1,2-a]pyrazine-8-carboxylate (compound **F-11,** 0.5 g, 1.2 mmol) in 1 M HCl in EA (20.0 mL) was stirred at rt for 16 hours, then the reaction was concentrated to give **Intermediate F** (400 mg) as a yellow solid.

The compound **F-3** was prepared according to the following scheme:

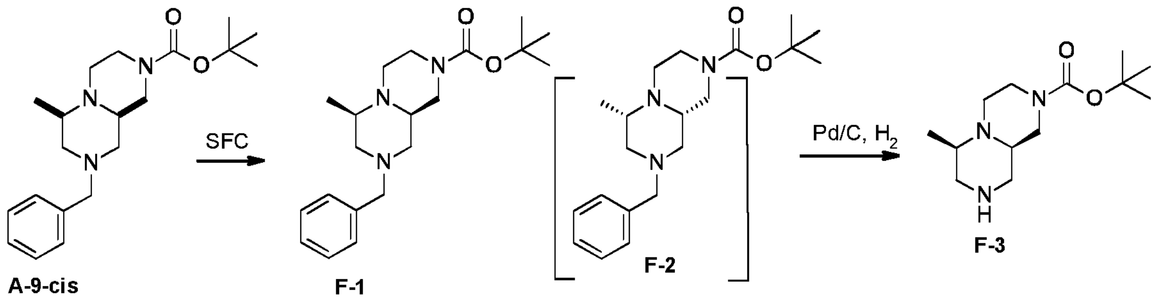


SFC (Gradient: 10% in EtOH (0.1% $NH_3H_2O$) in $CO_2$. Column: Daicel ChiralPak AD, 250×20 mm, 5 μm) separation of Compound **A-9-cis** (5.0 g) gave compound **F-1** (second peak, 2.2 g) and compound **F-2** (first peak, 2.2 g) as light yellow oil. A mixture of *tert*-butyl (*4R,9aR*)-2-benzyl-4-methyl-3,4,6,7,9,9a-hexahydro-*1H*-pyrazino[1,2-a]pyrazine-8-carboxylate (compound **F-1,** 2.2 g, 6.4 mmol) and $Pd(OH)_2$ /C (0.9 g) in THF (50.0 mL) was stirred at 50 °C for 6 hours under $H_2$. Then the reaction was filtered, and the filtrate was concentrated to give compound **F-3** (1.5) as a black oil. LCMS $(M+H)^+$: 256. The stereochemistry of compound **F-3** was confirmed by its derivative (compound **P).**

**Intermediate G**

**5-[(*4R*,*9aS*)-4-methyl-1,3,4,6,7,8,9,9a-octahydropyrazino[1,2-a]pyrazin-2-yl]-2-deuterio-quinoline-8-carbonitrile**

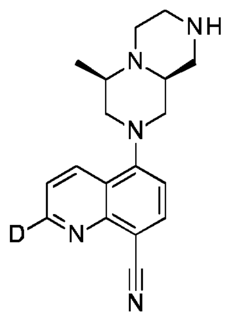

The title compound was prepared according to the following scheme:

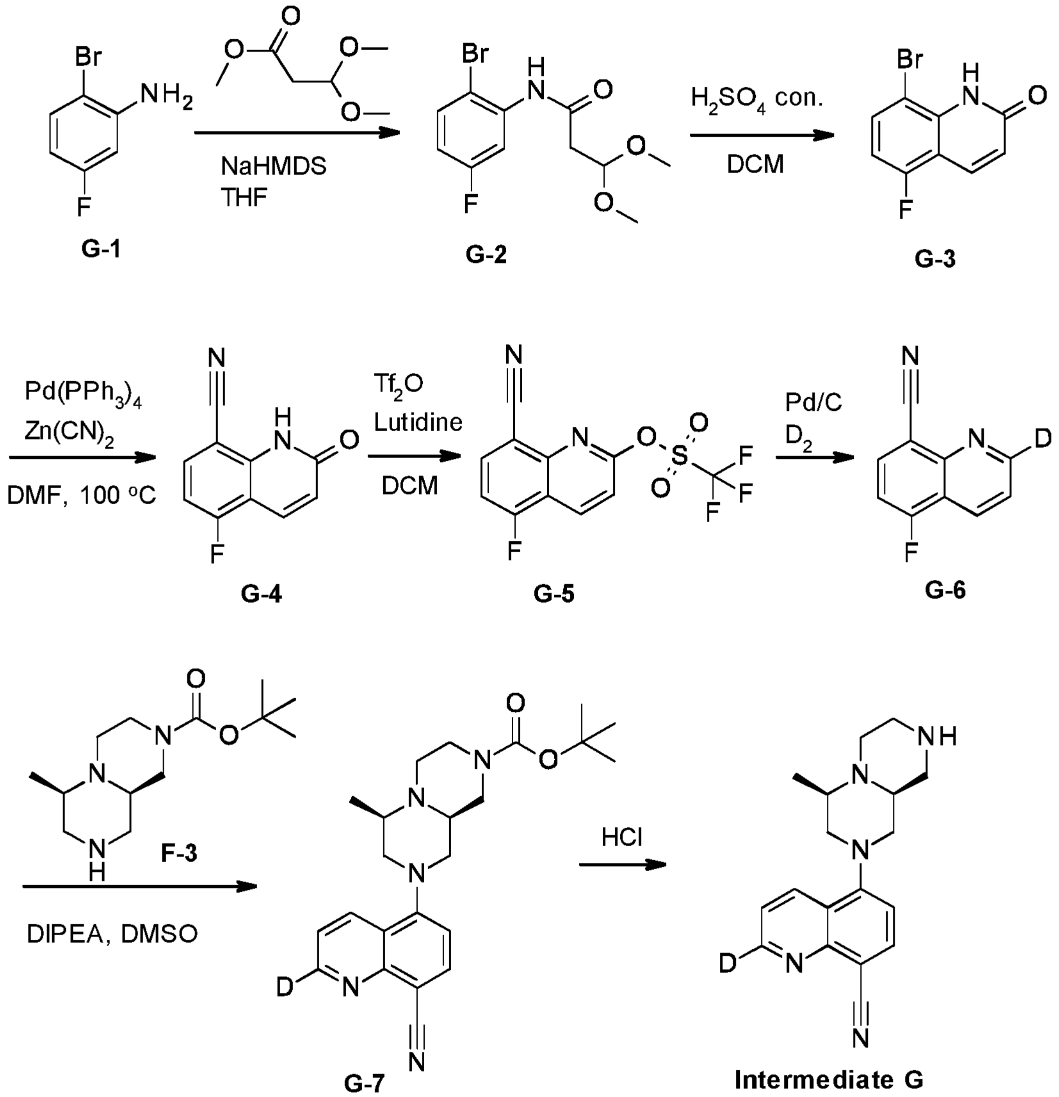


G-1 G-2 G-3 G-4 G-5 G-6 F-3 G-7 Intermediate G

**Step 1: Preparation of N-(2-bromo-5-fluoro-phenyl)-3,3-dimethoxy-propanamide (compound G-2)**

To a solution of 2-bromo-5-fluoroaniline (50 g, 263 mmol) and methyl 3,3-dimethoxypropionate, (45 mL, 316 mmol) in THF (150 mL) was added NaHMDS in THF (394 mL, 394 mmol) dropwise at 0°C. The mixture was stirred at the temperature for 10 minutes, and then it was warmed up to 15 °C and stirred for 18 hours. The reaction was quenched with sat. aqueous solution of $NH_4Cl$ and concentrated to about 300 mL. The solution was diluted with water and extracted with EtOAc. The organic layer was dried over $Na_2SO_4$ and concentrated to give compound **G-2** (100 g) as a brown oil. LCMS $(M+H)^+$: 306.

**Step 2: Preparation of 8-bromo-5-fluoro-*1H*-quinolin-2-one (compound G-3)**

A solution of N-(2-bromo-5-fluoro-phenyl)-3,3-dimethoxy-propanamide (compound **G-2,** 100 g, 238 mmol) in DCM (500 mL) was added to concentrated sulfuric acid (300 mL) at 0°C. The mixture was stirred at 15 °C for 2 hours, then poured slowly into 2000 mL ice-water, and a yellow precipitate appeared. The mixture was filtered, and the wet-cake was washed with 500 mL water, 200 mL isopropyl alcohol and 300 mL PE. The solid was dried to give compound **G-3** (50 g) as a yellow solid. LCMS $(M+H)^+$: 242.

**Step 3: Preparation of 5-fluoro-2-oxo-1*H*-quinoline-8-carbonitrile (compound G-4)**

A solution of 8-bromo-5-fluoro-1*H*-quinolin-2-one (compound **G-3,** 50 g, 206 mmol), zinc cyanide (4820 mg, 412 mmol), $Pd(PPh_3)_4$ (2428 mg, 21 mmol) in DMF was stirred at 120 °C for 5 hours. The reaction mixture was diluted with water and extracted with DCM. The organic layer was dried and concentrated to give the crude product, which was

purified by flash column to give compound **G-4** (29 g) as a yellow solid. LCMS $(M+H)^+$: 189.

**Step 4: Preparation of (8-cyano-5-fluoro-2-quinolyl) trifluoromethanesulfonate (compound G-5)**

To a solution of 5-fluoro-2-oxo-1*H*-quinoline-8-carbonitrile (compound **G-4,** 17 g, 90 mmol) and 2,6-dimethylpyridine (39 g, 361 mmol) in DCM was added trifluoromethanesulfonic anhydride (51 g, 181 mmol) dropwise at 0 °C. The mixture was stirred at 0 °C for 1 hour, and then the reaction was diluted with water, extracted with DCM. The organic layer was dried and concentrated. The residue was purified by flash column to give compound **G-5** (23.0 g) as a yellow solid. LCMS $(M+H)^+$: 321.

**Step 5: Preparation of 2-deuterio-5-fluoro-quinoline-8-carbonitrile (compound G-6)**

To a solution of (8-cyano-5-fluoro-2-quinolyl) trifluoromethanesulfonate (compound **G-5,** 23 g, 72 mmol) in THF (230 mL) and deuterium oxide (100 mL) was added potassium carbonate (20 g, 144 mmol) and Pd/C (6 g). The mixture was stirred at 40 °C for 5 hours under deuterium atmosphere (balloon). The mixture was filtered, and the filtrate was concentrated and purified by flash column to give compound **G-6** (11 g) as a light yellow solid. LCMS $(M+H)^+$: 174.

**Step 6-7: preparation of 5-[(*4R*,*9aS*)-4-methyl-1,3,4,6,7,8,9,9a-octahydropyrazino[1,2-a]pyrazin-2-yl]-2-deuterio-quinoline-8-carbonitrile (Intermediate G)**

The title compound was prepared in analogy to the preparation of **Intermediate F** by using 2-deuterio-5-fluoro-quinoline-8-carbonitrile (compound **G-6)** instead of 3,4-difluoropyrazolo[1,5-a]pyridine-7-carbonitrile (compound **F-10). Intermediate G** (400 mg) was obtained as a yellow solid. LCMS $(M+H)^+$: 309.

**Intermediate K**

**5-[(*4S*,*9aS*)-4-methyl-1,3,4,6,7,8,9,9a-octahydropyrazino[1,2-a]pyrazin-2-yl]-2-deuterio-quinoline-8-carbonitrile**

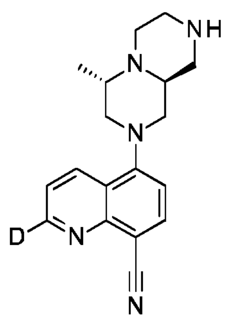

The title compound was prepared according to the following scheme:

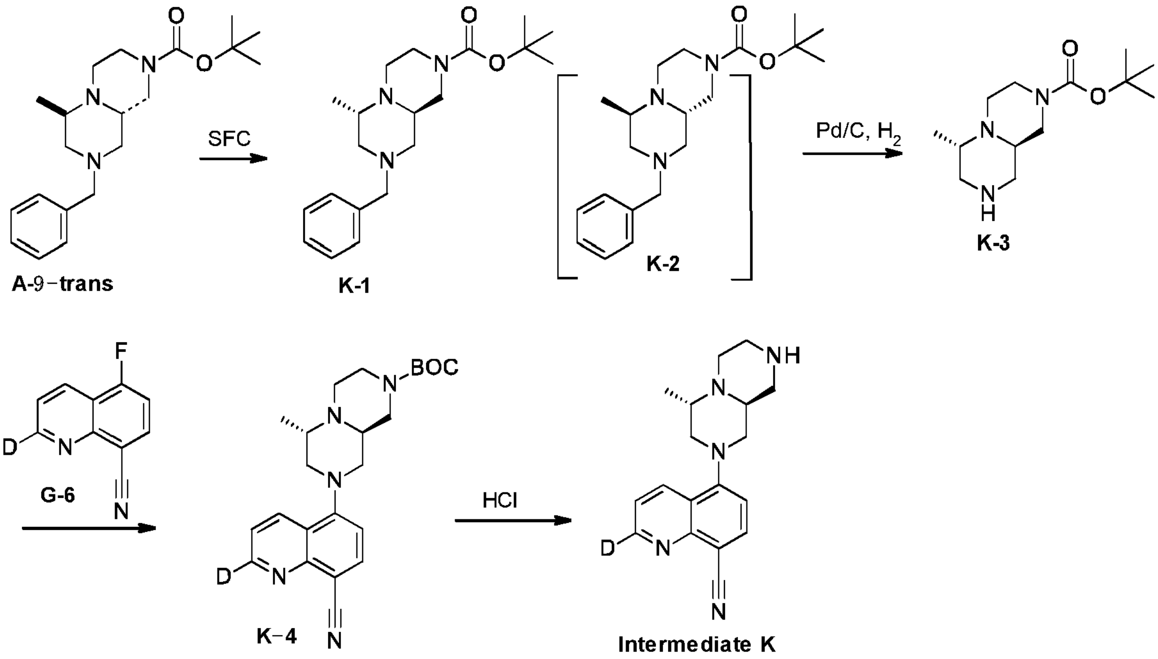

A-9-trans K-1 K-2 K-3 G-6 K-4 Intermediate K

**Step 1: Preparation of *tert*-butyl (*6S,9aR*)-6-methyl-1,3,4,6,7,8,9,9a-octahydropyrazino [1,2-a] pyrazine-2-carboxylate (compound K-3)**

SFC (Gradient: 8% in MeOH (0.1% NH3.$H_2O$) in $CO_2$. Column: Daicel ChiralPak AD, 250×50 mm, 10 µm) separation of Compound **A-9-trans** (7.0 g) gave compound **K-1** (first peak, 3.4 g) and compound **K-2** (second peak, 2.7 g) as light yellow oil.

A mixture of *tert*-butyl (*4S,9aR*)-2-benzyl-4-methyl-3,4,6,7,9,9a-hexahydro-1*H*-pyrazino[1,2-a]pyrazine-8-carboxylate (compound **K-1**, 3.4 g, 9.8 mmol) and $Pd(OH)_2$ /C (0.8 g) in THF was stirred at 50 °C for 2 hours under $H_2$. Then the reaction mixture was filtered, and the filtrate was concentrated to give compound **K-3** (2.5 g) as a black oil, LCMS $(M+H)^+$: 256. The stereochemistry **of K-3** was confirmed by its derivative (compound **N**).

**Step 2: Preparation of 5-[(*4S,9a5*)-4-methyl-1,3,4,6,7,8,9,9a-octahydropyrazino[1,2-a]pyrazin-2-yl]-2-deuterio-quinoline-8-carbonitrile (Intermediate K)**

The title compound was prepared in analogy to the preparation of **Intermediate G** by using *tert*-butyl (*6S,9aR*)-6-methyl-1,3,4,6,7,8,9,9a-octahydropyrazino[1,2-a]pyrazine-2-carboxylate (compound **K-3**) instead of *tert*-butyl (*6R,9aR*)-6-methyl-1,3,4,6,7,8,9,9a-octahydropyrazino[1,2-a]pyrazine-2-carboxylate (compound **F-3**). **Intermediate K** (700 mg) was obtained as a yellow solid. LCMS $(M+H)^+$: 309.

**Intermediate L**

**4-[(*4R,9aS*)-4-methyl-1,3,4,6,7,8,9,9a-octahydropyrazino[1,2-a]pyrazin-2-yl]-1-methyl-1,8-naphthyridin-2-one**

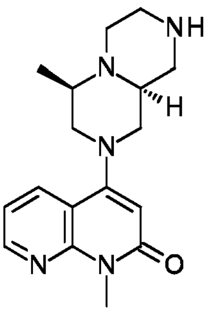

The title compound was prepared according to the following scheme:

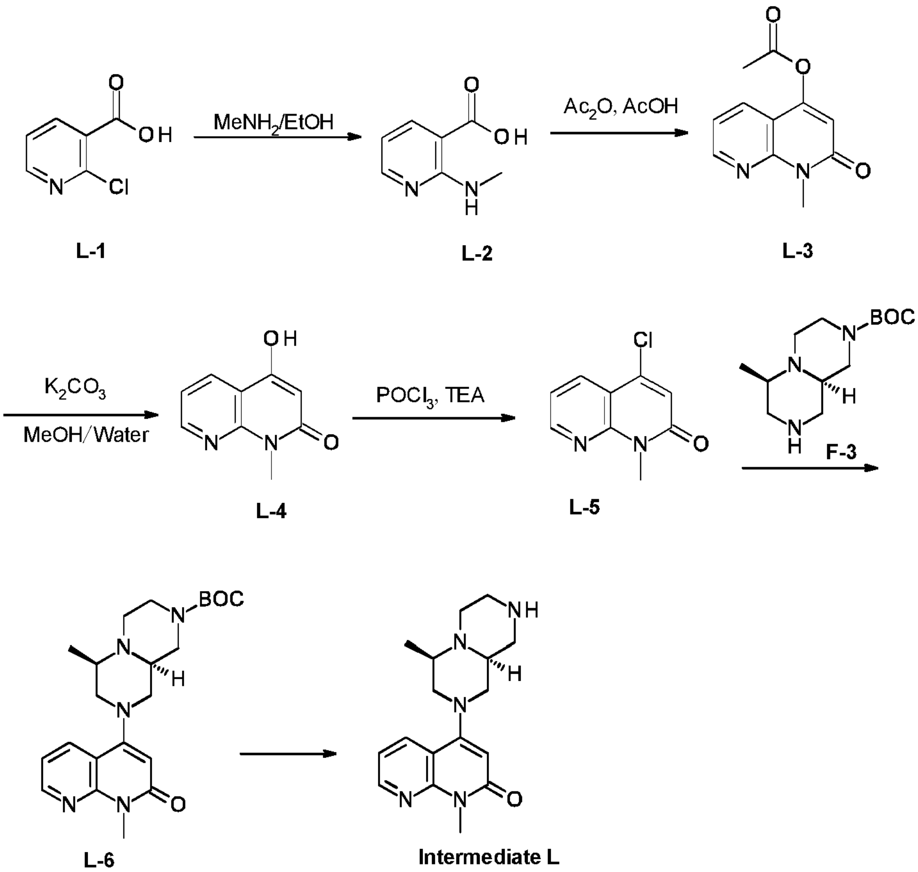


L-1 L-2 L-3 L-4 L-5 L-6 Intermediate L

**Step 1: preparation of 2-(methylamino)pyridine-3-carboxylic acid (compound L-2)**

2-chloronicotinic acid (compound **L-1**, 1.0 kg, 6347 mmol) was dissolved in 33% monomethylamine (386349 mmol) solution in ethanol. The reaction mixture was stirred in the autoclave at 80 °C for 80 hours. The reaction mixture was concentrated in *vacuo* to afford compound **L-2** (1.4 kg, crude). LCMS$(M+H)^+$: 153.

**Step 2: preparation of (1-methyl-2-oxo-1,8-naphthyridin-4-yl) acetate (compound L-3)**

A solution of 2-(methylamino)pyridine-3-carboxylic acid (compound **L-2**, 1.4 kg, crude) in acetic anhydride (10.0 L, 105789 mmol) and acetic acid (5.0 L) was heated to reflux for 2 hours. The reaction mixture was concentrated in *vacuo* to afford compound **L-3** (1.8 kg, crude). LCMS$(M+H)^+$: 219.

**Step 3: preparation of 4-hydroxy-1-methyl-1,8-naphthyridin-2-one (compound L-4)**

To a solution of (1-methyl-2-oxo-1,8-naphthyridin-4-yl) acetate (compound **L-3**, 1.8 kg, crude) in methanol (12.0 L) was added a solution of potassium carbonate (1.9 kg, 13748 mmol) in water (3.6 L). The mixture was stirred at 25 °C for 2 hours. Then the reaction mixture was concentrated under reduced pressure to remove the MeOH. The residue was acidified with HCl solution (6 *N*) to pH = 4-5, extracted with EA (1500 mL) for three times. The combined organic layers were washed with sat. brine (1500 mL), dried over $Na_2SO_4$, and concentrated in *vacuo* to afford compound **L-4** (450 g, 40.2% yield). LCMS$(M+H)^+$: 177, $^1H$ NMR (400 MHz, DMSO-$d_6$) $\delta$ ppm 11.68 (s, 1H), 8.63 (dd, *J* = 4.60, 1.8 Hz, 1H), 8.22 (dd, *J* = 7.8, 1.80 Hz, 1H), 7.27 (dd, *J* = 7.8, 4.6 Hz, 1H), 5.93 (s, 1H), 3.59 (s, 3H).

**Step 4: preparation of 4-chloro-1-methyl-1,8-naphthyridin-2-one (compound L-5)**

A solution of 4-hydroxy-1-methyl-1,8-naphthyridin-2-one (**compound L-4,** 150.0 g, 850 mmol) in phosphorus oxychloride (300 mL) was stirred at 100 °C for 2 hours. The reaction mixture was concentrated in reduced pressure to remove the phosphorus oxychloride. The residue was neutralized by adding saturated aqueous $NaHCO_3$ at room tem-

perature to pH = 7-8, and the mixture was extracted with DCM (1000 mL) twice. The combined organic layer was washed with sat. brine (500 mL), dried over $Na_2SO_4$ and concentrated in *vacuo* to give a crude product, which was purified by silica gel chromatography (PE/EtOAc = 1:0 to 7:1) to afford compound **L-5** (39 g, 24% yield). LCMS(M+H)$^+$: 195, $^1$H NMR (400 MHz, DMSO-$d_6$) $\delta$ ppm 8.75 (dd, *J* = 4.6, 1.6 Hz, 1H), 8.32 (dd, *J* = 7.9, 1.7 Hz, 1H), 7.44 (dd, *J* = 8.0, 4.6 Hz, 1H), 7.03 (s, 1H), 3.66 (s, 3H).

**Step 5-6: preparation of 4-[(*4R,9aS*)-4-methyl-1,3,4,6,7,8,9,9a-octahydropyrazino[1,2-a]pyrazin-2-yl]-1-methyl-1,8-naphthyridin-2-one (Intermediate L)**

The title compound was prepared in analogy to the preparation of **Intermediate F** by using 4-chloro-1-methyl-1,8-naphthyridin-2-one (compound **L-5)** instead of 3,4-difluoropyrazolo[1,5-a]pyridine-7-carbonitrile (compound **F-10**). **Intermediate L** (1.8 g) was obtained as a yellow solid. LCMS (M+H)$^+$: 314.

**Intermediate M**

**4-[(*4S,9aS*)-4-methyl-1,3,4,6,7,8,9,9a-octahydropyrazino[1,2-a]pyrazin-2-yl]-1-methyl-1,8-naphthyridin-2-one**

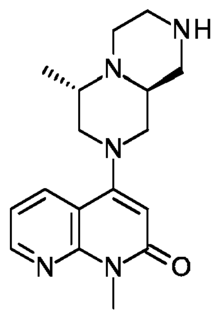

The title compound was prepared in analogy to the preparation of **Intermediate F** by using 4-chloro-1-methyl-1,8-naphthyridin-2-one instead of 3,4-difluoropyrazolo[1,5-a]pyridine-7-carbonitrile (compound **F-10**) and *tert*-butyl (*6S*,*9aR*)-6-methyl-1,3,4,6,7,8,9,9a-octahydropyrazino[1,2-a]pyrazine-2-carboxylate (compound **K-3**) instead of *tert*-butyl (*6R*,*9aR*)-6-methyl-1,3,4,6,7,8,9,9a-octahydropyrazino[1,2-a]pyrazine-2-carboxylate (compound **F-3**). **Intermediate M** (1.7 g) was obtained as a yellow solid. LCMS (M+H)$^+$: 314.

**Compound N**

**(*4S,9aR*)-4-methyl-2-(1-methyl-2-oxo-1,8-naphthyridin-4-yl)-*N*-(3,4,5-trifluorophenyl)-3,4,6,7,9,9a-hexahydro-*1H*-pyrazino[1,2-a]pyrazine-8-carboxamide**

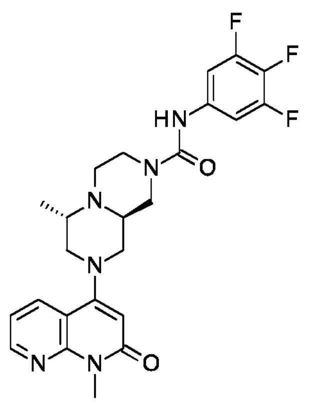

A mixture of 4-[(*4S*,*9aS*)-4-methyl-1,3,4,6,7,8,9,9a-octahydropyrazino[1,2-a]pyrazin-2-yl]-1-methyl-1,8-naphthyridin-2-one (Intermediate **M**, 200 mg, 638 μmol), phenyl N-(3,4,5-trifluorophenyl)carbamate (171 mg, 638 μmol) and DIPEA (412 mg, 3.2 mmol) in DMF (5 mL) was stirred at 60 °C overnight, then the reaction was diluted with EA, washed with water and brine, the organic layer was dried and concentrated, the residue was purified by silica gel to give Compound **N** as a yellow solid, 250 mg. LCMS $(M+H)^+$: 487.

**Compound P**

**(*4R*,*9aR*)-2-(8-cyano-2-deuterio-5-quinolyl)-4-methyl-*N*-(3,4,5-trifluorophenyl)-3,4,6,7,9,9a-hexahydro-*1H*-pyrazino[1,2-a]pyrazine-8-carboxamide**

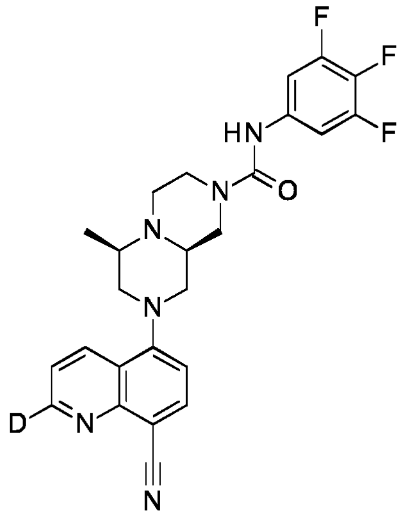

A mixture of 5-[(*4R*,*9aS*)-4-methyl-1,3,4,6,7,8,9,9a-octahydropyrazino[1,2-a]pyrazin-2-yl]-2-deuterio-quinoline-8-carbonitrile (Intermediate **G**, 100 mg, 324 μmol), phenyl (3,4,5-trifluorophenyl)carbamate (86.6 mg, 324 μmol) and DIPEA (170 μl, 973 μmol) in DMF (5 mL) was stirred at 50 °C for 2 hours. Then the reaction was diluted with EA, washed with water and brine, the organic layer was dried and concentrated, the residue was purified by silica gel to give compound **P** as a light yellow solid, 100 mg. LCMS $(M+H)^+$: 482.

**Example 1**

**5-[*cis*-4-methyl-8-(1,2,3,4-tetrahydroisoquinolin-7-ylmethyl)-3,4,6,7,9,9a-hexahydro-*1H*-pyrazino [1,2-a] pyrazin-2-yl] quinoline-8-carbonitrile**

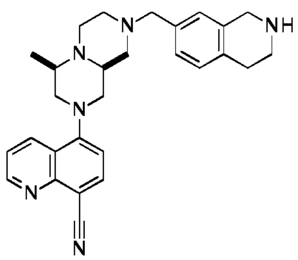

The title compound was prepared according to the following scheme:

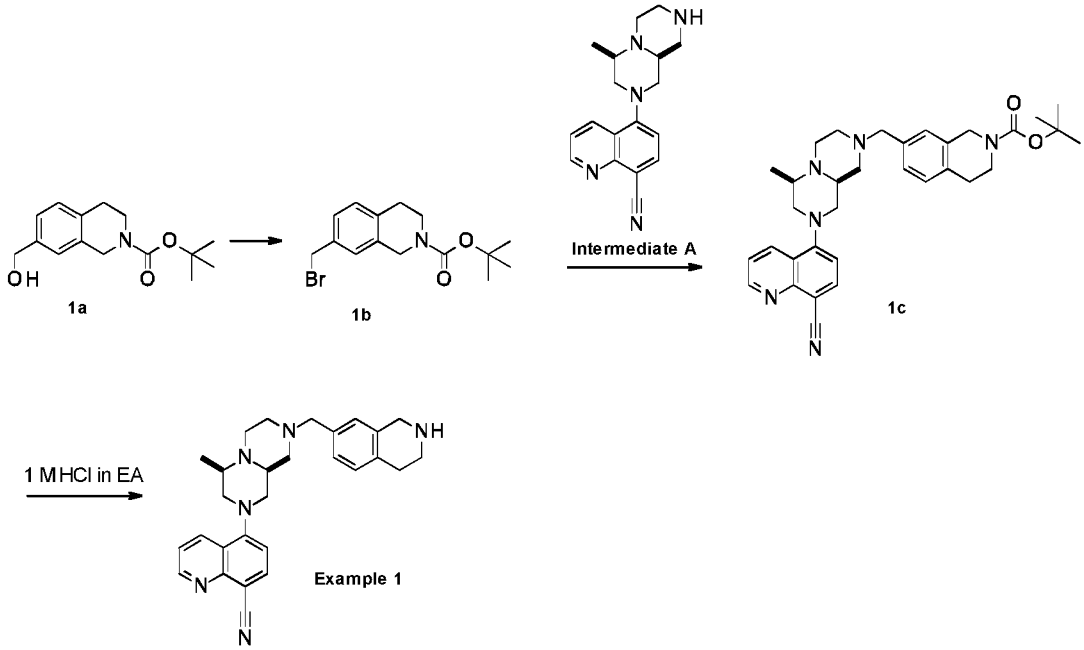


1a 1b 1c Example 1

**Step 1: Preparation of tert-butyl 7-(bromomethyl)-3,4-dihydro-*1H*-isoquinoline-2-carboxylate (compound 1b)**

To a solution of *tert*-butyl 7-(hydroxymethyl)-3,4-dihydro-1H-isoquinoline-2-carboxylate (compound **1a**, 1.35 g, 5.13 mmol) in DCM (30 mL) was added carbon tetrabromide (2.55 g, 7.69 mmol) at 0 °C. Then a solution of triphenylphosphine (2.02 g, 7.69 mmol) in DCM (5 mL) was added dropwise at 0 °C and the resulting mixture was stirred at 25 °C for 30 minutes. The reaction was concentrated and the residue was purified by flash column chromatography to afford compound **1b** (1.20 g) as a light yellow solid. LCMS$(M-56+H)^+$: 270, LCMS$(M-56+2+H)^+$: 272.

**Step 2: Preparation of tert-butyl 7-[[cis-2-(8-cyano-5-quinolyl)-4-methyl-3,4,6,7,9,9a-hexahydro-1H-pyrazino[1,2-a]pyrazin-8-yl]methyl]-3,4-dihydro-*1H*-isoquinoline-2-carboxylate (compound 1c)**

A mixture of 5-[*cis*-4-methyl-1,3,4,6,7,8,9,9a-octahydropyrazino[1,2-a]pyrazin-2-yl]quinoline-8-carbonitrile (Intermediate **A**, 30 mg, 98 μmol), *tert*-butyl 7-(bromomethyl)-3,4-dihydro-1H-isoquinoline-2-carboxylate (compound **1b**, 38 mg, 117 μmol) and sodium bicarbonate (25 mg, 293 μmol) in DMF (2 mL) was stirred at 100 °C for 16 hours. Then the reaction was filtered and the filtrate was purified by HPLC to give compound **1c** as a light yellow solid (30 mg), LCMS $(M+H)^+$: 553.

**Step 3: Preparation of 5-[cis-4-methyl-8-(1,2,3,4-tetrahydroisoquinolin-7-ylmethyl)-3,4,6,7,9,9a-hexahydro-*1H*-pyrazino[1,2-a]pyrazin-2-yl]quinoline-8-carbonitrile (Example 1)**

A mixture of *tert*-butyl 7-[[*cis*-2-(8-cyano-5-quinolyl)-4-methyl-3,4,6,7,9,9a-hexahydro-1H-pyrazino[1,2-a]pyrazin-8-yl]methyl]-3,4-dihydro-1H-isoquinoline-2-carboxylate (compound **1c**, 30 mg, 54 μmol) in 1 M HCl in EA (5 mL) was stirred at rt for 16 hours, then the reaction was concentrated to give Example **1** as a light yellow solid (26 mg). LCMS $(M+H)^+$: 453, $^1H$ NMR (400 MHz, METHANOL-d4) δ ppm: 8.99 (dd, *J* = 1.5, 4.6 Hz, 1H), 8.81 (dd, *J* = 1.4, 8.6 Hz, 1H), 8.21 (d, *J* = 8.1 Hz, 1H), 7.74 (dd, *J* = 4.5, 8.6 Hz, 1H), 7.55 - 7.41 (m, 2H), 7.38 (d, *J* = 8.1 Hz, 1H), 7.30 (d, *J* = 7.9 Hz, 1H), 4.55 - 4.38 (m, 2H), 4.37 - 4.25 (m, 3H), 4.17 - 4.03 (m, 1H), 3.87 - 3.53 (m, 8H), 3.44 - 3.32 (m, 4H), 3.07 (t, *J* = 6.2 Hz, 2H), 1.54 - 1.36 (m, 3H).

**Example 2**

**5-[*cis*-4-methyl-8-(1,2,3,4-tetrahydroisoquinolin-5-yl)-3,4,6,7,9,9a-hexahydro-*1H*-pyrazino [1,2-a] pyrazin-2-yl] quinoline-8-carbonitrile**

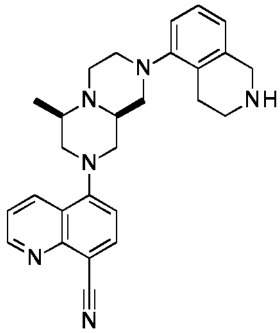

The title compound was prepared according to the following scheme:

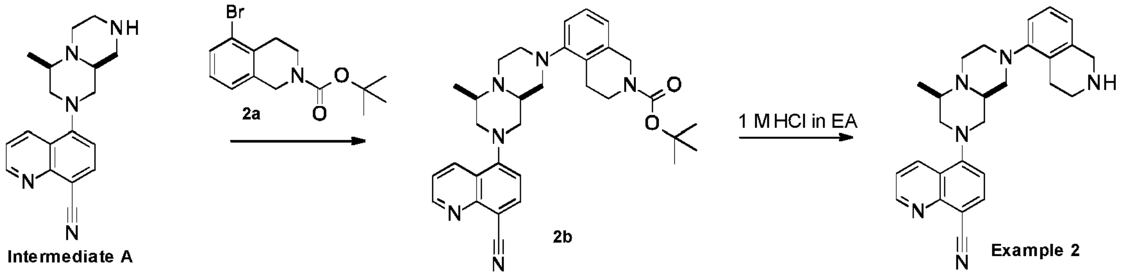

Intermediate A
2b
Example 2

**Step 1: Preparation of tert-butyl 5-[*cis*-2-(8-cyano-5-quinolyl)-4-methyl-3,4,6,7,9,9a-hexahydro-*1H*-pyrazino[1,2-a]pyrazin-8-yl]-3,4-dihydro-1H-isoquinoline-2-carboxylate (compound 2b)**

A mixture of 5-[cis-4-methyl-1,3,4,6,7,8,9,9a-octahydropyrazino[1,2-a]pyrazin-2-yl]quinoline-8-carbonitrile (Intermediate **A**, 30 mg, 98 μmol), tert-butyl 5-bromo-3,4-dihydro-1H-isoquinoline-2-carboxylate (compound **2a**, 46 mg, 146 μmol), RuPhos Pd G2 (8 mg, 10 μmol) and $Cs_2CO_3$ (95 mg, 293 μmol) in dioxane (5 mL) was charged with $N_2$, then the mixture was heated to 100 °C overnight. After cooling, the solid was filtered off and washed with EA (10 mL). The filtrate was concentrated and the residue was purified by prep-HPLC to give compound **2b** as a light yellow solid (25 mg), LCMS $(M+H)^+$: 539.

**Step 2: Preparation of 5-[*cis*-4-methyl-8-(1,2,3,4-tetrahydroisoquinolin-5-yl)-3,4,6,7,9,9a-hexahydro-*1H*-pyrazino[1,2-a]pyrazin-2-yl]quinoline-8-carbonitrile (Example 2)**

A mixture of *tert*-butyl 5-[*cis*-2-(8-cyano-5-quinolyl)-4-methyl-3,4,6,7,9,9a-hexahydro-1H-pyrazino[1,2-a]pyrazin-8-yl]-3,4-dihydro-1H-isoquinoline-2-carboxylate (compound **2b**, 25 mg, 46 μmol) in 1 M HCl in EA (5 mL) was stirred at rt for 16 hours, then the reaction was concentrated to give Example **2** as a light red solid (19 mg). LCMS $(M+H)^+$: 439, $^1H$ NMR (400 MHz, METHANOL-d4) δ ppm: 9.11 (dd, *J* = 1.1, 4.4 Hz, 1H), 8.96 (d, *J* = 7.5 Hz, 1H), 8.32 (d, *J* = 8.1 Hz, 1H), 7.86 (dd, *J* = 4.6, 8.5 Hz, 1H), 7.50 (d, *J* = 8.1 Hz, 1H), 7.40 - 7.31 (m, 1H), 7.26 (d, *J* = 7.8 Hz, 1H), 7.10 (d, *J* = 7.7 Hz, 1H), 4.40 (s, 2H), 4.19 (br t, *J* = 10.2 Hz, 1H), 4.09 - 3.97 (m, 2H), 3.87 - 3.72 (m, 2H), 3.63 - 3.37 (m, 8H), 3.30 - 3.13 (m, 3H), 1.58 (d, *J* = 6.5 Hz, 3H).

**Example 3**

**5-[*cis*-4-methyl-8-[(2-piperazin-1-yl-4-pyridyl)methyl]-3,4,6,7,9,9a-hexahydro-*1H*-pyrazino[1,2-a]pyrazin-2-yl]quinoline-8-carbonitrile**

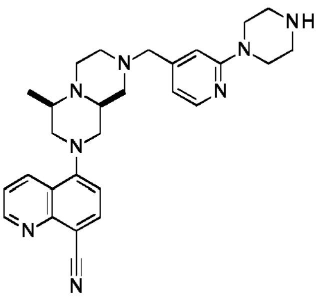

The title compound was prepared according to the following scheme:

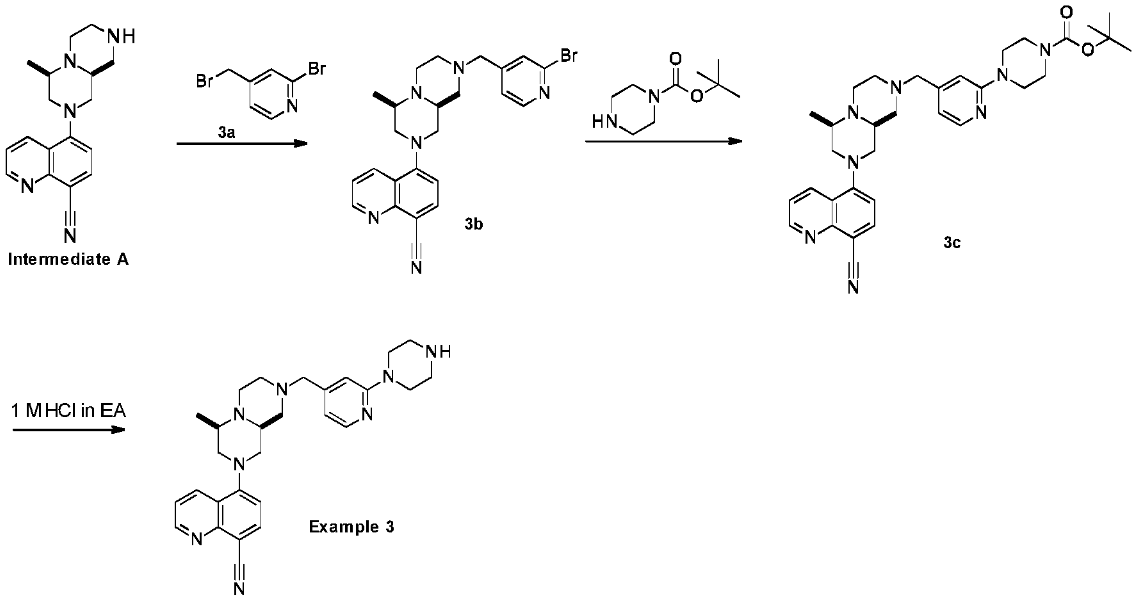


Intermediate A

3b

3c

Example 3

### Step 1: Preparation of 5-[*cis*-8-[(2-bromo-4-pyridyl)methyl]-4-methyl-3,4,6,7,9,9a-hexahydro-*1H*-pyrazino[1,2-a]pyrazin-2-yl]quinoline-8-carbonitrile (compound 3b)

A mixture of 2-bromo-4-(bromomethyl)pyridine (compound **3a**, 49 mg, 195 μmol), 5-(4-methyloctahydro-2H-pyrazino[1,2-a]pyrazin-2-yl)quinoline-8-carbonitrile (intermediate **A**, 30 mg, 98 μmol) and $K_2CO_3$ (41 mg, 293 μmol) in MeCN (5 mL) was stirred at rt for 16 hours. Then the reaction mixture was concentrated, and the residue was purified by silica gel column to give compound **3b** as a light yellow foam (30 mg). LCMS $(M+H)^+$: 477, LCMS $(M+2+H)^+$: 479.

### Step 2: Preparation of tert-butyl 4-[4-[[cis-2-(8-cyano-5-quinolyl)-4-methyl-3,4,6,7,9,9a-hexahydro-*1H*-pyrazino[1,2-a]pyrazin-8-yl]methyl]-2-pyridyl]piperazine-1-carboxylate (compound 3c)

A mixture of 5-[*cis*-8-[(2-bromo-4-pyridyl)methyl]-4-methyl-3,4,6,7,9,9a-hexahydro-*1H*-pyrazino[1,2-a]pyrazin-2-yl]quinoline-8-carbonitrile (compound **3b**, 30 mg, 63 μmol), *tert*-butyl piperazine-1-carboxylate (59 mg, 314 μmol), $Cs_2CO_3$ (61 mg, 189 μmol) and RuPhos Pd G2 (9 mg, 13 μmol) in dioxane (5 mL) was stirred at 100 °C for 16 hours. Then the reaction mixture was concentrated and the residue was purified by silica gel column to give compound 3c as a light yellow foam (20 mg). LCMS $(M+H)^+$: 583.

### Step 3: Preparation of 5-[*cis*-4-methyl-8-[(2-piperazin-1-yl-4-pyridyl)methyl]-3,4,6,7,9,9a-hexahydro-*1H*-pyrazino[1,2-a]pyrazin-2-yl]quinoline-8-carbonitrile (compound 3c)

A mixture of *tert*-butyl 4-[4-[[*cis*-2-(8-cyano-5-quinolyl)-4-methyl-3,4,6,7,9,9a-hexahydro-1H-pyrazino[1,2-a]pyrazin-8-yl]methyl]-2-pyridyl]piperazine-1-carboxylate (compound **3c**, 20 mg, 34 μmol) in 1 M HCl in EA (5 mL) was stirred at rt for 16 hours. After the reaction mixture was concentrated, the residue was dissolved in NaOH (1M, 5 mL) and extracted with DCM (10 mL). The organic layer was dried and concentrated, and the residue was lyophilized to give

Example **3** as a light yellow powder (10 mg). LCMS $(M+H)^+$: 483, $^1H$ NMR (400 MHz, METHANOL-d4) δ ppm: 8.96 (dd, *J* = 1.7, 4.3 Hz, 1H), 8.60 (dd, *J* = 1.7, 8.6 Hz, 1H), 8.12 (d, *J* = 8.1 Hz, 1H), 8.04 (d, *J* = 5.1 Hz, 1H), 7.62 (dd, *J* = 4.3, 8.6 Hz, 1H), 7.21 (d, *J* = 8.1 Hz, 1H), 6.82 (s, 1H), 6.73 (d, *J* = 5.3 Hz, 1H), 3.54 - 3.44 (m, 6H), 3.40 (br d, *J* = 11.0 Hz, 1H), 3.33 - 3.22 (m, 2H), 3.00 - 2.89 (m, 5H), 2.84 - 2.70 (m, 5H), 2.37 - 2.22 (m, 2H), 2.09 - 1.92 (m, 1H), 1.15 (d, *J* = 5.7 Hz, 3H).

**Example 4**

**5-[*cis*-8-isoindolin-4-yl-4-methyl-3,4,6,7,9,9a-hexahydro-*1H*-pyrazino[1,2-a]pyrazin-2-yl] quinoline-8-carbonitrile**

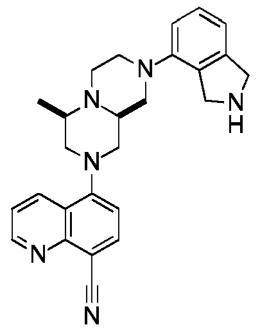

The title compound was prepared according to the following scheme:

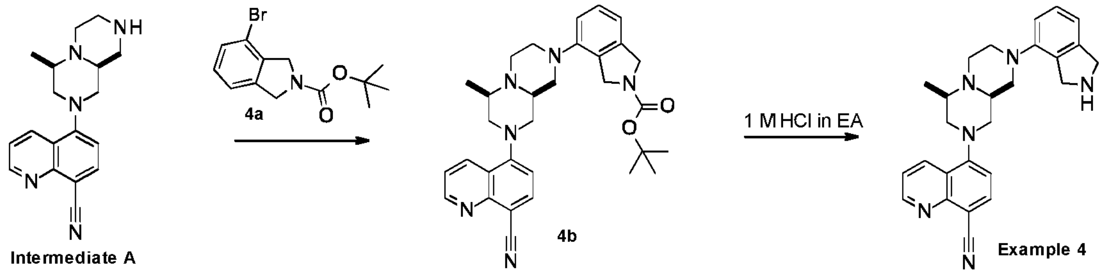


Intermediate A

4b

Example 4

Example **4** was prepared in analogy to the preparation of Example 2 by using compound **4a** instead of compound **2a**. Example **4** was obtained as a light brown solid (26 mg). LCMS $(M+H)^+$: 425, $^1H$ NMR (400 MHz, METHANOL-d4) δ ppm: 9.03 (dd, *J* = 1.7, 4.2 Hz, 1H), 8.74 (dd, *J* = 1.7, 8.6 Hz, 1H), 8.23 (d, *J* = 7.9 Hz, 1H), 7.72 (dd, *J* = 4.3, 8.6 Hz, 1H), 7.51 - 7.34 (m, 2H), 7.19 (dd, *J* = 7.6, 16.0 Hz, 2H), 4.70 (s, 2H), 4.64 (s, 2H), 4.21 - 3.89 (m, 3H), 3.85 - 3.71 (m, 2H), 3.62 - 3.37 (m, 4H), 3.25 - 3.10 (m, 2H), 1.54 (d, *J* = 6.6 Hz, 3H).

**Example 5**

**5-[(*4R*,*9aS*)-4-methyl-8-(1,2,3,4-tetrahydroisoquinolin-7-ylmethyl)-3,4,6,7,9,9a-hexahydro-*1H*-pyrazino[1,2-a]pyrazin-2-yl]quinoline-8-carbonitrile**

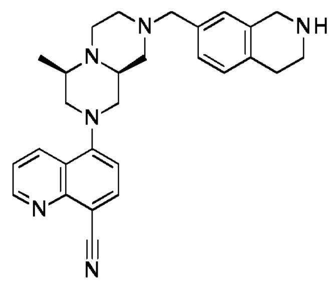

Example **5** was prepared in analogy to the preparation of Example **1**by using Intermediate **C** instead of Intermediate **A**. Example **5** was obtained as an orange solid (113 mg). LCMS $(M+H)^+$: 453, $^1$H NMR (400 MHz, METHANOL-d4) $\delta$ ppm: 9.09 (dd, *J* = 1.5, 4.5 Hz, 1H), 8.88 (dd, *J* = 1.6, 8.6 Hz, 1H), 8.30 (d, *J* = 8.1 Hz, 1H), 7.83 (dd, *J* = 4.5, 8.6 Hz, 1H), 7.65 - 7.53 (m, 2H), 7.48 (d, *J* = 8.1 Hz, 1H), 7.42 (d, *J* = 7.9 Hz, 1H), 4.63 - 4.48 (m, 2H), 4.48 - 4.36 (m, 3H), 4.22 (br d, *J* = 13.1 Hz, 1H), 3.98 - 3.60 (m, 8H), 3.58 - 3.39 (m, 4H), 3.19 (t, *J* = 6.3 Hz, 2H), 1.56 (d, *J* = 6.4 Hz, 3H).

**Example 6**

**5-[(*4R*,*9aS*)-4-methyl-8-(5,6,7,8-tetrahydro-1,6-naphthyridin-3-ylmethyl)-3,4,6,7,9,9a-hexahydro-*1H*-pyrazino[1,2-a]pyrazin-2-yl]quinoline-8-carbonitrile**

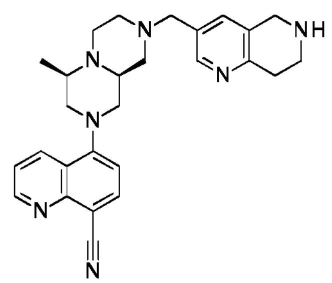

The title compound was prepared according to the following scheme:

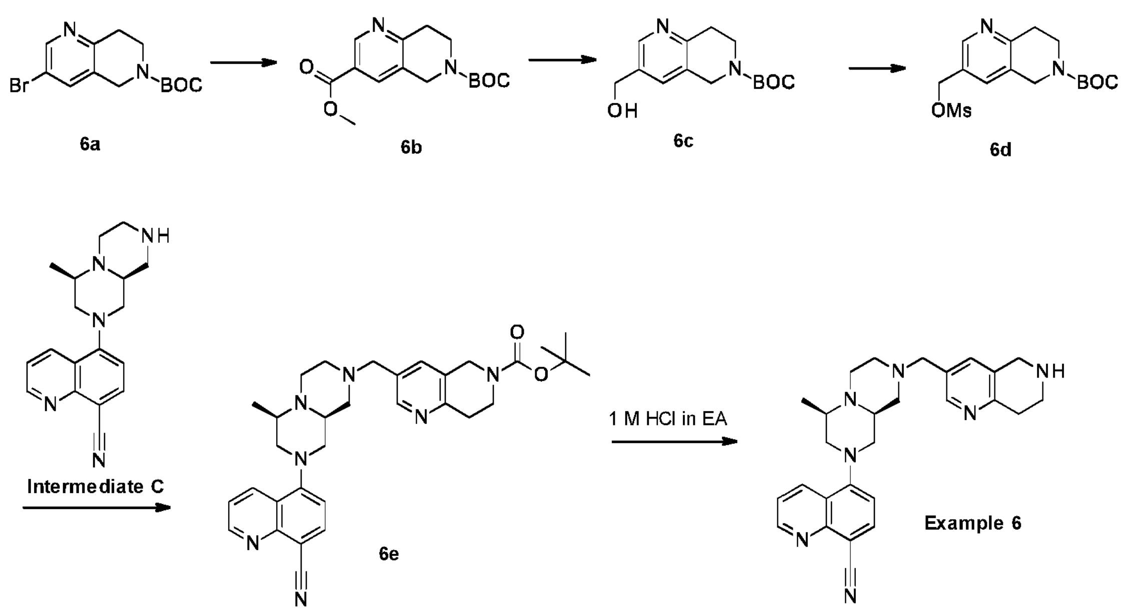

6a 6b 6c 6d 6e Example 6

**Step 1: Preparation of O6-*tert*-butyl O3-methyl 7,8-dihydro-*5H*-1,6-naphthyridine-3,6-dicarboxylate (compound 6b)**

To a solution of *tert*-butyl 3-bromo-7,8-dihydro-*5H*-1,6-naphthyridine-6-carboxylate (compound **6a**, 3.2 g, 10.2 mmol) in methanol (50.0 mL) was added 1,3-bis(diphenylphosphino)propane (1.7 g, 4.1 mmol), triethylamine (7.1 mL, 51.1 mmol) and palladium (II) acetate (0.9 mg, 4.0 mmol). The mixture was purged with nitrogen for three times. Then the reaction mixture was stirred at 100 °C for 16 hours under the carbon monoxide (2280 mmHg). The reaction mixture was filtered and the filtrate was concentrated under reduce pressure. The residue was purified by chromatography column to give compound **6b** (2.6 g) as a light yellow solid. LCMS $(M+H)^+$: 293.

**Step 2: Preparation of *tert*-butyl 3-(hydroxymethyl)-7,8-dihydro-*5H*-1,6-naphthyridine-6-carboxylate (compound 6c)**

To a solution of O6-*tert*-butyl O3-methyl 7,8-dihydro-*5H*-1,6-naphthyridine-3,6-dicarboxylate (compound **6**b, 2.5 g, 8.5 mmol) in DCM (30.0 mL) was added DIBAL-H (17.0 mL, 17.0 mmol) at 0°C. The reaction mixture was then stirred at 0°C for 2 hours. The reaction mixture was quenched with aqueous potassium sodium tartrate solution and extracted with DCM. The combined organic layer was washed with brine, dried and concentrated. The residue was purified by chromatography column to give compound **6c** (1.1 g) as a yellow solid. LCMS $(M+H)^+$: 265, $^1$H NMR (400MHz, CHLOROFORM-d) $\delta$ ppm: 8.38 (s, 1H), 7.46 (s, 1H), 5.30 (s, 1H), 4.71 (s, 2H), 4.60 (s, 2H), 3.75 (t, *J* = 5.9 Hz, 2H), 2.99 (br t, *J* = 5.7 Hz, 2H), 1.50 (s, 9H).

**Step 3: Preparation of *tert*-butyl 3-(methylsulfonyloxymethyl)-7,8-dihydro-*5H*-1,6-naphthyridine-6-carboxylate (compound 6d)**

A mixture of *tert*-butyl 3-(hydroxymethyl)-7,8-dihydro-*5H*-1,6-naphthyridine-6-carboxylate (compound **6c**, 200 mg, 757 $\mu$mol), DIPEA (396 $\mu$l, 2270 $\mu$mol) and methanesulfonic anhydride (264 mg, 1510 $\mu$mol) in DCM (10 mL) was stirred at rt for 16 hours. Then the reaction was diluted with DCM, washed with water, $K_2CO_3$ (1N in water) and brine, the organic layer was dried and concentrated to give compound **6d** as a light yellow oil (200 mg). LCMS $(M+H)^+$: 343.

**Step 4: Preparation of tert-butyl 3-[[(*4R*,*9aS*)-2-(8-cyano-5-quinolyl)-4-methyl-3,4,6,7,9,9a-hexahydro-1H-pyrazino[1,2-a]pyrazin-8-yl]methyl]-7,8-dihydro-*5H*-1,6-naphthyridine-6-carboxylate (compound 6e)**

A mixture of *tert*-butyl 3-(methylsulfonyloxymethyl)-7,8-dihydro-*5H*-1,6-naphthyridine-6-carboxylate (compound **6d**, 111 mg, 325 $\mu$mol), 5-[(*4R*,*9aS*)-4-methyl-1,3,4,6,7,8,9,9a-octahydropyrazino[1,2-a]pyrazin-2-yl]quinoline-8-carbonitrile (Intermediate **C**, 50 mg, 163 $\mu$mol) and $K_2CO_3$ (67 mg, 488 $\mu$mol) in MeCN (5 mL) was stirred at rt for 16 hours. Then the reaction was concentrated, the residue was purified by prep-HPLC to give compound **6e** as a light yellow solid (15 mg). LCMS $(M+H)^+$: 554.

**Step 5: Preparation of 5-[(*4R*,*9aS*)-4-methyl-8-(5,6,7,8-tetrahydro-1,6-naphthyridin-3-ylmethyl)-3,4,6,7,9,9a-hexahydro-1H-pyrazino[1,2-a]pyrazin-2-yl]quinoline-8-carbonitrile (Example 6)**

A mixture of *tert*-butyl 3-[[(*4R*,*9aS*)-2-(8-cyano-5-quinolyl)-4-methyl-3,4,6,7,9,9a-hexahydro-*1H*-pyrazino[1,2-a]pyrazin-8-yl]methyl]-7,8-dihydro-5H-1,6-naphthyridine-6-carboxylate (compound **6e**, 15 mg, 27 $\mu$mol) in 1 M HCl in EA (2 mL) was stirred at rt for 16 hours. Then the reaction was concentrated to give Example **6** as an orange solid (15 mg). LCMS $(M+H)^+$: 454. $^1$H NMR (400 MHz, METHANOL-d4) $\delta$ ppm: 9.08 (d, *J* = 3.8 Hz, 1H), 8.94 - 8.83 (m, 2H), 8.55 (s, 1H), 8.28 (d, *J* = 7.9 Hz, 1H), 7.82 (dd, *J* = 4.4, 8.4 Hz, 1H), 7.45 (d, *J* = 8.1 Hz, 1H), 4.68 (s, 2H), 4.21 - 3.95 (m, 4H), 3.94 - 3.86 (m, 1H), 3.80 - 3.65 (m, 4H), 3.54 - 3.46 (m, 2H), 3.45 - 3.35 (m, 5H), 3.13 - 3.00 (m, 1H), 2.86 - 2.75 (m, 1H), 1.54 (d, *J* = 6.5 Hz, 3H).

**Example 7**

**5-[(*4R*,*9aS*)-4-methyl-8-[2-(4-piperazin-1-ylphenyl)ethyl]-3,4,6,7,9,9a-hexahydro-*1H*pyrazino[1,2-a]pyrazin-2-yl]quinoline-8-carbonitrile**

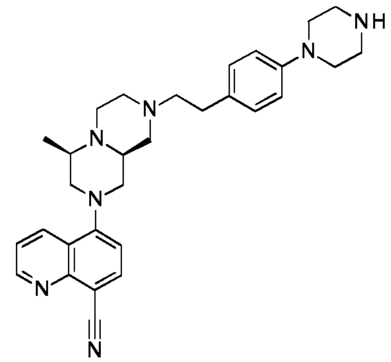

The title compound was prepared according to the following scheme:

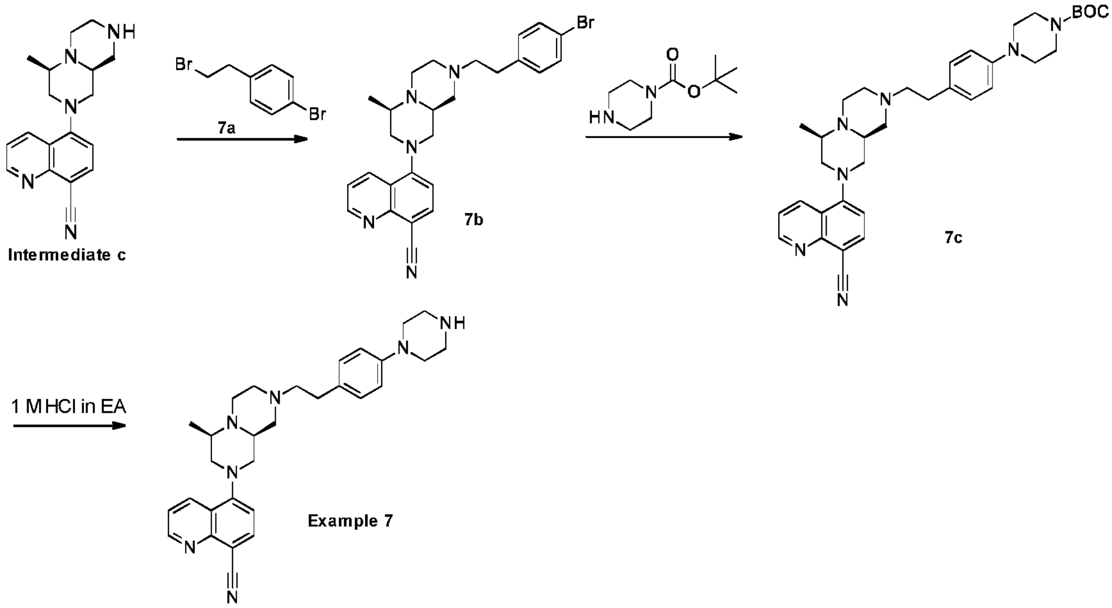


Intermediate c, 7b, 7c, Example 7

**Step 1: Preparation of 5-[(*4R*,*9aA*)-8-[2-(4-bromophenyl)ethyl]-4-methyl-3,4,6,7,9,9a-hexahydro-*1H*-pyrazino[1,2-a]pyrazin-2-yl]quinoline-8-carbonitrile (compound 7b)**

A mixture of 1-bromo-4-(2-bromoethyl)benzene (compound **7a**, 51.5 mg, 195 μmol), 5-[(*4R*,*9aS*)-4-methyl-1,3,4,6,7,8,9,9a-octahydropyrazino[1,2-a]pyrazin-2-yl]quinoline-8-carbonitrile (Intermediate **C**, 40 mg, 130 μmol) and $K_2CO_3$ (54 mg, 390 μmol) in MeCN (3 mL) was stirred at 80 °C for 16 hours. Then the reaction mixture was filtered and concentrated to give compound **7b** as a light yellow solid (60 mg), LCMS $(M+H)^+$: 491.

**Step 2: Preparation of 5-[(*4R*,*9aS*)-4-methyl-8-[2-(4-piperazin-1-ylphenyl)ethyl]-3,4,6,7,9,9a-hexahydro-*1H*-pyrazino[1,2-a]pyrazin-2-yl]quinoline-8-carbonitrile (Example 7)**

Example 7 was prepared in analogy to the preparation of Example **3** by using 5-[(*4R*,*9aS*)-8-[2-(4-bromophenyl)ethyl]-4-methyl-3,4,6,7,9,9a-hexahydro-1H-pyrazino[1,2-a]pyrazin-2-yl]quinoline-8-carbonitrile (compound **7b**) instead of compound **3b**. Example 7 was obtained as an orange solid (58 mg). LCMS $(M+H)^+$: 496, $^1H$ NMR (400 MHz, METHANOL-d4) δ ppm: 9.12 (dd, *J* = 1.5, 4.6 Hz, 1H), 8.96 (dd, *J* = 1.6, 8.6 Hz, 1H), 8.34 (d, *J* = 8.1 Hz, 1H), 7.88 (dd, *J* = 4.5, 8.6 Hz, 1H), 7.52 (d, *J* = 8.1 Hz, 1H), 7.31 (d, *J* = 8.6 Hz, 2H), 7.08 (d, *J* = 8.7 Hz, 2H), 4.48 - 4.36 (m, 1H), 4.26 (br d, *J* = 13.2 Hz, 1H), 4.14 - 3.94 (m, 3H), 3.88 - 3.75 (m, 3H), 3.72 - 3.62 (m, 2H), 3.59 - 3.48 (m, 4H), 3.48 - 3.38 (m, 8H), 3.22 - 3.09 (m, 2H), 1.58 (d, *J* = 6.4 Hz, 3H).

**Example 8**

**5-[(*4R,9aS*)-4-methyl-8-[(6-piperazin-1-yl-3-pyridyl)methyl]-3,4,6,7,9,9a-hexahydro-*1H*-pyrazino [1,2-a]pyrazin-2-yl]quinoline-8-carbonitrile**

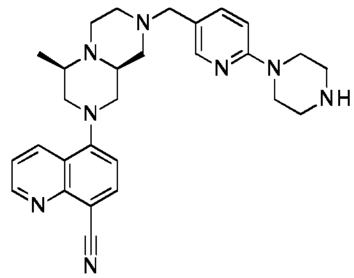

Example **8** was prepared in analogy to the preparation of Example **3** by using Intermediate **C** instead of Intermediate **A** and 2-bromo-5-(bromomethyl)pyridine instead of compound **3a**. Example **8** was obtained as an orange solid (17 mg). LCMS $(M+H)^+$: 483, $^1H$ NMR (400 MHz, METHANOL-d4) δ ppm: 9.09 (dd, *J* = 1.4, 4.5 Hz, 1H), 8.87 (dd, *J* = 1.5, 8.6 Hz, 1H), 8.37 - 8.24 (m, 3H), 7.82 (dd, *J* = 4.5, 8.6 Hz, 1H), 7.49 (dd, *J* = 8.7, 14.3 Hz, 2H), 4.43 - 4.11 (m, 4H), 4.09 - 4.03 (m, 4H), 3.99 - 3.86 (m, 1H), 3.80 - 3.66 (m, 4H), 3.58 - 3.47 (m, 6H), 3.45 - 3.38 (m, 3H), 1.55 (d, *J* = 6.48 Hz, 3H).

**Example 9**

**5-[(*4R,9aS*)-8-isoindolin-4-yl-4-methyl-3,4,6,7,9,9a-hexahydro-*1H*-pyrazino[1,2-a]pyrazin-2-yl] quinoline-8-carbonitrile**

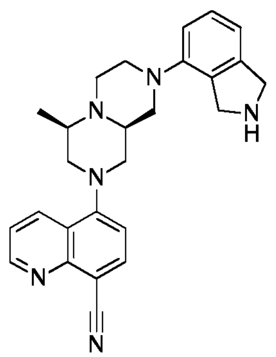

Example **9** was prepared in analogy to the preparation of Example **2** by using Intermediate **C** instead of Intermediate **A** and compound **4a** instead of compound **2a**. Example **9** was obtained as an orange solid (65 mg). LCMS $(M+H)^+$: 425, $^1H$ NMR (400 MHz, METHANOL-d4) δ ppm: 9.08 (dd, *J* = 1.6, 4.4 Hz, 1H), 8.84 (dd, *J* = 1.6, 8.6 Hz, 1H), 8.28 (d, *J* = 8.1 Hz, 1H), 7.78 (dd, *J* = 4.4, 8.6 Hz, 1H), 7.54 - 7.40 (m, 2H), 7.21 (dd, *J* = 7.8, 14.4 Hz, 2H), 4.73 (s, 2H), 4.66 (s, 2H), 4.16 (br t, *J* = 10.9 Hz, 1H), 4.10 - 3.96 (m, 2H), 3.86 - 3.74 (m, 2H), 3.62 - 3.53 (m, 2H), 3.51 - 3.37 (m, 4H), 3.28 - 3.19 (m, 1H), 1.58 (d, *J* = 6.48 Hz, 3H).

**Example 10**

**5-[(*4R,9aS*)-4-methyl-8-(1,2,3,4-tetrahydroisoquinolin-6-ylmethyl)-3,4,6,7,9,9a-hexahydro-*1H*-pyrazino[1,2-a]pyrazin-2-yl]quinoline-8-carbonitrile**

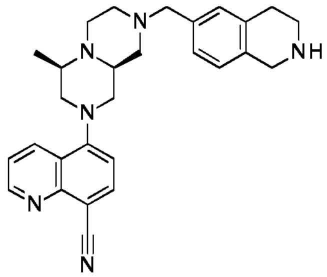

Example **10** was prepared in analogy to the preparation of Example **1** by using Intermediate **C** instead of Intermediate **A** and *tert*-butyl 6-(bromomethyl)-3,4-dihydro-*1H*-isoquinoline-2-carboxylate instead of compound **1b**. Example **10** was obtained as a light yellow solid (46 mg). LCMS $(M+H)^+$: 453, $^1H$ NMR (400 MHz, METHANOL-d4) δ ppm: 9.07 (dd, *J* = 1.6, 4.4 Hz, 1H), 8.81 (dd, *J* = 1.6, 8.7 Hz, 1H), 8.27 (d, *J* = 7.9 Hz, 1H), 7.78 (dd, *J* = 4.4, 8.6 Hz, 1H), 7.60 - 7.55 (m, 2H), 7.44 (d, *J* = 7.9 Hz, 1H), 7.38 (d, *J* = 7.8 Hz, 1H), 4.59 - 4.46 (m, 2H), 4.43 (s, 2H), 4.36 - 4.26 (m, 1H), 4.17 (br d, *J* = 13.3 Hz, 1H), 3.94 - 3.67 (m, 6H), 3.65 - 3.48 (m, 4H), 3.44 - 3.35 (m, 2H), 3.26 - 3.17 (m, 2H), 1.53 (d, *J* = 6.4 Hz, 3H).

## Example 11

**5-[(*4R*,*9aS*)-4-methyl-8-[2-(6-piperazin-1-yl-3-pyridyl)ethyl]-3,4,6,7,9,9a-hexahydro-*1H*-pyrazino [1,2-a] pyrazin-2-yl] quinoline-8-carbonitrile**

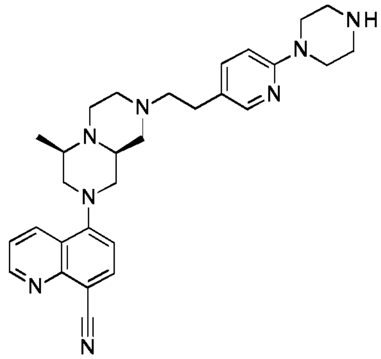

The title compound was prepared according to the following scheme:

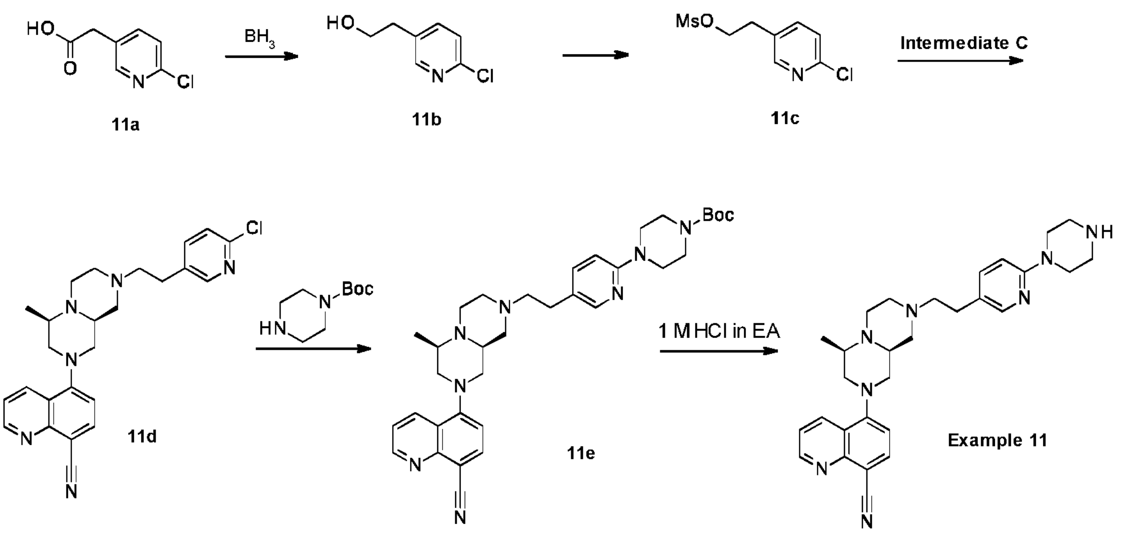


11a 11b 11c

11d 11e Example 11

**Step 1: Preparation of 2-(6-chloro-3-pyridyl)ethanol (compound 11b)**

A mixture of 2-(6-chloropyridin-3-yl)acetic acid (compound **11a**, 4 g, 23 mmol) in borane tetrahydrofuran complex (47 mL, 47 mmol) was stirred at rt for 1 hour. After the reaction was quenched with MeOH, the mixture was concentrated and the residue was purified by silica gel column to give compound **11b** as a colorless oil (4 g), LCMS $(M+H)^+$: 158.

**Step 2: Preparation of 2-(6-chloro-3-pyridyl)ethyl methanesulfonate (compound 11c)**

To a mixture of 2-(6-chloro-3-pyridyl)ethanol (compound **11b**, 4.0 g, 25.4 mmol) and DIPEA (13.3 ml, 76.1 mmol) in DCM (50.0 mL) was added methanesulfonic anhydride (6.6 g, 38.1 mmol) slowly at rt. After the reaction mixture was stirred at rt for 15 minutes., it was diluted with $NaHCO_3$ and extracted with EtOAc. The organic layer was dried and concentrated to give compound **11c** as a light brown oil (5.0 g), LCMS $(M+H)^+$: 236.

**Step 3: Preparation of 5-[(*4R*,*9aS*)-8-[2-(6-chloro-3-pyridyl)ethyl]-4-methyl-3,4,6,7,9,9a-hexahydro-*1H*-pyrazino[1,2-a]pyrazin-2-yl]quinoline-8-carbonitrile (compound 11d)**

A mixture of 2-(6-chloro-3-pyridyl)ethyl methanesulfonate (compound **11c**, 173 mg, 732 μmol), 5-[(*4R*,*9aS*)-4-methyl-1,3,4,6,7,8,9,9a-octahydropyrazino[1,2-a]pyrazin-2-yl]quinoline-8-carbonitrile (Intermediate **C**, 150 mg, 488 μmol) and potassium carbonate (202 mg, 1460 μmol,) in MeCN (10 mL) was stirred at 80 °C for 16 hours. Then the reaction was concentrated, and the residue was purified by silica gel column to give compound **11d** as a light yellow solid (150 mg), LCMS $(M+H)^+$: 447.

**Step 4: Preparation of 5-[(*4R*,*9aS*)-4-methyl-8-[2-[6-(4-methylpiperazin-1-yl)-3-pyridyl]ethyl]-3,4,6,7,9,9a-hexahydro-*1H*-pyrazino[1,2-a]pyrazin-2-yl]quinoline-8-carbonitrile (compound 11e)**

A mixture of 5-[(*4R*,*9aS*)-8-[2-(6-chloro-3-pyridyl)ethyl]-4-methyl-3,4,6,7,9,9a-hexahydro-*1H*-pyrazino[1,2-a]pyrazin-2-yl]quinoline-8-carbonitrile (compound **11d**, 60 mg, 134 μmol), *tert*-butyl piperazine-1-carboxylate (38 mg, 201 μmol), cesium carbonate (131 mg, 403 μmol) and RuPhos Pd G2 (19 mg, 27 μmol) in dioxane (5 mL) was stirred at 110 °C for 16 hours. Then the reaction was concentrated and the residue was purified by prep-HPLC to give compound **11e** as a light yellow powder (35 mg), LCMS $(M+H)^+$: 597.

**Step 5: Preparation of 5-[(*4R*,*9aS*)-4-methyl-8-[2-(6-piperazin-1-yl-3-pyridyl)ethyl]-3,4,6,7,9,9a-hexahydro-*1H*-pyrazino[1,2-a]pyrazin-2-yl]quinoline-8-carbonitrile (Example 11)**

A mixture of 5-[(*4R*,*9aS*)-4-methyl-8-[2-[6-(4-methylpiperazin-1-yl)-3-pyridyl]ethyl]-3,4,6,7,9,9a-hexahydro-1H-pyrazino[1,2-a]pyrazin-2-yl]quinoline-8-carbonitrile (compound **11e**, 35 mg, 59 μmol) in 1 M HCl in EA (5 mL) was stirred at rt for 16 hours. Then the reaction was concentrated, and the residue was lyophilized to give Example **11** as an orange solid (32 mg). LCMS $(M+H)^+$: 497. $^1H$ NMR (400 MHz, METHANOL-d4) δ ppm: 9.08 (dd, *J* = 1.6, 4.3 Hz, 1H), 8.83 (dd, *J* = 1.6, 8.6 Hz, 1H), 8.28 (d, *J* = 7.9 Hz, 1H), 8.24 - 8.11 (m, 2H), 7.80 (dd, *J* = 4.4, 8.7 Hz, 1H), 7.52 (d, *J* = 9.4 Hz, 1H), 7.46 (d, *J* = 7.9 Hz, 1H), 4.38 - 4.24 (m, 1H), 4.23 - 4.12 (m, 1H), 4.07 - 3.95 (m, 6H), 3.92 - 3.81 (m, 1H), 3.78 - 3.63 (m, 3H), 3.63 - 3.47 (m, 8H), 3.47 - 3.38 (m, 2H), 3.27 - 3.19 (m, 2H), 1.54 (d, *J* = 6.5 Hz, 3H).

**Example 12**

**5-[(*4R*,*9aS*)-8-[2-[6-[(*3R,4R*)-3-amino-4-methoxy-pyrrolidin-1-yl]-3-pyridyl]ethyl]-4-methyl-3,4,6,7,9,9a-hexahydro-1*H*-pyrazino[1,2-a]pyrazin-2-yl]quinoline-8-carbonitrile**

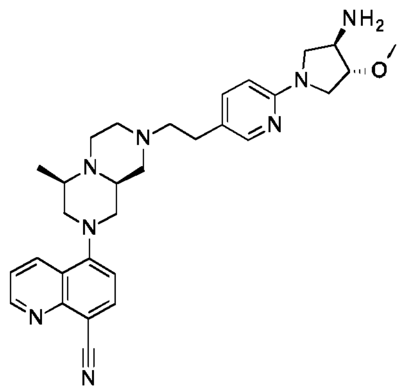

Example **12** was prepared in analogy to the preparation of Example **11** by using *tert*-butyl *N*-[(*3R*,*4R*)-4-methoxypyrrolidin-3-yl]carbamate instead of *tert*-butyl piperazine-1-carboxylate. Example **12** was obtained as an orange solid (34 mg). LCMS $(M+H)^+$: 527, $^1H$ NMR (400 MHz, METHANOL-d4) δ ppm: 9.08 (dd, *J* = 1.6, 4.4 Hz, 1H), 8.82 (dd, *J* = 1.6, 8.6 Hz, 1H), 8.28 (d, *J* = 8.1 Hz, 1H), 8.16 (dd, *J* = 2.1, 9.4 Hz, 1H), 8.07 (d, *J* = 1.6 Hz, 1H), 7.79 (dd, *J* = 4.3, 8.6 Hz, 1H), 7.46 (d, *J* = 8.1 Hz, 1H), 7.24 (d, *J* = 9.3 Hz, 1H), 4.36 - 4.23 (m, 2H), 4.21 - 4.09 (m, 4H), 4.05 - 3.94 (m, 2H), 3.91 - 3.82 (m, 2H), 3.81 - 3.64 (m, 4H), 3.63 - 3.45 (m, 7H), 3.43 - 3.36 (m, 2H), 3.26 - 3.18 (m, 2H), 1.54 (d, *J* = 6.4 Hz, 3H).

**Example 13**

**5-[(*4R*,*9aS*)-8-[2-[5-[(*3R*,*4R*)-3-amino-4-methoxy-pyrrolidin-1-yl]-2-pyridyl]ethyl]-4-methyl-3,4,6,7,9,9a-hexahydro-*1H*-pyrazino[1,2-a]pyrazin-2-yl]quinoline-8-carbonitrile**

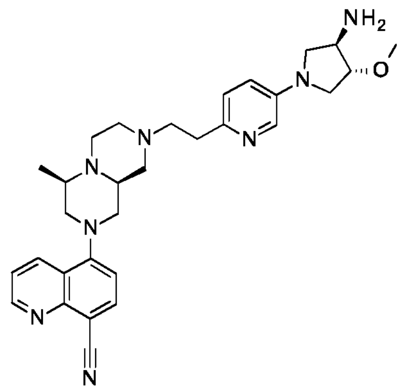

The title compound was prepared according to the following scheme:

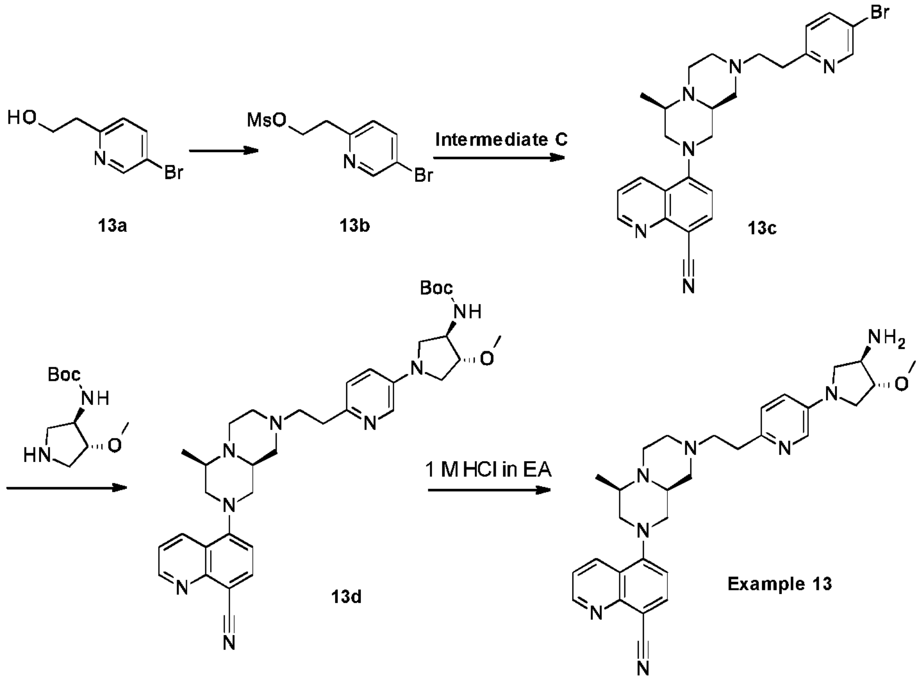


13a 13b 13c

13d Example 13

Example **13** was prepared in analogy to the preparation of Example **11** by using compound **13a** instead of compound **11b** and *tert*-butyl N-[(*3R,4R*)-4-methoxypyrrolidin-3-yl]carbamate instead of *tert*-butyl piperazine-1-carboxylate. Example **13** was obtained as an orange solid (22 mg). LCMS $(M+H)^+$: 527, $^1H$ NMR (400 MHz, METHANOL-d4) δ ppm: 9.07 (dd, *J* = 1.5, 4.3 Hz, 1H), 8.85 (dd, *J* = 1.5, 8.6 Hz, 1H), 8.27 (d, *J* = 7.9 Hz, 1H), 8.12 (d, *J* = 2.7 Hz, 1H), 7.91 - 7.86 (m, 1H), 7.80 (td, *J* = 4.1, 8.5 Hz, 2H), 7.45 (d, *J* = 8.1 Hz, 1H), 4.31 - 4.23 (m, 1H), 4.17 (br s, 1H), 4.04 (br dd, *J* = 3.7, 6.8 Hz, 2H), 3.96 (dd, *J* = 5.5, 11.1 Hz, 1H), 3.92 - 3.82 (m, 2H), 3.79 - 3.65 (m, 4H), 3.64 - 3.59 (m, 1H), 3.57 - 3.46 (m, 5H), 3.45 - 3.35 (m, 7H), 3.11 - 2.98 (m, 1H), 1.59 - 1.46 (m, 3H).

**Example 15**

**5-[(*4R*,*9aS*)-8-[[6-(1,4-diazepan-1-yl)-3-pyridyl]methyl]-4-methyl-3,4,6,7,9,9a-hexahydro-*1H*-pyrazino[1,2-a]pyrazin-2-yl]quinoline-8-carbonitrile**

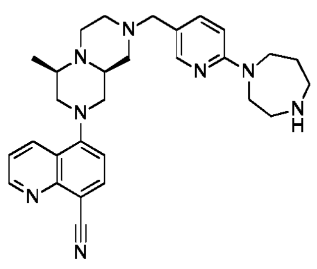

Example **15** was prepared in analogy to the preparation of Example **3** by using Intermediate **C** instead of Intermediate **A**, 2-bromo-5-(bromomethyl)pyridine instead of compound **3a** and *tert*-butyl 1,4-diazepane-1-carboxylate instead of tert-butyl piperazine-1-carboxylate. Example **15** was obtained as a light yellow solid (23 mg). LCMS $(M+H)^+$: 497, $^1H$ NMR (400 MHz, METHANOL-d4) δ ppm: 9.08 (dd, *J* = 1.5, 4.5 Hz, 1H), 8.86 (dd, *J* = 1.6, 8.6 Hz, 1H), 8.36 - 8.24 (m, 3H), 7.82 (dd, *J* = 4.5, 8.6 Hz, 1H), 7.51 (d, *J* = 9.4 Hz, 1H), 7.49 - 7.44 (m, 1H), 4.50 - 4.39 (m, 1H), 4.35 - 4.28 (m, 1H), 4.25 - 4.14 (m, 4H), 3.97 - 3.87 (m, 3H), 3.87 - 3.69 (m, 4H), 3.61 - 3.38 (m, 9H), 2.40 - 2.34 (m, 2H), 1.55 (d, *J* = 6.4 Hz, 3H).

**Example 16**

**5-[(*4R,9aR*)-4-methyl-8-(5,6,7,8-tetrahydro-2,6-naphthyridin-1-yl)-3,4,6,7,9,9a-hexahydro-*1H*-pyrazino[1,2-a]pyrazin-2-yl]quinoline-8-carbonitrile**

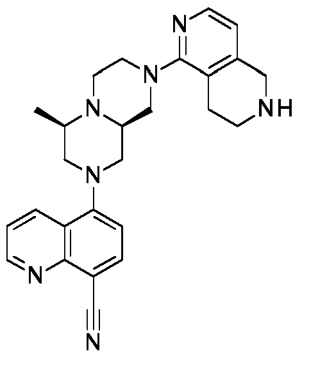

Example **16** was prepared in analogy to the preparation of Example **2** by using Intermediate **D** instead of Intermediate **A** and *tert*-butyl 5-chloro-3,4-dihydro-2,6-naphthyridine-2(1H)-carboxylate instead of compound **2a**. Example **16** was obtained as an orange solid (20 mg). LCMS $(M+H)^+$: 440, $^1H$ NMR (400 MHz, METHANOL-d4) δ ppm: 9.10 (dd, *J* = 1.6, 4.4 Hz, 1H), 8.99 (dd, *J* = 1.3, 8.6 Hz, 1H), 8.31 (d, *J* = 8.1 Hz, 1H), 8.26 (d, *J* = 5.9 Hz, 1H), 7.86 (dd, *J* = 4.5, 8.6 Hz, 1H), 7.51 (d, *J* = 8.1 Hz, 1H), 7.28 (d, *J* = 5.9 Hz, 1H), 4.57 (s, 3H), 4.09 - 3.74 (m, 7H), 3.67 - 3.47 (m, 5H), 3.28 - 3.17 (m, 3H), 1.85 (br d, *J* = 4.9 Hz, 3H).

**Example 17**

**5-[(*4R,9aS*)-8-[2-[6-[(*3R,4S*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile**

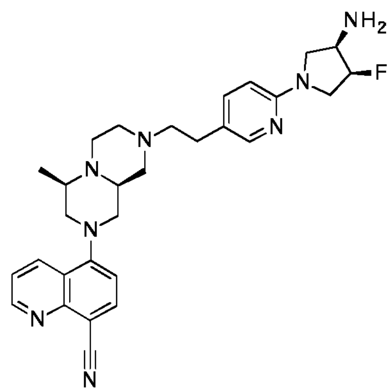

Example **17** was prepared in analogy to the preparation of Example **11** by using *tert*-butyl *N*-[(*3R,4S*)-4-fluoropyrrolidin-3-yl]carbamate instead of *tert*-butyl piperazine-1-carboxylate. Example **17** was obtained as an orange solid (8 mg). LCMS $(M+H)^+$: 515, $^1H$ NMR (400 MHz, METHANOL-d4) δ ppm: 9.28 - 9.06 (m, 2H), 8.42 (d, *J* = 8.2 Hz, 1H), 8.20 (dd, *J* = 2.0, 9.4 Hz, 1H), 8.14 (d, *J* = 1.6 Hz, 1H), 8.02 (dd, *J* = 4.9, 8.6 Hz, 1H), 7.60 (d, *J* = 8.2 Hz, 1H), 7.26 (d, *J* = 9.3 Hz, 1H), 5.73 (t, *J* = 2.8 Hz, 0.5H), 5.60 (t, *J* = 2.9 Hz, 0.5H), 4.64 - 4.49 (m, 1H), 4.44 - 4.26 (m, 3H), 4.24 - 4.03 (m, 5H), 3.97 - 3.48 (m, 10H), 3.32 - 3.25 (m, 2H), 1.61 (d, *J* = 6.5 Hz, 3H).

**Example 18**

**5-[(*4R,9aS*)-8-[2-[6-[(*3R,4R*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile**

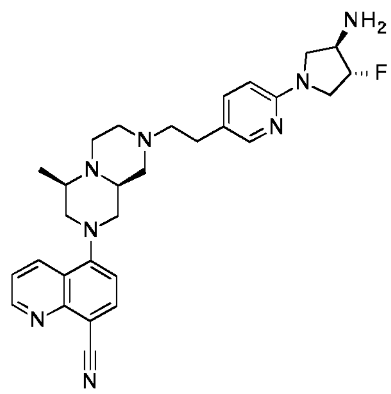

Example **18** was prepared in analogy to the preparation of Example **11** by using *tert*-butyl *N*-[(*3R*,*4R*)-4-fluoropyrrolidin-3-yl]carbamate instead of *tert*-butyl piperazine-1-carboxylate. Example **18** was obtained as an orange solid (5 mg). LCMS $(M+H)^+$: 515, $^1H$ NMR (400 MHz, METHANOL-d4) δ ppm: 8.95 (dd, *J* = 1.5, 4.3 Hz, 1H), 8.67 (dd, *J* = 1.6, 8.6 Hz, 1H), 8.14 (d, *J* = 7.9 Hz, 1H), 8.06 (dd, *J* = 2.1, 9.3 Hz, 1H), 7.98 (d, *J* = 1.6 Hz, 1H), 7.65 (dd, *J* = 4.4, 8.6 Hz, 1H), 7.32 (d, *J* = 8.1 Hz, 1H), 7.15 (d, *J* = 9.3 Hz, 1H), 5.55 (br, 0.5H), 5.42 (br, 0.5H), 4.32 - 4.08 (m, 3H), 4.07 - 3.91 (m, 3H), 3.89 - 3.74 (m, 3H), 3.71 - 3.54 (m, 3H), 3.50 - 3.25 (m, 7H), 3.14 - 3.04 (m, 2H), 1.38 (d, *J* = 6.4 Hz, 3H).

**Example 19**

**5-[(*4R,9aS*)-8-[2-[5-[(*3R,4S*)-3-amino-4-fluoro-pyrrolidin-1-yl]-2-pyridyl]ethyl]-4-methyl-3,4,6,7,9,9a-hexahydro-*1H*-pyrazino[1,2-a]pyrazin-2-yl]quinoline-8-carbonitrile**

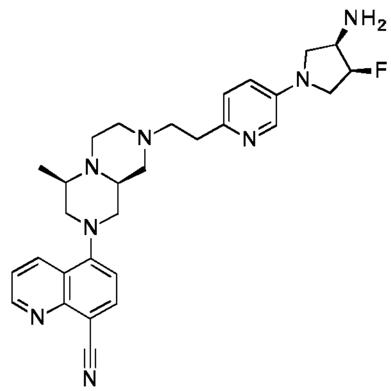

Example **19** was prepared in analogy to the preparation of Example **11** by using compound **13c** instead of compound **11d** and *tert*-butyl *N*-[(*3R*,*4S*)-4-fluoropyrrolidin-3-yl]carbamate instead of tert-butyl piperazine-1-carboxylate. Example **19** was obtained as an orange solid (48 mg). LCMS $(M+H)^+$: 515, $^1H$ NMR (400 MHz, METHANOL-d4) δ ppm: 9.11 (dd, *J* = 1.5, 4.5 Hz, 1H), 8.94 (dd, *J* = 1.5, 8.7 Hz, 1H), 8.32 (d, *J* = 7.9 Hz, 1H), 8.15 (d, *J* = 2.8 Hz, 1H), 7.95 - 7.78 (m, 3H), 7.50 (d, *J* = 8.1 Hz, 1H), 5.65 (br, 0.5H), 5.52 (br, 0.5H), 4.40 - 4.12 (m, 3H), 4.11 - 4.02 (m, 1H), 4.02 - 3.84 (m, 5H), 3.84 - 3.73 (m, 2H), 3.68 - 3.38 (m, 10H), 1.56 (d, *J* = 6.4 Hz, 3H).

**Example 20**

**5-[(*4R,9aS*)-4-methyl-8-[2-[5-[(*3S*)-3-methylpiperazin-1-yl]-2-pyridyl]ethyl]-3,4,6,7,9,9a-hexahydro-*1H*-pyrazino[1,2-a]pyrazin-2-yl]quinoline-8-carbonitrile**

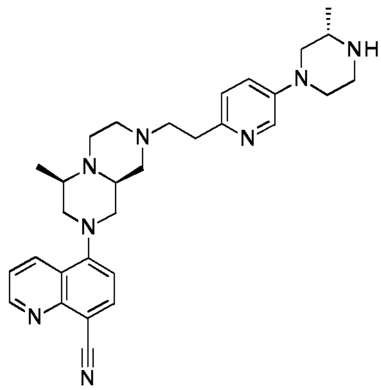

Example **20** was prepared in analogy to the preparation of Example **11** by using compound **13c** instead of compound **11d** and *tert*-butyl (*2S*)-2-methylpiperazine-1-carboxylate instead of tert-butyl piperazine-1-carboxylate. Example **20** was obtained as an orange solid (37 mg). LCMS $(M+H)^+$: 511, $^1H$ NMR (400 MHz, METHANOL-d4) $\delta$ ppm: 9.10 (d, *J* = 3.9 Hz, 1H), 8.92 (br d, *J* = 8.4 Hz, 1H), 8.50 (s, 1H), 8.31 (d, *J* = 8.1 Hz, 1H), 8.25 - 8.19 (m, 1H), 7.95 (d, *J* = 9.0 Hz, 1H), 7.84 (dd, *J* = 4.6, 8.4 Hz, 1H), 7.49 (d, *J* = 8.1 Hz, 1H), 4.37 - 4.24 (m, 1H), 4.18 - 4.06 (m, 3H), 3.95 - 3.70 (m, 5H), 3.64 - 3.35 (m, 12H), 3.29 - 3.20 (m, 1H), 3.16 - 3.06 (m, 1H), 1.55 (d, *J* = 6.2 Hz, 3H), 1.46 (d, *J* = 6.5 Hz, 3H).

**Example 21**

**5-[(*4R,9aS*)-4-methyl-8-[2-[6-[(*3S*)-3-methylpiperazin-1-yl]-2-pyridyl]ethyl]-3,4,6,7,9,9a-hexahydro-*1H*-pyrazino[1,2-a]pyrazin-2-yl]quinoline-8-carbonitrile**

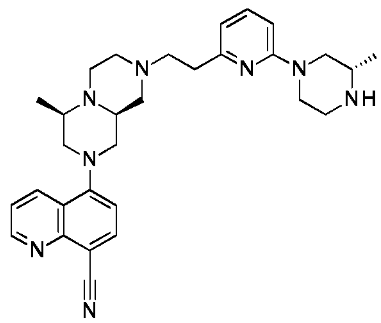

The title compound was prepared according to the following scheme:

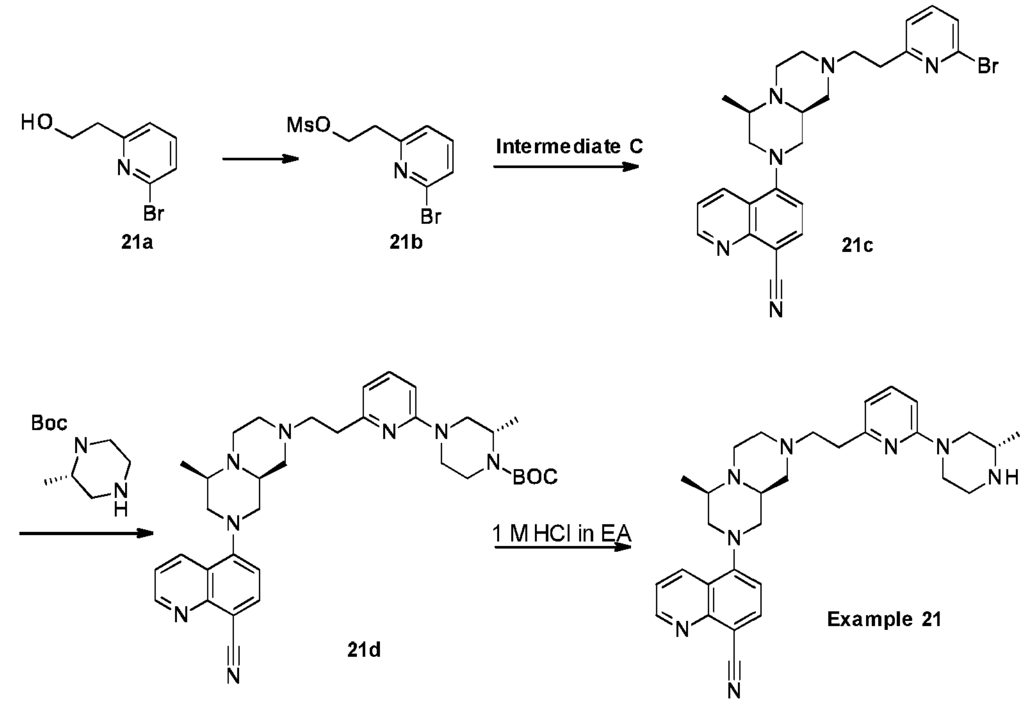


21a 21b 21c

21d Example 21

Example **21** was prepared in analogy to the preparation of Example **11** by using compound **21a** instead of compound **11b** and *tert*-butyl (*2S*)-2-methylpiperazine-1-carboxylate instead of *tert*-butyl piperazine-1-carboxylate. Example **21** was obtained as an orange solid (14 mg). LCMS $(M+H)^+$: 511, $^1H$ NMR (400 MHz, METHANOL-d4) δ ppm: 9.11 (dd, *J* = 1.5, 4.5 Hz, 1H), 8.95 (dd, *J* = 1.5, 8.7 Hz, 1H), 8.33 (d, *J* = 8.1 Hz, 1H), 8.00 (dd, *J* = 7.4, 8.9 Hz, 1H), 7.86 (dd, *J* = 4.5, 8.6 Hz, 1H), 7.51 (d, *J* = 8.1 Hz, 1H), 7.29 (d, *J* = 8.9 Hz, 1H), 7.10 (d, *J* = 7.3 Hz, 1H), 4.70 - 4.51 (m, 2H), 4.49 - 4.36 (m, 1H), 4.29 - 3.94 (m, 4H), 3.87 - 3.71 (m, 5H), 3.69 - 3.38 (m, 11H), 1.57 (d, *J* = 6.5 Hz, 3H), 1.47 (d, *J* = 6.5 Hz, 3H).

**Example 22**

**5-[(*4R*,*9aS*)-4-methyl-8-[2-[6-(6-methyl-2,6-diazaspiro[3.3]heptan-2-yl)-3-pyridyl]ethyl]-3,4,6,7,9,9a-hexahydro-*1H*-pyrazino[1,2-a]pyrazin-2-yl]quinoline-8-carbonitrile**

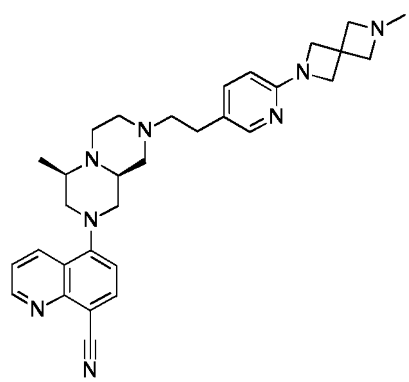

A mixture of 5-[(*4R*,*9aS*)-8-[2-(6-chloro-3-pyridyl)ethyl]-4-methyl-3,4,6,7,9,9a-hexahydro-*1H*-pyrazino[1,2-a]pyrazin-2-yl]quinoline-8-carbonitrile (compound **11d**, 50 mg, 112 μmol), 2-methyl-2,6-diazaspiro[3.3]heptane (12 mg, 112μmol ), cesium carbonate (109 mg, 336 μmol) and RuPhos Pd G2 (16 mg, 22 μmol) in dioxane (5 mL) was stirred at 110 °C for 16 hours. Then the reaction was concentrated and the residue was purified by prep-HPLC to give Example **22** as a light yellow solid (13 mg). LCMS $(M+H)^+$: 523, $^1H$ NMR (400 MHz, METHANOL-d4) δ ppm: 9.03 (dd, *J* = 1.6, 4.3 Hz, 1H), 8.66 (dd, *J* = 1.6, 8.6 Hz, 1H), 8.20 (d, *J* = 8.1 Hz, 1H), 7.96 (d, *J* = 1.8 Hz, 1H), 7.78 (dd, *J* = 2.0, 9.0 Hz,

1H), 7.68 (dd, *J* = 4.2, 8.6 Hz, 1H), 7.31 (d, *J* = 7.9 Hz, 1H), 6.69 (d, *J* = 8.9 Hz, 1H), 4.64 - 4.52 (m, 2H), 4.44 - 4.26 (m, 6H), 3.71 - 3.46 (m, 6H), 3.26 - 3.17 (m, 2H), 3.10 - 2.88 (m, 9H), 2.84 - 2.63 (m, 2H), 1.28 (br d, *J* = 6.1 Hz, 3H).

**Example 24**

**5-[(*4R,9aS*)-8-[2-[6-[(*3R,4S*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile**

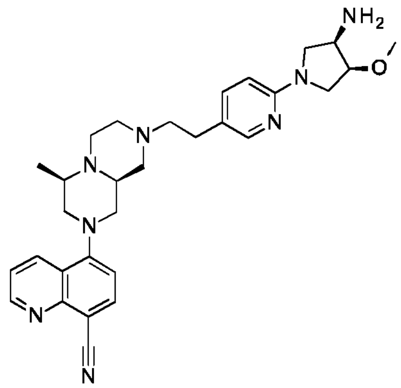

Example **24** was prepared in analogy to the preparation of Example **11** by using *tert*-butyl *N*-[(*3R,4S*)-4-methoxypyrrolidin-3-yl]carbamate instead of *tert*-butyl piperazine-1-carboxylate. Example **24** was obtained as an orange solid (17 mg). LCMS $(M+H)^+$: 527, $^1H$ NMR (400 MHz, METHANOL-d4) δ ppm: 9.07 (dd, *J* = 1.5, 4.3 Hz, 1H), 8.80 (dd, *J* = 1.5, 8.5 Hz, 1H), 8.27 (d, *J* = 7.9 Hz, 1H), 8.15 (dd, *J* = 2.1, 9.3 Hz, 1H), 8.06 (d, *J* = 1.6 Hz, 1H), 7.78 (dd, *J* = 4.3, 8.6 Hz, 1H), 7.44 (d, *J* = 8.1 Hz, 1H), 7.23 (d, *J* = 9.4 Hz, 1H), 4.41 - 4.35 (m, 1H), 4.28 - 4.07 (m, 4H), 4.02 - 3.90 (m, 3H), 3.89 - 3.67 (m, 6H), 3.65 - 3.38 (m, 9H), 3.25 - 3.14 (m, 2H), 1.52 (d, *J* = 6.4 Hz, 3H).

**Example 25**

**5-[(*4R,9aS*)-8-[2-[6-[(*3S,4S*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile**

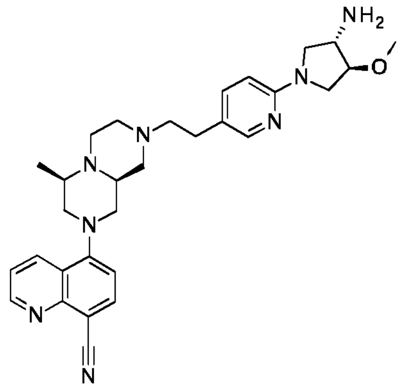

Example **25** was prepared in analogy to the preparation of Example **11** by using *tert*-butyl *N*-[(*3S,4S*)-4-methoxypyrrolidin-3-yl]carbamate instead of *tert*-butyl piperazine-1-carboxylate. Example **25** was obtained as an orange solid (23 mg). LCMS $(M+H)^+$: 527, $^1H$ NMR (400 MHz, METHANOL-d4) δ ppm: 9.09 (dd, *J* = 1.6, 4.4 Hz, 1H), 8.84 (dd, *J* = 1.6, 8.7 Hz, 1H), 8.29 (d, *J* = 8.1 Hz, 1H), 8.16 (dd, *J* = 2.1, 9.4 Hz, 1H), 8.08 (d, *J* = 1.6 Hz, 1H), 7.81 (dd, *J* = 4.5, 8.6 Hz, 1H), 7.47 (d, *J* = 7.9 Hz, 1H), 7.24 (d, *J* = 9.3 Hz, 1H), 4.40 - 4.28 (m, 2H), 4.23 - 4.07 (m, 4H), 4.02 (br d, *J* = 13.8 Hz, 2H), 3.96 - 3.81 (m, 2H), 3.80 - 3.68 (m, 4H), 3.65 - 3.50 (m, 7H), 3.48 - 3.37 (m, 2H), 3.27 - 3.18 (m, 2H), 1.55 (d,

*J* = 6.4 Hz, 3H).

**Example 26**

**5-[(*4R*,*9aS*)-8-[2-[4-[(*3R*,*4R*)-3-amino-4-methoxy-pyrrolidin-1-yl]phenyl]ethyl]-4-methyl-3,4,6,7,9,9a-hexahydro-*1H*-pyrazino[1,2-a]pyrazin-2-yl]quinoline-8-carbonitrile**

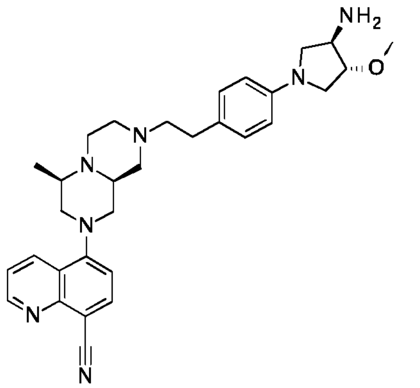

Example **26** was prepared in analogy to the preparation of Example **3** by using 5-[(*4R*,*9aS*)-8-[2-(4-bromophenyl)ethyl]-4-methyl-3,4,6,7,9,9a-hexahydro-*1H*-pyrazino[1,2-a]pyrazin-2-yl]quinoline-8-carbonitrile (compound **7b**) instead of compound **3b** and *tert*-butyl *N*-[(*3R*,*4R*)-4-methoxypyrrolidin-3-yl]carbamate instead of tert-butyl piperazine-1-carboxylate. Example **26** was obtained as a brown solid (27 mg). LCMS $(M+H)^+$: 526, $^1H$ NMR (400 MHz, METHANOL-d4) $\delta$ ppm: 9.01 (dd, *J* = 1.4, 4.6 Hz, 1H), 8.87 (dd, *J* = 1.3, 8.6 Hz, 1H), 8.23 (d, *J* = 8.1 Hz, 1H), 7.78 (dd, *J* = 4.6, 8.6 Hz, 1H), 7.41 (d, *J* = 8.2 Hz, 1H), 7.12 (d, *J* = 8.6 Hz, 2H), 6.57 (d, *J* = 8.7 Hz, 2H), 4.31 (br d, *J* = 1.3 Hz, 1H), 4.14 (br d, *J* = 13.0 Hz, 1H), 4.03 - 3.85 (m, 4H), 3.81 - 3.63 (m, 5H), 3.60 - 3.48 (m, 3H), 3.45 - 3.29 (m, 8H), 3.15 (dd, *J* = 3.5, 10.7 Hz, 1H), 3.06 - 2.93 (m, 2H), 1.47 (d, *J* = 6.4 Hz, 3H).

**Example 27**

**5-[(*4R*,*9aS*)-4-methyl-8-[(6-piperazin-1-yl-2-pyridyl)methyl]-3,4,6,7,9,9a-hexahydro-*1H*-pyrazino[1,2-a]pyrazin-2-yl]quinoline-8-carbonitrile**

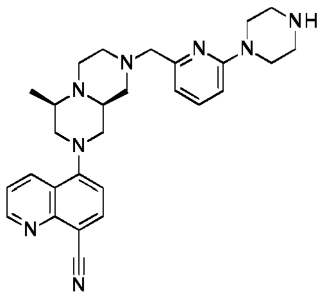

Example **27** was prepared in analogy to the preparation of Example **3** by using Intermediate **C** instead of Intermediate **A** and 2-bromo-6-(bromomethyl)pyridine instead of compound **3a**. Example **27** was obtained as a light brown solid (20 mg). LCMS $(M+H)^+$: 483, $^1H$ NMR (400 MHz, METHANOL-d4) $\delta$ ppm: 9.12 (dd, *J* = 1.5, 4.6 Hz, 1H), 8.97 (dd, *J* = 1.5, 8.6 Hz, 1H), 8.32 (d, *J* = 7.9 Hz, 1H), 7.91 - 7.77 (m, 2H), 7.50 (d, *J* = 8.1 Hz, 1H), 7.11 (d, *J* = 8.8 Hz, 1H), 7.02 (d, *J* = 7.2 Hz, 1H), 4.63 - 4.51 (m, 1H), 4.47 (s, 2H), 4.23 - 4.12 (m, 1H), 4.08 - 3.97 (m, 5H), 3.94 - 3.70 (m, 6H), 3.56 - 3.37 (m, 7H), 1.57 (d, *J* = 6.5 Hz, 3H).

**Example 28**

**5-[(*4R,9aS*)-4-methyl-8-[2-[4-(5-oxa-2,8-diazaspiro[3.5]nonan-2-yl)phenyl]ethyl]-3,4,6,7,9,9a-hexahydro-*1H*-pyrazino[1,2-a]pyrazin-2-yl]quinoline-8-carbonitrile**

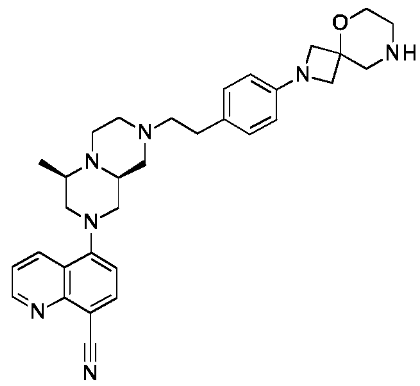

Example **28** was prepared in analogy to the preparation of Example **3** by using 5-[(*4R*,*9aS*)-8-[2-(4-bromophenyl)ethyl]-4-methyl-3,4,6,7,9,9a-hexahydro-*1H*-pyrazino[1,2-a]pyrazin-2-yl]quinoline-8-carbonitrile (compound **7b**) instead of compound **3b** and *tert*-butyl 5-oxa-2,8-diazaspiro[3.5]nonane-8-carboxylate instead of *tert*-butyl piperazine-1-carboxylate. Example **28** was obtained as a brown solid (27 mg). LCMS $(M+H)^+$: 538, $^1H$ NMR (400 MHz, METHANOL-d4) $\delta$ ppm: 8.90 (dd, *J* = 1.7, 4.2 Hz, 1H), 8.52 (dd, *J* = 1.7, 8.6 Hz, 1H), 8.06 (d, *J* = 8.1 Hz, 1H), 7.56 (dd, *J* = 4.2, 8.6 Hz, 1H), 7.17 (d, *J* = 8.1 Hz, 1H), 7.06 (d, *J* = 8.6 Hz, 2H), 6.43 (d, *J* = 8.4 Hz, 2H), 3.90 (d, *J* = 8.6 Hz, 2H), 3.86 - 3.79 (m, 2H), 3.62 (d, *J* = 7.9 Hz, 3H), 3.54 - 3.31 (m, 6H), 3.28 - 3.22 (m, 2H), 3.18 - 3.13 (m, 2H), 3.11 - 3.03 (m, 1H), 2.99 - 2.69 (m, 7H), 2.49 - 2.36 (m, 1H), 1.10 (d, *J* = 5.9 Hz, 3H).

**Example 30**

**5-[(*4R*,*9aR*)-4-methyl-8-(4-piperazin-1-ylpyrimidin-2-yl)-3,4,6,7,9,9a-hexahydro-*1H*-pyrazino[1,2-a]pyrazin-2-yl]quinoline-8-carbonitrile**

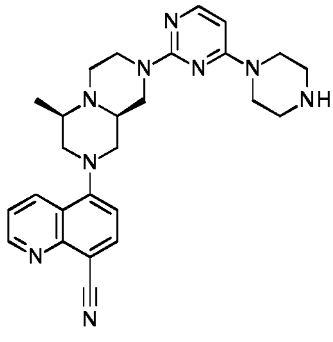

The title compound was prepared according to the following scheme:

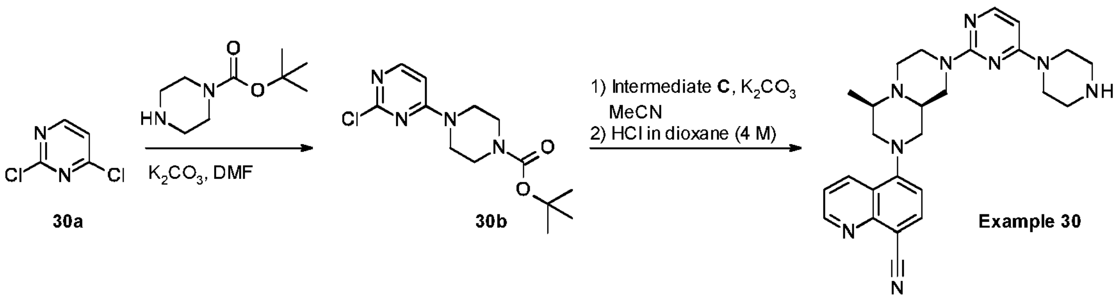


30a 30b Example 30

**Step 1: Preparation of tert-butyl 4-(2-chloropyrimidin-4-yl)piperazine-1-carboxylate (compound 30b)**

A mixture of 2,4-dichloropyrimidine (compound **30a**, 100 mg, 671 μmol), $K_2CO_3$ (185 mg, 1340 μmol) and *tert*-butyl piperazine-1-carboxylate (138 mg, 738 μmol) in DMF (3 mL) was stirred at 50 °C for 2 hours. Then the reaction mixture was diluted with EtOAc (40 mL) and washed with water. The organic layer was dried over $Na_2SO_4$ and concentrated. The residue was purified by flash column chromatography to give compound **30b** (100 mg) as a white solid. LCMS$(M+H)^+$: 299, LCMS$(M+H+2)^+$: 301.

**Step 2: Preparation of 5-[(*4R*,*9aR*)-4-methyl-8-(4-piperazin-1-ylpyrimidin-2-yl)-3,4,6,7,9,9a-hexahydro-tH-pyrazino[1,2-a]pyrazin-2-yl]quinoline-8-carbonitrile**

A mixture of *tert*-butyl 4-(2-chloropyrimidin-4-yl)piperazine-1-carboxylate (compound **30b**, 43 mg, 143 μmol), $K_2CO_3$ (36 mg, 260 μmol) and 5-[(*4R*,*9as*)-4-methyl-1,3,4,6,7,8,9,9a-octahydropyrazino[1,2-a]pyrazin-2-yl]quinoline-8-carbonitrile (Intermediate C, 40 mg, 130 μmol) in MeCN (1 mL) was stirred at 120 °C overnight. Then the reaction mixture was concentrated and the residue was purified by flash column chromatography to give the coupling product, which was dissolved in dioxane (3 mL) and treated with a solution of HCl in dioxane (4 M, 2 mL). After the reaction mixture was stirred at rt for 2 hours, it was concentrated to give Example **30** (60 mg) as a yellow solid. LCMS $(M+H)^+$: 470. $^1H$ NMR (400 MHz, METHANOL-$d_4$) δ ppm: 8.99 (dd, *J* = 1.3, 4.5 Hz, 1H), 8.85 (dd, *J* = 1.4, 8.6 Hz, 1H), 8.21 (d, *J* = 7.9 Hz, 1H), 7.85 (d, *J* = 7.5 Hz, 1H), 7.76 (dd, *J* = 4.6, 8.6 Hz, 1H), 7.41 (d, *J* = 8.1 Hz, 1H), 6.62 (d, *J* = 7.6 Hz, 1H), 4.75 - 4.62 (m, 2H), 4.26 - 4.13 (m, 2H), 4.13 - 4.04 (m, 1H), 4.00 (br d, *J* = 12.5 Hz, 1H), 3.95 (br s, 1H), 3.90 - 3.82 (m, 1H), 3.82 - 3.62 (m, 3H), 3.59 - 3.45 (m, 2H), 3.45 - 3.25 (m, 7H), 1.48 (d, *J* = 6.4 Hz, 3H).

**Example 31**

**5-[(*4R*,*9aR*)-4-methyl-8-(6-methyl-2-piperazin-1-yl-pyrimidin-4-yl)-3,4,6,7,9,9a-hexahydro-1*H*-pyrazino[1,2-a]pyrazin-2-yl]quinoline-8-carbonitrile**

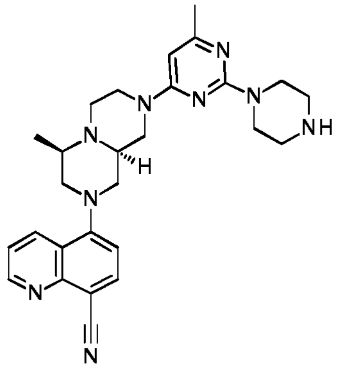

The title compound was prepared according to the following scheme:

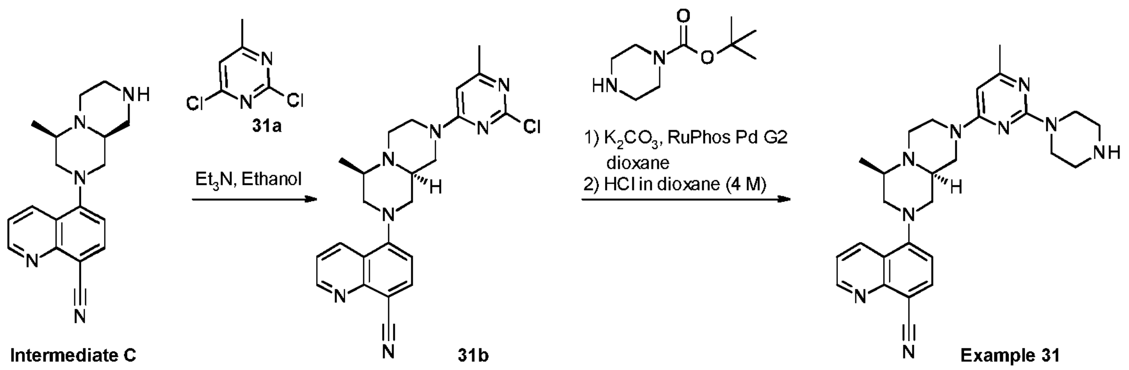


Intermediate C 31b Example 31

**Step 1: Preparation of 5-[(*4R*,*9aR*)-8-(2-chloro-6-methyl-pyrimidin-4-yl)-4-methyl-3,4,6,7,9,9a-hexahydro-1*H*-pyrazino[1,2-a]pyrazin-2-yl]quinoline-8-carbonitrile (compound 31b)**

A solution of 2,4-dichloro-6-methylpyrimidine (compound **31a**, 29 mg, 179 μmol), 5-[(*4R*,*9aS*)-4-methyl-1,3,4,6,7,8,9,9a-octahydropyrazino[1,2-a]pyrazin-2-yl]quinoline-8-carbonitrile (Intermediate **C**, 50 mg, 163 μmol) and $Et_3N$ (16.5 mg, 163 μmol) in ethanol (4 mL) was stirred at rt for 12 hours. Then the mixture was concentrated and purified by flash column chromatography to give compound **31b** (60 mg) as a yellow oil. LCMS$(M+H)^+$: 434, LCMS$(M+H+2)^+$: 436.

**Step 2: Preparation of 5-[(*4R*,*9aR*)-4-methyl-8-(6-methyl-2-piperazin-1-yl-pyrimidin-4-yl)-3,4,6,7,9,9a-hexahydro-1*H*-pyrazino[1,2-a]pyrazin-2-yl]quinoline-8-carbonitrile (Example 31)**

To a solution of 5-[(*4R*,*9aR*)-8-(2-chloro-6-methyl-pyrimidin-4-yl)-4-methyl-3,4,6,7,9,9a-hexahydro-1*H*-pyrazino[1,2-a]pyrazin-2-yl]quinoline-8-carbonitrile (compound **31b**, 60 mg, 138 μmol) in dioxane (5 mL) was added *tert*-butyl piperazine-1-carboxylate (31 mg, 166 μmol) and $K_2CO_3$ (38 mg, 277 μmol). The suspension was bubbled with $N_2$ for 5 minutes, then RuPhos Pd G2 (11 mg, 14 μmol) was added. The reaction mixture was stirred at 100 °C overnight and then concentrated. The residue was purified by prep-HPLC to give the coupling product, which was dissolved in dioxane (5 mL) and treated with a solution of HCl in dioxane (4 M, 2 mL). After the reaction mixture was stirred at rt for 2 hours, it was concentrated to give Example **31** (17 mg) as a yellow solid. LCMS $(M+H)^+$: 484. $^1$H NMR (400 MHz, METHANOL-d4) $\delta$ ppm: 9.09 (br d, *J* = 3.8 Hz, 1H), 8.90 (br d, *J* = 8.1 Hz, 1H), 8.30 (d, *J* = 7.7 Hz, 1H), 7.83 (br dd, *J* = 4.2, 8.1 Hz, 1H), 7.50 (br d, *J* = 7.7 Hz, 1H), 6.68 (s, 1H), 5.38 - 5.22 (m, 1H), 4.69 - 4.54 (m, 1H), 4.23 - 4.06 (m, 6H), 4.03 - 3.83 (m, 3H), 3.81 - 3.74 (m, 1H), 3.55 - 3.37 (m, 7H), 3.61 - 3.36 (m, 1H), 2.49 (s, 3H), 1.59 (br d, *J* = 5.9 Hz, 3H).

**Example 32**

**5-[(*4R*,*9aS*)-4-methyl-8-(2-piperazin-1-yl-4-pyridyl)-3,4,6,7,9,9a-hexahydro-1*H*-pyrazino [1,2-a] pyrazin-2-yl] quinoline-8-carbonitrile**

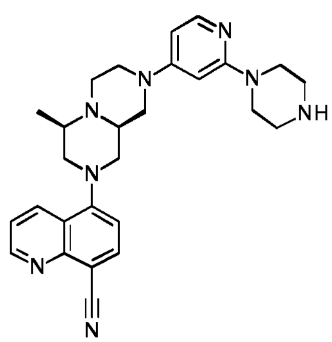

The title compound was prepared according to the following scheme:

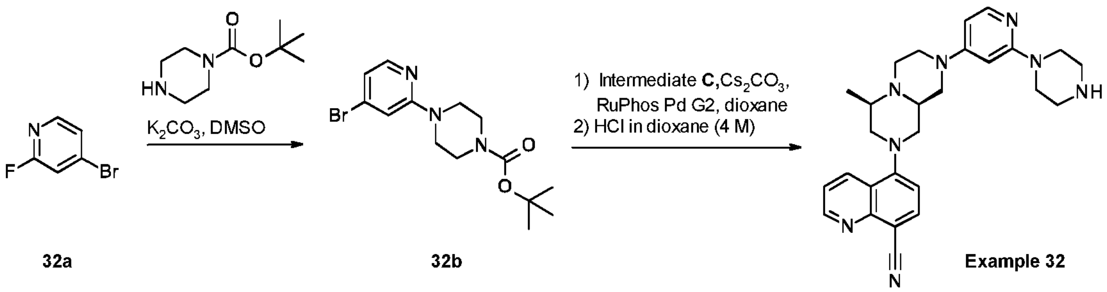


32a 32b Example 32

**Step 1: Preparation of *tert*-butyl 4-(4-bromo-2-pyridyl)piperazine-1-carboxylate (compound 32b)**

A solution of *tert*-butyl piperazine-1-carboxylate (111 mg, 597 μmol), 4-bromo-2-fluoropyridine (compound **32a**, 70 mg, 398 μmol) and $K_2CO_3$ (165 mg, 1.19 mmol) in DMSO (3 mL) was stirred at 100 °C overnight. The mixture was diluted with EtOAc (40 mL) and washed with water. The organic layer was dried over $Na_2SO_4$ and concentrated. The residue was purified by flash column chromatography to give compound **32b** (110 mg) as a white solid. LCMS(M+H)$^+$: 342, LCMS(M+H+2)$^+$: 344.

**Step 2: Preparation of 5-[(*4R*,*9aS*)-4-methyl-8-(2-piperazin-1-yl-4-pyridyl)-3,4,6,7,9,9a-hexahydro-1*H*-pyrazino[1,2-a]pyrazin-2-yl]quinoline-8-carbonitrile (compound 32)**

To a solution of *tert*-butyl 4-(4-bromo-2-pyridyl)piperazine-1-carboxylate (compound **32b**, 67 mg, 195 μmol) in dioxane (5 mL) was added 5-[(*4R*,*9aS*)-4-methyl-1,3,4,6,7,8,9,9a-octahydropyrazino[1,2-a]pyrazin-2-yl]quinoline-8-carbonitrile (Intermediate **C**, 40 mg, 130 μmol) and $Cs_2CO_3$ (127 mg, 390 μmol). The suspension was bubbled with $N_2$ for 5 minutes, then Ruphos Pd G2 (10 mg, 13 μmol) was added. After the reaction mixture was heated at 100 °C overnight, it was concentrated. The residue was purified by flash column chromatography to give the coupling product, which was dissolved in dioxane (5 mL) and treated with a solution of HCl in dioxane (4 M, 2 mL). After the yellow suspension was stirred at rt for 2 hours, it was concentrated to give Example **32** (46 mg) as a yellow solid. LCMS (M+H)$^+$: 469. $^1$H NMR (400 MHz, METHANOL-d4) $\delta$ ppm: 8.98 (dd, *J* = 1.5, 4.4 Hz, 1H), 8.80 (dd, *J* = 1.5, 8.6 Hz, 1H), 8.19 (d, *J* = 7.9 Hz, 1H), 7.76 - 7.69 (m, 2H), 7.39 (d, *J* = 8.1 Hz, 1H), 6.81 (dd, *J* = 1.9, 7.4 Hz, 1H), 6.48 (d, *J* = 1.8 Hz, 1H), 4.63 - 4.46 (m, 2H), 4.05 - 3.94 (m, 2H), 3.85 (br s, 1H), 3.82 - 3.77 (m, 4H), 3.71 - 3.60 (m, 2H), 3.58 - 3.55 (m, 1H), 3.46 - 3.26 (m, 8H), 1.47 (d, *J* = 6.5 Hz, 3H).

**Example 33**

**5-[(*4R*,*9aR*)-4-methyl-8-(2-piperazin-1-ylpyrimidin-4-yl)-3,4,6,7,9,9a-hexahydro-1*H*-pyrazino [1,2-a] pyrazin-2-yl] quinoline-8-carbonitrile**

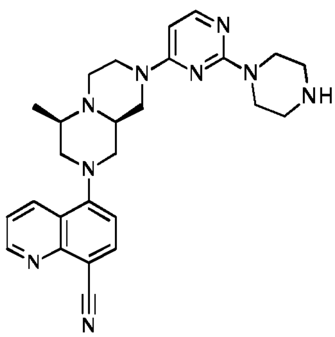

The title compound was prepared according to the following scheme:

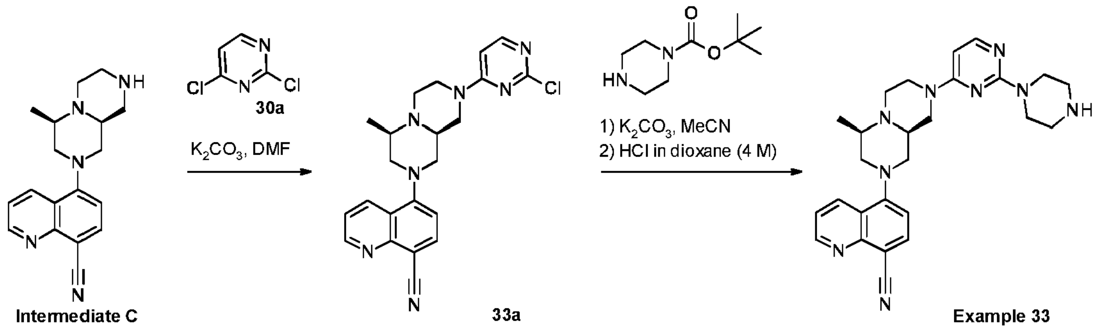


**Step 1: Preparation of 5-[(*4R,9aR*)-8-(2-chloropyrimidin-4-yl)-4-methyl-3,4,6,7,9,9a-hexahydro- 1H-pyrazino [1,2-a] pyrazin-2-yl] quinoline-8-carbonitrile (compound 33a)**

A mixture of 2,4-dichloropyrimidine (compound **30a**, 27 mg, 179 μmol), $K_2CO_3$ (45 mg, 325 μmol) and 5-[(*4R,9aS*)-4-methyl-1,3,4,6,7,8,9,9a-octahydropyrazino[1,2-a]pyrazin-2-yl]quinoline-8-carbonitrile (Intermediate **C**, 50 mg, 163 μmol) in DMF (3 mL) was stirred at 50 °C for 2 hours, then the reaction was diluted with EtOAc (40 mL) and washed with water. The organic layer was dried over $Na_2SO_4$ and concentrated to give compound **33a** (crude 70 mg) as a yellow oil. LCMS$(M+H)^+$: 420, LCMS$(M+H+2)^+$: 422.

**Step 2: Preparation of 5-[(*4R,9aR*)-4-methyl-8-(2-piperazin-1-ylpyrimidin-4-yl)-3,4,6,7,9,9a-hexahydro-1*H*-pyrazino[1,2-a]pyrazin-2-yl]quinoline-8-carbonitrile (compound 33)**

A suspension of 5-[(*4R,9aR*)-8-(2-chloropyrimidin-4-yl)-4-methyl-3,4,6,7,9,9a-hexahydro-1*H*-pyrazino[1,2-a]pyrazin-2-yl]quinoline-8-carbonitrile (compound **33a**, 68 mg, 162 μmol), $K_2CO_3$ (45 mg, 324 μmol) and *tert*-butyl piperazine-1-carboxylate (36 mg, 194 μmol) in MeCN (1 mL) was stirred at 120 °C overnight and then concentrated. The residue was purified by prep-HPLC to give the coupling product, which was dissolved in dioxane (2 mL) and treated with a solution of HCl in dioxane (4 M, 2 mL). The yellow suspension was stirred at rt for 2 hours. The reaction mixture was concentrated to give Example **33** (32 mg) as a yellow solid. LCMS $(M+H)^+$: 470. $^1H$ NMR (400 MHz, METHANOL-$d_4$) δ ppm: 8.98 (d, *J* = 3.9 Hz, 1H), 8.79 (br d, *J* = 8.3 Hz, 1H), 8.19 (d, *J* = 7.9 Hz, 1H), 7.85 (d, *J* = 7.5 Hz, 1H), 7.72 (dd, *J* = 4.5, 8.5 Hz, 1H), 7.39 (d, *J* = 7.9 Hz, 1H), 6.68 (br d, *J* = 7.5 Hz, 1H), 5.31 - 5.11 (m, 1H), 4.59 - 4.43 (m, 1H), 4.00 (br s, 6H), 3.84 (br d, *J* = 2.8 Hz, 2H), 3.70 - 3.61 (m, 2H), 3.60 - 3.52 (m, 2H), 3.33 (br s, 6H), 1.47 (br d, *J* = 6.2 Hz, 3H).

**Example 34**

**5-[(*4R,9aR*)-4-methyl-8-(4-piperazin-1-yl-2-pyridyl)-3,4,6,7,9,9a-hexahydro-1*H*-pyrazino[1,2-a]pyrazin-2-yl]quinoline-8-carbonitrile**

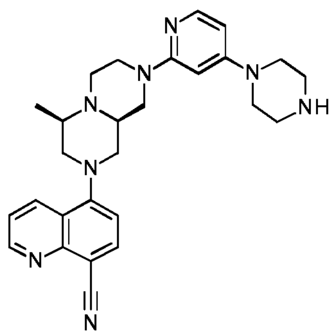

The title compound was prepared according to the following scheme:

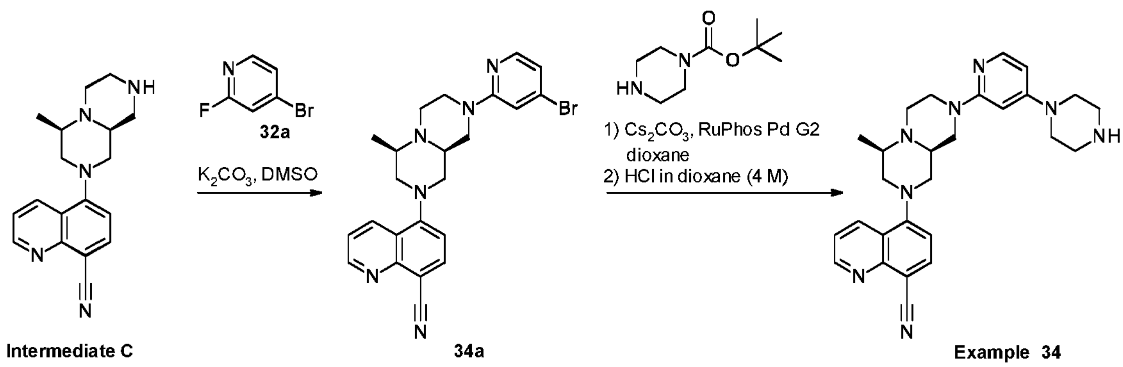


Intermediate C 34a Example 34

**Step 1: Preparation of 5-[(*4R*,*9aR*)-8-(4-bromo-2-pyridyl)-4-methyl-3,4,6,7,9,9a-hexahydro-1*H*-pyrazino[1,2-a]pyrazin-2-yl]quinoline-8-carbonitrile (compound 34a)**

To a solution of 5-[(*4R*,*9aS*)-4-methyl-1,3,4,6,7,8,9,9a-octahydropyrazino[1,2-a]pyrazin-2-yl]quinoline-8-carbonitrile (Intermediate **C**, 40 mg, 130 μmol) in DMSO (3 mL) was added 4-bromo-2-fluoropyridine (compound **32a**, 28 mg, 156 μmol) and $K_2CO_3$ (54 mg, 390 μmol). The reaction mixture was stirred at 120 °C overnight, and then the reaction was diluted with EtOAc (40 mL), washed with water, the organic layer was dried over $Na_2SO_4$ and concentrated. The residue was purified by flash column chromatography to give compound **34a** (52 mg) as a yellow oil. LCMS$(M+H)^+$: 463, LCMS$(M+H+2)^+$: 465.

**Step 2: Preparation of 5-[(*4R*,*9aR*)-4-methyl-8-(4-piperazin-1-yl-2-pyridyl)-3,4,6,7,9,9a-hexahydro-1*H*-pyrazino[1,2-a]pyrazin-2-yl]quinoline-8-carbonitrile (Example 34)**

To a solution of 5-[(4*R*,9*aR*)-8-(4-bromo-2-pyridyl)-4-methyl-3,4,6,7,9,9a-hexahydro-1*H*-pyrazino[1,2-a]pyrazin-2-yl]quinoline-8-carbonitrile (compound **34a**, 50 mg, 108 μmol) in dioxane (5 mL) was added *tert*-butyl piperazine-1-carboxylate (30 mg, 162 μmol) and $Cs_2CO_3$ (105 mg, 324 μmol). The suspension was bubbled with $N_2$ for 5 minutes, then Ruphos Pd G2 (8 mg, 10 μmol) was added. After the reaction mixture was heated at 100 °C overnight, it was filtered and the filtrate was concentrated. The residue was purified by prep-HPLC to give the coupling product, which was then dissolved in dioxane (5 mL), and treated with a solution of HCl in dioxane (4 M, 2 mL), the yellow suspension was stirred at rt for 2 hours. The reaction mixture was concentrated to give Example **34** (13 mg) as a yellow solid. LCMS $(M+H)^+$: 469. $^1H$ NMR (400 MHz, METHANOL-d4) $\delta$ ppm: 9.06 (dd, *J* = 1.7, 4.3 Hz, 1H), 8.78 (dd, *J* = 1.6, 8.6 Hz, 1H), 8.26 (d, *J* = 7.9 Hz, 1H), 7.86 (d, *J* = 7.5 Hz, 1H), 7.75 (dd, *J* = 4.3, 8.6 Hz, 1H), 7.46 (d, *J* = 8.1 Hz, 1H), 6.88 (dd, *J* = 2.5, 7.5 Hz, 1H), 6.57 (d, *J* = 2.3 Hz, 1H), 4.50 - 4.36 (m, 2H), 4.13 (br d, *J* = 12.0 Hz, 2H), 4.03 - 3.97 (m, 4H), 3.97 - 3.91 (m, 1H), 3.88 - 3.74 (m, 3H), 3.55 (br d, *J* = 13.1 Hz, 1H), 3.45 - 3.38 (m, 7H), 1.59 (d, *J* = 6.5 Hz, 3H).

**Example 35**

**5-(8-isoindolin-4-yl-3,4,6,7,9,9a-hexahydro-1*H*-pyrazino[1,2-a]pyrazin-2-yl)quinoline-8-carbonitrile**

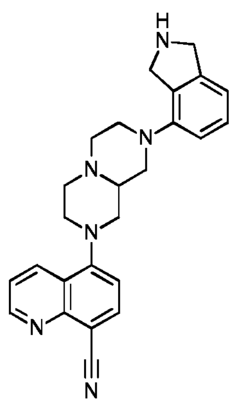

The title compound was prepared according to the following scheme:

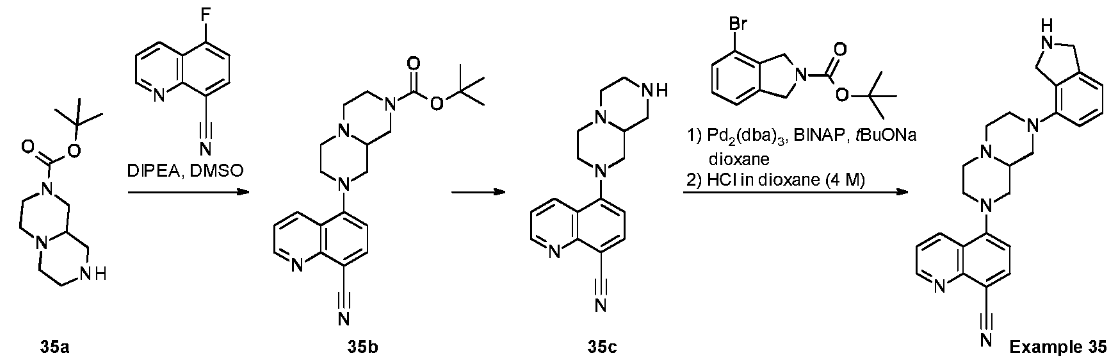


35a 35b 35c Example 35

**Step 1: Preparation of *tert*-butyl 8-(8-cyano-5-quinolyl)-3,4,6,7,9,9a-hexahydro-1*H*-pyrazino [1,2-a] pyrazine-2-carboxylate (compound 35b)**

A solution of 5-fluoroquinoline-8-carbonitrile (258 mg, 1.5 mmol), *tert*-butyl 1,3,4,6,7,8,9,9a-octahydropyrazino[1,2-a]pyrazine-2-carboxylate (compound **35a**, 241 mg, 1 mmol) and DIPEA (241 mg, 1 mmol) in DMSO (5 mL) was stirred at 120 °C overnight, and then diluted with EtOAc (40 mL), washed with water, dried over $Na_2SO_4$. The organic layer was concentrated and the residue was purified by flash column chromatography to give compound **35b** (390 mg) as a yellow oil. LCMS$(M+H)^+$:394.

**Step 2: Preparation of 5-(1,3,4,6,7,8,9,9a-octahydropyrazino[1,2-a]pyrazin-2-yl)quinoline-8-carbonitrile (compound 35c)**

To a solution of *tert*-butyl 8-(8-cyano-5-quinolyl)-3,4,6,7,9,9a-hexahydro-1*H*-pyrazino[1,2-a]pyrazine-2-carboxylate (compound **35b**, 390 mg, 1mmol) in dioxane (4 mL), was added HCl in dioxane (4 M, 2 mL) slowly. The yellow suspension was stirred at rt for 2 hours and concentrated. The residue was dissolved in MeOH (4 mL), few drops of NaOMe in MeOH was added to adjust the system to slightly basic, and then $NaHCO_3$ solid was added, the suspension was stirred at rt for 30 minutes. The suspension was filtered and concentrated to give compound **35c** (280 mg) as a yellow oil. LCMS$(M+H)^+$:294.

**Step 3: Preparation of 5-(8-isoindolin-4-yl-3,4,6,7,9,9a-hexahydro-1*H*-pyrazino[1,2-a]pyrazin-2-yl)quinoline-8-carbonitrile (compound 35)**

To a solution of 5-(1,3,4,6,7,8,9,9a-octahydropyrazino[1,2-a]pyrazin-2-yl)quinoline-8-carbonitrile (compound **35c**, 50 mg, 170 μmol) in dioxane (5 mL) was added *tert*-butyl 4-bromoisoindoline-2-carboxylate (76 mg, 256 μmol) and tBuONa (49 mg, 511 μmol). The suspension was bubbled with $N_2$ for 5 minutes, then $Pd_2(dba)_3$ (16 mg, 17 μmol) and BINAP (21 mg, 34 μmol) was added. After the reaction mixture was stirred at 100 °C overnight, it was concentrated. The residue was purified by prep-HPLC to give coupling product, which was dissolved in dioxane (5 mL), and then treated with a solution of HCl in dioxane (4 M, 2 mL), the yellow suspension was stirred at rt for 2 hours. The reaction mixture was concentrated to give Example **35** (25 mg) as a yellow solid. LCMS $(M+H)^+$: 411. $^1H$ NMR (400 MHz, METHANOL-d4) $\delta$ppm: 8.94 (dd, *J* = 1.7, 4.3 Hz, 1H), 8.65 (dd, *J* = 1.7, 8.6 Hz, 1H), 8.13 (d, *J* = 8.1 Hz, 1H), 7.62 (dd, *J* = 4.3, 8.6 Hz, 1H), 7.38 - 7.30 (m, 2H), 7.09 (dd, *J* = 7.8, 13.8 Hz, 2H), 4.60 (s, 2H), 4.54 (s, 2H), 4.04 - 3.93 (m, 1H), 3.70 - 3.62 (m, 5H), 3.54 - 3.49 (m, 1H), 3.48 - 3.43 (m, 1H), 3.41 (br d, *J* = 3.7 Hz, 1H), 3.39 - 3.32 (m, 1H), 3.31 - 3.25 (m, 1H), 3.23 (br d, *J*= 1.7 Hz, 1H), 3.07 (dd, *J* = 11.0, 13.3 Hz, 1H).

**Example 36**

**5-[(*4R*,*9aR*)-8-[2-[(*3R*,*4R*)-3-amino-4-methoxy-pyrrolidin-1-yl]pyrimidin-4-yl]-4-methyl-3,4,6,7,9,9a-hexahydro-1*H*-pyrazino[1,2-a]pyrazin-2-yl]quinoline-8-carbonitrile**

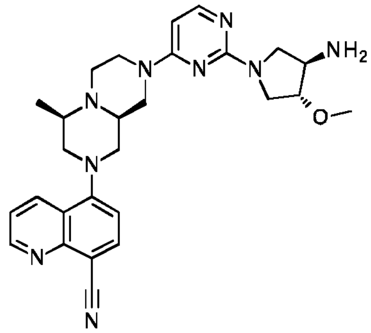

The title compound was prepared in analogy to the preparation of Example **33** by using *tert*-butyl *N*-[(*3R*,*4R*)-4-methoxypyrrolidin-3-yl]carbamate instead of *tert*-butyl piperazine-1-carboxylate. Example **36** (18 mg) was obtained as a yellow solid. LCMS $(M+H)^+$: 500. $^1H$ NMR (400 MHz, METHANOL-d4) $\delta$ ppm: 9.06 (dd, *J* = 1.6, 4.3 Hz, 1H), 8.74 (d, *J* = 8.4 Hz, 1H), 8.25 (d, *J* = 7.9 Hz, 1H), 7.94 (d, *J* = 7.6 Hz, 1H), 7.73 (dd, *J* = 4.2, 8.5 Hz, 1H), 7.45 (d, *J* = 7.9 Hz, 1H), 6.75 (d, *J* = 7.6 Hz, 1H), 4.94 (br s, 2H), 4.32 - 4.19 (m, 1H), 4.14 - 3.96 (m, 5H), 3.92 - 3.64 (m, 6H), 3.63 - 3.58 (m, 1H), 3.55 - 3.35 (m, 6H), 1.56 (d, *J* = 6.4 Hz, 3H).

**Example 37**

**5-[(*4R*,*9aR*)-8-[4-[(*3R*,*4R*)-3-amino-4-methoxy-pyrrolidin-1-yl]pyrimidin-2-yl]-4-methyl-3,4,6,7,9,9a-hexahydro-1*H*-pyrazino[1,2-a]pyrazin-2-yl]quinoline-8-carbonitrile**

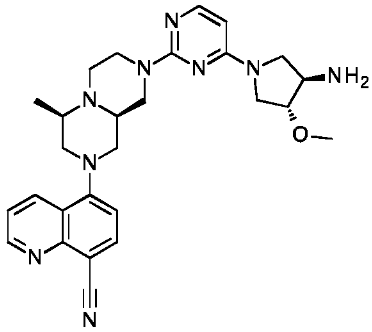

The title compound was prepared in analogy to the preparation of Example **30** by using *tert*-butyl *N*-[(*3R*,*4R*)-4-methoxypyrrolidin-3-yl]carbamate instead of *tert*-butyl piperazine-1-carboxylate. Example **37** (24 mg) was obtained as a yellow solid. LCMS $(M+H)^+$: 500. $^1H$ NMR (400 MHz, METHANOL-d4) $\delta$ ppm: 9.08 (d, *J* = 4.4 Hz, 1H), 8.90 - 8.82 (m, 1H), 8.29 (dd, *J* = 2.2, 7.9 Hz, 1H), 7.92 (d, *J* = 7.3 Hz, 1H), 7.85 - 7.76 (m, 1H), 7.50 (d, *J* = 8.1 Hz, 1H), 6.48 (dd, *J* = 3.9, 7.3 Hz, 1H), 4.86 - 4.75 (m, 2H), 4.29 - 4.05 (m, 6H), 4.05 - 3.90 (m, 2H), 3.87 - 3.75 (m, 4H), 3.72 - 3.57 (m, 2H), 3.51 - 3.49 (m, 3H), 3.48 - 3.39 (m, 2H), 1.59 (d, *J* = 6.4 Hz, 3H).

**Example 38**

**5-[2-(4-methyl-6-piperazin-1-yl-3-pyridyl)-3,4,6,7,9,9a-hexahydro-1*H*-pyrazino[1,2-a]pyrazin-8-yl]quinoline-8-carbonitrile**

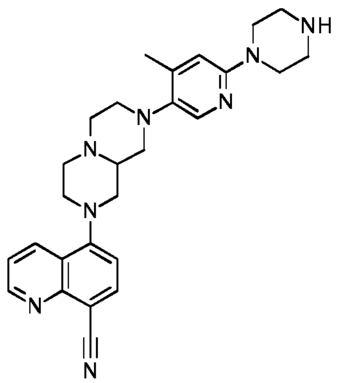

The title compound was prepared according to the following scheme:

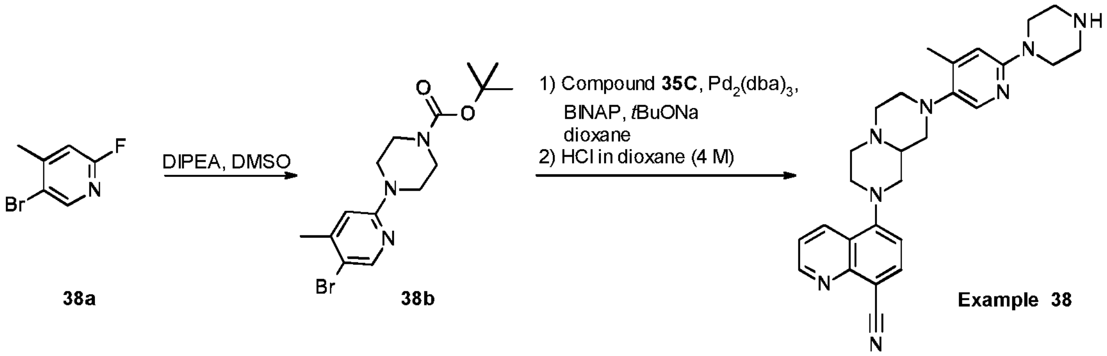


38a 38b Example 38

**Step 1: Preparation of *tert*-butyl 4-(5-bromo-4-methyl-2-pyridyl)piperazine-1-carboxylate (compound 38b)**

A solution of 5-bromo-2-fluoro-4-methyl-pyridine (compound **38a**, 190 mg, 1 mmol), *tert*-butyl piperazine-1-carboxylate (223 mg, 1.2 mmol) and DIPEA (616 mg, 5 mmol) in DMSO (5 mL) was stirred at 120 °C overnight, then diluted with EtOAc (40 mL). The organic layer was washed with water, dried over $Na_2SO_4$ and concentrated. The residue was purified by flash chromatography to give compound **38b** (282 mg) as a white solid. LCMS$(M+H)^+$: 356, LCMS$(M+H+2)^+$: 358.

**Step 2: Preparation of 5-[2-(4-methyl-6-piperazin-1-yl-3-pyridyl)-3,4,6,7,9,9a-hexahydro-1*H*-pyrazino[1,2-a]pyrazin-8-yl]quinoline-8-carbonitrile (compound 38)**

To a solution of *tert*-butyl 4-(5-bromo-4-methyl-2-pyridyl)piperazine-1-carboxylate (compound **38b**, 63 mg, 177 μmol) in dioxane (5 mL) was added 5-(1,3,4,6,7,8,9,9a-octahydropyrazino[1,2-a]pyrazin-2-yl)quinoline-8-carbonitrile (compound **35c**, 40 mg, 136 μmol) and tBuONa (26 mg, 273 μmol). The suspension was bubbled with $N_2$ for 5 minutes, then $Pd_2(dba)_3$ (13 mg, 14 μmol) and BINAP (17 mg, 27 μmol) was added. After he reaction mixture was stirred at 110 °C overnight, it was concentrated. The residue was purified by prep-HPLC to give the coupling product, which was dissolved in dioxane (5 mL), and then treated with a solution of HCl in dioxane (4 M, 2 mL), the yellow suspension was stirred at rt for 2 hours. The reaction mixture was concentrated to give Example **38** (4 mg) as a yellow solid. LCMS $(M+H)^+$: 469. $^1H$ NMR (400 MHz, METHANOL-d4) $\delta$ ppm: 9.08 (dd, *J* = 1.4, 4.3 Hz, 1H), 8.84 (dd, *J* = 1.5, 8.6 Hz, 1H), 8.28 (d, *J* = 7.9 Hz, 1H), 7.86 (s, 1H), 7.80 (dd, *J* = 4.4, 8.6 Hz, 1H), 7.50 - 7.44 (m, 2H), 4.20 - 4.10 (m, 1H), 4.03 - 3.96 (m, 4H), 3.83 - 3.74 (m, 5H), 3.82 - 3.73 (m, 1H), 3.62 (s, 3H), 3.53 - 3.45 (m, 7H), 3.30 - 3.22 (m, 1H), 2.61 (s, 3H).

**Example 39**

**5-[(*4R*,*9aR*)-8-[4-(3-aminoazetidin-1-yl)pyrimidin-2-yl]-4-methyl-3,4,6,7,9,9a-hexahydro-*1H*-pyrazino[1,2-a]pyrazin-2-yl]quinoline-8-carbonitrile**

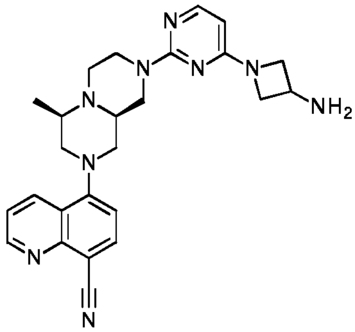

The title compound was prepared in analogy to the preparation of Example **30** by using *tert*-butyl *N*-(azetidin-3-yl)carbamate instead of *tert*-butyl piperazine-1-carboxylate. Example **39** (17 mg) was obtained as a yellow solid. LCMS $(M+H)^+$: 456. $^1H$ NMR (400 MHz, METHANOL-d4) $\delta$ ppm: 8.96 (dd, *J* = 1.4, 4.3 Hz, 1H), 8.71 (br d, *J* = 8.6 Hz, 1H), 8.16 (d, *J* = 7.9 Hz, 1H), 7.76 (d, *J* = 7.2 Hz, 1H), 7.67 (dd, *J* = 4.3, 8.4 Hz, 1H), 7.36 (d, *J* = 8.1 Hz, 1H), 6.18 (d, *J* = 7.3 Hz, 1H), 4.72 - 4.49 (m, 4H), 4.31 - 4.19 (m, 3H), 4.09 - 3.96 (m, 2H), 3.90 - 3.79 (m, 1H), 3.73 - 3.62 (m, 3H), 3.56 (s, 1H), 3.50 (s, 1H), 3.47 - 3.40 (m, 1H), 3.36 - 3.25 (m, 3H), 1.46 (d, *J* = 6.4 Hz, 3H).

**Example 40**

**5-[(*4R*,*9aS*)-8-[2-[(*3S*,*4R*)-3-amino-4-fluoro-pyrrolidin-1-yl]-4-pyridyl]-4-methyl-3,4,6,7,9,9a-hexahydro-1*H*-pyrazino[1,2-a]pyrazin-2-yl]quinoline-8-carbonitrile**

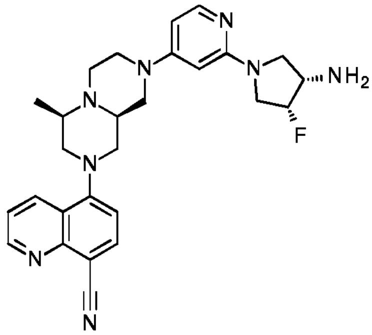

The title compound was prepared in analogy to the preparation of Example **32** by using *tert*-butyl *N*-[(*3S*,*4R*)-4-fluoropyrrolidin-3-yl]carbamate instead of *tert*-butyl piperazine-1-carboxylate. Example **40** (19 mg) was obtained as a yellow solid. LCMS $(M+H)^+$: 487. $^1H$ NMR (400 MHz, METHANOL-d4) $\delta$ ppm: 9.03 (dd, *J* = 1.6, 4.3 Hz, 1H), 8.68 (dd, *J* = 1.7, 8.6 Hz, 1H), 8.21 (d, *J* = 8.1 Hz, 1H), 7.75 - 7.62 (m, 2H), 7.36 (d, *J* = 8.1 Hz, 1H), 6.82 (dd, *J* = 2.4, 7.6 Hz, 1H), 6.07 (d, *J* = 2.3 Hz, 1H), 5.68 - 5.45 (m, 1H), 4.43 - 4.19 (m, 3H), 4.18 - 4.11 (m, 1H), 4.10 - 3.88 (m, 2H), 3.78 - 3.57 (m, 4H), 3.48 - 3.37 (m, 1H), 3.32 - 3.22 (m, 2H), 3.20 - 3.01 (m, 3H), 2.82 - 2.69 (m, 1H), 1.37 (d, *J* = 6.4 Hz, 3H).

**Example 41**

**5-[(*4R*,*9aS*)-8-[2-(3-aminoazetidin-1-yl)-4-pyridyl]-4-methyl-3,4,6,7,9,9a-hexahydro-1*H*-pyrazino[1,2-a]pyrazin-2-yl]quinoline-8-carbonitrile**

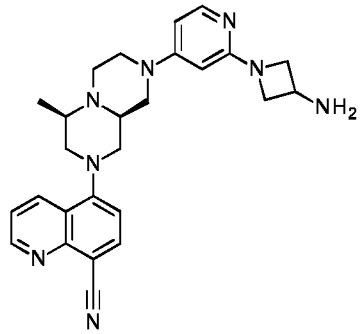

The title compound was prepared in analogy to the preparation of Example **32** by using *tert*-butyl *N*-(azetidin-3-yl)carbamate instead of *tert*-butyl piperazine-1-carboxylate. Example **41** (6 mg) was obtained as a yellow solid. LCMS $(M+H)^+$: 455. $^1H$ NMR (400 MHz, METHANOL-d4) $\delta$ ppm: 8.94 (dd, *J* = 1.6, 4.4 Hz, 1H), 8.72 - 8.64 (m, 1H), 8.15 (d, *J* = 7.9 Hz, 1H), 7.67 - 7.59 (m, 2H), 7.35 (d, *J* = 8.1 Hz, 1H), 6.69 (dd, *J* = 2.6, 7.3 Hz, 1H), 5.99 (d, *J* = 2.2 Hz, 1H), 4.57 - 4.39 (m, 4H), 4.27 - 4.19 (m, 2H), 4.01 - 3.93 (m, 2H), 3.89 (br d, *J* = 3.3 Hz, 1H), 3.85 - 3.73 (m, 2H), 3.70 - 3.53 (m, 4H), 3.50 (s, 1H), 3.36 - 3.24 (m, 3H), 1.46 (d, *J* = 6.4 Hz, 3H).

## Example 42

**5-[(*4R*,*9aS*)-4-methyl-8-(5,6,7,8-tetrahydro-2,7-naphthyridin-4-yl)-3,4,6,7,9,9a-hexahydro-1*H*-pyrazino[1,2-a]pyrazin-2-yl]quinoline-8-carbonitrile**

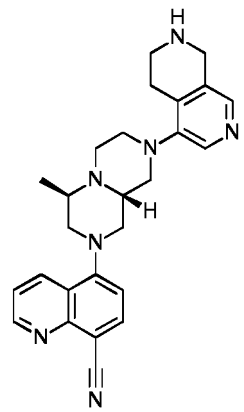

The title compound was prepared according to the following scheme:

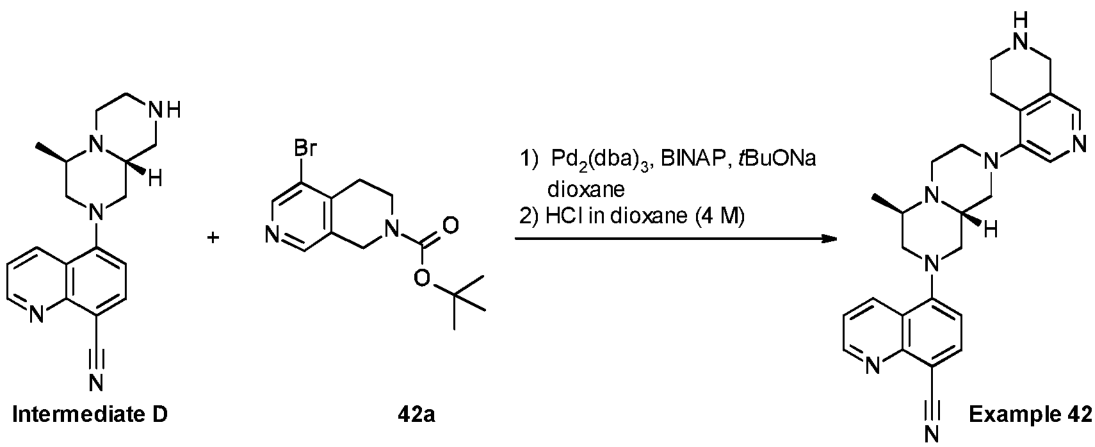


**Intermediate D** **42a** **Example 42**

To a solution of 5-[(4*R*,9a*R*)-4-methyl-1,3,4,6,7,8,9,9a-octahydropyrazino[1,2-a]pyrazin-2-yl]quinoline-8-carbonitrile (Intermediate **D**, 50 mg, 163 μmol) in dioxane (5 mL) was added *tert*-butyl 5-bromo-3,4-dihydro-1*H*-2,7-naphthyridine-2-carboxylate (compound **42a**, 61 mg, 195 μmol, CAS: 1251012-16-4, vendor: Bepharm) and *t*-BuONa (31 mg, 325 μmol). The suspension was bubbled with $N_2$ for 5 minutes, then $Pd_2(dba)_3$ (15 mg, 16 μmol) and BINAP (20 mg, 33 μmol) was added. After being stirred at 110 °C overnight, the reaction mixture was concentrated. The residue was purified by prep-HPLC to give the coupling product, which was dissolved in dioxane (5 mL), and then treated with a solution of HCl in dioxane (4 M, 2 mL), the yellow suspension was stirred at rt for 2 hours.. The reaction mixture was concentrated to give Example **42** (4 mg) as a yellow solid. LCMS $(M+H)^+$: 440. $^1H$ NMR (400 MHz, METHANOL-d4) $\delta$ ppm: 8.95 (d, *J* = 4.2 Hz, 1H), 8.78 - 8.72 (m, 1H), 8.59 - 8.52 (m, 2H), 8.14 (d, *J* = 7.9 Hz, 1H), 7.66 (dd, *J* = 4.3, 8.6 Hz, 1H), 7.35 (d, *J* = 7.9 Hz, 1H), 4.53 (s, 2H), 3.95 - 3.88 (m, 1H), 3.75 - 3.62 (m, 5H), 3.57 - 3.48 (m, 8H), 3.41 - 3.31 (m, 3H), 1.76 (br d, *J* = 6.6 Hz, 3H).

**Example 43**

**5-[(*4R*,*9aR*)-4-methyl-8-(5-methyl-4-piperazin-1-yl-pyrimidin-2-yl)-3,4,6,7,9,9a-hexahydro-1*H*-pyrazino[1,2-a]pyrazin-2-yl]quinoline-8-carbonitrile**

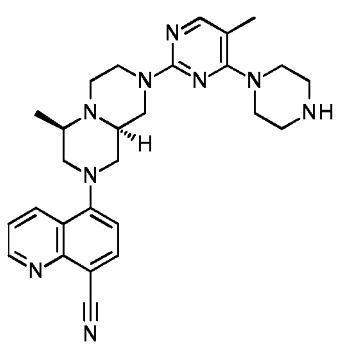

The title compound was prepared according to the following scheme:

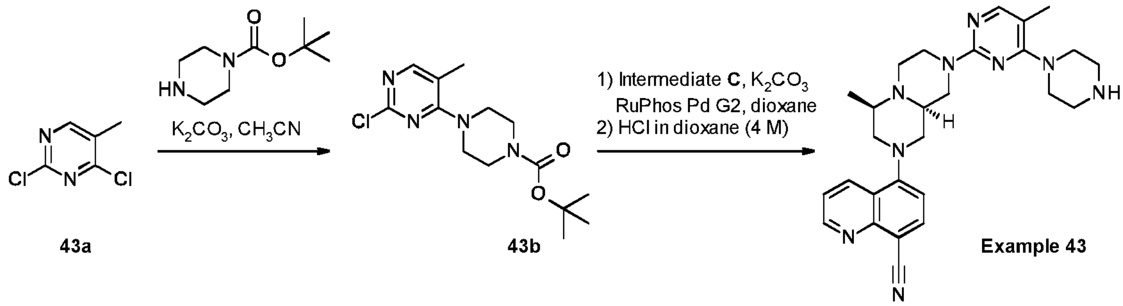

**43a** **43b** **Example 43**

**Step 1: Preparation of *tert*-butyl 4-(2-chloro-5-methyl-pyrimidin-4-yl)piperazine-1-carboxylate (compound 43b)**

To a solution of 2,4-dichloro-5-methyl-pyrimidine (compound **43a**, 53 mg, 322 μmol) in $CH_3CN$ (3 mL) was added *tert*-butyl piperazine-1-carboxylate (50 mg, 268 μmol) and $K_2CO_3$ (74 mg, 537 μmol). The reaction mixture was stirred at rt overnight, then concentrated. The residue was purified by flash column chromatography to give compound **43b** (70 mg) as a white solid. LCMS$(M+H)^+$: 313, LCMS$(M+H+2)^+$: 315.

**Step 2: Preparation of 5-[(*4R*,*9aR*)-4-methyl-8-(5-methyl-4-piperazin-1-yl-pyrimidin-2-yl)-3,4,6,7,9,9a-hexahydro-1*H*-pyrazino[1,2-a]pyrazin-2-yl]quinoline-8-carbonitrile (Example 43)**

To a solution of *tert*-butyl 4-(2-chloro-5-methyl-pyrimidin-4-yl)piperazine-1-carboxylate (compound **43b**, 56 mg, 179 μmol) in dioxane (5 mL) was added 5-[(4*R*,9a*S*)-4-methyl-1,3,4,6,7,8,9,9a-octahydropyrazino[1,2-a]pyrazin-2-yl]quinoline-8-carbonitrile (Intermediate **C**, 50 mg, 163 μmol) and $K_2CO_3$ (45 mg, 325 μmol). The suspension was bubbled with $N_2$ for 5 minutes, then Ruphos Pd G2 (13 mg, 16 μmol) was added. The reaction mixture was stirred at 100 °C

overnight, the solid was filtered off and the filtrate was concentrated. The residue was purified by prep-HPLC (Boc group was removed during the separation by using TFA system) to give Example **43** (20 mg) as a yellow solid. LCMS $(M+H)^+$: 484. $^1H$ NMR (400 MHz, METHANOL-d4) $\delta$ ppm: 8.91 (dd, *J* = 1.6, 4.3 Hz, 1H), 8.57 (dd, *J* = 1.5, 8.6 Hz, 1H), 8.09 (d, *J* = 7.9 Hz, 1H), 7.80 (s, 1H), 7.58 (dd, *J* = 4.3, 8.6 Hz, 1H), 7.28 (d, *J* = 8.1 Hz, 1H), 4.72 - 4.53 (m, 2H), 3.85 (br d, *J* = 12.2 Hz, 1H), 3.82 - 3.75 (m, 4H), 3.72 - 3.54 (m, 4H), 3.44 - 3.31 (m, 1H), 3.30 - 3.23 (m, 4H), 3.20 - 2.94 (m, 4H), 2.14 (s, 3H), 1.37 (d, *J* = 6.4 Hz, 3H).

**Example 44**

**5-[(*4R*,*9aR*)-4-methyl-8-(5,6,7,8-tetrahydro-2,7-naphthyridin-4-yl)-3,4,6,7,9,9a-hexahydro-1*H*-pyrazino[1,2-a]pyrazin-2-yl]quinoline-8-carbonitrile**

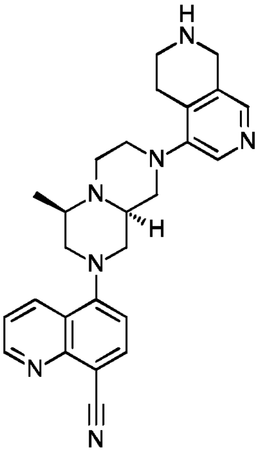

The title compound was prepared in analogy to the preparation of Example **42** by using 5-[(*4R*,*9aS*)-4-methyl-1,3,4,6,7,8,9,9a-octahydropyrazino[1,2-a]pyrazin-2-yl]quinoline-8-carbonitrile (Intermediate **C**) instead of 5-[(*4R*,*9aR*)-4-methyl-1,3,4,6,7,8,9,9a-octahydropyrazino[1,2-a]pyrazin-2-yl]quinoline-8-carbonitrile (Intermediate **D**). Example **44** (25 mg) was obtained as a yellow solid. LCMS $(M+H)^+$: 440. $^1H$ NMR (400 MHz, METHANOL-d4) $\delta$ ppm: 8.95 (dd, *J* = 1.6, 4.4 Hz, 1H), 8.72 (dd, *J* = 1.6, 8.7 Hz, 1H), 8.58 (s, 1H), 8.55 (s, 1H), 8.15 (d, *J* = 7.9 Hz, 1H), 7.66 (dd, *J* = 4.3, 8.6 Hz, 1H), 7.35 (d, *J* = 8.1 Hz, 1H), 4.53 (s, 2H), 4.16 (br t, *J* = 10.9 Hz, 1H), 3.98 (br d, *J* = 11.4 Hz, 1H), 3.94 - 3.86 (m, 1H), 3.72 - 3.63 (m, 2H), 3.60 - 3.40 (m, 7H), 3.39 - 3.32 (m, 4H), 3.32 - 3.25 (m, 1H), 1.48 (d, *J*= 6.5 Hz, 3H).

**Example 45**

**5-[(*4R*,*9aR*)-8-[2-[(*3R*,*4R*)-3-amino-4-methoxy-pyrrolidin-1-yl]-6-methyl-pyrimidin-4-yl]-4-methyl-3,4,6,7,9,9a-hexahydro-1*H*-pyrazino[1,2-a]pyrazin-2-yl]quinoline-8-carbonitrile**

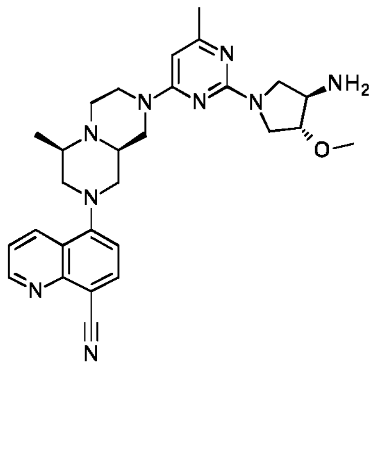

The title compound was prepared in analogy to the preparation of Example **31** by using *tert*-butyl *N*-[(*3R*,*4R*)-4-methoxypyrrolidin-3-yl]carbamate instead of *tert*-butyl piperazine-1-carboxylate. Example **45** (30 mg) was obtained as a yellow solid. LCMS $(M+H)^+$: 514. $^1$H NMR (400 MHz, METHANOL-d4) $\delta$ ppm: 8.94 (dd, *J* = 1.5, 4.3 Hz, 1H), 8.62 (dd, *J* = 1.4, 8.6 Hz, 1H), 8.13 (d, *J* = 7.9 Hz, 1H), 7.62 (dd, *J* = 4.3, 8.6 Hz, 1H), 7.34 (d, *J* = 7.9 Hz, 1H), 6.51 (s, 1H), 5.35 - 5.05 (m, 1H), 4.61 - 4.41 (m, 1H), 4.20 - 4.09 (m, 1H), 4.06 - 3.59 (m, 12H), 3.38 (td, *J* = 1.6, 3.2 Hz, 4H), 3.26 (br s, 2H), 2.36 (s, 3H), 1.45 (br d, *J* = 6.4 Hz, 3H).

**Example 46**

**5-[2-[6-[(*6R*)-6-amino-1,4-oxazepan-4-yl]-4-methyl-3-pyridyl]-3,4,6,7,9,9a-hexahydro-1*H*-pyrazino[1,2-a]pyrazin-8-yl]quinoline-8-carbonitrile**

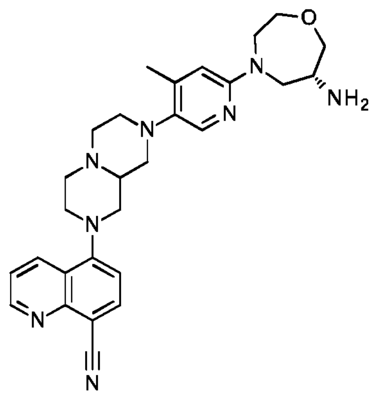

The title compound was prepared in analogy to the preparation of Example **38** by using *tert*-butyl *N*-[(*6R*)-1,4-oxazepan-6-yl]carbamate (catalog NO: PB97931, vendor: PharmaBlock) instead of *tert*-butyl piperazine-1-carboxylate. Example **46** (2 mg) was obtained as a yellow solid. LCMS $(M+H)^+$: 499. $^1$H NMR (400 MHz, METHANOL-d4) $\delta$ ppm: 8.96 (d, *J* = 3.4 Hz, 1H), 8.71 (d, *J* = 7.7 Hz, 1H), 8.16 (d, *J* = 7.9 Hz, 1H), 7.70 - 7.64 (m, 2H), 7.37 (s, 1H), 7.34 (d, *J* = 8.1 Hz, 1H), 4.25 - 4.16 (m, 2H), 4.12 - 3.88 (m, 6H), 3.85 - 3.72 (m, 4H), 3.70 - 3.62 (m, 5H), 3.50 (s, 2H), 3.40 - 3.25 (m, 4H), 3.17 - 3.08 (m, 1H), 2.50 (s, 3H).

**Example 47**

**5-[*trans*-4-methyl-8-(1,2,3,4-tetrahydroisoquinolin-5-yl)-3,4,6,7,9,9a-hexahydro-1*H*-pyrazino[1,2-a]pyrazin-2-yl]quinoline-8-carbonitrile**

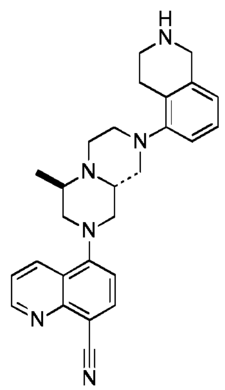

The title compound was prepared in analogy to the preparation of Example **2** by using Intermediate **B** instead of Intermediate **A**. Example **47** (3.9 mg) was obtained as a yellow solid. LCMS $(M+H)^+$: 439. $^1$H NMR (400 MHz,

METHANOL-d4) δ ppm: 9.04 (dd, *J* = 1.5, 4.3 Hz, 1H), 8.78 (br d, *J* = 8.2 Hz, 1H), 8.23 (d, *J* = 7.9 Hz, 1H), 7.73 (dd, *J* = 4.3, 8.7 Hz, 1H), 7.42 (br d, *J* = 7.7 Hz, 1H), 7.39 - 7.33 (m, 1H), 7.25 (br s, 1H), 7.09 (d, *J* = 7.8 Hz, 1H), 4.39 (s, 3H), 4.02 (br s, 1H), 3.89 - 3.34 (m, 9H), 3.31 - 2.94 (m, 5H), 1.87 (br s, 3H).

**Example 53**

**5-[(*4R*,*9aS*)-8-[2-[(*6S*)-6-amino-1,4-oxazepan-4-yl]-4-pyridyl]-4-methyl-3,4,6,7,9,9a-hexahydro-1*H*-pyrazino[1,2-a]pyrazin-2-yl]quinoline-8-carbonitrile**

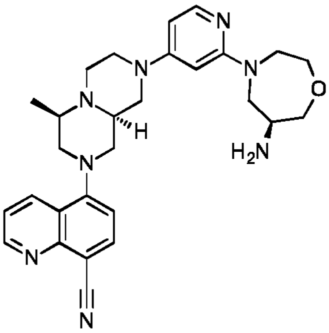

The title compound was prepared in analogy to the preparation of Example **32** by using *tert*-butyl *N*-[(*6S*)-1,4-oxazepan-6-yl]carbamate (catalog NO: PB97932, vendor: PharmaBlock) instead of *tert*-butyl piperazine-1-carboxylate. Example **53** (10 mg) was obtained as a yellow solid. LCMS $(M+H)^+$: 499. $^1H$ NMR (400 MHz, METHANOL-d4) δ ppm: 8.95 (dd, *J* = 1.5, 4.3 Hz, 1H), 8.69 (s, 1H), 8.15 (d, *J* = 7.9 Hz, 1H), 7.68 - 7.63 (m, 2H), 7.36 (d, *J* = 7.9 Hz, 1H), 6.75 (dd, *J* = 2.1, 7.6 Hz, 1H), 6.44 (d, *J* = 1.8 Hz, 1H), 4.72 - 4.63 (m, 1H), 4.62 - 4.53 (m, 1H), 4.22 - 4.14 (m, 1H), 4.06 - 3.94 (m, 4H), 3.94 - 3.85 (m, 3H), 3.83 - 3.63 (m, 6H), 3.63 - 3.56 (m, 1H), 3.41 - 3.24 (m, 4H), 1.47 (d, *J* = 6.4 Hz, 3H).

**Example 54**

**5-[(*4R*,*9aS*)-8-[2-[(*6R*)-6-amino-1,4-oxazepan-4-yl]-4-pyridyl]-4-methyl-3,4,6,7,9,9a-hexahydro-1*H*-pyrazino[1,2-a]pyrazin-2-yl]quinoline-8-carbonitrile**

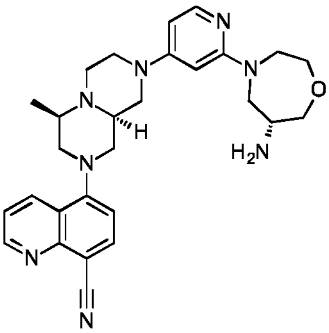

The title compound was prepared in analogy to the preparation of Example **32** by using *tert*-butyl *N*-[(*6R*)-1,4-oxazepan-6-yl]carbamate (catalog NO: PB97931, vendor: PharmaBlock) instead of *tert*-butyl piperazine-1-carboxylate. Example **54** (10 mg) was obtained as a yellow solid. LCMS $(M+H)^+$: 499. $^1H$ NMR (400 MHz, METHANOL-d4) δ ppm: 8.95 (dd, *J*= 1.5, 4.3 Hz, 1H), 8.74 (dd, *J* = 1.5, 8.6 Hz, 1H), 8.16 (d, *J* = 7.9 Hz, 1H), 7.71 - 7.60 (m, 2H), 7.36 (d, *J* = 8.1 Hz, 1H), 6.74 (dd, *J* = 2.0, 7.6 Hz, 1H), 6.48 (d, *J* = 1.3 Hz, 1H), 4.74 (br d, *J* = 1.7 Hz, 1H), 4.53 (br d, *J* = 14.5 Hz, 1H), 4.21 - 4.13 (m, 1H), 4.07 - 3.91 (m, 5H), 3.90 - 3.87 (m, 1H), 3.87 - 3.80 (m, 2H), 3.79 - 3.73 (m, 2H), 3.72 (s, 1H), 3.66 (br dd, *J* = 4.0, 13.5 Hz, 2H), 3.62 - 3.56 (m, 1H), 3.41 - 3.24 (m, 4H), 1.47 (d, *J* = 6.4 Hz, 3H).

**Example 55**

**5-[(*4R,9aR*)-8-[2-(3-amino-3-methyl-azetidin-1-yl)-6-methyl-pyrimidin-4-yl]-4-methyl-3,4,6,7,9,9a-hexahydro-1*H*-pyrazino[1,2-a]pyrazin-2-yl]quinoline-8-carbonitrile**

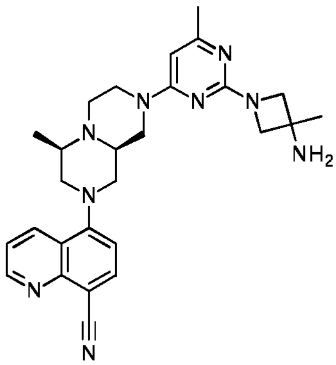

The title compound was prepared according to the following scheme:

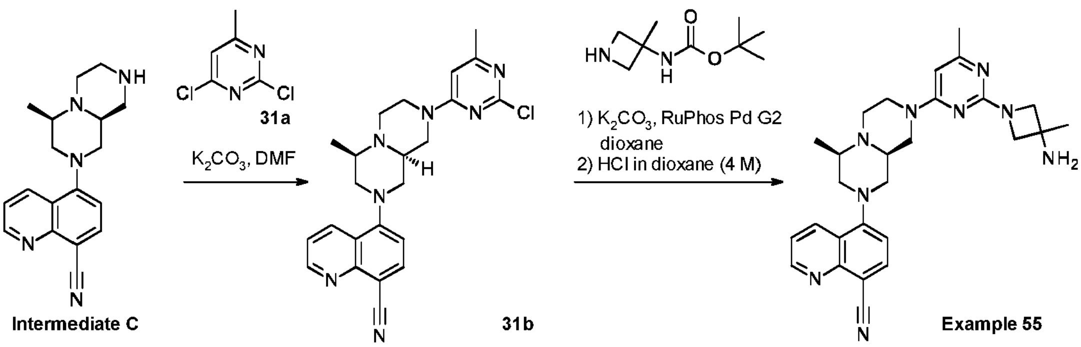


Intermediate C

31b

Example 55

**Step 1: Preparation of 5-[(*4R*,*9aR*)-8-(2-chloro-6-methyl-pyrimidin-4-yl)-4-methyl-3,4,6,7,9,9a-hexahydro-1*H*-pyrazino[1,2-a]pyrazin-2-yl]quinoline-8-carbonitrile (compound 31b)**

A solution of 2,4-dichloro-6-methylpyrimidine (compound **31a**, 117 mg, 716 μmol), 5-[(*4R*,*9aS*)-4-methyl-1,3,4,6,7,8,9,9a-octahydropyrazino[1,2-a]pyrazin-2-yl]quinoline-8-carbonitrile (Intermediate **C**, 200 mg, 651 μmol) and $K_2CO_3$ (269 mg, 1.95 mmol) in DMF (3 mL) was stirred at 50 °C for 2 hours, then the reaction was diluted with EtOAc (40 mL), washed with water. The organic layer was dried and concentrated, the residue was purified by flash column chromatography to give compound **31b** (170 mg) as a yellow oil. LCMS(M+H)$^+$: 434, LCMS(M+H+2)$^+$: 436.

**Step 2: Preparation of 5-[(*4R*,*9aR*)-8-[2-(3-amino-3-methyl-azetidin-1-yl)-6-methyl-pyrimidin-4-yl]-4-methyl-3,4,6,7,9,9a-hexahydro-1*H*-pyrazino[1,2-a]pyrazin-2-yl]quinoline-8-carbonitrile (Example 55)**

To a solution of 5-[(*4R*,*9aR*)-8-(2-chloro-6-methyl-pyrimidin-4-yl)-4-methyl-3,4,6,7,9,9a-hexahydro-1*H*-pyrazino[1,2-a]pyrazin-2-yl]quinoline-8-carbonitrile (compound **31b**, 50 mg, 115 μmol) in dioxane (5 mL) was added *tert*-butyl *N*-(3-methylazetidin-3-yl)carbamate (26 mg, 138 μmol) and $K_2CO_3$ (32 mg, 230 μmol). The suspension was bubbled with $N_2$ for 5 minutes, then Ruphos Pd G2 (9 mg, 12 μmol) was added. After being stirred at 110 °C overnight, the reaction mixture was concentrated. The residue was purified by prep-HPLC to give the coupling product, which was dissolved in dioxane (5 mL), and then treated with a solution of HCl in dioxane (4 M, 2 mL), the yellow suspension was stirred at rt for 2 hours. The reaction mixture was concentrated to give Example **55** (10 mg) as a yellow solid. LCMS (M+H)$^+$: 484. $^1$H NMR (400 MHz, METHANOL-d4) $\delta$ ppm: 8.95 (dd, *J* = 1.5, 4.3 Hz, 1H), 8.69 (dd, *J* = 1.6, 8.7 Hz, 1H), 8.16 (d, *J* = 7.9 Hz, 1H), 7.66 (dd, *J* = 4.4, 8.6 Hz, 1H), 7.36 (d, *J* = 8.1 Hz, 1H), 6.50 (s, 1H), 4.52 - 4.41 (m, 1H), 4.40 - 4.19 (m, 4H), 3.97 (br d, *J* = 11.5 Hz, 2H), 3.90 - 3.60 (m, 4H), 3.57 - 3.44 (m, 2H), 3.41 - 3.25 (m, 3H), 2.32 (br s, 3H), 1.63 (br s, 3H), 1.46 (br d, *J* = 6.2 Hz, 3H).

**Example 56**

**5-[(*4R,9aS*)-8-[2-(3-amino-3-methyl-azetidin-1-yl)-4-pyridyl]-4-methyl-3,4,6,7,9,9a-hexahydro-1*H*-pyrazino[1,2-a]pyrazin-2-yl]quinoline-8-carbonitrile**

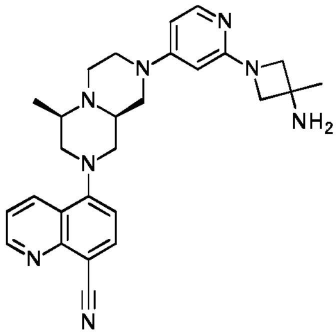

The title compound was prepared in analogy to the preparation of Example **32** by using *tert*-butyl *N*-(3-methylazetidin-3-yl)carbamate instead of *tert*-butyl piperazine-1-carboxylate and TFA/DCM (1:2) instead of 1 M HCl in EA. Example **56** (6 mg) was obtained as a yellow solid. LCMS $(M+H)^+$: 469. $^1H$ NMR (400 MHz, METHANOL-d4) $\delta$ ppm: 8.90 (td, *J* = 1.5, 4.2 Hz, 1H), 8.57 (dd, *J* = 1.5, 8.6 Hz, 1H), 8.09 (dd, *J* = 1.0, 7.9 Hz, 1H), 7.65 - 7.54 (m, 2H), 7.26 (d, *J* = 7.9 Hz, 1H), 6.67 (dd, *J* = 2.4, 7.6 Hz, 1H), 5.88 (d, *J* = 2.1 Hz, 1H), 4.38 - 4.23 (m, 4H), 4.20 - 4.11 (m, 2H), 3.81 (br d, *J* = 12.3 Hz, 1H), 3.68 - 3.34 (m, 5H), 3.19 - 3.01 (m, 3H), 3.01 - 2.86 (m, 1H), 1.62 (s, 3H), 1.40 - 1.30 (m, 3H).

**Example 57**

**5-[(*4R,9aS*)-4-methyl-8-[(5-piperazin-1-yl-3-pyridyl)methyl]-3,4,6,7,9,9a-hexahydro-*1H*-pyrazino[1,2-a]pyrazin-2-yl]quinoline-8-carbonitrile**

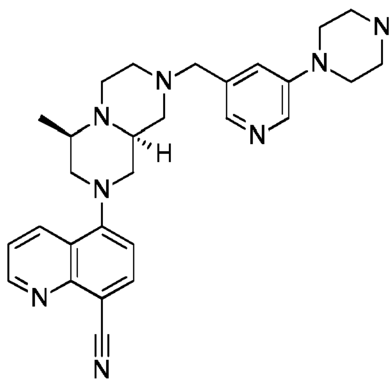

The title compound was prepared in analogy to the preparation of Example **3** by using Intermediate **C** instead of Intermediate **A** and 3-bromo-5-(bromomethyl)pyridine instead of compound **3a.** Example **57** was obtained as a yellow foam (20 mg). LCMS $(M+H)^+$: 483, $^1H$ NMR (400 MHz, METHANOL-$d_4$) $\delta$ ppm: 9.15 - 9.06 (m, 1H), 8.99 - 8.88 (m, 1H), 8.57 (d, *J*= 2.4 Hz, 1H), 8.46 (br s, 1H), 8.42 (s, 1H), 8.31 (d, *J*= 8.1 Hz, 1H), 7.93 - 7.80 (m, 1H), 7.48 (d, *J* = 8.1 Hz, 1H), 4.31 - 4.21 (m, 2H), 3.91 - 3.82 (m, 4H), 3.80 - 3.53 (m, 11H), 3.51 - 3.43 (m, 5H), 1.55 (d, *J* = 6.5 Hz, 3H).

**Example 58**

**5-[(*4R,9aS*)-4-methyl-8-[2-[6-[(*3R*)-3-methylpiperazin-1-yl]-2-pyridyl]ethyl]-3,4,6,7,9,9a-hexahydro-*1H*-pyrazino[1,2-a]pyrazin-2-yl]quinoline-8-carbonitrile**

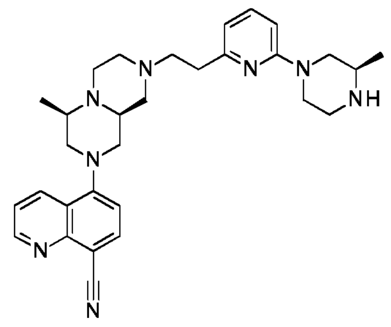

The title compound was prepared in analogy to the preparation of Example **11** by using compound **21c** instead of compound **11d** and *tert*-butyl (*2R*)-2-methylpiperazine-1-carboxylate instead of *tert*-butyl piperazine-1-carboxylate. Example **58** was obtained as an orange solid (11 mg). LCMS $(M+H)^+$: 511, $^1H$ NMR (400 MHz, METHANOL-d4) δ ppm: 9.08 (dd, *J*= 1.5, 4.4 Hz, 1H), 8.88 - 8.78 (m, 1H), 8.27 (d, *J* = 7.9 Hz, 1H), 7.88 (br t, *J* = 7.1 Hz, 1H), 7.79 (dd, *J*= 4.3, 8.7 Hz, 1H), 7.45 (d, *J* = 7.9 Hz, 1H), 7.14 (br d, *J*= 8.7 Hz, 1H), 6.99 (d, *J* = 7.0 Hz, 1H), 4.66 - 4.47 (m, 2H), 4.24 - 4.08 (m, 2H), 4.03 - 3.78 (m, 3H), 3.78 - 3.63 (m, 5H), 3.62 - 3.48 (m, 4H), 3.47 - 3.37 (m, 6H), 3.26 - 3.17 (m, 1H), 1.52 (d, *J* = 6.1 Hz, 3H), 1.44 (d, *J* = 6.6 Hz, 3H).

**Example 59**

**5-[(*4R,9aS*)-4-methyl-8-[2-[6-(5-oxa-2,8-diazaspiro[3.5]nonan-2-yl)-3-pyridyl]ethyl]-3,4,6,7,9,9a-hexahydro-*1H*-pyrazino[1,2-a]pyrazin-2-yl]quinoline-8-carbonitrile**

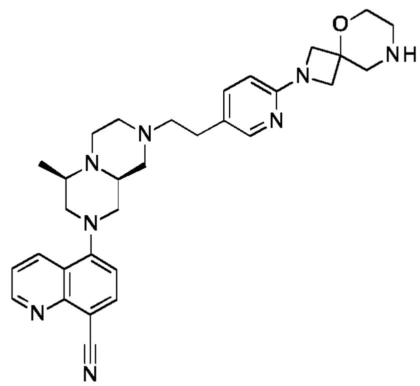

The title compound was prepared in analogy to the preparation of Example **3** by using 5-[(*4R*,*9aS*)-8-[2-(6-chloro-3-pyridyl)ethyl]-4-methyl-3,4,6,7,9,9a-hexahydro-1H-pyrazino[1,2-a]pyrazin-2-yl]quinoline-8-carbonitrile (compound **11d**) instead of compound **3b,** *tert*-butyl 5-oxa-2,8-diazaspiro[3.5]nonane-8-carboxylate instead of *tert*-butyl piperazine-1-carboxylate and TFA/DCM (1:2) instead of 1 M HCl in EA. Example **59** was obtained as a light yellow solid (36 mg). LCMS $(M+H)^+$: 539, $^1H$ NMR (400 MHz, METHANOL-d4) δ ppm: 8.99 (dd, *J* = 1.6, 4.3 Hz, 1H), 8.64 (dd, *J* = 1.7, 8.6 Hz, 1H), 8.16 (d, *J* = 8.1 Hz, 1H), 7.92 (d, *J* = 2.0 Hz, 1H), 7.66 (dd, *J* = 4.2, 8.6 Hz, 1H), 7.51 (dd, *J* = 2.2, 8.6 Hz, 1H), 7.25 (d, *J* = 8.1 Hz, 1H), 6.46 (d, *J* = 8.7 Hz, 1H), 3.96 (d, *J* = 8.9 Hz, 2H), 3.81 (d, *J*= 8.7 Hz, 2H), 3.74 - 3.65 (m, 2H), 3.48 - 3.37 (m, 3H), 3.14 - 3.07 (m, 1H), 3.01 (s, 2H), 2.97 (br d, *J* = 11.0 Hz, 1H), 2.90 - 2.70 (m, 8H), 2.66 - 2.54 (m, 2H), 2.33 (br t, *J*= 6.6 Hz, 2H), 2.04 (br t, *J*= 10.5 Hz, 1H), 1.19 (d, *J*= 5.9 Hz, 3H).

**Example 60**

**5-[(*4R,9aS*)-4-methyl-8-[(2-methyl-6-piperazin-1-yl-3-pyridyl)methyl]-3,4,6,7,9,9a-hexahydro-*1H*-pyrazino[1,2-a]pyrazin-2-yl]quinoline-8-carbonitrile**

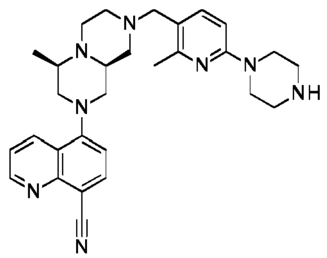

The title compound was prepared according to the following scheme:

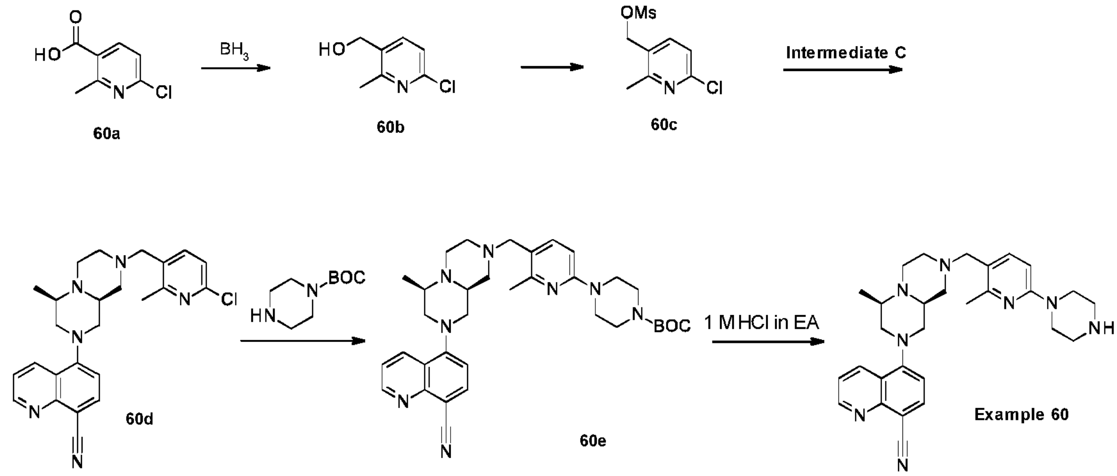


60a 60b 60c

60d 60e Example 60

The title compound was prepared in analogy to the preparation of Example **11** by using compound **60a** instead of compound **11a.** Example **60** was obtained as a yellow solid (47 mg). LCMS $(M+H)^+$: 497, $^1H$ NMR (400 MHz, METHANOL-d4) δ ppm: 8.99 (dd, *J*= 1.6, 4.6 Hz, 1H), 8.83 (dd, *J* = 1.6, 8.7 Hz, 1H), 8.27 - 8.13 (m, 2H), 7.75 (dd, *J* = 4.6, 8.6 Hz, 1H), 7.39 (d, *J* = 8.1 Hz, 1H), 7.26 (d, *J* = 9.4 Hz, 1H), 4.37 - 4.15 (m, 3H), 4.12 - 4.03 (m, 1H), 4.02 - 3.93 (m, 4H), 3.89 - 3.78 (m, 1H), 3.73 - 3.61 (m, 4H), 3.54 - 3.44 (m, 2H), 3.43 - 3.32 (m, 7H), 2.76 - 2.65 (m, 3H), 1.45 (d, *J*= 6.4 Hz, 3H).

**Example 61**

**5-[(*4R*,*9aS*)-4-methyl-8-(1,2,3,4-tetrahydroisoquinolin-7-ylmethyl)-3,4,6,7,9,9a-hexahydro-*1H*-pyrazino[1,2-a]pyrazin-2-yl]-2-deuterio-quinoline-8-carbonitrile**

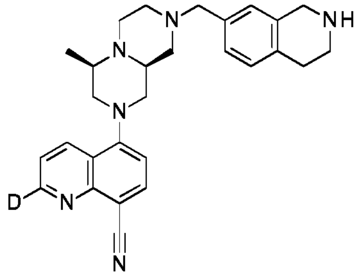

The title compound was prepared in analogy to the preparation of Example **1** by using Intermediate **G** instead of Intermediate **A.** Example **61** was obtained as an orange solid (165 mg). LCMS $(M+H)^+$: 454, $^1H$ NMR (400 MHz, METHANOL-$d_4$) $\delta$ ppm: 8.89 (d, *J*= 8.7 Hz, 1H), 8.31 (d, *J* = 8.1 Hz, 1H), 7.83 (d, *J* = 8.6 Hz, 1H), 7.61 - 7.55 (m, 2H), 7.48 (d, *J* = 8.1 Hz, 1H), 7.42 (d, *J*= 7.9 Hz, 1H), 4.68 - 4.49 (m, 2H), 4.45 (s, 2H), 4.43 - 4.34 (m, 1H), 4.22 (br d, *J*= 13.1 Hz, 1H), 3.95 (br d, *J* = 2.6 Hz, 1H), 3.91 - 3.58 (m, 7H), 3.54 (t, *J* = 6.3 Hz, 2H), 3.51 - 3.41 (m, 2H), 3.18 (t, *J* = 6.4 Hz, 2H), 1.56 (d, *J*= 6.4 Hz, 3H).

**Example 62**

**5-[(*4R,9aS*)-8-[2-[6-[(*3R,4R*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]-2-deuterio-quinoline-8-carbonitrile**

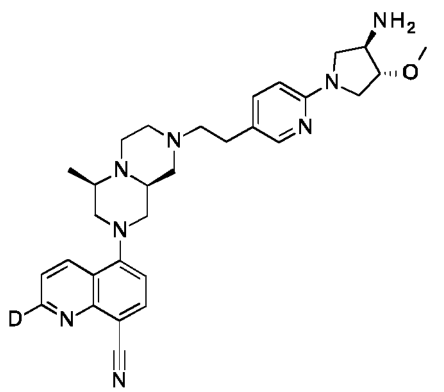

The title compound was prepared according to the following scheme:

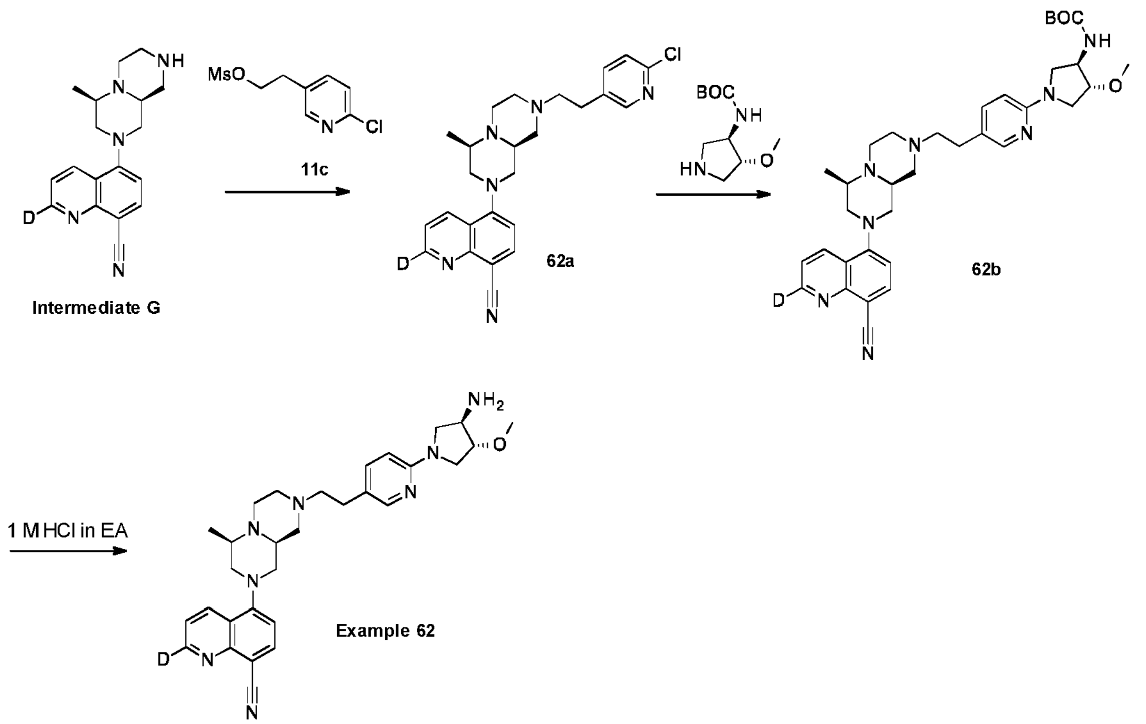


Intermediate G

62a

62b

Example 62

The title compound was prepared in analogy to the preparation of Example **11** by using *tert*-butyl *N*-[(*3R*,*4R*)-4-methoxypyrrolidin-3-yl]carbamate instead of *tert*-butyl piperazine-1-carboxylate and Intermediate **G** instead of Intermediate **C.** Example **62** was obtained as an orange solid (86 mg). LCMS $(M+H)^+$: 528, $^1H$ NMR (400 MHz, METHANOL-$d_4$) $\delta$ ppm: 8.93 (d, *J* = 8.7 Hz, 1H), 8.32 (d, *J* = 7.9 Hz, 1H), 8.16 (dd, *J* = 2.0, 9.4 Hz, 1H), 8.11 - 8.07 (m, 1H), 7.86 (d,

*J* = 8.6 Hz, 1H), 7.51 (d, *J* = 8.1 Hz, 1H), 7.24 (d, *J* = 9.4 Hz, 1H), 4.45 (br t, *J*= 11.1 Hz, 1H), 4.38 - 4.31 (m, 1H), 4.26 (br d, *J* = 12.7 Hz, 1H), 4.19 - 4.05 (m, 5H), 4.03 - 3.95 (m, 1H), 3.90 - 3.59 (m, 9H), 3.56 - 3.44 (m, 5H), 3.25 (t, *J* = 7.9 Hz, 2H), 1.58 (d, *J*= 6.5 Hz, 3H).

**Example 63**

**5-[(*4R,9aS*)-8-[2-[6-[(*3R,4S*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]-2-deuterio-quinoline-8-carbonitrile**

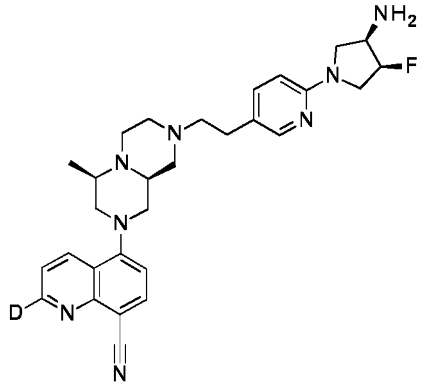

The title compound was prepared in analogy to the preparation of Example **11** by using tert-butyl *N*-[(*3R*,*4S*)-4-fluoropyrrolidin-3-yl]carbamate instead of *tert*-butyl piperazine-1-carboxylate and Intermediate **G** instead of Intermediate **C.** Example **63** was obtained as an orange solid (60 mg). LCMS $(M+H)^+$: 516, $^1H$ NMR (400 MHz, METHANOL-d4) δ ppm: 9.03 (d, *J* = 8.7 Hz, 1H), 8.37 (d, *J*= 8.1 Hz, 1H), 8.19 (dd, *J*= 2.1, 9.4 Hz, 1H), 8.12 (d, *J*= 1.6 Hz, 1H), 7.93 (d, *J* = 8.6 Hz, 1H), 7.55 (d, *J* = 8.1 Hz, 1H), 7.26 (d, *J* = 9.4 Hz, 1H), 5.71 (t, *J* = 2.9 Hz, 0.5H), 5.58 (t, *J* = 3.1 Hz, 0.5H), 4.50 (br t, *J* = 11.2 Hz, 1H), 4.41 - 4.23 (m, 3H), 4.22 - 3.98 (m, 5H), 3.96 - 3.41 (m, 10H), 3.27 (t, *J* = 8.0 Hz, 2H), 1.59 (d, *J*= 6.4 Hz, 3H).

**Example 64**

**5-[(*4R,9aS*)-4-methyl-8-[2-[6-(6-methyl-2,6-diazaspiro[3.3]heptan-2-yl)-3-pyridyl]ethyl]-3,4,6,7,9,9a-hexahydro-*1H*-pyrazino[1,2-a]pyrazin-2-yl]-2-deuterio-quinoline-8-carbonitrile**

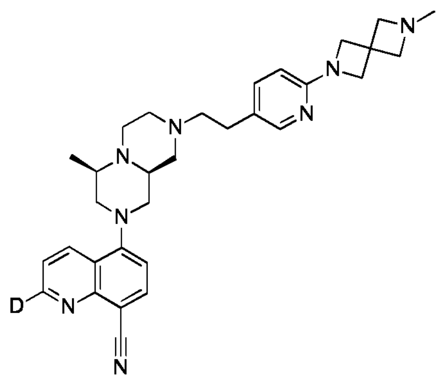

The title compound was prepared in analogy to the preparation of Example **22** by using compound **62a** instead of Intermediate **11d.** Example **64** was obtained as a light yellow solid (70 mg). LCMS $(M+H)^+$: 524, $^1H$ NMR (400 MHz, METHANOL-d4) δ ppm: 8.52 (d, *J* = 8.7 Hz, 1H), 8.04 (d, *J* = 8.1 Hz, 1H), 7.79 (d, *J* = 2.0 Hz, 1H), 7.54 (d, *J* = 8.6 Hz, 1H), 7.37 (dd, *J* = 2.3, 8.6 Hz, 1H), 7.13 (d, *J* = 8.1 Hz, 1H), 6.29 (d, *J* = 8.4 Hz, 1H), 3.94 (s, 4H), 3.36 (s, 4H), 3.34 - 3.25 (m, 2H), 3.19 - 3.10 (m, 1H), 3.04 - 2.93 (m, 1H), 2.85 (br d, *J* = 11.0 Hz, 1H), 2.75 - 2.57 (m, 6H), 2.52 - 2.43 (m,

2H), 2.31 - 2.16 (m, 5H), 1.91 (br t, *J* = 10.5 Hz, 1H), 1.07 (d, *J* = 6.0 Hz, 3H).

**Example 65**

**5-[(*4R*,*9aS*)-8-[2-[6-(3-amino-3-methyl-azetidin-1-yl)-3-pyridyl]ethyl]-4-methyl-3,4,6,7,9,9a-hexahydro-*1H*-pyrazino[1,2-a]pyrazin-2-yl]quinoline-8-carbonitrile**

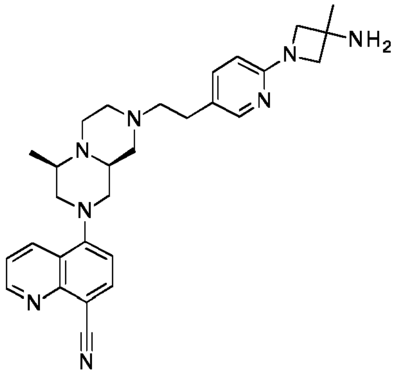

The title compound was prepared in analogy to the preparation of Example **11** by using *tert*-butyl *N*-(3-methylazetidin-3-yl)carbamate instead of *tert*-butyl piperazine-1-carboxylate and TFA/DCM (1:2) instead of 1 M HCl in EA. Example **65** was obtained as a light yellow solid (40 mg). LCMS $(M+H)^+$: 497, $^1H$ NMR (400 MHz, METHANOL-d4) δ ppm: 8.99 (dd, *J* = 1.6, 4.3 Hz, 1H), 8.64 (dd, *J* = 1.7, 8.6 Hz, 1H), 8.16 (d, *J* = 8.1 Hz, 1H), 7.91 (d, *J* = 1.8 Hz, 1H), 7.66 (dd, *J* = 4.3, 8.6 Hz, 1H), 7.50 (dd, *J* = 2.3, 8.6 Hz, 1H), 7.25 (d, *J* = 8.1 Hz, 1H), 6.44 (d, *J* = 8.6 Hz, 1H), 3.93 - 3.81 (m, 4H), 3.49 - 3.37 (m, 2H), 3.29 (br d, *J* = 2.7 Hz, 1H), 3.16 - 3.07 (m, 1H), 2.97 (br d, *J* = 11.0 Hz, 1H), 2.88 - 2.68 (m, 6H), 2.65 - 2.54 (m, 2H), 2.41 - 2.27 (m, 2H), 2.04 (t, *J* = 10.5 Hz, 1H), 1.54 (s, 3H), 1.18 (d, *J* = 6.0 Hz, 3H).

**Example 66**

**4-[(*4R*,*9aS*)-8-[2-[4-[(*3R*,*4R*)-3-amino-4-methoxy-pyrrolidin-1-yl]phenyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile**

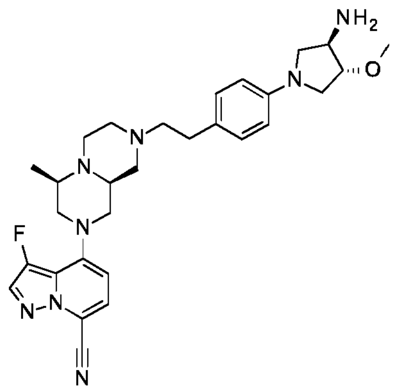

The title compound was prepared according to the following scheme:

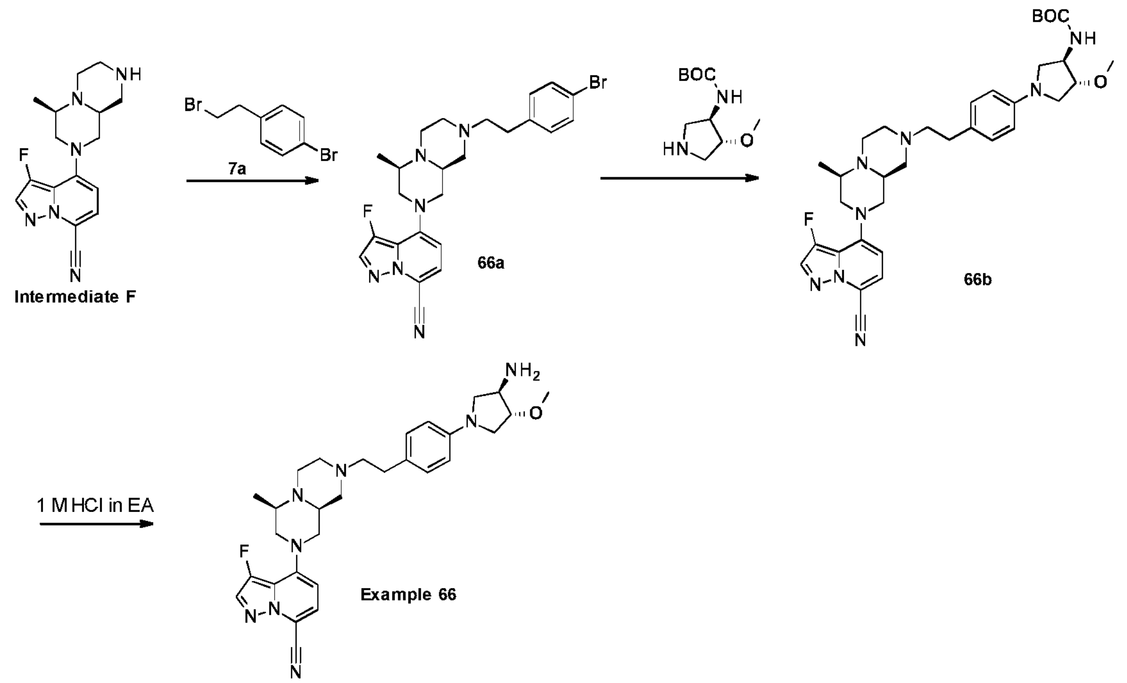


The title compound was prepared in analogy to the preparation of Example **11** by using *tert*-butyl *N*-[(*3R*,*4R*)-4-methoxypyrrolidin-3-yl]carbamate instead of *tert*-butyl piperazine-1-carboxylate, compound **7a** instead of compound **11c** and Intermediate **F** instead of Intermediate **C.** Example **66** was obtained as a yellow solid (55 mg). LCMS $(M+H)^+$: 533, $^1H$ NMR (400 MHz, METHANOL-d4) δ ppm: 8.08 (d, *J* = 3.5 Hz, 1H), 7.50 (d, *J* = 7.9 Hz, 1H), 7.23 (d, *J* = 8.7 Hz, 2H), 6.68 (dd, *J* = 3.9, 8.3 Hz, 3H), 4.25 - 4.10 (m, 2H), 3.99 (br d, *J* = 11.6 Hz, 3H), 3.94 - 3.84 (m, 4H), 3.78 - 3.54 (m, 5H), 3.52 - 3.38 (m, 8H), 3.27 (dd, *J* = 3.5, 10.8 Hz, 1H), 3.17 - 3.04 (m, 2H), 1.53 (d, *J* = 6.4 Hz, 3H).

### Example 67

**4-[(*4R*,*9aS*)-8-[2-[6-[(*3R*,*4R*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]-3-fluoro-pyrazolo[1,5-a] pyridine-7-carbonitrile**

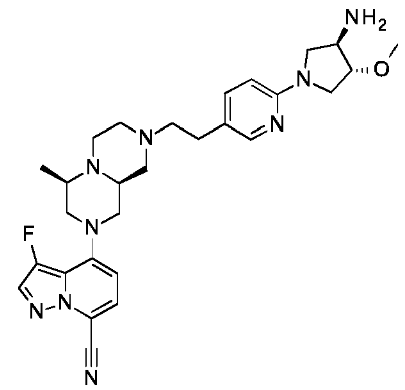

The title compound was prepared according to the following scheme:

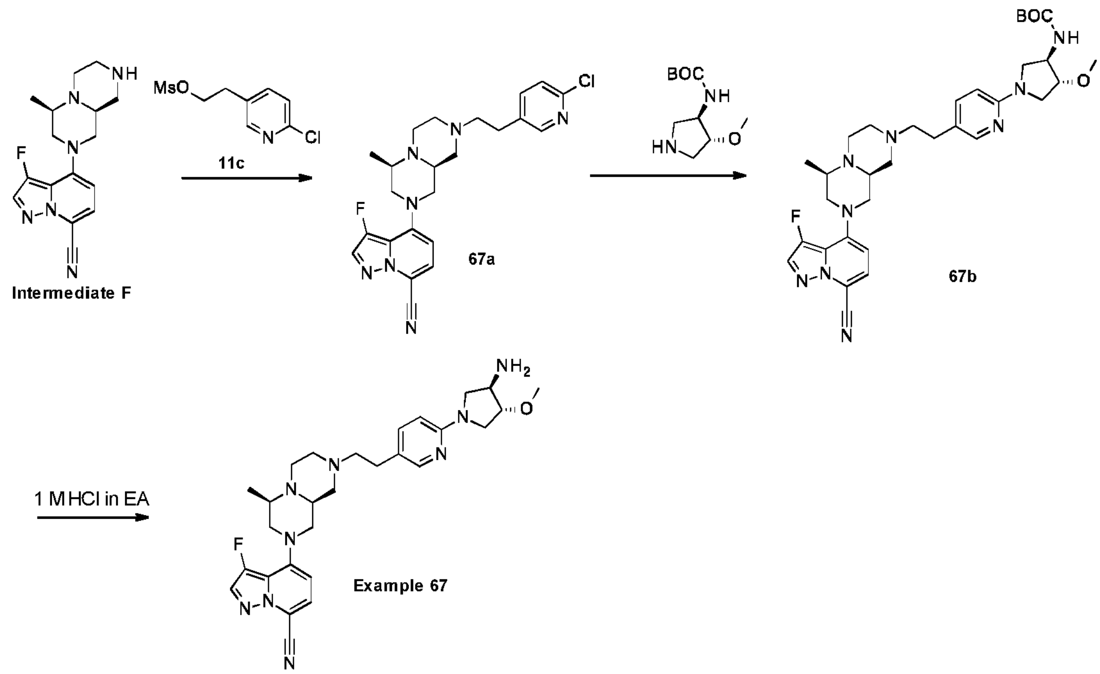

Intermediate F
67a
67b
Example 67

The title compound was prepared in analogy to the preparation of Example **11** by using *tert*-butyl *N*-[(*3R*,*4R*)-4-methoxypyrrolidin-3-yl]carbamate instead of *tert*-butyl piperazine-1-carboxylate and Intermediate **F** instead of Intermediate **C.** Example **67** was obtained as a yellow solid (55 mg). LCMS $(M+H)^+$: 534, $^1H$ NMR (400 MHz, METHANOL-d4) δ ppm: 8.09 - 8.00 (m, 1H), 7.98 - 7.92 (m, 2H), 7.38 (d, *J* = 7.8 Hz, 1H), 7.12 (d, *J* = 9.3 Hz, 1H), 6.56 (d, *J* = 7.9 Hz, 1H), 4.27 - 4.16 (m, 1H), 4.06 - 3.90 (m, 5H), 3.89 - 3.71 (m, 5H), 3.68 - 3.62 (m, 1H), 3.60 - 3.34 (m, 9H), 3.33 - 3.25 (m, 2H), 3.14 - 2.99 (m, 2H), 1.41 (d, *J* = 6.4 Hz, 3H).

### Example 68

**4-[(*4R*,*9aS*)-8-[2-[4-[(*3R,4R*)-3-amino-4-methoxy-pyrrolidin-1-yl]phenyl]ethyl]-4-methyl-3,4,6,7,9,9a-hexahydro-*1H*-pyrazino[1,2-a]pyrazin-2-yl] pyrazolo[1,5-a]pyridine-7-carbonitrile**

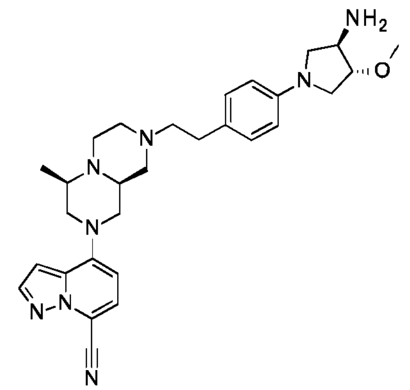

The title compound was prepared according to the following scheme:

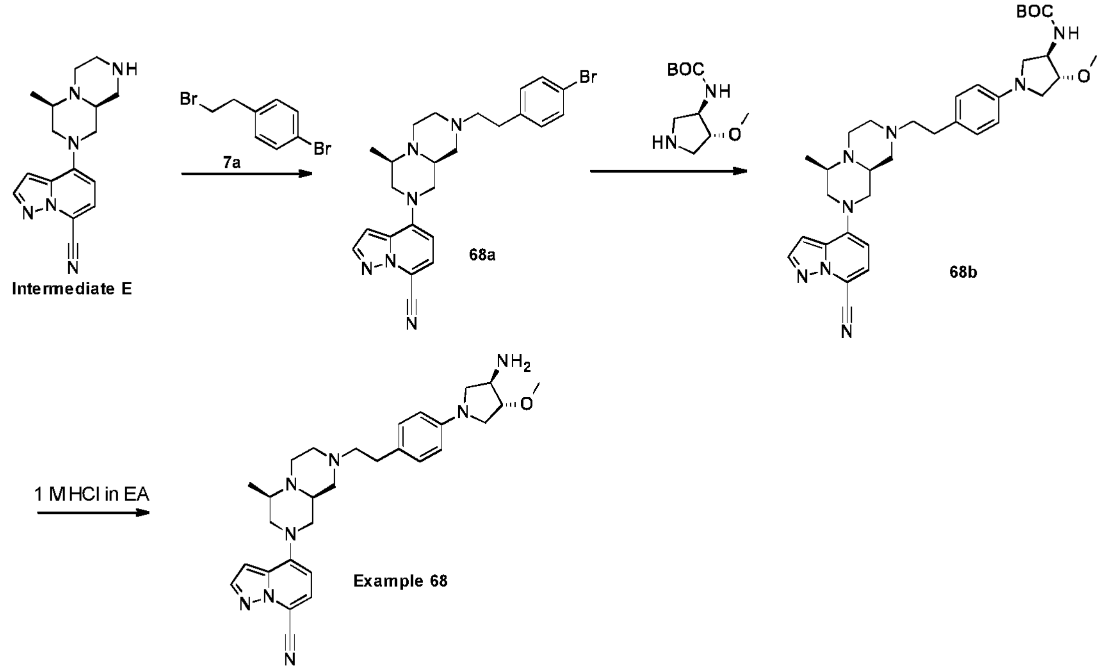


Intermediate E

68a

68b

Example 68

The title compound was prepared in analogy to the preparation of Example **11** by using *tert-butyl N*-[(*3R*,*4R*)-4-methoxypyrrolidin-3-yl]carbamate instead of *tert*-butyl piperazine-1-carboxylate, compound **7a** instead of compound **11c** and Intermediate **E** instead of Intermediate **C.** Example **68** was obtained as an orange solid (70 mg). LCMS $(M+H)^+$: 515, $^1H$ NMR (400 MHz, METHANOL-d4) δ ppm: 8.11 (d, *J* = 2.4 Hz, 1H), 7.55 (d, *J* = 7.9 Hz, 1H), 7.23 (d, *J* = 8.6 Hz, 2H), 7.03 (d, *J* = 2.4 Hz, 1H), 6.79 (d, *J* = 7.9 Hz, 1H), 6.69 (d, *J* = 8.6 Hz, 2H), 4.34 - 3.98 (m, 7H), 3.94 - 3.76 (m, 4H), 3.70 - 3.59 (m, 3H), 3.58 - 3.42 (m, 8H), 3.31 - 3.22 (m, 1H), 3.17 - 3.07 (m, 2H), 1.59 (d, *J* = 6.5 Hz, 3H).

**Example 69**

**4-[(*4R*,*9aS*)-4-methyl-8-(1,2,3,4-tetrahydroisoquinolin-7-ylmethyl)-3,4,6,7,9,9a-hexahydro-*1H*-pyrazino[1,2-a]pyrazin-2-yl]-3-fluoro-pyrazolo[1,5-a]pyridine-7-carbonitrile**

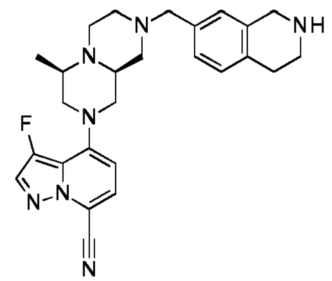

The title compound was prepared in analogy to the preparation of Example **1** by using Intermediate **E** instead of Intermediate **A.** Example **69** was obtained as an orange solid (8 mg). LCMS $(M+H)^+$: 460, $^1H$ NMR (400 MHz, METHANOL-d4) δ ppm: 7.92 (d, *J* = 3.7 Hz, 1H), 7.45 - 7.34 (m, 3H), 7.28 (d, *J* = 8.1 Hz, 1H), 6.49 (d, *J* = 7.9 Hz, 1H), 4.32 (s, 2H), 4.21 (br s, 2H), 3.77 - 3.57 (m, 3H), 3.50 - 3.37 (m, 5H), 3.15 - 2.82 (m, 8H), 1.27 (br d, *J* = 6.2 Hz, 3H).

**Example 70**

**5-[(*4R*,*9aS*)-4-methyl-8-(1,2,3,4-tetrahydroisoquinolin-5-ylmethyl)-3,4,6,7,9,9a-hexahydro-*1H*-pyrazino[1,2-a]pyrazin-2-yl]quinoline-8-carbonitrile**

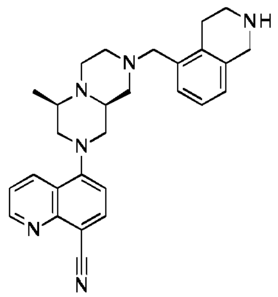

The title compound was prepared in analogy to the preparation of Example **1** by using Intermediate **C** instead of Intermediate **A** and *tert*-butyl 5-(bromomethyl)-3,4-dihydroisoquinoline-2(*1H*)-carboxylate (CAS: 2031269-14-2, Catalog NO: PB98143, PharmaBlock) instead of compound **1b.** Example **70** was obtained as an orange foam (10 mg). LCMS $(M+H)^+$: 453, $^1H$ NMR (400 MHz, METHANOL-$d_4$) $\delta$ ppm: 9.23 - 9.13 (m, 1H), 9.11 - 9.04 (m, 1H), 8.40 (d, *J* = 8.1 Hz, 1H), 8.01 - 7.91 (m, 1H), 7.75 - 7.65 (m, 1H), 7.57 (d, *J* = 8.1 Hz, 1H), 7.49 - 7.35 (m, 2H), 4.73 - 4.52 (m, 3H), 4.44 (s, 2H), 4.08 - 3.89 (m, 4H), 3.87 - 3.50 (m, 9H), 3.47 - 3.39 (m, 2H), 1.60 (d, *J* = 6.4 Hz, 3H)

**Example 71**

**5-[(*4R*,*9aS*)-4-methyl-8-(1,2,3,4-tetrahydroisoquinolin-8-ylmethyl)-3,4,6,7,9,9a-hexahydro-1H-pyrazino[1,2-a]pyrazin-2-yl]quinoline-8-carbonitrile**

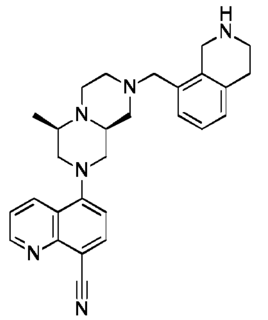

The title compound was prepared in analogy to the preparation of Example **1** by using Intermediate **C** instead of Intermediate **A** and *tert*-butyl 8-(bromomethyl)-3,4-dihydroisoquinoline-2(*1H*)-carboxylate (CAS: 2268818-17-1, Catalog NO: PB98142, PharmaBlock) instead of compound **1b.** Example **71** was obtained as an orange foam (18 mg). LCMS $(M+H)^+$: 453, $^1H$ NMR (400 MHz, METHANOL-$d_4$) $\delta$ ppm: 9.13 - 9.01 (m, 1H), 8.87 - 8.71 (m, 1H), 8.26 (d, *J* = 7.9 Hz, 1H), 7.91 - 7.66 (m, 1H), 7.52 - 7.27 (m, 4H), 4.79 - 4.59 (m, 2H), 4.29 - 3.83 (m, 5H), 3.81 - 3.34 (m, 11H), 3.19 (br t, *J* = 6.4 Hz, 2H), 1.53 (d, *J* = 6.5 Hz, 3H).

**Example 72**

**5-[(*4R*,*9aS*)-8-(isoindolin-4-ylmethyl)-4-methyl-3,4,6,7,9,9a-hexahydro-*1H*-pyrazino[1,2-a]pyrazin-2-yl]quinoline-8-carbonitrile**

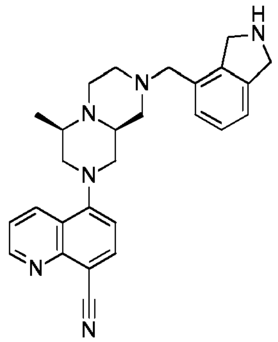

The title compound was prepared in analogy to the preparation of Example 1 by using Intermediate **C** instead of Intermediate **A** and *tert*-butyl 4-(bromomethyl)isoindoline-2-carboxylate (CAS: 1123176-01-1 , Catalog NO: PB98141, PharmaBlock) instead of compound **1b.** Example **72** was obtained as an orange foam (20 mg). LCMS $(M+H)^+$: 439, $^1H$ NMR (400 MHz, METHANOL-$d_4$) $\delta$ ppm: 9.14 - 9.05 (m, 1H), 8.94 - 8.81 (m, 1H), 8.30 (d, *J* = 7.9 Hz, 1H), 7.88 - 7.75 (m, 1H), 7.62 (br d, *J* = 6.0 Hz, 1H), 7.57 - 7.51 (m, 2H), 7.47 (d, *J* = 8.1 Hz, 1H), 4.98 (br d, *J* = 8.6 Hz, 2H), 4.70 (s, 2H), 4.43 - 4.26 (m, 3H), 4.13 (br d, *J* = 12.0 Hz, 1H), 4.00 - 3.88 (m, 1H), 3.82 - 3.56 (m, 6H), 3.48 - 3.36 (m, 3H), 1.55 (d, *J* = 6.5 Hz, 3H).

**Example 73**

**5-[(*4R,9aS*)-8-[2-(3-amino-3-methyl-azetidin-1-yl)-6-methyl-4-pyridyl]-4-methyl-3,4,6,7,9,9a-hexahydro-*1H*-pyrazino[1,2-a]pyrazin-2-yl]quinoline-8-carbonitrile**

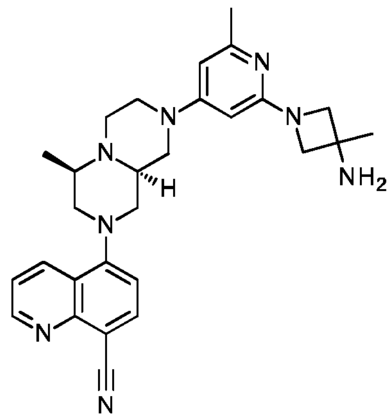

The title compound was prepared in analogy to the preparation of Example **32** by using tert-butyl N-(3-methylazetidin-3-yl)carbamate instead of *tert*-butyl piperazine-1-carboxylate, 4-bromo-2-fluoro-6-methylpyridine instead of 4-bromo-2-fluoropyridine and TFA/DCM (1:2) instead of 1 M HCl in EA. Example **73** (35 mg) was obtained as a yellow foam. LCMS $(M+H)^+$: 483. $^1H$ NMR (400 MHz, METHANOL-d4) $\delta$ ppm: 8.88 (dd, *J* = 1.6, 4.3 Hz, 1H), 8.54 (dd, *J* = 1.7, 8.6 Hz, 1H), 8.05 (d, *J* = 8.1 Hz, 1H), 7.55 (dd, *J* = 4.2, 8.6 Hz, 1H), 7.16 (d, *J* = 8.1 Hz, 1H), 6.12 (d, *J* = 1.7 Hz, 1H), 5.48 (d, *J* = 2.0 Hz, 1H), 3.81 (br d, *J* = 11.7 Hz, 1H), 3.77 - 3.69 (m, 3H), 3.64 (d, *J* = 7.8 Hz, 2H), 3.46 - 3.38 (m, 1H), 3.37 - 3.25 (m, 2H), 2.89 - 2.61 (m, 5H), 2.51 (s, 1H), 2.18 (s, 4H), 1.38 (s, 3H), 1.11 (d, *J* = 6.1 Hz, 3H).

**Example 74**

**4-[(*4R,9aS*)-8-[2-[6-(3-amino-3-methyl-azetidin-1-yl)-3-pyridyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile**

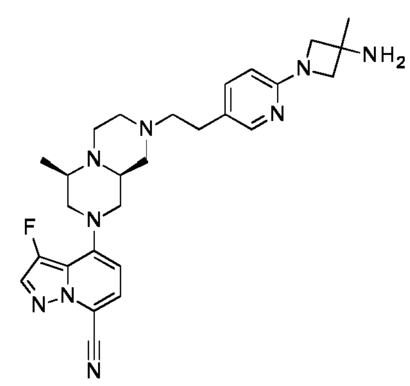

The title compound was prepared in analogy to the preparation of Example **11** by using *tert*-butyl *N*-(3-methylazetidin-3-yl)carbamate instead of *tert*-butyl piperazine-1-carboxylate, Intermediate **F** instead of Intermediate **C** and TFA/DCM (1:2) instead of 1 M HCl in EA. Example **74** was obtained as a light yellow solid (14 mg). LCMS $(M+H)^+$: 504, $^1H$ NMR (400 MHz, METHANOL-d4) δ ppm: 7.88 (d, *J* = 3.1 Hz, 1H), 7.79 (d, *J*= 1.8 Hz, 1H), 7.37 (dd, *J* = 2.3, 8.5 Hz, 1H), 7.31 (d, *J* = 7.9 Hz, 1H), 6.38 (d, *J* = 7.9 Hz, 1H), 6.31 (d, *J* = 8.6 Hz, 1H), 3.83 - 3.73 (m, 2H), 3.71 - 3.63 (m, 2H), 3.52 - 3.34 (m, 2H), 3.17 - 3.09 (m, 1H), 2.95 (br dd, *J*= 1.7, 11.0 Hz, 1H), 2.84 (br d, *J* = 11.0 Hz, 1H), 2.72 - 2.57 (m, 4H), 2.56 - 2.37 (m, 4H), 2.25 - 2.05 (m, 2H), 1.91 (t, *J* = 10.8 Hz, 1H), 1.41 (s, 3H), 1.06 (d, *J*= 6.2 Hz, 3H).

**Example 75**

**4-[(*4R*,*9aS*)-8-[2-[6-[(*3R*,*4S*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile**

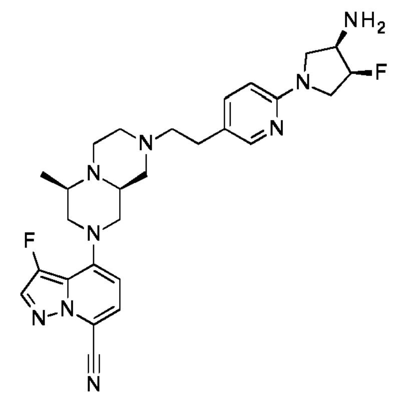

The title compound was prepared in analogy to the preparation of Example **11** by using *tert*-butyl *N*-(3-methylazetidin-3-yl)carbamate instead of *tert*-butyl piperazine-1-carboxylate and Intermediate **F** instead of Intermediate **C.** Example **74** was obtained as a light yellow solid (21 mg). LCMS $(M+H)^+$: 522, $^1H$ NMR (400 MHz, METHANOL-d4) δ ppm: 8.00 (d, *J*= 3.5 Hz, 1H), 7.93 (d, *J*= 2.1 Hz, 1H), 7.49 (dd, *J*= 2.3, 8.7 Hz, 1H), 7.42 (d, *J*= 7.9 Hz, 1H), 6.50 (d, *J* = 8.2 Hz, 2H), 5.22 - 5.16 (m, 0.5H), 5.05 (t, *J*= 3.1 Hz, 0.5H), 3.89 - 3.77 (m, 2H), 3.74 - 3.67 (m, 1H), 3.65 - 3.49 (m, 3H), 3.27 (br d, *J*= 11.0 Hz, 1H), 3.18 (t, *J*= 9.6 Hz, 1H), 3.12 - 3.05 (m, 1H), 2.97 (br d, *J*= 11.0 Hz, 1H), 2.84 - 2.69 (m, 4H), 2.67 - 2.52 (m, 4H), 2.40 - 2.18 (m, 2H), 2.03 (t, *J*= 10.8 Hz, 1H), 1.18 (d, *J*= 6.2 Hz, 3H).

**Example 76**

**5-[(*4R*,*9aS*)-8-[[6-(3-amino-3-methyl-azetidin-1-yl)-2-methyl-3-pyridyl]methyl]-4-methyl-3,4,6,7,9,9a-hexahydro-*1H*-pyrazino[1,2-a]pyrazin-2-yl]quinoline-8-carbonitrile**

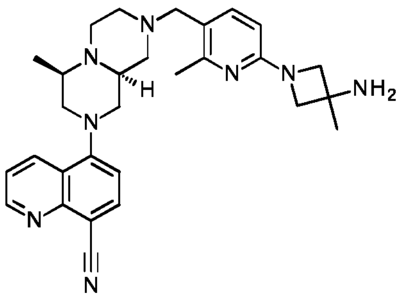

The title compound was prepared according to the following scheme:

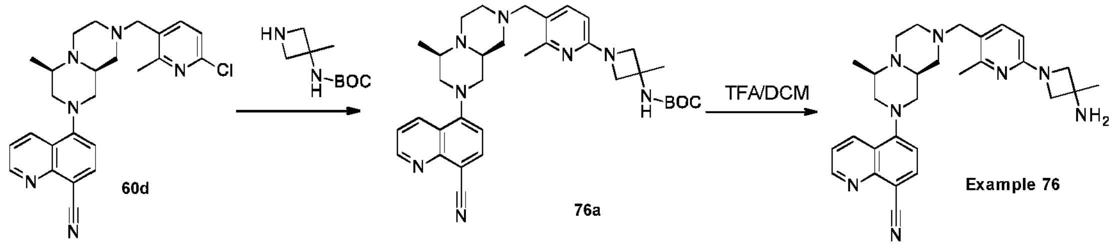


The title compound was prepared in analogy to the preparation of Example **11** by using *tert*-butyl *N*-(3-methylazetidin-3-yl)carbamate instead of *tert*-butyl piperazine-1-carboxylate, compound **60d** instead of compound **11d** and TFA/DCM (1:2) instead of 1 M HCl in EA. Example **76** was obtained as a light yellow solid (25 mg). LCMS $(M+H)^+$: 497, $^1H$ NMR (400 MHz, METHANOL-d4) δ ppm: 9.02 (dd, *J*= 1.6, 4.3 Hz, 1H), 8.67 (dd, *J*= 1.6, 8.6 Hz, 1H), 8.20 (d, *J* = 7.9 Hz, 1H), 7.94 (d, *J* = 9.0 Hz, 1H), 7.68 (dd, *J* = 4.3, 8.6 Hz, 1H), 7.36 (d, *J* = 7.9 Hz, 1H), 6.73 (d, *J*= 8.9 Hz, 1H), 4.48 (dd, *J*= 2.5, 10.3 Hz, 2H), 4.40 - 4.29 (m, 2H), 3.95 - 3.84 (m, 1H), 3.83 - 3.58 (m, 6H), 3.28 - 3.06 (m, 5H), 2.78 - 2.68 (m, 1H), 2.63 (s, 3H), 2.48 (br t, *J* = 11.6 Hz, 1H), 1.74 (s, 3H), 1.45 (d, *J* = 6.4 Hz, 3H).

### Example 77

**5-[(*4R,9aS*)-8-[2-[6-[(*4aR,7aR*)-3,4,4a,5,7,7a-hexahydro-*2H*-pyrrolo[3,4-b][1,4]oxazin-6-yl]-3-pyridyl]ethyl]-4-methyl-3,4,6,7,9,9a-hexahydro-*1H*-pyrazino[1,2-a]pyrazin-2-yl]-2-deuterio-quinoline-8-carbonitrile**

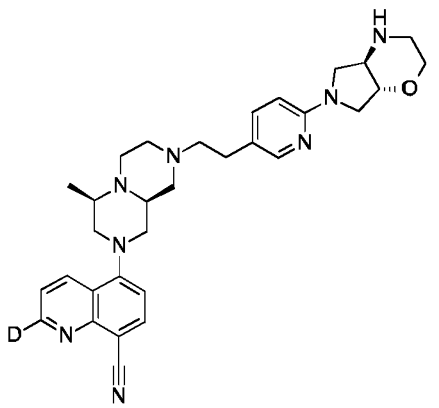

The title compound was prepared in analogy to the preparation of Example **11** by using *tert*-butyl (*4aR*,*7aR*)-3,4a,5,6,7,7a-hexahydro-*2H*-pyrrolo[3,4-b][1,4]oxazine-4-carboxylate (CAS: 1932337-68-2, catalog: PBXA8123, vendor: Pharmablock) instead of *tert*-butyl piperazine-1-carboxylate and Intermediate **G** instead of Intermediate **C.** Example **77** was obtained as a light yellow solid (44 mg). LCMS $(M+H)^+$: 540, $^1H$ NMR (400 MHz, METHANOL-d4) δ ppm: 8.65 (d, *J*= 8.7 Hz, 1H), 8.16 (d, *J*= 8.1 Hz, 1H), 7.91 (d, *J*= 2.2 Hz, 1H), 7.66 (d, *J* = 8.6 Hz, 1H), 7.48 (dd, *J* = 2.3, 8.6 Hz, 1H), 7.25 (d, *J* = 8.1 Hz, 1H), 6.48 (d, *J* = 8.7 Hz, 1H), 3.99 (dd, *J* = 2.4, 11.9 Hz, 1H), 3.84 - 3.71 (m, 3H), 3.69 - 3.61 (m, 1H), 3.47 - 3.36 (m, 3H), 3.26 - 3.08 (m, 3H), 3.04 - 2.94 (m, 4H), 2.88 - 2.67 (m, 6H), 2.64 - 2.54 (m, 2H), 2.40 -

2.29 (m, 2H), 2.11 - 2.00 (m, 1H), 1.19 (d, *J*= 6.0 Hz, 3H).

**Example 78**

**5-[(*4R*,*9aS*)-4-methyl-8-[(6-piperazin-1-yl-3-pyridyl)methyl]-3,4,6,7,9,9a-hexahydro-*1H*-pyrazino[1,2-a]pyrazin-2-yl]-2-deuterio-quinoline-8-carbonitrile**

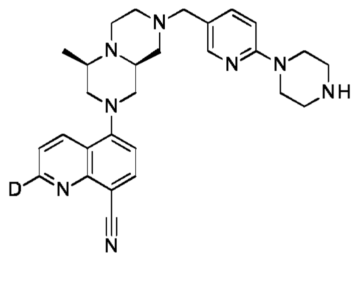

Example **78** was prepared in analogy to the preparation of Example **3** by using Intermediate **G** instead of Intermediate **A** and 2-bromo-5-(bromomethyl)pyridine instead of compound **3a.** Example **78** was obtained as a light yellow solid (15 mg). LCMS $(M+H)^+$: 484, $^1$H NMR (400 MHz, METHANOL-d4) δ ppm: 8.61 (d, *J* = 8.7 Hz, 1H), 8.14 (d, *J*= 8.1 Hz, 1H), 8.06 (d, *J* = 2.2 Hz, 1H), 7.68 - 7.55 (m, 2H), 7.23 (d, *J* = 8.1 Hz, 1H), 6.84 (d, *J* = 8.8 Hz, 1H), 3.58 - 3.45 (m, 6H), 3.44 - 3.39 (m, 1H), 3.29 - 3.23 (m, 2H), 3.00 - 2.92 (m, 5H), 2.88 - 2.64 (m, 5H), 2.37 - 2.21 (m, 2H), 2.00 (br t, *J* = 10.3 Hz, 1H), 1.17 (d, *J*= 6.0 Hz, 3H).

**Example 79**

**5-[(*4R*,*9aS*)-8-[[6-(3-amino-3-methyl-azetidin-1-yl)-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile**

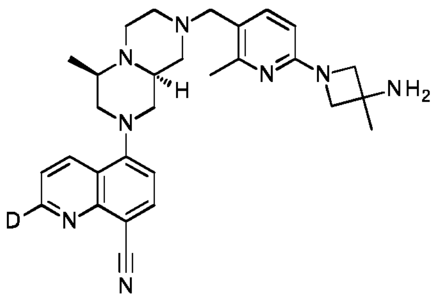

The title compound was prepared in analogy to the preparation of Example **11** by using *tert*-butyl *N*-(3-methylazetidin-3-yl)carbamate instead of *tert*-butyl piperazine-1-carboxylate, Intermediate **G** instead of Intermediate **C** and TFA/DCM (1:2) instead of 1 M HCl in EA. Example **79** was obtained as a light yellow solid (27 mg). LCMS $(M+H)^+$: 498, $^1$H NMR (400 MHz, METHANOL-d4) δ ppm: 8.61 (d, *J* = 8.6 Hz, 1H), 8.14 (d, *J*= 8.1 Hz, 1H), 7.63 (d, *J*= 8.6 Hz, 1H), 7.42 (d, *J* = 8.3 Hz, 1H), 7.23 (d, *J*= 8.1 Hz, 1H), 6.24 (d, *J*= 8.3 Hz, 1H), 3.95 - 3.85 (m, 2H), 3.84 - 3.75 (m, 2H), 3.48 - 3.38 (m, 3H), 3.30 - 3.22 (m, 2H), 2.92 (br d, *J*= 8.9 Hz, 1H), 2.88 - 2.69 (m, 5H), 2.46 (s, 3H), 2.34 - 2.22 (m, 2H), 2.07 - 1.95 (m, 1H), 1.52 (s, 3H), 1.17 (d, *J* = 6.1 Hz, 3H).

**Example 80**

**5-[(*4R*,*9aS*)-4-methyl-8-[[2-methyl-6-(6-methyl-2,6-diazaspiro[3.3]heptan-2-yl)-3-pyridyl]methyl]-3,4,6,7,9,9a-hexahydro-*1H*-pyrazino[1,2-a]pyrazin-2-yl]-2-deuterio-quinoline-8-carbonitrile**

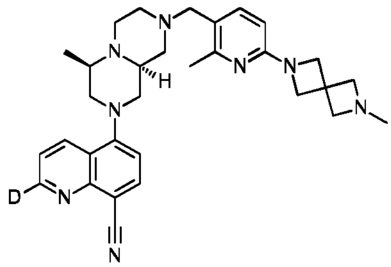

The title compound was prepared according to the following scheme:

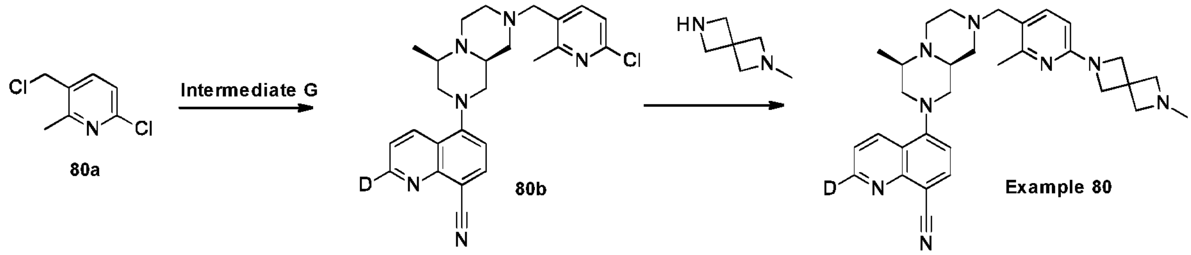


80a

80b

Example 80

**Step 1: preparation of 5-[(*4R*,*9aS*)-8-[(6-chloro-2-methyl-3-pyridyl)methyl]-4-methyl-3,4,6,7,9,9a-hexahydro-*1H*-pyrazino[1,2-a]pyrazin-2-yl]-2-deuterio-quinoline-8-carbonitrile (compound 80b)**

A mixture of 5-[(*4R*,*9aS*)-4-methyl-1,3,4,6,7,8,9,9a-octahydropyrazino[1,2-a]pyrazin-2-yl]-2-deuterio-quinoline-8-carbonitrile (**intermediate G**, 500 mg, 1.6 mmol), 6-chloro-3-(chloromethyl)-2-methylpyridine (428 mg, 2.4 mmol) and $K_2CO_3$ (672 mg, 4.9 mmol) in MeCN (10 mL) was stirred at 80 °C for 16 hours. Then the reaction was concentrated, the residue was purified by silica gel to give compound **80b** as a light yellow solid, 500 mg. LCMS $(M+H)^+$: 448.

**Step 2: preparation of 5-[(*4R*,*9aS*)-4-methyl-8-[[2-methyl-6-(6-methyl-2,6-diazaspiro[3.3]heptan-2-yl)-3-pyridyl]methyl]-3,4,6,7,9,9a-hexahydro-*1H*-pyrazino[1,2-a]pyrazin-2-yl]-2-deuterio-quinoline-8-carbonitrile (Example 80)**

A mixture of 5-[(*4R*,*9aS*)-8-[(6-chloro-2-methyl-3-pyridyl)methyl]-4-methyl-3,4,6,7,9,9a-hexahydro-*1H*-pyrazino[1,2-a]pyrazin-2-yl]-2-deuterio-quinoline-8-carbonitrile (compound **80b,** 80 mg, 179 μmol), 2-methyl-2,6-diazaspiro[3.3]heptane (30 mg, 268 μmol), $Cs_2CO_3$ (116 mg, 357 μmol) and Ruphos Pd G2 (13.9 mg, 17.9 μmol) in dioxane (5 mL) was stirred at 120 °C for 16 hours. Then the reaction was concentrated and the residue was purified by HPLC-preparation to give Example **80** as light yellow powder, 30 mg. LCMS $(M+H)^+$: 524, $^1H$ NMR (400 MHz, METHANOL-d4) $\delta$ ppm: 8.61 (d, *J* = 8.6 Hz, 1H), 8.14 (d, *J*= 7.9 Hz, 1H), 7.63 (d, *J* = 8.6 Hz, 1H), 7.42 (d, *J*= 8.3 Hz, 1H), 7.23 (d, *J*= 8.1 Hz, 1H), 6.23 (d, *J*= 8.3 Hz, 1H), 4.06 (s, 4H), 3.48 (s, 4H), 3.46 - 3.38 (m, 3H), 3.31 - 3.22 (m, 2H), 2.96 - 2.87 (m, 1H), 2.84 - 2.68 (m, 5H), 2.45 (s, 3H), 2.36 (s, 3H), 2.32 - 2.23 (m, 2H), 2.05 - 1.93 (m, 1H), 1.17 (d, *J* = 6.1 Hz, 3H).

**Example 81**

**5-[(*4R*,*9aS*)-8-[[6-[(*3R*)-3-amino-3-methyl-pyrrolidin-1-yl]-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile**

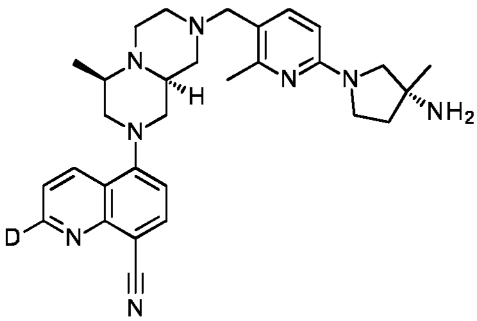

The title compound was prepared according to the following scheme:

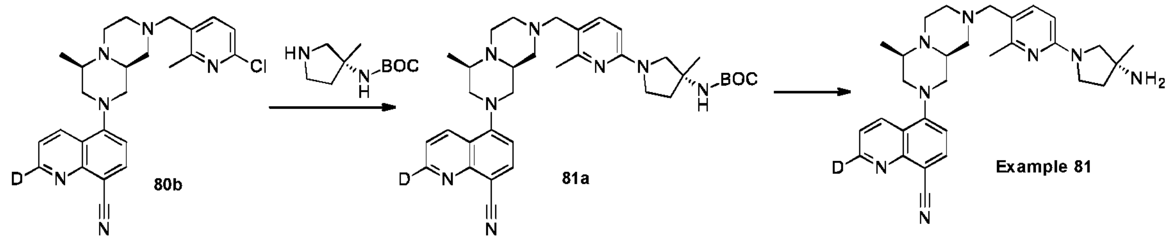

A mixture of 5-[(*4R*,*9aS*)-8-[(6-chloro-2-methyl-3-pyridyl)methyl]-4-methyl-3,4,6,7,9,9a-hexahydro-*1H*-pyrazino[1,2-a]pyrazin-2-yl]-2-deuterio-quinoline-8-carbonitrile (compound **80b,** 80 mg, 179 μmol), *tert*-butyl (*R*)-(3-methylpyrrolidin-3-yl)carbamate (53.6 mg, 268 μmol,), $Cs_2CO_3$ (116 mg, 357 μmol) and Ruphos Pd G2 (14 mg, 18 μmol) in dioxane (5 mL) was stirred at 120 °C for 16 hours. The reaction was concentrated and the residue was purified by HPLC-preparation to give compound **81a** (40 mg) as a light yellow solid. Then compound **81a** (40 mg, 65.4 μmol) was treated with 1 M HCl in EA (3 mL) and stirred at rt for 16 hours. The reaction mixture was concentrated to give Example **81** (32 mg) as an orange solid. LCMS $(M+H)^+$: 512, $^1H$ NMR (400 MHz, METHANOL-$d_4$) $\delta$ ppm: 8.90 (d, *J*= 8.6 Hz, 1H), 8.31 (d, *J*= 7.9 Hz, 1H), 8.21 (d, *J* = 9.3 Hz, 1H), 7.84 (d, *J* = 8.6 Hz, 1H), 7.49 (d, *J* = 8.1 Hz, 1H), 7.07 (d, *J* = 9.4 Hz, 1H), 4.40 - 4.22 (m, 3H), 4.19 - 4.10 (m, 1H), 4.03 - 3.89 (m, 4H), 3.87 - 3.82 (m, 1H), 3.82 - 3.70 (m, 4H), 3.65 - 3.52 (m, 2H), 3.51 - 3.35 (m, 3H), 2.80 (s, 3H), 2.50 - 2.42 (m, 2H), 1.66 (s, 3H), 1.56 (d, *J* = 6.5 Hz, 3H).

**Example 82**

**5-[(*4R*,*9aS*)-8-[[6-[[(*3S*,*4R*)-4-fluoropyrrolidin-3-yl]amino]-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile**

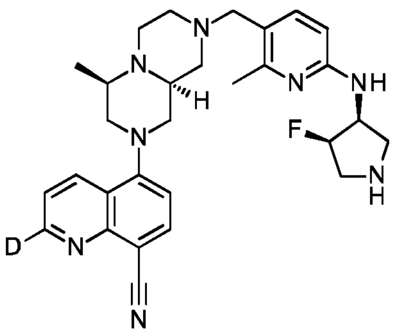

The title compound was prepared in analogy to the preparation of Example **81** by using *tert*-butyl (*3S*,*4R*)-3-amino-4-fluoropyrrolidine-1-carboxylate instead of *tert*-butyl (*R*)-(3-methylpyrrolidin-3-yl)carbamate. Example **82** was obtained as an orange solid, 32 mg. LCMS $(M+H)^+$: 516, $^1H$ NMR (400 MHz, METHANOL-$d_4$) $\delta$ ppm: 8.88 (d, *J*= 8.7 Hz, 1H), 8.30 (d, *J* = 8.1 Hz, 1H), 8.24 (d, *J* = 9.2 Hz, 1H), 7.83 (d, *J* = 8.7 Hz, 1H), 7.48 (d, *J* = 7.9 Hz, 1H), 7.21 (d, *J*= 9.2 Hz, 1H), 5.57 (t, *J*= 2.9 Hz, 0.5H), 5.44 (t, *J*= 2.9 Hz, 0.5H), 5.19 - 5.01 (m, 1H), 4.36 - 4.17 (m, 3H), 4.13 (br d, *J*=

11.9 Hz, 1H), 4.01 - 3.83 (m, 3H), 3.82 - 3.60 (m, 5H), 3.58 - 3.37 (m, 5H), 3.27 - 3.21 (m, 1H), 2.79 (s, 3H), 1.56 (d, *J*= 6.4 Hz, 3H).

**Example 83**

**5-[(*4R,9aS*)-4-methyl-8-[[2-methyl-6-(9-oxa-3,7-diazabicyclo[3.3.1]nonan-3-yl)-3-pyridyl]methyl]-3,4,6,7,9,9a-hexahydro-*1H*-pyrazino[1,2-a]pyrazin-2-yl]-2-deuterio-quinoline-8-carbonitrile**

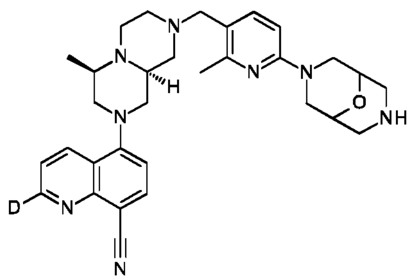

The title compound was prepared in analogy to the preparation of Example **81** by using *tert*-butyl 9-oxa-3,7-diazabicyclo[3.3.1]nonane-3-carboxylate instead of *tert*-butyl (R)-(3-methylpyrrolidin-3-yl)carbamate. Example **83** was obtained as an orange solid, 12 mg. LCMS $(M+H)^+$: 540, $^1H$ NMR (400 MHz, METHANOL-$d_4$) $\delta$ ppm: 8.84 (d, *J*= 8.6 Hz, 1H), 8.28 (d, *J* = 7.9 Hz, 2H), 7.80 (d, *J*= 8.6 Hz, 1H), 7.45 (d, *J*= 7.9 Hz, 1H), 7.34 (d, *J*= 9.2 Hz, 1H), 4.41 (br s, 2H), 4.30 (br d, *J*= 12.8 Hz, 2H), 4.16 - 3.98 (m, 4H), 3.94 - 3.82 (m, 1H), 3.81 - 3.63 (m, 7H), 3.61 - 3.38 (m, 8H), 2.84 (s, 3H), 1.54 (d, *J*= 6.4 Hz, 3H).

**Example 84**

**5-[(*4R,9aS*)-8-[2-[6-[(*3R*)-3-amino-3-methyl-pyrrolidin-1-yl]-3-pyridyl]ethyl]-4-methyl-3,4,6,7,9,9a-hexahydro-1H-pyrazino[1,2-a]pyrazin-2-yl]-2-deuterio-quinoline-8-carbonitrile**

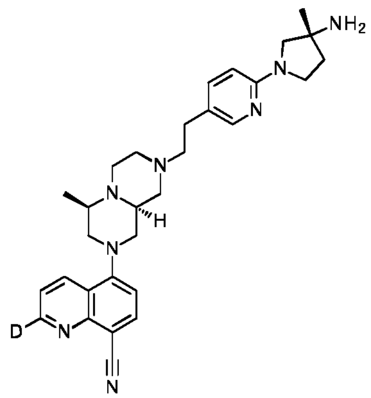

The title compound was prepared in analogy to the preparation of Example **11** by using *tert*-butyl (*R*)-(3-methylpyrrolidin-3-yl)carbamate instead of *tert*-butyl piperazine-1-carboxylate and Intermediate **G** instead of Intermediate **C.** Example **84** was obtained as an orange solid, 50 mg. LCMS $(M+H)^+$: 512, $^1H$ NMR (400 MHz, METHANOL-d4) $\delta$ ppm: 8.85 (d, *J* = 8.7 Hz, 1H), 8.28 (d, *J* = 8.1 Hz, 1H), 8.19 - 8.11 (m, 1H), 8.07 (s, 1H), 7.81 (d, *J* = 8.6 Hz, 1H), 7.47 (d, *J* = 8.1 Hz, 1H), 7.21 (d, *J* = 9.4 Hz, 1H), 4.44 - 4.31 (m, 1H), 4.22 (br d, *J* = 13.3 Hz, 1H), 4.06 (br t, *J*= 10.9 Hz, 2H), 3.98 - 3.83 (m, 4H), 3.81 - 3.71 (m, 4H), 3.69 - 3.54 (m, 4H), 3.54 - 3.38 (m, 2H), 3.27 - 3.17 (m, 2H), 2.55 - 2.38 (m, 2H), 1.67 (s, 3H), 1.56 (d, *J*= 6.4 Hz, 3H).

**Example 85**

**5-[(*4R*,9*a*S)-4-methyl-8-[[2-methyl-6-[(*2S*)-2-methylpiperazin-1-yl]-3-pyridyl]methyl]-3,4,6,7,9,9a-hexahydro-1H-pyrazino[1,2-a]pyrazin-2-yl]quinoline-8-carbonitrile**

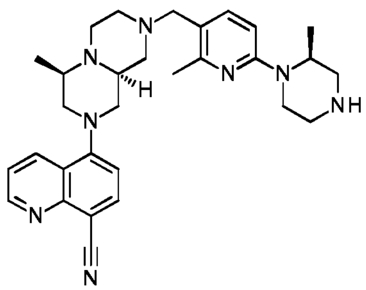

The title compound was prepared in analogy to the preparation of Example **11** by using *tert*-butyl (*3S*)-3-methylpiperazine-1-carboxylate instead of *tert*-butyl piperazine-1-carboxylate and compound **60d** instead of compound **11d.** Example **85** was obtained as a light yellow solid, 5 mg. LCMS $(M+H)^+$: 511, $^1H$ NMR (400 MHz, METHANOL-d4) $\delta$ ppm: 9.01 - 8.94 (m, 1H), 8.69 - 8.56 (m, 1H), 8.14 (d, *J* = 7.9 Hz, 1H), 7.71 - 7.59 (m, 1H), 7.41 (d, *J* = 8.6 Hz, 1H), 7.23 (d, *J* = 8.1 Hz, 1H), 6.53 (d, *J* = 8.6 Hz, 1H), 4.45 (br d, *J* = 4.8 Hz, 1H), 3.99 - 3.88 (m, 1H), 3.55 - 3.38 (m, 5H), 3.32 - 3.21 (m, 1H), 3.12 - 2.86 (m, 6H), 2.85 - 2.67 (m, 7H), 2.37 - 2.23 (m, 2H), 2.04 - 1.93 (m, 1H), 1.20 - 1.13 (m, 6H).

**Example 86**

**5-[(*4R*,*9aS*)-4-methyl-8-[[2-methyl-6-[(*3R*)-3-methylpiperazin-1-yl]-3-pyridyl]methyl]-3,4,6,7,9,9a-hexahydro-*1H*-pyrazino[1,2-a]pyrazin-2-yl]quinoline-8-carbonitrile**

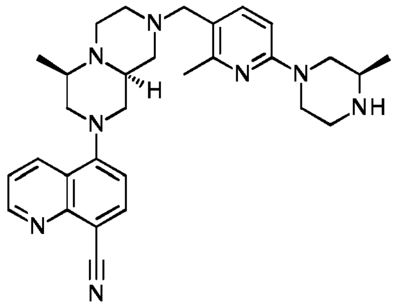

The title compound was prepared in analogy to the preparation of Example **11** by using *tert*-butyl (*2R*)-2-methylpiperazine-1-carboxylate instead of *tert*-butyl piperazine-1-carboxylate and compound **60d** instead of compound **11d.** Example **86** was obtained as a light yellow solid, 15 mg. LCMS $(M+H)^+$: 511, $^1H$ NMR (400 MHz, METHANOL-$d_4$) $\delta$ ppm 9.02 - 8.91 (m, 1H), 8.67 - 8.54 (m, 1H), 8.14 (d, *J* = 8.1 Hz, 1H), 7.68 - 7.56 (m, 1H), 7.42 (d, *J* = 8.6 Hz, 1H), 7.23 (d, *J*= 8.1 Hz, 1H), 6.59 (d, *J*= 8.4 Hz, 1H), 4.23 - 4.04 (m, 2H), 3.54 - 3.36 (m, 3H), 3.32 - 3.21 (m, 2H), 3.10 - 3.02 (m, 1H), 2.98 - 2.66 (m, 9H), 2.51 - 2.38 (m, 4H), 2.37 - 2.20 (m, 2H), 2.00 (t, *J* = 10.2 Hz, 1H), 1.18 - 1.15 (m, 6H).

**Example 87**

**5-[(*4R*,*9aS*)-4-methyl-8-[(4-methyl-6-piperazin-1-yl-3-pyridyl)methyl]-3,4,6,7,9,9a-hexahydro-*1H*-pyrazino[1,2-a]pyrazin-2-yl]-2-deuterio-quinoline-8-carbonitrile**

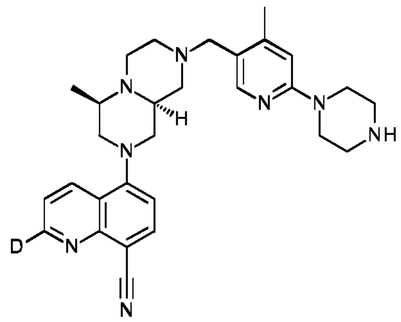

The title compound was prepared according to the following scheme:

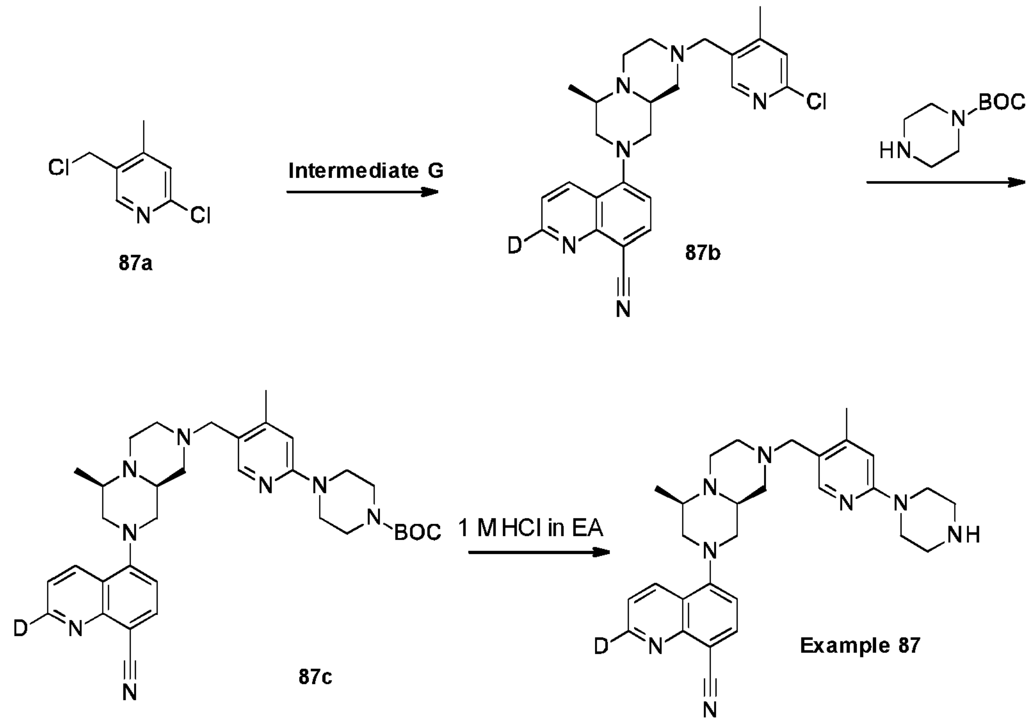


87a

87b

87c

Example 87

The title compound was prepared in analogy to the preparation of Example **11** by using compound **87a** instead of compound **11c** and intermediate **G** instead of intermediate **C.** Example **87** was obtained as an orange solid, 27 mg. LCMS $(M+H)^+$: 498, $^1H$ NMR (400 MHz, METHANOL-$d_4$) $\delta$ ppm: 8.98 (d, *J*= 8.6 Hz, 1H), 8.34 (d, *J*= 8.1 Hz, 1H), 8.24 (s, 1H), 7.89 (d, *J*= 8.6 Hz, 1H), 7.51 (d, *J*= 8.1 Hz, 1H), 7.44 (s, 1H), 4.31 - 4.09 (m, 4H), 4.08 - 4.02 (m, 4H), 3.98 - 3.90 (m, 1H), 3.77 (br d, *J*= 12.7 Hz, 2H), 3.67 (br d, *J*= 9.7 Hz, 2H), 3.59 - 3.36 (m, 8H), 3.22 - 3.09 (m, 1H), 2.66 (s, 3H), 1.56 (*J* = 6.5 Hz, 3H).

**Example 88**

**5-[(*4R*,*9aS*)-4-methyl-8-[(3-methyl-6-piperazin-1-yl-2-pyridyl)methyl]-3,4,6,7,9,9a-hexahydro-*1H*-pyrazino[1,2-a]pyrazin-2-yl]-2-deuterio-quinoline-8-carbonitrile**

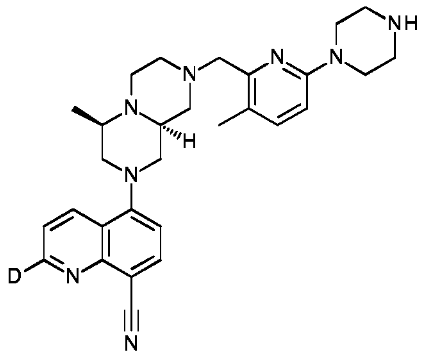

The title compound was prepared in analogy to the preparation of Example **11** by using 6-chloro-2-(chloromethyl)-3-methyl-pyridine instead of compound **11c** and intermediate **G** instead of intermediate **C.** Example **88** was obtained as an orange solid, 16 mg. LCMS $(M+H)^+$: 498, $^1H$ NMR (400 MHz, METHANOL-d4) $\delta$ ppm: 8.94 (d, *J*= 8.7 Hz, 1H), 8.30 (d, *J*= 7.9 Hz, 1H), 7.84 (d, *J* = 8.6 Hz, 1H), 7.71 (d, *J* = 8.8 Hz, 1H), 7.48 (d, *J* = 8.1 Hz, 1H), 7.06 (d, *J* = 8.8 Hz, 1H), 4.69 - 4.57 (m, 1H), 4.53 (d, *J* = 2.1 Hz, 2H), 4.17 (br d, *J*= 12.5 Hz, 1H), 4.09 - 4.03 (m, 1H), 4.00 - 3.89 (m, 5H), 3.89 - 3.67 (m, 5H), 3.59 - 3.39 (m, 7H), 2.32 (s, 3H), 1.57 (d, *J*= 6.5 Hz, 3H).

**Example 89**

**5-[(*4R*,*9aS*)-8-[2-[2-[(*3R*,*4S*)-3-amino-4-fluoro-pyrrolidin-1-yl]-4-pyridyl]ethyl]-4-methyl-3,4,6,7,9,9a-hexahydro-*1H*-pyrazino[1,2-a]pyrazin-2-yl]-2-deuterio-quinoline-8-carbonitrile**

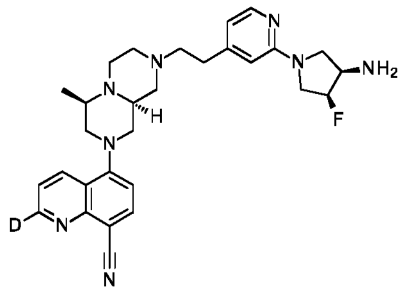

The title compound was prepared in analogy to the preparation of Example **11** by using 2-(2-chloro-4-pyridyl)acetic acid instead of compound **11a,** intermediate **G** instead of intermediate **C** and *tert*-butyl *N*-[(*3R*,*4S*)-4-fluoropyrrolidin-3-yl]carbamate instead of *tert*-butyl piperazine-1-carboxylate. Example **89** was obtained as an orange solid, 60 mg. LCMS $(M+H)^+$: 516, $^1H$ NMR (400 MHz, METHANOL-d4) $\delta$ ppm: 8.93 (d, *J*= 8.6 Hz, 1H), 8.32 (d, *J*= 7.9 Hz, 1H), 8.01 (d, *J* = 6.6 Hz, 1H), 7.86 (d, *J* = 8.6 Hz, 1H), 7.51 (d, *J* = 8.1 Hz, 1H), 7.35 (s, 1H), 7.18 - 7.11 (m, 1H), 5.71 (br s, 0.5H), 5.58 (t, *J* = 3.1 Hz, 0.5H), 4.45 (br t, *J*= 11.2 Hz, 1H), 4.39 - 4.16 (m, 4H), 4.15 - 4.04 (m, 3H), 4.03 - 3.94 (m, 1H), 3.89 - 3.63 (m, 8H), 3.57 - 3.37 (m, 4H), 1.58 (d, *J* = 6.4 Hz, 3H).

**Example 90**

**5-[(*4R*,*9aS*)-8-[[6-(3,6-diazabicyclo[3.1.1]heptan-3-yl)-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile**

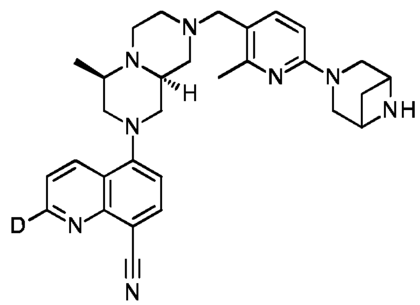

The title compound was prepared in analogy to the preparation of Example **81** by using tert-butyl 3,6-diazabicyclo[3.1.1]heptane-6-carboxylate instead of *tert*-butyl (*R*)-(3-methylpyrrolidin-3-yl)carbamate and TFA/DCM (1:2) instead of 1 M HCl in EA. Example **90** was obtained as a light solid, 15 mg. LCMS $(M+H)^+$: 510, $^1H$ NMR (400 MHz, METHANOL-$d_4$) $\delta$ ppm: 8.63 (d, *J* = 8.6 Hz, 1H), 8.17 (d, *J*= 7.9 Hz, 1H), 7.83 (d, *J* = 8.9 Hz, 1H), 7.65 (d, *J* = 8.6 Hz, 1H), 7.32 (d, *J*= 8.1 Hz, 1H), 6.85 (d, *J*= 8.8 Hz, 1H), 4.59 (d, *J*= 6.2 Hz, 2H), 4.20 - 4.02 (m, 4H), 3.95 (br s, 2H), 3.83 - 3.73 (m, 1H), 3.66 - 3.50 (m, 3H), 3.48 - 3.33 (m, 3H), 3.17 - 2.97 (m, 3H), 2.96 - 2.83 (m, 2H), 2.70 - 2.57 (m, 4H), 2.01 - 1.94 (m, 1H), 1.36 (d, *J* = 6.4 Hz, 3H).

**Example 91**

**5-[(*4R*,*9aS*)-8-[6-(3,8-diazabicyclo[3.2.1]octan-3-yl)-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile**

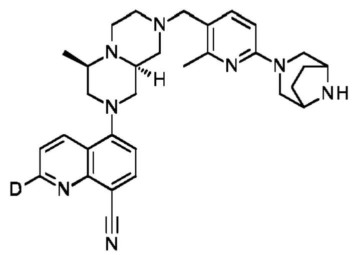

The title compound was prepared in analogy to the preparation of Example **81** by using tert-butyl 3,8-diazabicyclo[3.2.1]octane-8-carboxylate instead of *tert*-butyl (R)-(3-methylpyrrolidin-3-yl)carbamate. Example **91** was obtained as an orange solid, 15 mg. LCMS $(M+H)^+$: 524, $^1H$ NMR (400 MHz, METHANOL-$d_4$) $\delta$ ppm: 8.80 (d, *J*= 8.6 Hz, 1H), 8.26 (d, *J* = 7.9 Hz, 1H), 8.17 (d, *J* = 9.2 Hz, 1H), 7.77 (d, *J* = 8.6 Hz, 1H), 7.43 (d, *J* = 8.1 Hz, 1H), 7.26 (d, *J* = 9.0 Hz, 1H), 4.38 - 4.25 (m, 4H), 4.17 - 3.93 (m, 4H), 3.91 - 3.79 (m, 1H), 3.76 - 3.61 (m, 4H), 3.56 - 3.34 (m, 4H), 3.29 - 3.08 (m, 2H), 3.01 - 2.84 (m, 1H), 2.77 (s, 3H), 2.28 - 2.09 (m, 4H), 1.53 (d, *J* = 6.4 Hz, 3H).

**Example 92**

**5-[(*4R*,*9aS*)-8-[[6-[(*1S*,*4S*)-2,5-diazabicyclo[2.2.1]heptan-2-yl]-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile**

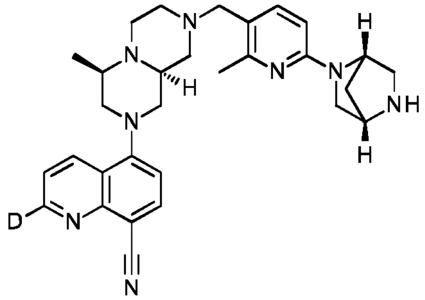

The title compound was prepared in analogy to the preparation of Example **81** by using *tert*-butyl (*1S*,*4S*)-2,5-diazabicyclo[2.2.1]heptane-2-carboxylate instead of *tert*-butyl (*R*)-(3-methylpyrrolidin-3-yl)carbamate. Example **92** was obtained as a light brown solid, 15 mg. LCMS $(M+H)^+$: 510, $^1H$ NMR (400 MHz, METHANOL-$d_4$) $\delta$ ppm: 8.88 (d, *J*= 8.6 Hz, 1H), 8.30 (d, *J* = 7.9 Hz, 1H), 8.21 (d, *J* = 9.4 Hz, 1H), 7.83 (d, *J* = 8.7 Hz, 1H), 7.48 (d, *J* = 8.1 Hz, 1H), 7.15 (br d, *J*= 9.0 Hz, 1H), 5.30 (br s, 1H), 4.74 (s, 1H), 4.39 - 4.08 (m, 4H), 4.04 - 3.86 (m, 3H), 3.82 - 3.60 (m, 5H), 3.58 - 3.36 (m, 5H), 3.30 - 3.15 (m, 1H), 2.79 (s, 3H), 2.47 - 2.37 (m, 1H), 2.31 - 2.21 (m, 1H), 1.55 (d, *J*= 6.4 Hz, 3H).

### Example 93

**4-[(*4R*,*9aR*)-4-methyl-8-(3-methyl-5-piperazin-1-yl-2-pyridyl)-3,4,6,7,9,9a-hexahydro-*1H*-pyrazino[1,2-a]pyrazin-2-yl]-1-methyl-1,8-naphthyridin-2-one**

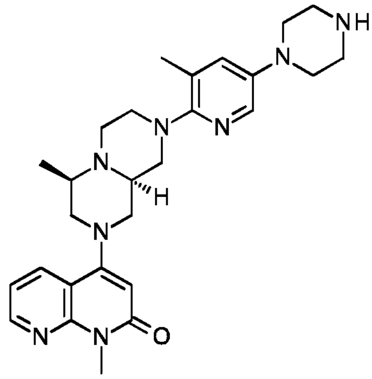

The title compound was prepared according to the following scheme:

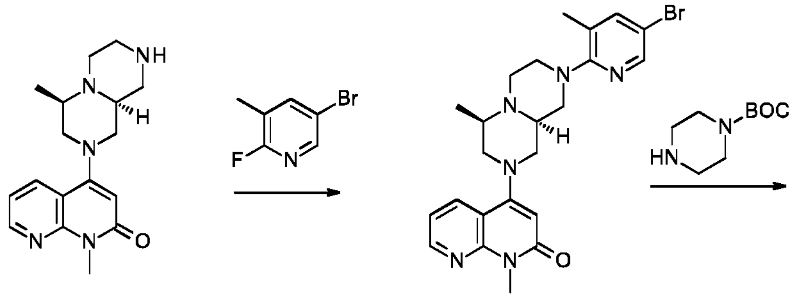

Intermediate L 93a

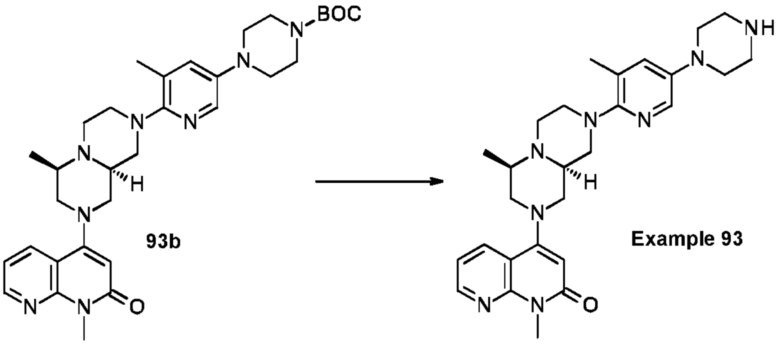

93b Example 93

**Step 1: preparation of 4-[(*4R*,*9aR*)-8-(5-bromo-3-methyl-2-pyridyl)-4-methyl-3,4,6,7,9,9a-hexahydro-*1H*-pyrazino[1,2-a]pyrazin-2-yl]-1-methyl-1,8-naphthyridin-2-one (compound 93a)**

A mixture of 4-[(*4R*,*9aS*)-4-methyl-1,3,4,6,7,8,9,9a-octahydropyrazino[1,2-a]pyrazin-2-yl]-1-methyl-1,8-naphthyridin-2-one (intermediate **L,** 150 mg, 479 μmol), 5-bromo-2-fluoro-3-methylpyridine (136 mg, 718 μmol) and DIPEA (309 mg, 2.39 mmol) in NMP (5 mL) was stirred at 180 °C for 16 hours. Then the reaction was diluted with EA, washed with water and brine, the organic layer was dried and concentrated. The residue was purified by silica gel to give compound **93a** as light brown foam, 100 mg. LCMS $(M+H)^+$: 483.

**Step 2: preparation of *tert*-butyl 4-[6-[(*4R*,*9aR*)-4-methyl-2-(1-methyl-2-oxo-1,8-naphthyridin-4-yl)-3,4,6,7,9,9a-hexahydro-*1H*-pyrazino[1,2-a]pyrazin-8-yl]-5-methyl-3-pyridyl]piperazine-1-carboaylate (compound 93b)**

A mixture of 4-[(*4R*,*9aR*)-8-(5-bromo-3-methyl-2-pyridyl)-4-methyl-3,4,6,7,9,9a-hexahydro-*1H*-pyrazino[1,2-a]pyrazin-2-yl]-1-methyl-1,8-naphthyridin-2-one (compound **93a,** 100 mg, 207 μmol), *tert*-butyl piperazine-1-carboxylate (57.8 mg, 310 μmol), $Cs_2CO_3$ (135 mg, 414 μmol) and Ruphos Pd G2 (16.1 mg, 20.7 μmol) in dioxane (5 mL) was stirred at 115 °C for 16 hours. Then the reaction was concentrated and the residue was purified by HPLC to give compound **93b** as a light yellow solid, 30 mg. LCMS $(M+H)^+$: 589.

**Step 3: preparation of 4-[(*4R*,*9aR*)-4-methyl-8-(3-methyl-5-piperazin-1-yl-2-pyridyl)-3,4,6,7,9,9a-hexahydro-*1H*-pyrazino[1,2-a]pyrazin-2-yl]-1-methyl-1,8-naphthyridin-2-one (Example 93)**

A mixture of *tert*-butyl 4-[6-[(*4R*,*9aR*)-4-methyl-2-(1-methyl-2-oxo-1,8-naphthyridin-4-yl)-3,4,6,7,9,9a-hexahydro-*1H*-pyrazino[1,2-a]pyrazin-8-yl]-5-methyl-3-pyridyl]piperazine-1-carboxylate (compound **93b,** 15 mg, 25.5 μmol) in 1 M HCl in EA (2 mL) was stirred at rt for 16 hours, then the reaction was concentrated to give Example **93** as a yellow solid, 14 mg. LCMS $(M+H)^+$: 489, $^1H$ NMR (400 MHz, METHANOL-$d_4$) $\delta$ ppm: 8.74 - 8.66 (m, 1H), 8.44 - 8.37 (m, 1H), 8.14 (d, *J* = 2.6 Hz, 1H), 7.85 (d, *J* = 2.9 Hz, 1H), 7.45 - 7.35 (m, 1H), 6.34 (s, 1H), 4.25 - 4.15 (m, 1H), 4.08 (br d, *J*= 12.3 Hz, 1H), 4.01 - 3.87 (m, 3H), 3.87 - 3.77 (m, 6H), 3.69 - 3.59 (m, 5H), 3.56 - 3.41 (m, 5H), 3.40 - 3.33 (m, 2H), 2.55 (s, 3H), 1.59 (d, *J*= 6.5 Hz, 3H).

**Example 94**

**5-[(*4R,9aS*)-8-[[6-[(*1S*,*4S*)-2,5-diazabicyclo[2.2.1]heptan-2-yl]-4-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile**

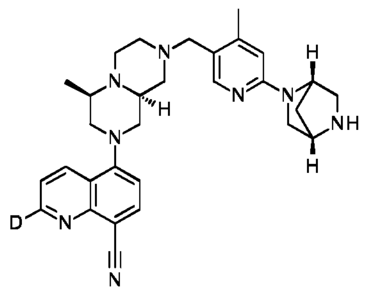

The title compound was prepared in analogy to the preparation of Example **81** by using tert-butyl (IS, 4S)-2,5-diazabicyclo[2.2. 1]heptane-2-carboxylate instead of *tert*-butyl (R)-(3-methylpyrrolidin-3-yl)carbamate and compound **87b** instead of compound **80b.** Example **94** was obtained as an orange solid, 33 mg. LCMS $(M+H)^+$: 510, $^1H$ NMR (400 MHz, METHANOL-$d_4$) $\delta$ ppm: 8.94 (d, *J* = 8.6 Hz, 1H), 8.33 (d, *J* = 8.1 Hz, 1H), 8.13 (br s, 1H), 7.87 (d, *J* = 8.7 Hz, 1H), 7.49 (d, *J* = 8.1 Hz, 1H), 7.23 (br s, 1H), 5.19 (s, 1H), 4.74 (s, 1H), 4.27 - 4.02 (m, 4H), 3.99 - 3.84 (m, 3H), 3.81 - 3.70 (m, 2H), 3.69 - 3.56 (m, 3H), 3.54 - 3.36 (m, 5H), 3.12 - 2.97 (m, 1H), 2.66 (s, 3H), 2.46 - 2.38 (m, 1H), 2.31 - 2.20 (m, 1H), 1.56 (d, *J* = 6.5 Hz, 3H).

**Example 95**

**5-[(*4R*,*9aR*)-8-[5-[(*1S*,*4S*)-2,5-diazabicyclo[2.2.1]heptan-2-yl]-3-methyl-2-pyridyl]-4-methyl-3,4,6,7,9,9a-hexahydro-1H-pyrazino[1,2-a]pyrazin-2-yl]-2-deuterio-quinoline-8-carbonitrile**

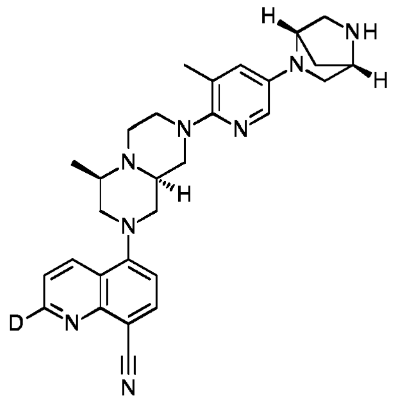

The title compound was prepared in analogy to the preparation of Example **93** by using *tert*-butyl (*1S*,*4S*)-2,5-diazabicyclo[2.2.1]heptane-2-carboxylate instead of *tert*-butyl piperazine-1-carboxylate and intermediate **G** instead of intermediate **L.** Example **95** was obtained as a dark brown solid, 8 mg. LCMS $(M+H)^+$: 496, $^1H$ NMR (400 MHz, METHANOL-$d_4$) $\delta$ ppm: 8.80 (d, *J* = 8.7 Hz, 1H), 8.25 (d, *J* = 8.1 Hz, 1H), 7.81 - 7.70 (m, 2H), 7.62 (d, *J* = 3.1 Hz, 1H), 7.44 (d, *J* = 7.9 Hz, 1H), 4.83 (s, 1H), 4.60 (s, 1H), 4.31 - 4.15 (m, 1H), 4.07 (br d, *J* = 12.3 Hz, 1H), 4.01 - 3.94 (m, 1H), 3.84 - 3.66 (m, 6H), 3.63 - 3.35 (m, 7H), 2.52 (s, 3H), 2.36 - 2.23 (m, 1H), 2.13 (br d, *J* = 11.5 Hz, 1H), 1.57 (d, *J* = 6.5 Hz, 3H).

**Example 96**

**5-[(*4R,9aR*)-8-[5-[(*3R,4S*)-3-amino-4-methoxy-pyrrolidin-1-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

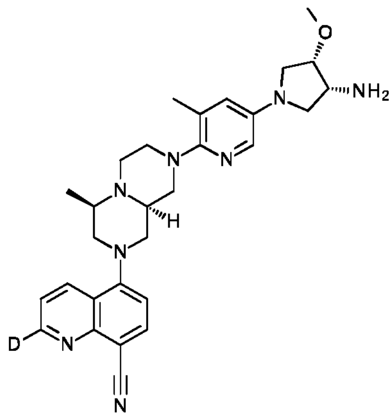

The title compound was prepared in analogy to the preparation of Example **93** by using *tert*-butyl *N*-[(*3R,4S*)-4-methoxypyrrolidin-3-yl]carbamate instead of *tert*-butyl piperazine-1-carboxylate and intermediate **G** instead of intermediate **L.** Example **96** was obtained as a dark brown solid, 14 mg. LCMS $(M+H)^+$: 514, $^1$H NMR (400 MHz, METHANOL-$d_4$) $\delta$ ppm: 8.86 (d, *J*= 8.6 Hz, 1H), 8.27 (d, *J*= 8.1 Hz, 1H), 7.79 (d, *J*= 8.6 Hz, 1H), 7.71 (d, *J*= 2.8 Hz, 1H), 7.52 (d, *J*= 2.9 Hz, 1H), 7.46 (d, *J*= 8.1 Hz, 1H), 4.35 - 4.19 (m, 2H), 4.16 - 4.02 (m, 2H), 4.02 - 3.93 (m, 1H), 3.87 - 3.72 (m, 6H), 3.70 - 3.56 (m, 3H), 3.55 - 3.36 (m, 7H), 2.53 (s, 3H), 1.58 (d, *J*= 6.5 Hz, 3H).

**Example 97**

**5-[(*4R*,*9aR*)-8-[5-[(*3R*)-3-amino-3-methyl-pyrrolidin-1-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

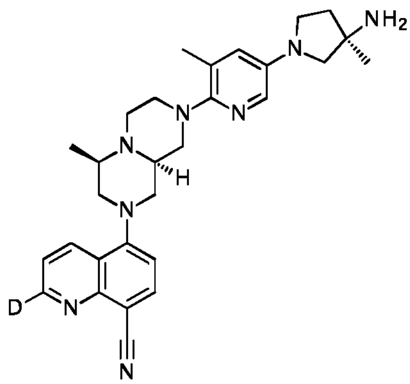

The title compound was prepared in analogy to the preparation of Example **93** by using *tert*-butyl (*R*)-(3-methylpyrrolidin-3-yl)carbamate instead of *tert*-butyl piperazine-1-carboxylate and intermediate **G** instead of intermediate **L.** Example **97** was obtained as a light brown solid, 5 mg. LCMS $(M+H)^+$: 498, $^1$H NMR (400 MHz, METHANOL-$d_4$) $\delta$ ppm 8.79 (d, *J* = 8.6 Hz, 1H), 8.25 (d, *J* = 7.9 Hz, 1H), 7.74 (d, *J* = 8.7 Hz, 1H), 7.69 (d, *J* = 2.7 Hz, 1H), 7.51 (d, *J* = 3.1 Hz, 1H), 7.44 (d, *J* = 8.1 Hz, 1H), 4.28 - 4.16 (m, 1H), 4.12 - 4.04 (m, 1H), 4.02 - 3.92 (m, 1H), 3.83 - 3.69 (m, 6H), 3.67 - 3.36 (m, 7H), 2.53 (s, 3H), 2.34 (br d, *J*= 7.9 Hz, 2H), 1.65 - 1.51 (m, 6H).

**Example 98**

**5-[(*4R,9aS*)-8-[[6-[(*4aR,7aR*)-3,4,4a,5,7,7a-hexahydro-*2H*-pyrrolo[3,4-b][1,4]oxazin-6-yl]-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile**

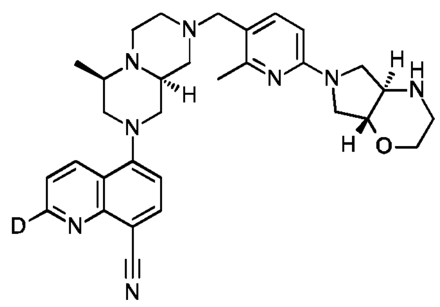

The title compound was prepared in analogy to the preparation of Example **81** by using *tert*-butyl (*4aR*,*7aR*)-3,4a,5,6,7,7a-hexahydro-*2H*-pyrrolo[3,4-b][1,4]oxazine-4-carboxylate (CAS: 1932337-68-2, catalog: PBXA8123, vendor: Pharmablock) instead of *tert*-butyl (R)-(3-methylpyrrolidin-3-yl)carbamate. Example **98** was obtained as a light yellow solid, 30 mg. LCMS $(M+H)^+$: 540, $^1H$ NMR (400 MHz, METHANOL-$d_4$) $\delta$ ppm: 8.58 (d, *J*= 8.6 Hz, 1H), 8.12 (d, *J* = 7.9 Hz, 1H), 7.61 (d, *J* = 8.6 Hz, 1H), 7.37 (d, *J* = 8.6 Hz, 1H), 7.21 (d, *J* = 8.1 Hz, 1H), 6.26 (d, *J*= 8.4 Hz, 1H), 4.05 - 3.92 (m, 1H), 3.84 - 3.69 (m, 3H), 3.68 - 3.55 (m, 1H), 3.49 - 3.36 (m, 3H), 3.28 - 3.06 (m, 4H), 3.05 - 2.88 (m, 4H), 2.85 - 2.67 (m, 5H), 2.44 (s, 3H), 2.35 - 2.20 (m, 2H), 1.97 (t, *J*= 10.0 Hz, 1H), 1.15 (d, *J*= 6.1 Hz, 3H).

**Example 99**

**5-[(*4R,9aR*)-8-[5-[(*3R,4R*)-3-amino-4-methoxy-pyrrolidin-1-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

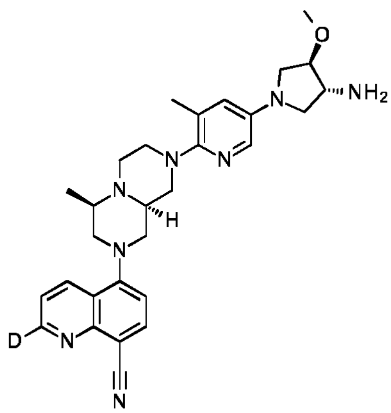

The title compound was prepared in analogy to the preparation of Example **93** by using *tert*-butyl *N*-[(*3R*,*4R*)-4-methoxypyrrolidin-3-yl]carbamate instead of *tert*-butyl piperazine-1-carboxylate and intermediate **G** instead of intermediate **L.** Example **99** was obtained as an orange solid, 5 mg. LCMS $(M+H)^+$: 514, $^1H$ NMR (400 MHz, METHANOL-d4) $\delta$ ppm 8.79 (d, *J* = 8.6 Hz, 1H), 8.25 (d, *J*= 7.9 Hz, 1H), 7.77 - 7.66 (m, 2H), 7.54 (d, *J*= 2.9 Hz, 1H), 7.44 (d, *J* = 8.1 Hz, 1H), 4.27 - 4.16 (m, 2H), 4.14 - 4.04 (m, 1H), 4.04 - 3.94 (m, 2H), 3.94 - 3.86 (m, 1H), 3.83 - 3.70 (m, 6H), 3.61 - 3.35 (m, 9H), 2.53 (s, 3H), 1.57 (d, J = 6.4 Hz, 3H).

**Example 100**

**5-[(*4R,9aR*)-8-[5-[(*4aR,7aR*)-3,4,4a,5,7,7a-hexahydro-*2H*-pyrrolo[3,4-b][1,4]oxazin-6-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

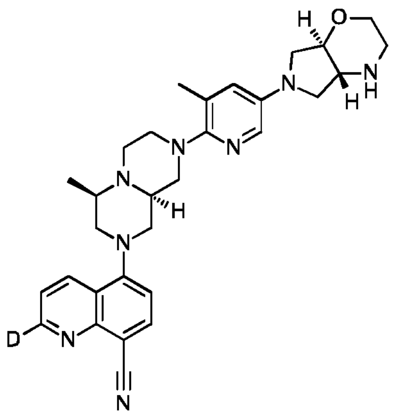

The title compound was prepared in analogy to the preparation of Example **93** by using *tert*-butyl (*4aR*,*7aR*)-3,4a,5,6,7,7a-hexahydro-*2H*-pyrrolo[3,4-b][1,4]oxazine-4-carboxylate (CAS: 1932337-68-2, catalog: PBXA8123, vendor: Pharmablock) instead of *tert*-butyl piperazine-1-carboxylate and intermediate **G** instead of intermediate **L.** Example **100** was obtained as an orange solid, 5 mg. LCMS $(M+H)^+$: 526, $^1H$ NMR (400 MHz, METHANOL-$d_4$) $\delta$ ppm 8.75 (d, *J*= 8.6 Hz, 1H), 8.23 (d, *J*= 7.9 Hz, 1H), 7.72 (d, *J*= 8.6 Hz, 1H), 7.61 (d, *J*= 2.7 Hz, 1H), 7.52 (d, *J* = 2.9 Hz, 1H), 7.42 (d, *J* = 7.9 Hz, 1H), 4.32 - 4.11 (m, 3H), 4.10 - 3.92 (m, 3H), 3.90 - 3.60 (m, 8H), 3.58 - 3.35 (m, 8H), 2.51 (s, 3H), 1.56 (d, *J*= 6.5 Hz, 3H).

**Example 101**

**5-[(*4R,9aR*)-8-[5-(6-amino-2-azaspiro[3.3]heptan-2-yl)-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

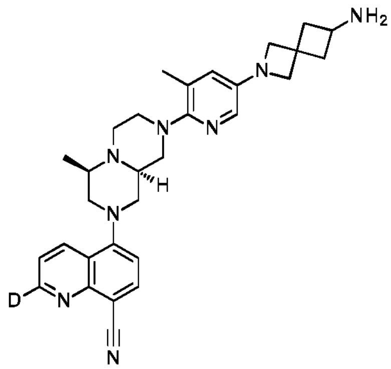

The title compound was prepared in analogy to the preparation of Example **93** by using *tert*-butyl *N*-(2-azaspiro[3.3]heptan-6-yl)carbamate instead of *tert*-butyl piperazine-1-carboxylate, intermediate **G** instead of intermediate **L** and TFA/DCM (1:2) instead of 1 M HCl in EA. Example **101** was obtained as a light yellow solid, 30 mg. LCMS $(M+H)^+$: 510, $^1H$ NMR (400 MHz, METHANOL-d4) $\delta$ ppm: 8.72 (d, *J*= 8.7 Hz, 1H), 8.23 (d, *J*= 7.9 Hz, 1H), 7.71 (d, *J* = 8.7 Hz, 1H), 7.47 - 7.35 (m, 2H), 7.07 (d, *J*= 2.6 Hz, 1H), 4.11 (br s, 1H), 4.06 - 3.99 (m, 3H), 3.92 (s, 3H), 3.82 - 3.71 (m, 3H), 3.66 - 3.53 (m, 2H), 3.46 - 3.37 (m, 2H), 3.31 - 3.21 (m, 3H), 2.75 - 2.65 (m, 2H), 2.49 - 2.36 (m, 5H), 1.53 (d, *J* = 6.5 Hz, 3H).

**Example 102**

**5-[(*4R,9aR*)-4-methyl-8-[3-methyl-5-(6-methyl-2,6-diazaspiro[3.3]heptane-2-carbonyl)-2-pyridyl]-3,4,6,7,9,9a-hexahydro-*1H*-pyrazino[1,2-a]pyrazin-2-yl]-2-deuterio-quinoline-8-carbonitrile**

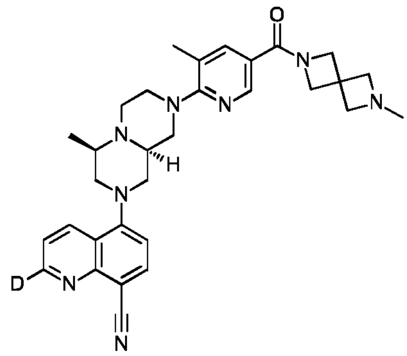

The title compound was prepared according to the following scheme:

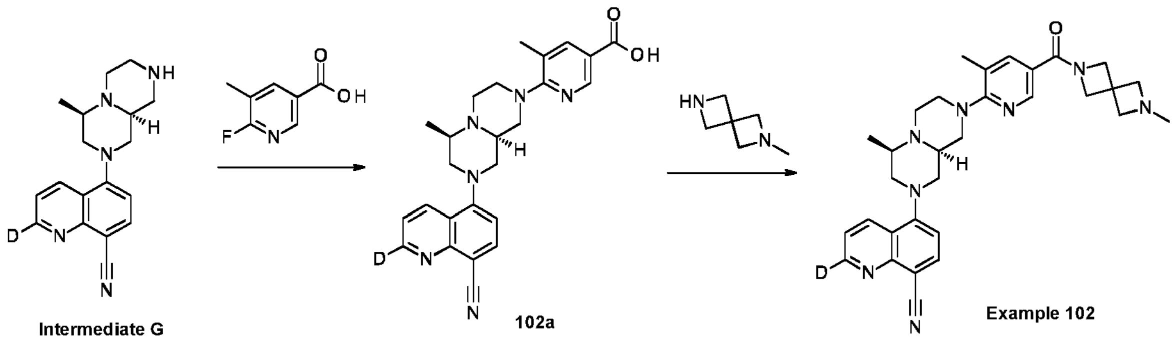

Intermediate G

102a

Example 102

**Step 1: preparation of 6-[(*4R*,*9aR*)-2-(8-cyano-2-deuterio-5-quinolyl)-4-methyl-3,4,6,7,9,9a-hexahydro-*1H*-pyrazino[1,2-a]pyrazin-8-yl]-5-methyl-pyridine-3-carboxylic acid (compound 102a)**

A mixture of 5-[(*4R*,*9aS*)-4-methyl-1,3,4,6,7,8,9,9a-octahydropyrazino[1,2-a]pyrazin-2-yl]-2-deuterio-quinoline-8-carbonitrile **(intermediate G,** 100 mg, 324 μmol), 6-fluoro-5-methylnicotinic acid (101 mg, 648 μmol) and DIPEA (210 mg, 1.62 mmol) in DMSO (5 mL) was stirred at 120 °C for 16 hours, then the reaction was purified by flash preparation (TFA in water, MeCN) to give compound **102a** as a light yellow solid, 100 mg, LCMS $(M+H)^+$: 444.

**Step 2: preparation of 5-[(*4R*,*9aR*)-4-methyl-8-[3-methyl-5-(6-methyl-2,6-diazaspiro[3.3]heptane-2-carbonyl)-2-pyridyl]-3,4,6,7,9,9a-hexahydro-*1H*-pyrazino[1,2-a]pyrazin-2-yl]-2-deuterio-quinoline-8-carbonitrile (Example 102)**

A mixture of 6-[(*4R*,*9aR*)-2-(8-cyano-2-deuterio-5-quinolyl)-4-methyl-3,4,6,7,9,9a-hexahydro-*1H*-pyrazino[1,2-a]pyrazin-8-yl]-5-methyl-pyridine-3-carboxylic acid (compound **102a,** 100 mg, 225 μmol), 2-methyl-2,6-diazaspiro[3.3]heptane (50.6 mg, 451 μmol), HATU (103 mg, 271 μmol) and DIPEA (87.4 mg, 676 μmol) in DMF (5 mL) was stirred at rt for 1 hour, then the reaction was diluted with EA, washed with water and brine. The organic layer was dried and concentrated, the residue was purified by HPLC-preparation to give Example **102** as a light yellow solid, 45 mg. LCMS $(M+H)^+$: 538, $^1$H NMR (400 MHz, METHANOL-$d_4$) $\delta$ ppm: 8.62 (d, $J$ = 8.6 Hz, 1H), 8.35 (d, $J$ = 2.1 Hz, 1H), 8.11 (d, $J$ = 8.1 Hz, 1H), 7.74 (d, $J$ = 1.7 Hz, 1H), 7.62 (d, $J$ = 8.6 Hz, 1H), 7.23 (d, $J$ = 8.1 Hz, 1H), 4.48 (br s, 2H), 4.21 (br s, 2H), 3.74 - 3.64 (m, 1H), 3.58 (br d, $J$= 12.1 Hz, 1H), 3.49 - 3.39 (m, 6H), 3.35 (br d, $J$= 11.4 Hz, 1H), 3.15 - 3.01 (m, 1H), 2.94 - 2.74 (m, 5H), 2.44 - 2.36 (m, 1H), 2.34 (s, 3H), 2.31 (s, 3H), 1.19 (d, $J$= 5.6 Hz, 3H).

**Example 103**

**5-[(*4S,9aR*)-4-methyl-8-[3-methyl-5-(6-methyl-2,6-diazaspiro[3.3]heptane-2-carbonyl)-2-pyridyl]-3,4,6,7,9,9a-hexahydro-*1H*-pyrazino[1,2-a]pyrazin-2-yl]-2-deuterio-quinoline-8-carbonitrile**

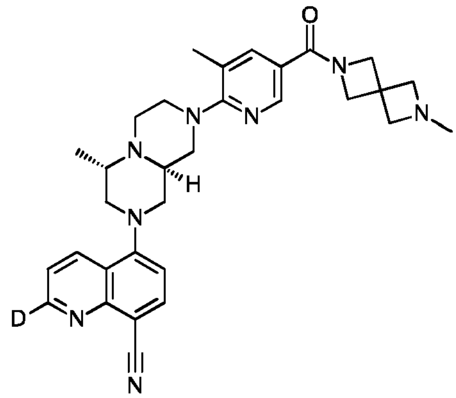

The title compound was prepared in analogy to the preparation of Example **102** by using **intermediate K** instead of **intermediate G.** Example **103** was obtained as a light yellow solid, 35 mg. LCMS $(M+H)^+$: 538, $^1H$ NMR (400 MHz, METHANOL-$d_4$) $\delta$ ppm: 8.73 (d, *J*= 8.6 Hz, 1H), 8.36 (d, *J*= 2.3 Hz, 1H), 8.15 (d, *J*= 7.9 Hz, 1H), 7.78 - 7.72 (m, 1H), 7.65 (d, *J*= 8.6 Hz, 1H), 7.27 (d, *J*= 8.1 Hz, 1H), 4.49 (br s, 2H), 4.22 (br s, 2H), 3.69 - 3.54 (m, 2H), 3.48 (s, 4H), 3.44 - 3.32 (m, 5H), 3.16 - 2.97 (m, 2H), 2.86 - 2.72 (m, 3H), 2.36 (s, 3H), 2.34 (s, 3H), 1.46 (d, *J* = 6.5 Hz, 3H).

**Example 105**

**5-[(*4R,9aS*)-8-[2-[(*3R*)-3-amino-3-methyl-pyrrolidin-1-yl]-4-pyridyl]-4-methyl-3,4,6,7,9,9a-hexahydro-*1H*-pyrazino[1,2-a]pyrazin-2-yl]quinoline-8-carbonitrile**

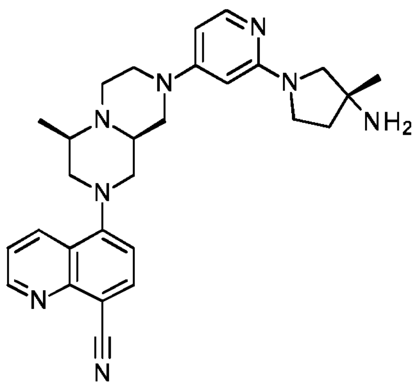

The title compound was prepared in analogy to the preparation of Example **32** by using *tert*-butyl *N*-[(*3R*)-3-methylpyrrolidin-3-yl]carbamate instead of *tert*-butyl piperazine-1-carboxylate. Example **105** (20 mg) was obtained as an orange solid. LCMS $(M+H)^+$: 483. $^1H$ NMR (400 MHz, METHANOL-d4) $\delta$ ppm: 9.06 (dd, *J* = 1.6, 4.3 Hz, 1H), 8.77 (dd, *J* = 1.7, 8.6 Hz, 1H), 8.26 (d, *J*= 7.9 Hz, 1H), 7.78 - 7.72 (m, 2H), 7.46 (d, *J*= 8.1 Hz, 1H), 6.83 (dd, *J*= 2.2, 7.6 Hz, 1H), 6.19 (d, *J*= 2.2 Hz, 1H), 4.68 - 4.52 (m, 2H), 4.15 - 4.02 (m, 2H), 3.95 - 3.83 (m, 4H), 3.82 - 3.64 (m, 4H), 3.48 - 3.35 (m, 4H), 2.45 - 2.36 (m, 2H), 1.64 (s, 3H), 1.58 (d, *J*= 6.5 Hz, 3H).

**Example 106**

**5-[(*4S*,*9aS*)-4-methyl-8-[4-[(*2R*)-morpholin-2-yl]phenyl]-3,4,6,7,9,9a-hexahydro-*1H*-pyrazino[1,2-a]pyrazin-2-yl]-2-deuterio-quinoline-8-carbonitrile**

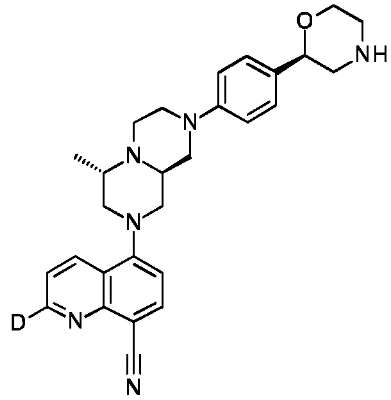

The title compound was prepared in analogy to the preparation of Example **2** by using 5-[(*4S*,*9aS*)-4-methyl-1,3,4,6,7,8,9,9a-octahydropyrazino[1,2-a]pyrazin-2-yl]-2-deuterio-quinoline-8-carbonitrile (Intermediate **K**) instead of 5-[*cis*-4-methyl-1,3,4,6,7,8,9,9a-octahydropyrazino[1,2-a]pyrazin-2-yl]quinoline-8-carbonitrile (Intermediate **A**), and *tert*-butyl (*2R*)-2-(4-bromophenyl)morpholine-4-carboxylate (CAS:1312566-00-9, procedure see Patent WO 2014041007 A1 20140320) instead of *tert*-butyl 5-bromo-3,4-dihydro-*1H*-isoquinoline-2-carboxylate (Compound **2a**). Example **106** (11 mg) was obtained as an orange solid. LCMS $(M+H)^+$: 470. $^1H$ NMR (400 MHz, METHANOL-d4) $\delta$ ppm: 8.71 (d, *J* = 8.6 Hz, 1H), 8.13 (d, *J* = 8.1 Hz, 1H), 7.65 (d, *J*= 8.6 Hz, 1H), 7.27 - 7.21 (m, 3H), 6.97 (d, *J*= 8.8 Hz, 2H), 4.38 (dd, *J* = 2.3, 10.5 Hz, 1H), 3.95 (dd, *J* = 2.4, 11.6 Hz, 1H), 3.73 (dt, *J* = 3.2, 11.4 Hz, 1H), 3.62 (br t, *J* = 12.8 Hz, 2H), 3.44 (br d, *J*= 11.4 Hz, 1H), 3.34 (br d, *J* = 2.6 Hz, 2H), 3.30 - 3.25 (m, 2H), 3.03 - 2.79 (m, 6H), 2.78 - 2.66 (m, 2H), 2.51 (br, 1H), 1.43 (d, *J*= 6.5 Hz, 3H).

**Example 107**

**5-[(*4S*,*9aS*)-4-methyl-8-[4-[(*2S*)-morpholin-2-yl]phenyl]-3,4,6,7,9,9a-hexahydro-*1H*-pyrazino[1,2-a]pyrazin-2-yl]-2-deuterio-quinoline-8-carbonitrile**

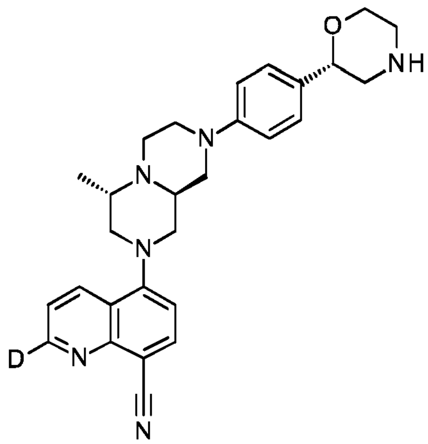

The title compound was prepared in analogy to the preparation of Example **2** by using 5-[(*4S*,*9aS*)-4-methyl-1,3,4,6,7,8,9,9a-octahydropyrazino[1,2-a]pyrazin-2-yl]-2-deuterio-quinoline-8-carbonitrile (Intermediate **K**) instead of 5-[*cis*-4-methyl-1,3,4,6,7,8,9,9a-octahydropyrazino[1,2-a]pyrazin-2-yl]quinoline-8-carbonitrile (Intermediate **A**) and

*tert*-butyl (2S)-2-(4-bromophenyl)morpholine-4-carboxylate (CAS:1131220-37-5, procedure see Patent WO 2014041007 A1 20140320) instead of *tert*-butyl 5-bromo-3,4-dihydro-*1H*-isoquinoline-2-carboxylate (Compound **2a**). Example **107** (10 mg) was obtained as an orange solid. LCMS $(M+H)^+$: 470. $^1H$ NMR (400 MHz, METHANOL-d4) $\delta$ ppm: 8.83 (d, *J*=8.4 Hz, 1H), 8.26 (d, *J*=7.9 Hz, 1H), 7.77 (d, *J*=8.6 Hz, 1H), 7.45 (d, *J*=7.9 Hz, 1H), 7.38 (br d, *J*=8.7 Hz, 2H), 7.12 (d, *J*=8.7 Hz, 2H), 4.8-4.8 (m, 1H), 4.4-4.3 (m, 1H), 4.24 (dd, *J*=3.3, 13.2 Hz, 1H), 4.1-3.9 (m, 4H), 3.9-3.6 (m, 6H), 3.5-3.4 (m, 2H), 3.3-3.2 (m, 1H), 3.2-3.1 (m, 2H), 2.98 (br, 1H), 1.82 (d, *J*=6.8 Hz, 3H).

**Example 108**

**5-[(*4S,9aR*)-8-[5-(3,6-diazabicyclo[3.1.1]heptan-3-yl)-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

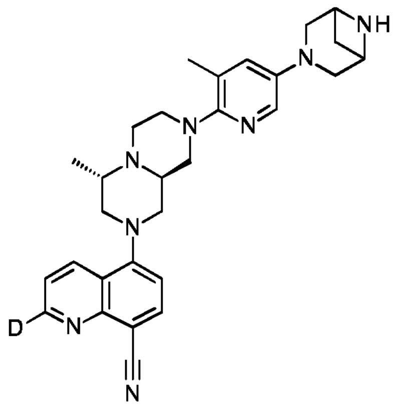

The title compound was prepared according to the following scheme:

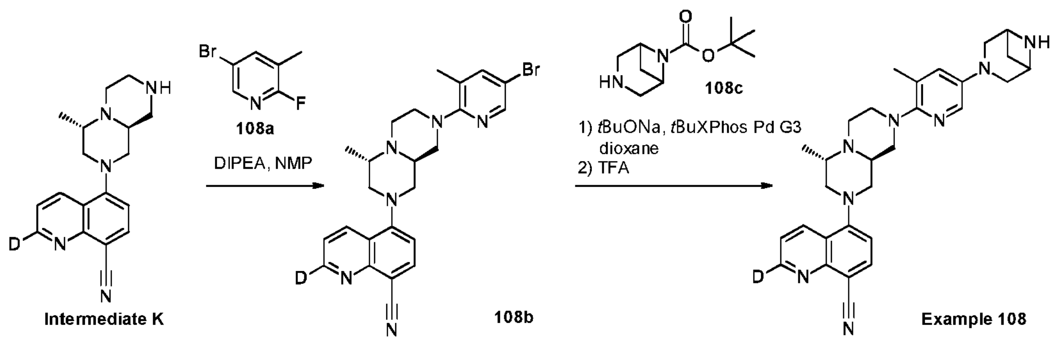


Intermediate K

108b

Example 108

**Step 1: Preparation of 5-[(*4S*,*9aR*)-8-(5-bromo-3-methyl-2-pyridyl)-4-methyl-3,4,6,7,9,9a-hexahydro-*1H*-pyrazino[1,2-a]pyrazin-2-yl]-2-deuterio-quinoline-8-carbonitrile (compound 108b)**

A solution of 5-bromo-2-fluoro-3-methylpyridine (compound **108a,** 462 mg, 2.43 mmol), 5-[(*4S*,*9aS*)-4-methyl-1,3,4,6,7,8,9,9a-octahydropyrazino[1,2-a]pyrazin-2-yl]-2-deuterio-quinoline-8-carbonitrile (Intermediate **K,** 500 mg, 1.62 mmol) and DIPEA (1.1 g, 8.1 mmol) in NMP (6 mL) was stirred at 185 °C for 20 hours, then the reaction was diluted with EtOAc (40 mL), washed with water. The organic layer was dried and concentrated, the residue was purified by flash column chromatography to give compound **108b** (640 mg) as an orange oil. LCMS$(M+1)^+$: 478, LCMS$(M+3)^+$: 480.

**Step 2: Preparation of 5-[(*4S*,*9aR*)-8-[5-(3,6-diazabicyclo[3.1.1]heptan-3-yl)-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile (compound 108)**

To a solution of 5-[(*4S*,*9aR*)-8-(5-bromo-3-methyl-2-pyridyl)-4-methyl-3,4,6,7,9,9a-hexahydro-*1H*-pyrazino[1,2-a]pyrazin-2-yl]-2-deuterio-quinoline-8-carbonitrile (compound **108b,** 100 mg, 209 μmol) in dioxane (4 mL) was added tert-butyl 3,6-diazabicyclo[3.1.1]-heptane-6-carboxylate (83 mg, 418 μmol) and tBuONa (41 mg, 418 μmol). The suspension was bubbled with $N_2$ for 5 minutes, then tBuXPhos Pd G3 (33 mg, 41.8 μmol) was added. After the reaction

mixture was heated at 110 °C overnight, it was filtered and the filtrate was concentrated. The residue was dissolved in DCM/TFA (4 mL, 1:1), after stirring at r.t. for 10 mins, the reaction mixture was concentrated and the residue was purified by prep-HPLC to give Example **108** (11 mg) as a light yellow foam. LCMS $(M+H)^+$: 496. $^1$H NMR (400 MHz, METHANOL-d4) $\delta$ ppm: 8.79 (d, *J*= 8.6 Hz, 1H), 8.24 (d, *J*= 7.9 Hz, 1H), 7.80 (d, *J*= 2.6 Hz, 1H), 7.73 (d, *J*= 8.7 Hz, 1H), 7.42 (d, *J*= 8.2 Hz, 1H), 7.22 (d, *J*= 2.8 Hz, 1H), 4.58 (d, *J*= 6.4 Hz, 2H), 4.43 - 4.30 (m, 1H), 3.95 - 3.90 (m, 2H), 3.84 - 3.78 (m, 2H), 3.52 - 3.43 (m, 8H), 3.30 - 3.24 (m, 2H), 3.12 - 3.03 (m, 2H), 2.43 (s, 3H), 2.05 (br, 1H), 1.85 (br, 3H).

**Example 109**

**5-[(*4R,9aR*)-8-[5-[2-[(*3S,4S*)-3-amino-4-methoxy-pyrrolidin-1-yl]ethyl]-2-pyridyl]-4-methyl-3,4,6,7,9,9a-hexahydro-*1H*-pyrazino[1,2-a]pyrazin-2-yl]-2-deuterio-quinoline-8-carbonitrile**

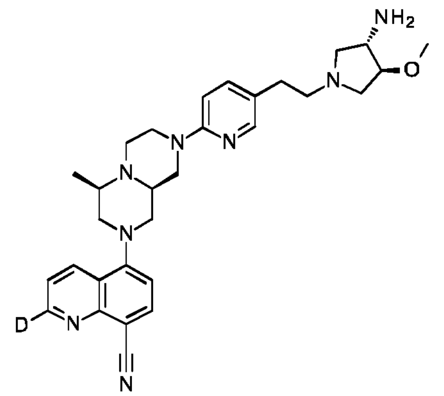

The title compound was prepared according to the following scheme:

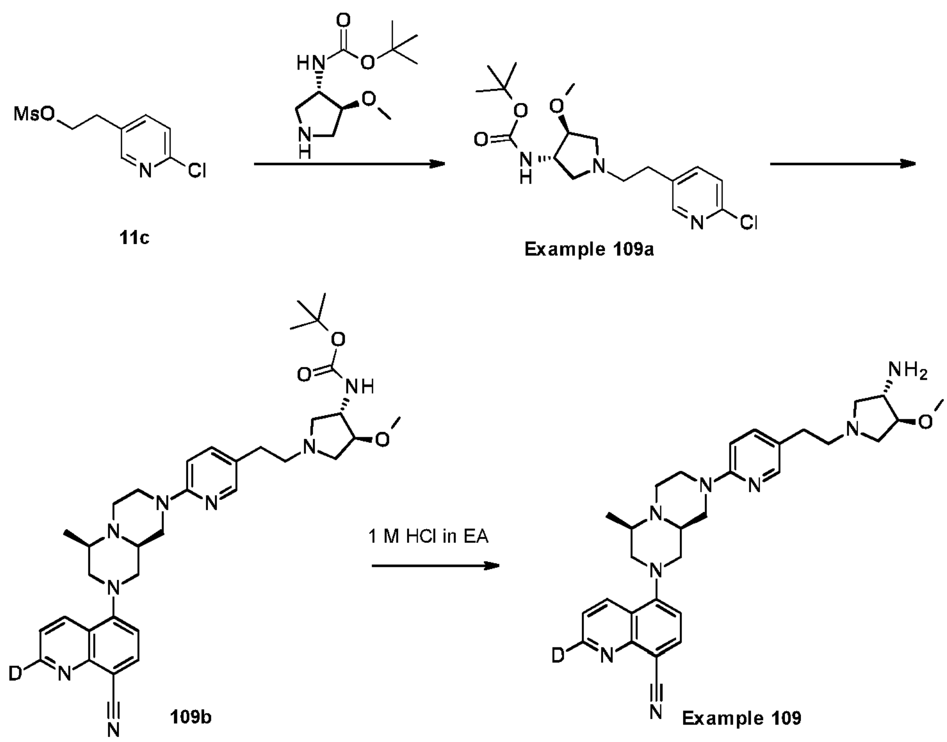


11c

Example 109a

109b

Example 109

**Step 1: Preparation of *tert*-butyl *N*-[(*3S*,*4S*)-1-[2-(6-chloro-3-pyridyl)ethyl]-4-methoxy-pyrrolidin-3-yl]carbamate (compound 109a)**

A mixture of 2-(6-chloro-3-pyridyl)ethyl methanesulfonate (compound **11c,** 288 mg, 1.1 mmol), *tert*-butyl *N*-[(3*S*,4*S*)-4-methoxypyrrolidin-3-yl]carbamate (216 mg, 1 mmol) and potassium carbonate (276 mg, 2 mmol,) in MeCN (6 mL) was stirred at 80 °C for 16 hours. Then the mixture was filtered, the filtrate was concentrated, the residue was purified by silica gel column to give compound **109a** as a yellow oil (278 mg), LCMS $(M)^+$: 356, LCMS$(M+2)^+$: 358.

**Step 2: Preparation of *tert-butyl N*-[(*3S*,*4S*)-1-[2-[6-[(*4R*,*9aR*)-2-(8-cyano-2-deuterio-5-quinolyl)-4-methyl-3,4,6,7,9,9a-hexahydro-*1H*-pyrazino[1,2-a]pyrazin-8-yl]-3-pyridyl]ethyl]-4-methoxy-pyrrolidin-3-yl]carbamate (compound 109b)**

A mixture of tert-butyl *N*-[(*3S*,*4S*)-1-[2-(6-chloro-3-pyridyl)ethyl]-4-methoxy-pyrrolidin-3-yl]carbamate (compound **109a,** 90 mg, 253 μmol), 5-[(*4R*,*9aS*)-4-methyl-1,3,4,6,7,8,9,9a-octahydropyrazino[1,2-a]pyrazin-2-yl]-2-deuterio-quinoline-8-carbonitrile (Intermediate **G,** 60 mg, 195 μmol), cesium carbonate (190 mg, 584 μmol) and RuPhos Pd G2 (28 mg, 39 μmol) in dioxane (6 mL) was stirred at 110 °C overnight. Then the reaction was concentrated and the residue was purified by silica gel column to give compound **109b** as a yellow oil (65 mg), LCMS $(M+H)^+$: 628.

**Step 3: Preparation of 5-[(*4R*,*9aR*)-8-[5-[2-[(*3S*,*4S*)-3-amino-4-methoxy-pyrrolidin-1-yl]ethyl]-2-pyridyl]-4-methyl-3,4,6,7,9,9a-hexahydro-*1H*-pyrazino[1,2-a]pyrazin-2-yl]-2-deuterio-quinoline-8-carbonitrile (compound 109)**

A mixture of tert-butyl *N*-[(*3S*,*4S*)-1-[2-[6-[(*4R*,*9aR*)-2-(8-cyano-2-deuterio-5-quinolyl)-4-methyl-3,4,6,7,9,9a-hexahydro-*1H*-pyrazino[1,2-a]pyrazin-8-yl]-3-pyridyl]ethyl]-4-methoxy-pyrrolidin-3-yl]carbamate (compound **109b,** 65 mg, 104 μmol) in 1 M HCl in EA (5 mL) was stirred at rt for 16 hours. Then the reaction was concentrated, and the residue was lyophilized to give Example **109** as an orange solid (57 mg). LCMS $(M+H)^+$: 528. $^1$H NMR (400 MHz, METHANOL-d4) $\delta$ ppm: 8.83 (d, *J*= 8.6 Hz, 1H), 8.28 (d, *J*= 7.9 Hz, 1H), 8.23 - 8.17 (m, 2H), 7.78 (d, *J*= 8.6 Hz, 1H), 7.57 (d, *J*= 9.3 Hz, 1H), 7.49 (d, *J*= 7.9 Hz, 1H), 4.62 (br dd, *J*= 11.3, 12.3 Hz, 2H), 4.32 (td, *J*= 2.7, 5.2 Hz, 1H), 4.26 - 4.03 (m, 4H), 4.01 - 3.62 (m, 10H), 3.54 - 3.40 (m, 5H), 3.36 - 3.34 (m, 1H), 3.24 - 3.16 (m, 2H), 1.59 (d, *J*= 6.4 Hz, 3H).

**Example 110**

**5-[(*4S*,*9aR*)-8-[5-[(*6R*)-6-amino-1,4-oxazepan-4-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

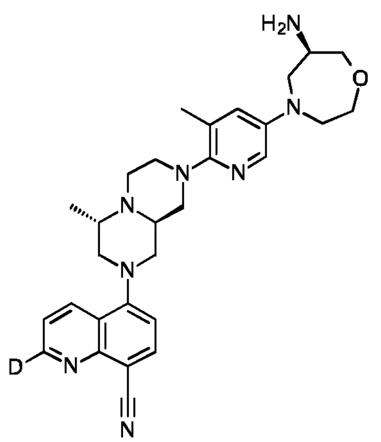

The title compound was prepared in analogy to the preparation of Example **108** by using *tert*-butyl *N*-[(*6R*)-1,4-oxazepan-6-yl]carbamate instead of *tert*-butyl 3,6-diazabicyclo[3.1.1]-heptane-6-carboxylate (Compound **108c**). Example **110** (11 mg) was obtained as an orange solid. LCMS $(M+H)^+$: 514. $^1$H NMR (400 MHz, METHANOL-d4) $\delta$ ppm: 8.82 (br d, *J*= 7.8 Hz, 1H), 8.23 (d, *J*= 7.9 Hz, 1H), 7.77 (s, 2H), 7.73 (d, *J*= 8.6 Hz, 1H), 7.42 (d, *J*= 8.1 Hz, 1H), 4.49 - 4.35 (m, 1H), 4.15 - 4.04 (m, 3H), 3.98 (br dd, *J* = 2.1, 14.1 Hz, 2H), 3.87 (br d, *J* = 3.3 Hz, 1H), 3.85 - 3.81 (m, 2H), 3.80 - 3.66 (m, 9H), 3.62 - 3.54 (m, 3H), 2.49 (s, 3H), 1.83 (br, 3H).

**Example 111**

**5-[(*4S*,*9aR*)-8-[5-[(*1S*,*4S*)-2,5-diazabicyclo[2.2.1]heptan-2-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

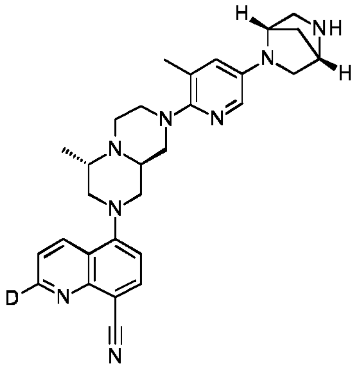

The title compound was prepared in analogy to the preparation of Example **108** by using *tert*-butyl (*1S*,*4S*)-2,5-diazabicyclo[2.2.1]heptane-2-carboxylate instead of *tert*-butyl 3,6-diazabicyclo[3.1.1]-heptane-6-carboxylate (Compound **108c**). Example **111** (11 mg) was obtained as an orange solid. LCMS $(M+H)^+$: 496. $^1H$ NMR (400 MHz, METHANOL-d4) $\delta$ ppm: 9.17 - 8.97 (m, 1H), 8.32 (d, *J* = 7.8 Hz, 1H), 7.88 (br d, *J* = 1.7 Hz, 1H), 7.80 (br s, 1H), 7.62 (br s, 1H), 7.51 (br d, *J* = 7.9 Hz, 1H), 4.86 - 4.81 (m, 2H), 4.60 (br, 1H), 4.57 - 4.45 (m, 1H), 4.02 (br d, *J* = 3.2 Hz, 1H), 3.92 - 3.83 (m, 2H), 3.83 - 3.71 (m, 4H), 3.67 - 3.49 (m, 4H), 3.42 (br, 2H), 3.29 - 3.24 (m, 1H), 2.55 (br, 3H), 2.32 (br d, *J*= 10.9 Hz, 1H), 2.14 (br d, *J*= 11.0 Hz, 1H), 1.83 (br d, *J*= 5.6 Hz, 3H).

**Example 112**

**5-[(*4S*,*9aR*)-8-[6-(3,8-diazabicyclo[3.2.1]octan-3-yl)-3-pyridyl]-4-methyl-3,4,6,7,9,9a-hexahydro-*1H*-pyrazino[1,2-a]pyrazin-2-yl]-2-deuterio-quinoline-8-carbonitrile**

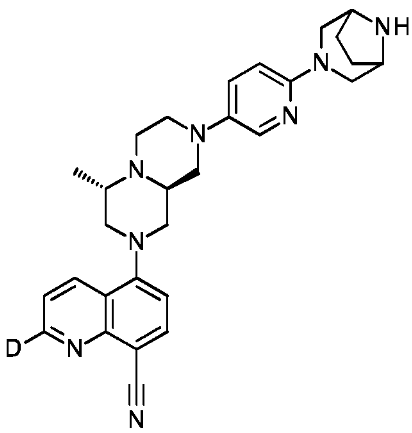

The title compound was prepared in analogy to the preparation of Example **32** by using 5-bromo-2-fluoropyridine instead of 4-bromo-2-fluoropyridine (compound **32a**), *tert*-butyl 3,8-diazabicyclo[3.2.1]octane-8-carboxylate instead of *tert*-butyl piperazine-1-carboxylate, and 5-[(4*S*,9a*S*)-4-methyl-1,3,4,6,7,8,9,9a-octahydropyrazino[1,2-a]pyrazin-2-yl]-2-deuterio-quinoline-8-carbonitrile (Intermediate **K**) instead of 5-[(4*R*,9a*S*)-4-methyl-1,3,4,6,7,8,9,9a-octahydropyrazino[1,2-a]pyrazin-2-yl]quinoline-8-carbonitrile (Intermediate **C**). Example **112** was obtained as an orange solid (90 mg). LCMS $(M+H)^+$: 496, $^1H$ NMR (400 MHz, METHANOL-$d_4$) $\delta$ ppm: 8.79 (d, *J* = 8.6 Hz, 1H), 8.23 (d, *J* = 7.9 Hz, 1H), 8.12 (dd, *J*= 2.9, 9.8 Hz, 1H), 7.75 (d, *J* = 8.7 Hz, 1H), 7.71 (d, *J* = 2.7 Hz, 1H), 7.46 - 7.38 (m, 2H), 4.40 - 4.26 (m, 5H), 4.09 (br d, *J*= 12.6 Hz, 3H), 3.76 (br d, *J*= 12.1 Hz, 3H), 3.61 (br, 4H), 3.46 - 3.36 (m, 1H), 3.22 - 3.03 (m, 2H),

2.25 - 2.17 (m, 2H), 2.16 - 2.09 (m, 2H), 1.80 (br, 3H).

**Example 113**

**4-[(*4S*,*9aR*)-4-methyl-8-[3-methyl-5-(6-methyl-2,6-diazaspiro[3.3]heptan-2-yl)-2-pyridyl]-3,4,6,7,9,9a-hexahydro-*1H*-pyrazino[1,2-a]pyrazin-2-yl]-1-methyl-1,8-naphthyridin-2-one**

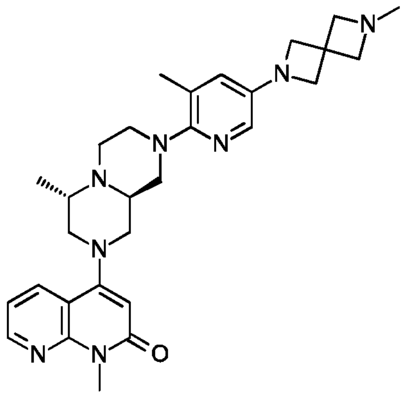

The title compound was prepared in analogy to the preparation of Example **108** by using 2-methyl-2,6-diazaspiro[3.3]heptane instead of *tert*-butyl 3,6-diazabicyclo[3.1.1]-heptane-6-carboxylate, 4-[(*4S*,*9aS*)-4-methyl-1,3,4,6,7,8,9,9a-octahydropyrazino[1,2-a]pyrazin-2-yl]-1-methyl-1,8-naphthyridin-2-one (Intermediate **M**) instead of 5-[(*4S*,*9aS*)-4-methyl-1,3,4,6,7,8,9,9a-octahydropyrazino[1,2-a]pyrazin-2-yl]-2-deuterio-quinoline-8-carbonitrile (Intermediate **K**). Example **113** (15 mg) was obtained as a white solid. LCMS $(M+H)^+$: 515. $^1H$ NMR (400 MHz, METHANOL-d4) $\delta$ ppm: 8.63 (dd, *J* = 1.8, 4.6 Hz, 1H), 8.30 (dd, *J* = 1.8, 8.0 Hz, 1H), 7.35 (d, *J* = 2.8 Hz, 1H), 7.32 (dd, *J* = 4.6, 8.1 Hz, 1H), 6.80 (d, *J* = 2.3 Hz, 1H), 6.16 (s, 1H), 3.97 (s, 4H), 3.91 (s, 4H), 3.76 (s, 3H), 3.37 (br d, *J*= 11.1 Hz, 2H), 3.29 - 3.21 (m, 2H), 3.20 - 3.08 (m, 3H), 3.05 - 2.94 (m, 2H), 2.78 (br d, *J*= 9.0 Hz, 1H), 2.75 - 2.66 (m, 2H), 2.64 (s, 3H), 2.29 (s, 3H), 1.41 (d, *J*= 6.6 Hz, 3H).

**Example 114**

**5-[(*4S*,*9aR*)-8-[5-[(*4aR*,*7aR*)-3,4,4a,5,7,7a-hexahydro-*2H*-pyrrolo[3,4-b][1,4]oxazin-6-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

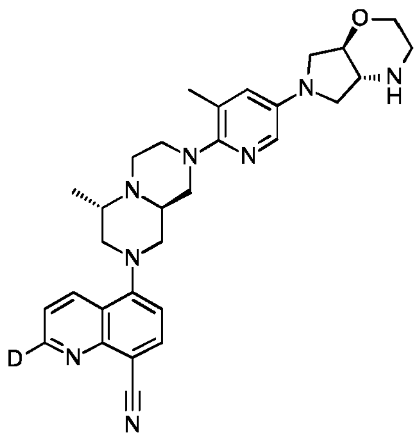

The title compound was prepared in analogy to the preparation of Example **108** by using *tert*-butyl (*4aR*,*7aR*)-3,4a,5,6,7,7a-hexahydro-*2H*-pyrrolo[3,4-b][1,4]oxazine-4-carboxylate instead of *tert*-butyl 3,6-diazabicyclo[3.1.1]-heptane-6-carboxylate. Example **114** (30 mg) was obtained as an orange solid. LCMS $(M+H)^+$: 526. $^1H$ NMR (400 MHz,

METHANOL-d4) $\delta$ ppm: 9.07 - 8.88 (m, 1H), 8.28 (d, *J*= 7.9 Hz, 1H), 7.81 (br d, *J* = 8.6 Hz, 1H), 7.72 (br, 1H), 7.61 - 7.53 (m, 1H), 7.48 (d, *J*= 8.1 Hz, 1H), 4.51 (br t, *J*= 10.1 Hz, 1H), 4.33 - 4.18 (m, 2H), 4.06 (dt, *J* = 2.7, 12.7 Hz, 2H), 3.87 (td, *J*= 8.0, 19.7 Hz, 4H), 3.77 (br d, *J*= 10.8 Hz, 3H), 3.73 - 3.65 (m, 2H), 3.65 - 3.59 (m, 2H), 3.58 - 3.49 (m, 3H), 3.49 - 3.41 (m, 1H), 3.41 - 3.35 (m, 1H), 3.25 (br t, *J*= 12.1 Hz, 1H), 2.56 (s, 3H), 1.86 (d, *J*= 6.7 Hz, 3H).

**Example 115**

**5-[(*4S*,*9aR*)-8-[6-[(*3R*,*4R*)-3-amino-4-methoxy-pyrrolidin-1-yl]-3-pyridyl]-4-methyl-3,4,6,7,9,9a-hexahydro-*1H*-pyrazino[1,2-a]pyrazin-2-yl]-2-deuterio-quinoline-8-carbonitrile**

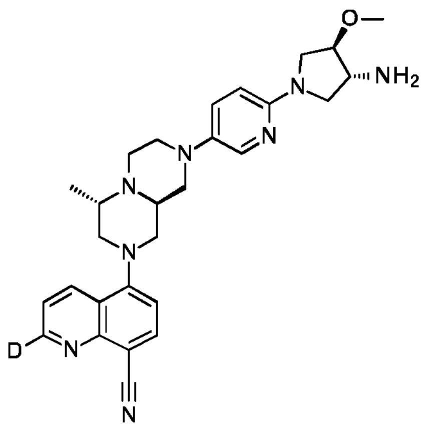

The title compound was prepared in analogy to the preparation of Example **32** by using 5-bromo-2-fluoropyridine instead of 4-bromo-2-fluoropyridine (compound **32a**), *tert*-butyl *N*-[(*3R*,*4R*)-4-methoxypyrrolidin-3-yl]carbamate instead of *tert*-butyl piperazine-1-carboxylate, and 5-[(*4S*,*9aS*)-4-methyl-1,3,4,6,7,8,9,9a-octahydropyrazino[1,2-a]pyrazin-2-yl]-2-deuterio-quinoline-8-carbonitrile (Intermediate **K**) instead of 5-[(4*R*,9a*S*)-4-methyl-1,3,4,6,7,8,9,9a-octahydropyrazino[1,2-a]pyrazin-2-yl]quinoline-8-carbonitrile (Intermediate **C**). Example **115** was obtained as a light yellow foam (13 mg). LCMS $(M+H)^+$: 500, $^1H$ NMR (400 MHz, METHANOL-$d_4$) $\delta$ ppm: 8.72 (d, *J*= 8.6 Hz, 1H), 8.14 (d, *J*= 8.1 Hz, 1H), 7.76 (d, *J*= 2.7 Hz, 1H), 7.65 (d, *J*= 8.6 Hz, 1H), 7.41 (dd, *J*= 2.9, 9.2 Hz, 1H), 7.26 (d, *J*= 8.1 Hz, 1H), 6.51 (d, *J* = 9.2 Hz, 1H), 3.79 - 3.72 (m, 2H), 3.61 (dd, *J* = 5.8, 10.2 Hz, 1H), 3.52 (td, *J* = 2.8, 5.7 Hz, 1H), 3.46 - 3.32 (m, 10H), 3.29 - 3.27 (m, 1H), 3.24 (dd, *J* = 3.2, 10.1 Hz, 1H), 3.07 - 2.97 (m, 1H), 2.92 - 2.81 (m, 2H), 2.75 (t, *J*= 10.8 Hz, 1H), 2.50 (br t, *J* = 10.8 Hz, 1H), 1.44 (d, *J*= 6.5 Hz, 3H).

**Example 117**

**5-[(*4S*,*9aR*)-8-[6-[2-[(*3R*,*4S*)-3-amino-4-fluoro-pyrrolidin-1-yl]ethyl]-3-pyridyl]-4-methyl-3,4,6,7,9,9a-hexahydro-1*H*-pyrazino[1,2-a]pyrazin-2-yl]-2-deuterio-quinoline-8-carbonitrile**

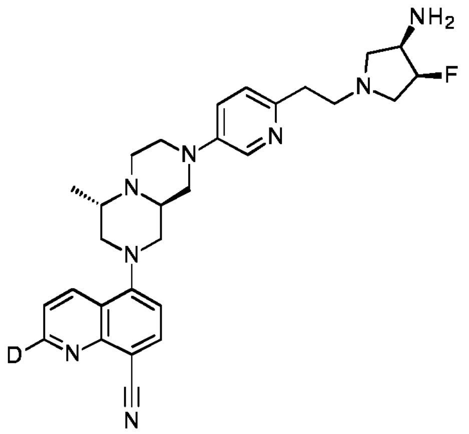

The title compound was prepared in analogy to the preparation of Example **109** by using *tert*-butyl *N*-[(*3R,4S*)-4-fluoropyrrolidin-3-yl]carbamate instead of *tert*-butyl *N*-[(*3S,4S*)-4-methoxypyrrolidin-3-yl]carbamate, 2-(5-bromo-2-pyridyl)ethyl methanesulfonate (compound **13b**) instead of 2-(6-chloro-3-pyridyl)ethyl methanesulfonate (compound **11c**), and 5-[(*4S*,*9aS*)-4-methyl-1,3,4,6,7,8,9,9a-octahydropyrazino[1,2-a]pyrazin-2-yl]-2-deuterio-quinoline-8-carbonitrile (Intermediate **K**) instead of 5-[(*4R*,*9aS*)-4-methyl-1,3,4,6,7,8,9,9a-octahydropyrazino[1,2-a]pyrazin-2-yl]-2-deuterio-quinoline-8-carbonitrile (Intermediate **G**). Example **117** was obtained as a yellow solid (48 mg). LCMS $(M+H)^+$: 516, $^1H$ NMR (400 MHz, METHANOL-$d_4$) $\delta$ ppm: 8.96 (br d, *J* = 7.1 Hz, 1H), 8.27 (d, *J* = 8.1 Hz, 1H), 8.18 (dd, *J* = 2.1, 9.4 Hz, 1H), 8.10 (d, *J*= 1.8 Hz, 1H), 7.81 (d, *J*= 8.6 Hz, 1H), 7.46 (d, *J*= 8.1 Hz, 1H), 7.25 (d, *J* = 9.3 Hz, 1H), 5.73 - 5.55 (m, 1H), 4.61 - 4.49 (m, 1H), 4.40 - 4.24 (m, 3H), 4.21 - 4.09 (m, 2H), 4.03 - 3.72 (m, 9H), 3.68 - 3.58 (m, 2H), 3.48 - 3.35 (m, 2H), 3.27 - 3.18 (m, 2H), 1.67 (br, 3H).

**Example 118**

**5-[(*4S*,*9aR*)-8-[5-(3-amino-3-methyl-azetidin-1-yl)-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile**

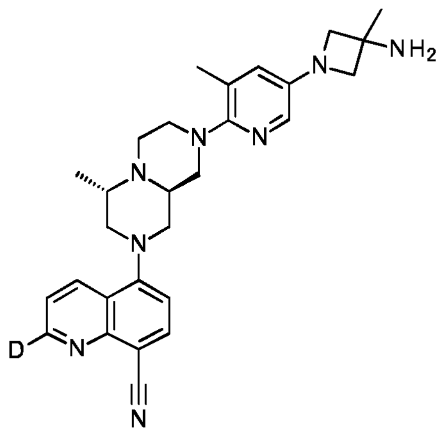

The title compound was prepared in analogy to the preparation of Example **108** by using *tert*-butyl *N*-(3-methylazetidin-3-yl)carbamate instead of *tert*-butyl 3,6-diazabicyclo[3.1.1]-heptane-6-carboxylate. Example **118** (20 mg) was obtained as a light yellow foam. LCMS $(M+H)^+$: 484. $^1H$ NMR (400 MHz, METHANOL-d4) $\delta$ ppm: 8.71 - 8.62 (m, 1H), 8.12 (dd, *J* = 1.2, 7.9 Hz, 1H), 7.67 (d, *J* = 2.8 Hz, 0.6H), 7.61 (dd, *J*= 1.5, 8.6 Hz, 1H), 7.39 (d, *J* = 2.7 Hz, 0.4H), 7.30 (dd, *J*= 2.4, 7.9 Hz, 1H), 7.14 (br, 0.6H), 6.83 (br, 0.4H), 4.46 (d, *J*= 6.4 Hz, 1H), 4.23 (br, 1H), 3.93 - 3.87 (m, 1H), 3.84 - 3.77 (m, 3H), 3.73 - 3.67 (m, 1H), 3.65 - 3.46 (m, 4H), 3.44 - 3.32 (m, 4H), 2.97 (td, *J*= 6.5, 10.5 Hz, 1H), 2.31 (s, 1.8H), 2.25 (s, 1.2H), 1.99 - 1.83 (m, 1.2H), 1.72 (br, 3H), 1.61 - 1.49 (m, 1.8H).

**Example 121**

**5-[(4*S*,9a*R*)-4-methyl-8-(5,6,7,8-tetrahydro-1,6-naphthyridin-2-yl)-3,4,6,7,9,9a-hexahydro-1*H*-pyrazino[1,2-a]pyrazin-2-yl]-2-deuterio-quinoline-8-carbonitrile**

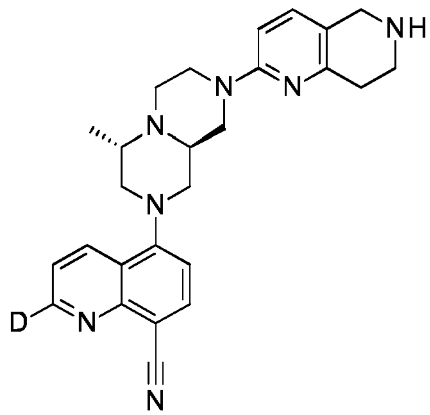

The preparation of Example **121** was the same as Example **2** by using Intermediate **K** instead of Intermediate **A** and *tert*-butyl 2-chloro-7,8-dihydro-5*H*-1,6-naphthyridine-6-carboxylate instead of *tert*-butyl 5-bromo-3,4-dihydro-1*H*-isoquinoline-2-carboxylate (compound **2a**). Example **121** was obtained as a light brown solid (84 mg). LCMS $(M+H)^+$: 441, $^1H$ NMR (400 MHz, METHANOL-$d_4$) $\delta$ ppm: 8.76 (d, *J*= 8.7 Hz, 1H), 8.21 (dd, *J*= 1.0, 8.1 Hz, 1H), 7.72 (d, *J* = 8.7 Hz, 1H), 7.48 (d, *J* = 8.6 Hz, 1H), 7.39 (dd, *J*= 1.9, 7.9 Hz, 1H), 6.91 (br d, *J* = 8.8 Hz, 1H), 4.49 (br, 2H), 4.30 (s, 2H), 4.02 - 3.81 (m, 2H), 3.73 - 3.62 (m, 2H), 3.59 (t, *J*= 6.5 Hz, 2H), 3.52 - 3.36 (m, 4H), 3.27 - 3.12 (m, 2H), 3.09 (t, *J*= 6.4 Hz, 2H), 1.66 (br, 3H).

**Example 122**

**5-[(4*S*,9a*R*)-8-[5-[2-[(3*S*,4*S*)-3-amino-4-methoxy-pyrrolidin-1-yl]ethyl]-2-pyridyl]-4-methyl-3,4,6,7,9,9a-hexahydro-1*H*-pyrazino[1,2-a]pyrazin-2-yl]-2-deuterio-quinoline-8-carbonitrile**

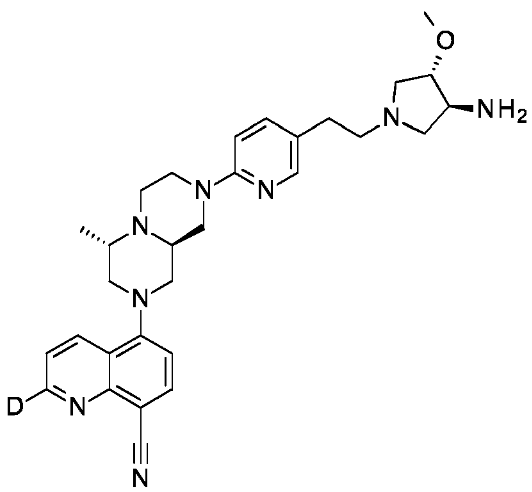

The preparation of Example **122** was the same as Example **2** by using Intermediate **K** instead of Intermediate **A** and *tert*-butyl *N*-[(3*S*,4*S*)-1-[2-(6-chloro-3-pyridyl)ethyl]-4-methoxy-pyrrolidin-3-yl]carbamate (compound **122a)** instead of *tert*-butyl 5-bromo-3,4-dihydro-1*H*-isoquinoline-2-carboxylate (compound **2a**). Example **122** was obtained as a light brown solid (64 mg). LCMS $(M+H)^+$: 528, $^1H$ NMR (400 MHz, METHANOL-$d_4$) $\delta$ ppm: 8.76 (d, *J*= 8.6 Hz, 1H), 8.21

(d, $J$ = 7.9 Hz, 1H), 8.08 (d, $J$= 2.2 Hz, 1H), 7.71 (d, $J$= 8.6 Hz, 1H), 7.63 (dd, $J$ = 2.1, 8.7 Hz, 1H), 7.38 (d, $J$= 7.9 Hz, 1H), 6.99 (d, $J$= 8.8 Hz, 1H), 4.37 (br t, $J$= 11.4 Hz, 2H), 4.01 - 3.96 (m, 1H), 3.95 - 3.81 (m, 2H), 3.74 - 3.70 (m, 1H), 3.66 (br t, $J$= 12.0 Hz, 2H), 3.44 (br d, $J$= 13.6 Hz, 2H), 3.41 (s, 4H), 3.39 - 3.34 (m, 2H), 3.27 - 3.11 (m, 3H), 3.00 - 2.87 (m, 4H), 2.86 - 2.79 (m, 2H), 1.64 (br d, $J$= 6.6 Hz, 3H).

The compound **122a** was prepared according to the following scheme:

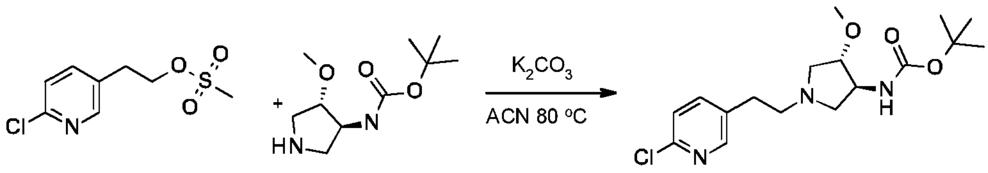


**11c** **122a**

To a tube was added 2-(6-chloropyridin-3-yl)ethyl methanesulfonate (288 mg, 1.1 mmol, Eq: 1.1), *tert*-butyl *N*-[(3*S*,4*S*)-4-methoxypyrrolidin-3-yl]carbamate (216 mg, 1 mmol), $K_2CO_3$ (276 mg, 2 mmol) and Acetonitrile (3 mL). The suspension was heated to 80 °C for 16 hours. The mixture was filtered; the filtrate was concentrated to give an oil. Then the oil was purified by silica gel to give compound **122a** (278 mg) as a yellow oil. LCMS(M+H)$^+$: 356.

**Example 123**

**5-[(4*S*,9*aR*)-8-[5-[[(3*R*,4*R*)-3-amino-4-methoxy-pyrrolidin-1-yl]methyl]-6-methyl-2-pyridyl]-4-methyl-3,4,6,7,9,9a-hexahydro-1*H*-pyrazino[1,2-a]pyrazin-2-yl]-2-deuterio-quinoline-8-carbonitrile**

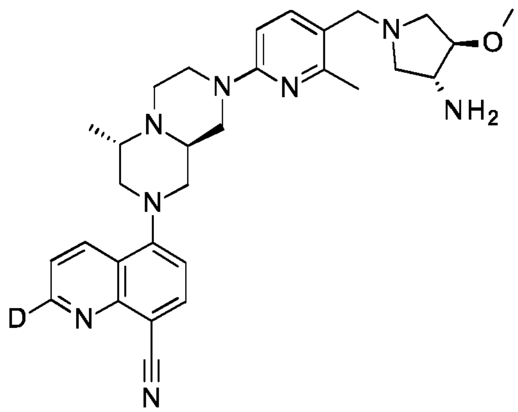

The preparation of Example **123** was the same as Example **2** by using Intermediate **K** instead of Intermediate **A** and *tert*-butyl N-[(3*R*,4*R*)-1-[(6-chloro-2-methyl-3-pyridyl)methyl]-4-methoxy-pyrrolidin-3-yl]carbamate (compound **123a)** instead of *tert*-butyl 5-bromo-3,4-dihydro-1*H*-isoquinoline-2-carboxylate (compound **2a**). Example **123** was obtained as a light brown solid (82 mg). LCMS (M+H)$^+$: 528, $^1$H NMR (400 MHz, METHANOL-$d_4$) δ ppm: 8.64 (d, $J$= 8.7 Hz, 1H), 8.08 (dd, $J$ = 2.1, 7.9 Hz, 1H), 7.65 (br d, $J$ = 8.8 Hz, 1H), 7.62 - 7.56 (m, 1H), 7.29 (d, $J$= 8.1 Hz, 1H), 6.83 (br d, $J$= 8.7 Hz, 1H), 4.63 - 4.40 (m, 2H), 4.32 (s, 2H), 4.12 (br s, 3H), 3.90 (br d, $J$= 5.1 Hz, 1H), 3.88 - 3.69 (m, 2H), 3.69 - 3.53 (m, 4H), 3.53 - 3.35 (m, 5H), 3.33 (s, 3H), 3.20 - 3.07 (m, 1H), 2.45 (s, 3H), 1.63 (br d, $J$ = 5.3 Hz, 3H).

The compound **123a** was prepared according to the following scheme:

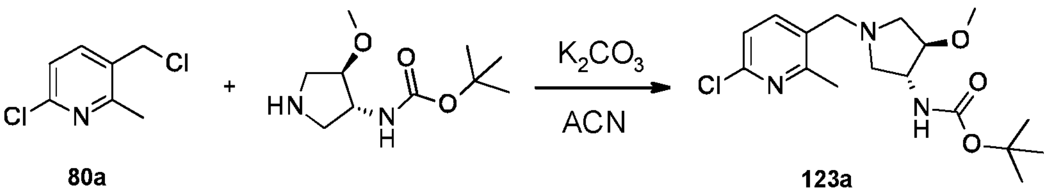


**80a** **123a**

To a tube was added 6-chloro-3-(chloromethyl)-2-methylpyridine (88 mg, 500 μmol), *tert*-butyl *N*-[(3*R*,4*R*)-4-methoxypyrrolidin-3-yl]carbamate (108 mg, 500 μmol), $K_2CO_3$ (138 mg, 1000 μmol) and Acetonitrile (3 mL). The suspension was heated to 86 °C for 2 hours. The mixture was filtered; the filtrate was concentrated to give an oil. Then the oil was purified by silica gel to give compound **123a** (170 mg) as a pale yellow oil. LCMS $(M+H)^+$: 356

**Example 124**

**5-[(*4R*,*9aS*)-8-[2-[6-[(*4aR*,*7aR*)-3,4,4a,5,7,7a-hexahydro-2*H*-pyrrolo[3,4-b][1,4]oxazin-6-yl]-3-pyridyl]ethyl]-4-methyl-3,4,6,7,9,9a-hexahydro-1*H*-pyrazino[1,2-a]pyrazin-2-yl] quinoline-8-carbonitrile**

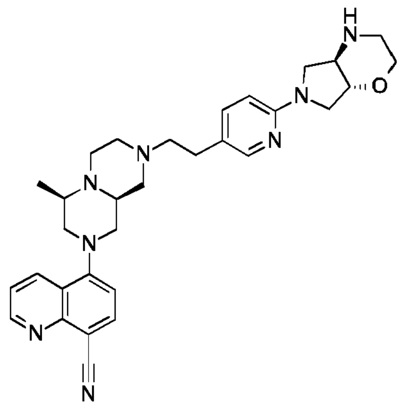

The title compound was prepared in analogy to the preparation of Example **11** by using *tert*-butyl (*4aR*,*7aR*)-3,4a,5,6,7,7a-hexahydro-*2H*-pyrrolo[3,4-b][1,4]oxazine-4-carboxylate (CAS: 1932337-68-2, catalog: PBXA8123, vendor: Pharmablock) instead of *tert*-butyl piperazine-1-carboxylate. Example **124** was obtained as a light yellow solid (60 mg). LCMS $(M+H)^+$: 539, $^1H$ NMR (400 MHz, METHANOL-d4) δ ppm: 9.09 (dd, *J*= 1.6, 4.5 Hz, 1H), 8.90 (dd, *J* = 1.5, 8.6 Hz, 1H), 8.30 (d, *J* = 7.9 Hz, 1H), 8.15 (dd, *J* = 2.1, 9.4 Hz, 1H), 8.07 (d, *J*= 1.7 Hz, 1H), 7.84 (dd, *J* = 4.5, 8.6 Hz, 1H), 7.48 (d, *J* = 8.1 Hz, 1H), 7.21 (d, *J* = 9.3 Hz, 1H), 4.53 - 4.37 (m, 1H), 4.37 - 4.20 (m, 3H), 4.18 - 3.94 (m, 6H), 3.87 - 3.35 (m, 14H), 3.23 (t, *J*= 8.1 Hz, 2H), 1.56 (d, *J* = 6.5 Hz, 3H).

**Example 125**

**5-[(*4S*,*9aS*)-4-methyl-8-[4-[(*2R*)-morpholin-2-yl]phenyl]-3,4,6,7,9,9a-hexahydro-*1H*-pyrazino[1,2-a]pyrazin-2-yl]quinoline-8-carbonitrile**

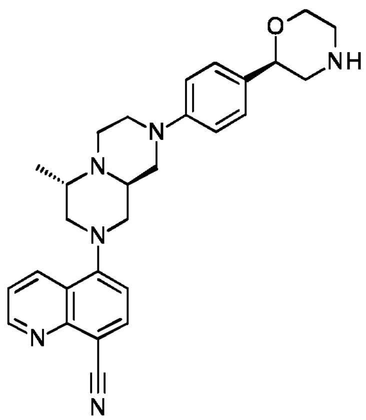

The title compound was prepared according to the following scheme:

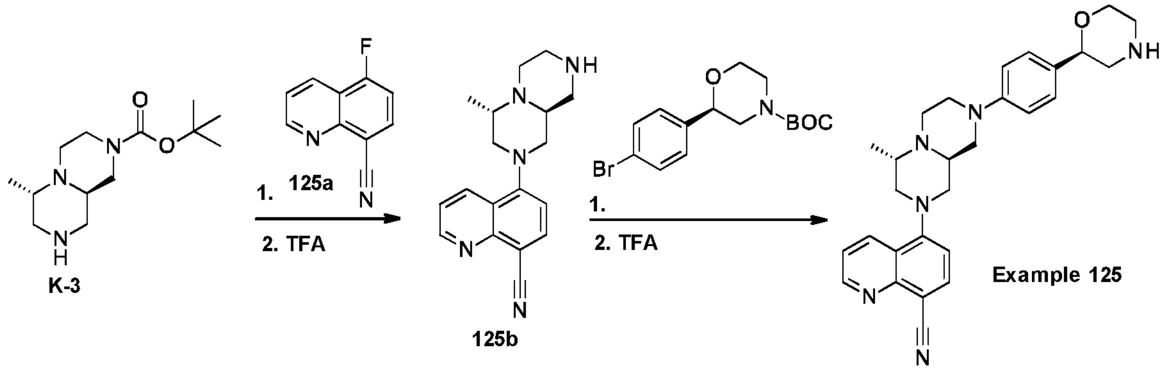


K-3 125b Example 125

**Step 1: Preparation of 5-[(*4S*,*9aS*)-4-methyl-1,3,4,6,7,8,9,9a-octahydropyrazino[1,2-a]pyrazin-2-yl]quinoline-8-carbonitrile (compound 125b)**

A solution of *tert*-butyl (*6S*,*9aR*)-6-methyl-1,3,4,6,7,8,9,9a-octahydropyrazino[1,2-a]pyrazine-2-carboxylate (compound **K-3**, 751 mg, 2.94 mmol), 5-fluoroquinoline-8-carbonitrile (compound **125a,** 460 mg, 2.67 mmol) and DIPEA (1.7 g, 13.4 mmol) in DMSO (10 mL) was stirred at 130 °C overnight, then the reaction was diluted with EtOAc (40 mL), washed with water. The organic layer was dried and concentrated, the residue was purified by flash column chromatography to give 1.1 g orange oil. The orange oil was dissolved in DCM/TFA (8 mL, 1:1) and stirred at rt for 10 mins, then the reaction mixture was concentrated. The residue was dissolved in NaOH (2 N), then extracted with DCM/*i*PrOH (2:1), the organic phase was dried and concentrated to give compound **125b** (700 mg) as a yellow solid. LCMS $(M+H)^+$: 308.

**Step 2: Preparation of 5-[(*4S*,*9aS*)-4-methyl-8-[4-[(2*R*)-morpholin-2-yl]phenyl]-3,4,6,7,9,9a-hexahydro-*1H*-pyrazino[1,2-a]pyrazin-2-yl]quinoline-8-carbonitrile (Example 125)**

To a solution of 5-[(*4S*,*9aS*)-4-methyl-1,3,4,6,7,8,9,9a-octahydropyrazino[1,2-a]pyrazin-2-yl]quinoline-8-carbonitrile (compound **125b,** 180 mg, 586 μmol) in dioxane (6 mL) was added *tert*-butyl (2*R*)-2-(4-bromophenyl)morpholine-4-carboxylate (CAS:1312566-00-9, procedure see Patent WO 2014041007 A1 20140320, 240 mg, 703 μmol) and $Cs_2CO_3$ (572 mg, 1.76 mmol). The suspension was bubbled with $N_2$ for 5 minutes, then RuPhos Pd G2 (46 mg, 58.6 μmol) was added. After the reaction mixture was heated at 100 °C overnight, it was filtered and the filtrate was concentrated. The residue was dissolved in DCM/TFA (4 mL, 1:1), after stirring at r.t. for 10 mins, the reaction mixture was concentrated and the residue was purified by prep-HPLC to give Example **125** (38 mg) as a yellow foam. LCMS $(M+H)^+$: 469. $^1H$ NMR (400 MHz, METHANOL-d4) $\delta$ ppm: 9.00 - 8.93 (m, 1H), 8.71 (dd, *J* = 1.3, 8.6 Hz, 1H), 8.14 (dd, *J* = 1.4, 8.0 Hz, 1H), 7.69 - 7.61 (m, 1H), 7.29 - 7.20 (m, 3H), 6.97 (d, *J*= 8.6 Hz, 2H), 4.38 (dd, *J*= 2.3, 10.5 Hz, 1H), 4.03 - 3.87 (m, 1H), 3.73 (dt, *J*= 3.2, 11.4 Hz, 1H), 3.62 (br t, *J*= 12.5 Hz, 2H), 3.44 (br d, *J*= 11.5 Hz, 1H), 3.35 (br s, 2H), 3.27 (br s, 2H), 3.02 - 2.80 (m, 6H), 2.79 - 2.65 (m, 2H), 2.51 (br t, *J*= 10.7 Hz, 1H), 1.43 (d, *J* = 6.2 Hz, 3H).

**Example 126**

The following tests were carried out in order to determine the activity of the compounds of formula (I) and (Ia) in HEK293-Blue-hTLR-7/8/9 cells assay.

**HEK293-Blue-hTLR-7 cells assay:**

A stable HEK293-Blue-hTLR-7 cell line was purchased from InvivoGen (Cat.#: hkb-htlr7, San Diego, California, USA). These cells were originally designed for studying the stimulation of human TLR7 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR7 cells with TLR7 ligands. Therefore, the reporter expression was declined by TLR7 antagonist under the stimulation of a ligand, such as R848 (Resiquimod), for incubation of 20 hrs. The cell culture supernatant SEAP reporter activity was determined using QUANTI-Blue™ kit (Cat.#: rep-qb1, Invivogen, San Diego, Ca, USA) at a wavelength of 640 nm, a detection medium that turns purple or blue in the presence of alkaline phosphatase.

HEK293-Blue-hTLR7 cells were incubated at a density of 250,000~450,000 cells/mL in a volume of 170 μL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of

20 μL test compound in a serial dilution in the presence of final DMSO at 1% and 10 μL of 20uM R848 in above DMEM, perform incubation under 37 °C in a $CO_2$ incubator for 20 hrs. Then 20 μL of the supernatant from each well was incubated with 180 μL Quanti-blue substrate solution at 37 °C for 2 hrs and the absorbance was read at 620~655 nm using a spectrophotometer. The signaling pathway that TLR7 activation leads to downstream NF-κB activation has been widely accepted, and therefore similar reporter assay was modified for evaluating TLR7 antagonist.

**HEK293-Blue-hTLR-8 cells assay:**

A stable HEK293-Blue-hTLR-8 cell line was purchased from InvivoGen (Cat.#: hkb-htlr8, San Diego, California, USA). These cells were originally designed for studying the stimulation of human TLR8 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR8 cells with TLR8 ligands. Therefore, the reporter expression was declined by TLR8 antagonist under the stimulation of a ligand, such as R848, for incubation of 20 hrs. The cell culture supernatant SEAP reporter activity was determined using QUANTI-Blue™ kit (Cat.#: rep-qb1, Invivogen, San Diego, Ca, USA) at a wavelength of 640 nm, a detection medium that turns purple or blue in the presence of alkaline phosphatase.

HEK293-Blue-hTLR8 cells were incubated at a density of 250,000~450,000 cells/mL in a volume of 170 μL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 μL test compound in a serial dilution in the presence of final DMSO at 1% and 10 μL of 60uM R848 in above DMEM, perform incubation under 37 °C in a $CO_2$ incubator for 20 hrs. Then 20 μL of the supernatant from each well was incubated with 180 μL Quanti-blue substrate solution at 37°C for 2 hrs and the absorbance was read at 620~655 nm using a spectrophotometer. The signaling pathway that TLR8 activation leads to downstream NF-κB activation has been widely accepted, and therefore similar reporter assay was modified for evaluating TLR8 antagonist.

**HEK293-Blue-hTLR-9 cells assay:**

A stable HEK293-Blue-hTLR-9 cell line was purchased from InvivoGen (Cat.#: hkb-htlr9, San Diego, California, USA). These cells were originally designed for studying the stimulation of human TLR9 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR9 cells with TLR9 ligands. Therefore, the reporter expression was declined by TLR9 antagonist under the stimulation of a ligand, such as ODN2006 (Cat.#: tlrl-2006-1, Invivogen, San Diego, California, USA), for incubation of 20 hrs. The cell culture supernatant SEAP reporter activity was determined using QUANTI-Blue™ kit (Cat.#: rep-qb1, Invivogen, San Diego, California, USA) at a wavelength of 640 nm, a detection medium that turns purple or blue in the presence of alkaline phosphatase.

HEK293-Blue-hTLR9 cells were incubated at a density of 250,000~450,000 cells/mL in a volume of 170 μL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 μL test compound in a serial dilution in the presence of final DMSO at 1% and 10 μL of 20uM ODN2006 in above DMEM, perform incubation under 37 °C in a $CO_2$ incubator for 20 hrs. Then 20 μL of the supernatant from each well was incubated with 180 μL Quanti-blue substrate solution at 37 °C for 2 h and the absorbance was read at 620-655 nm using a spectrophotometer. The signaling pathway that TLR9 activation leads to downstream NF-κB activation has been widely accepted, and therefore similar reporter assay was modified for evaluating TLR9 antagonist.

The compounds of formula (I) or (Ia) have human TLR7 and/or TLR8 inhibitory activities ($IC_{50}$ value) <0.5 μM. Moreover, compounds of this invention also have human TLR9 inhibitory activity <0.5μM. Activity data of the compounds of the present invention were shown in Table 2.

**Table 2. The activity of the compounds of present invention in HEK293-Blue-hTLR-7/8/9 cells assays**

| Example No | HEK/hTLR7 $IC_{50}$ (μM) | HEK/hTLR8 $IC_{50}$ (μM) | HEK/hTLR9 $IC_{50}$ (μM) |
|---|---|---|---|
| 1 | 0.017 | 0.010 | 0.058 |
| 2 | 0.007 | <0.003 | 0.054 |
| 3 | 0.020 | 0.012 | 0.110 |
| 4 | 0.010 | <0.003 | 0.079 |
| 5 | 0.017 | 0.012 | <0.032 |

(continued)

| Example No | HEK/hTLR7 $IC_{50}$ ($\mu$M) | HEK/hTLR8 $IC_{50}$ ($\mu$M) | HEK/hTLR9 $IC_{50}$ ($\mu$M) |
|---|---|---|---|
| 6 | 0.014 | 0.014 | 0.069 |
| 7 | 0.005 | 0.030 | <0.032 |
| 8 | 0.022 | 0.052 | 0.041 |
| 9 | 0.003 | 0.001 | 0.079 |
| 10 | 0.026 | 0.012 | 0.043 |
| 11 | 0.007 | 0.021 | 0.037 |
| 12 | 0.012 | 0.014 | 0.047 |
| 13 | 0.026 | 0.009 | 0.040 |
| 15 | 0.049 | 0.028 | 0.041 |
| 16 | 0.006 | 0.009 | 0.120 |
| 17 | 0.006 | 0.004 | 0.047 |
| 18 | 0.010 | 0.020 | 0.065 |
| 19 | 0.010 | 0.031 | 0.048 |
| 20 | 0.014 | 0.051 | 0.056 |
| 21 | 0.019 | 0.053 | 0.056 |
| 22 | 0.026 | 0.058 | 0.047 |
| 24 | 0.008 | 0.015 | 0.045 |
| 25 | 0.013 | 0.025 | 0.064 |
| 26 | 0.018 | 0.008 | 0.087 |
| 27 | 0.021 | 0.013 | 0.049 |
| 28 | 0.012 | 0.029 | 0.098 |
| 30 | 0.016 | 0.021 | 0.072 |
| 31 | 0.022 | 0.024 | 0.085 |
| 32 | 0.016 | 0.011 | 0.034 |
| 33 | 0.010 | 0.008 | 0.064 |
| 34 | 0.020 | 0.014 | 0.042 |
| 35 | 0.029 | 0.008 | 0.098 |
| 36 | 0.013 | 0.015 | 0.068 |
| 37 | 0.021 | 0.026 | 0.062 |
| 38 | 0.002 | 0.008 | 0.093 |
| 39 | 0.021 | 0.017 | 0.095 |
| 40 | 0.007 | 0.007 | <0.032 |
| 41 | 0.035 | 0.007 | 0.045 |
| 42 | 0.006 | 0.002 | 0.050 |
| 43 | 0.009 | 0.013 | 0.078 |
| 44 | 0.004 | 0.001 | 0.050 |
| 45 | 0.018 | 0.028 | 0.081 |
| 46 | 0.014 | 0.013 | 0.078 |

(continued)

| Example No | HEK/hTLR7 $IC_{50}$ (μM) | HEK/hTLR8 $IC_{50}$ (μM) | HEK/hTLR9 $IC_{50}$ (μM) |
|---|---|---|---|
| 47 | 0.010 | 0.006 | 0.117 |
| 53 | 0.013 | 0.02 | < 0.032 |
| 54 | 0.023 | 0.026 | 0.043 |
| 55 | 0.015 | 0.040 | 0.048 |
| 56 | 0.013 | 0.008 | < 0.032 |
| 57 | 0.023 | 0.022 | 0.094 |
| 58 | 0.020 | 0.040 | 0.051 |
| 59 | 0.009 | 0.028 | 0.048 |
| 60 | 0.008 | 0.014 | 0.053 |
| 61 | 0.016 | 0.008 | 0.064 |
| 62 | 0.023 | 0.017 | 0.043 |
| 63 | 0.022 | 0.049 | 0.078 |
| 64 | 0.023 | 0.032 | 0.032 |
| 65 | 0.019 | 0.028 | 0.050 |
| 66 | 0.062 | 0.039 | 0.116 |
| 67 | 0.065 | 0.084 | 0.083 |
| 68 | 0.053 | 0.077 | 0.041 |
| 69 | 0.066 | 0.052 | 0.142 |
| 70 | 0.011 | 0.012 | 0.097 |
| 71 | 0.045 | 0.021 | 0.079 |
| 72 | 0.012 | 0.015 | 0.089 |
| 73 | 0.025 | 0.018 | <0.032 |
| 74 | 0.028 | 0.062 | 0.048 |
| 75 | 0.034 | 0.084 | 0.065 |
| 76 | 0.016 | 0.005 | 0.050 |
| 77 | 0.021 | 0.024 | 0.044 |
| 78 | 0.035 | 0.017 | <0.032 |
| 79 | 0.019 | 0.006 | 0.046 |
| 80 | 0.023 | 0.005 | <0.032 |
| 81 | 0.016 | 0.005 | <0.032 |
| 82 | 0.012 | 0.003 | 0.041 |
| 83 | 0.017 | 0.007 | 0.051 |
| 84 | 0.016 | 0.019 | <0.032 |
| 85 | 0.019 | 0.010 | 0.097 |
| 86 | 0.015 | 0.012 | 0.062 |
| 87 | 0.017 | 0.015 | 0.052 |
| 88 | 0.040 | 0.010 | 0.047 |
| 89 | 0.028 | 0.044 | 0.047 |

(continued)

| Example No | HEK/hTLR7 $IC_{50}$ ($\mu$M) | HEK/hTLR8 $IC_{50}$ ($\mu$M) | HEK/hTLR9 $IC_{50}$ ($\mu$M) |
|---|---|---|---|
| 90 | 0.013 | 0.008 | 0.033 |
| 91 | 0.015 | 0.020 | 0.045 |
| 92 | 0.006 | 0.009 | 0.034 |
| 93 | 0.010 | 0.061 | 0.186 |
| 94 | 0.025 | 0.008 | 0.099 |
| 95 | 0.002 | 0.006 | 0.142 |
| 96 | 0.002 | 0.005 | 0.189 |
| 97 | 0.002 | 0.004 | 0.140 |
| 98 | 0.024 | 0.010 | 0.058 |
| 99 | 0.001 | 0.002 | 0.155 |
| 100 | 0.001 | 0.003 | 0.173 |
| 101 | 0.003 | 0.004 | 0.081 |
| 102 | 0.004 | 0.002 | 0.154 |
| 103 | 0.006 | 0.004 | 0.087 |
| 105 | 0.026 | 0.007 | <0.032 |
| 106 | 0.002 | 0.004 | 0.128 |
| 107 | 0.003 | 0.004 | 0.146 |
| 108 | 0.003 | 0.006 | 0.098 |
| 109 | 0.005 | 0.004 | 0.111 |
| 110 | 0.007 | 0.006 | 0.070 |
| 111 | 0.006 | 0.005 | 0.119 |
| 112 | 0.004 | 0.010 | 0.082 |
| 113 | 0.006 | 0.142 | 0.126 |
| 114 | 0.002 | 0.010 | 0.110 |
| 115 | 0.010 | 0.017 | 0.140 |
| 117 | 0.023 | 0.024 | 0.042 |
| 118 | 0.004 | 0.006 | 0.148 |
| 121 | 0.005 | 0.001 | 0.085 |
| 122 | 0.009 | 0.003 | 0.051 |
| 123 | 0.027 | 0.006 | 0.107 |
| 124 | 0.014 | 0.051 | 0.055 |
| 125 | 0.001 | 0.007 | 0.120 |

**Example 127**

**hERG channel inhibition assay:**

The hERG channel inhibition assay is a highly sensitive measurement that identifies compounds exhibiting hERG inhibition related to cardiotoxicity in vivo. The hERG $K^+$ channels were cloned in humans and stably expressed in a CHO (Chinese hamster ovary) cell line. $CHO_{hERG}$ cells were used for patch-clamp (voltage-clamp, whole-cell)

experiments. Cells were stimulated by a voltage pattern to activate hERG channels and conduct $I_{KhERG}$ currents (rapid delayed outward rectifier potassium current of the hERG channel). After the cells were stabilized for a few minutes, the amplitude and kinetics of $I_{KhERG}$ were recorded at a stimulation frequency of 0.1 Hz (6 bpm). Thereafter, the test compound was added to the preparation at increasing concentrations. For each concentration, an attempt was made to reach a steady-state effect, usually, this was achieved within 3-10 min at which time the next highest concentration was applied. The amplitude and kinetics of $I_{KhERG}$ are recorded in each concentration of the drug which were compared to the control values (taken as 100%). (references: Redfern WS, Carlsson L, Davis AS, Lynch WG, MacKenzie I, Palethorpe S, Siegl PK, Strang I, Sullivan AT, Wallis R, Camm AJ, Hammond TG. 2003; Relationships between preclinical cardiac electrophysiology, clinical QT interval prolongation and torsade de pointes for a broad range of drugs: evidence for a provisional safety margin in drug development. Cardiovasc. Res. 58:32-45, Sanguinetti MC, Tristani-Firouzi M. 2006; hERG potassium channels and cardiac arrhythmia. Nature 440:463-469, Webster R, Leishman D, Walker D. 2002; Towards a drug concentration effect relationship for QT prolongation and torsades de pointes. Curr. Opin. Drug Discov. Devel. 5:116-26).

Results of hERG are given in Table 3. A safety ratio (hERG $IC_{20}$ /$EC_{50}$) > 30 suggests a sufficient window to differentiate the pharmacology by inhibiting TLR7/8/9 pathways from the potential hERG related cardiotoxicity. The calculation of hERG $IC_{20}$ / TLR7/8/9 $IC_{50}$ below serves as early selectivity index to assess hERG liability.

**Table 3. hERG and safety ratio results**

| Example No | hERG $IC_{20}$ (μM) | hERG $IC_{50}$ (μM) | hERG $IC_{20}$/ TLR7 $IC_{50}$ | hERG $IC_{20}$/ TLR8 $IC_{50}$ | hERG $IC_{20}$/ TLR9 $IC_{50}$ |
|---|---|---|---|---|---|
| 1 | >10 | >20 | >588 | >1000 | >172 |
| 5 | >10 | >20 | >588 | >833 | >313 |
| 8 | 8.63 | >20 | 392 | 166 | 210 |
| 9 | 5.17 | >20 | 1723 | 5170 | 65 |
| 12 | >10 | >20 | >833 | >714 | >213 |
| 17 | >10 | >20 | >1667 | >2500 | >213 |
| 22 | >10 | >20 | >385 | >172 | >213 |
| 25 | >10 | >20 | >769 | >400 | >156 |
| 30 | 4.53 | >10 | 283 | 216 | 63 |
| 34 | 4.74 | >10 | 237 | 339 | 113 |
| 36 | 5.60 | >20 | 431 | 373 | 82 |
| 37 | 4.50 | >10 | 214 | 173 | 73 |
| 38 | >10 | >20 | >5000 | >1250 | >108 |
| 39 | 5 | >10 | 238 | 294 | 53 |
| 41 | 9.64 | >20 | 275 | 1377 | 214 |
| 42 | 4.35 | >10 | 725 | 2175 | 87 |
| 45 | >10 | >20 | >556 | >357 | >124 |
| 55 | >10 | >20 | >680 | >249 | >209 |
| 69 | >10 | >20 | >152 | >192 | >70 |
| 77 | >10 | >20 | >476 | >416 | >227 |
| 79 | >10 | >20 | >526 | >1666 | >217 |
| 80 | >10 | >20 | >434 | >2000 | >312 |
| 82 | >10 | >20 | >833 | >3333 | >243 |
| 83 | >10 | >20 | >588 | >1428 | >196 |
| 92 | >10 | >20 | >1666 | >1111 | >294 |
| 93 | >10 | >20 | >1000 | >163 | >53 |

(continued)

| Example No | hERG $IC_{20}$ (μM) | hERG $IC_{50}$ (μM) | hERG $IC_{20}$/ TLR7 $IC_{50}$ | hERG $IC_{20}$/ TLR8 $IC_{50}$ | hERG $IC_{20}$/ TLR9 $IC_{50}$ |
|---|---|---|---|---|---|
| 98 | >10 | >20 | >416 | >1000 | >172 |
| 106 | >10 | >20 | >5000 | >2500 | >78 |
| 109 | >10 | >20 | >2000 | >2500 | >90 |
| 111 | >10 | >20 | >1666 | >2000 | >84 |
| 112 | >10 | >20 | >2500 | >1000 | >122 |
| 113 | >10 | >20 | >1666 | >70 | >79 |
| 115 | >10 | >20 | >1000 | >588 | >71 |
| 118 | 7.66 | >20 | 1914 | 1276 | 51 |
| 121 | >10 | >20 | >2000 | >10000 | >117 |

**Example 128**

**3T3 in vitro phototoxicity assay**

Phototoxicity is defined as a toxic response that is elicited after the first exposure of the skin to certain chemicals and subsequent exposure to light, or that is induced similarly by skin irradiation after systemic administration of a chemical substance. The assay used in this study is designed to detect the phototoxic potential of a chemical by using a simple in vitro cytotoxicity assay with Balb/c 3T3 mouse fibroblasts. The principle of this test is a comparison of the cytotoxicity of a chemical when tested with and without exposure to a non-toxic dose of UVA-light. Cytotoxicity is expressed as a dose dependent reduction of the growth rate of cells as determined by uptake of the vital dye Neutral Red one day after treatment.

**1. Method**

**Preparation of stock solution and dosage of test item**

A small amount of substance was weighed and formulated freshly in DMSO just before the start of the exposure of the cells. This stock solution or appropriate dilutions with DMSO were added to the cell suspensions to obtain the required final concentrations. All solutions were generally prepared in Eppendorf caps and discarded after use.

Reference substance

Chlorpromazine (HCL) (Sigma, Batch/Lot No.: 120M1328V) , test concentration: 300 μg/mL, Solvent: PBS / 3% DMSO

**Measurement of UV absorption spectrum**

The absorption spectra as such or with UV-A or with UV-B pre-irradiation were recorded between 240 nm and 400 nm with a Lambda-2 spectral photometer (Perkin Elmer).

| | | | |
|---|---|---|---|
| UV radiation sources: | for UV-A: | Sol 500 with filter H1 | |
| | | Main spectrum: | 315-690 nm |
| | | Irradiance: | approx. 1.67 $mW/cm^2$ |
| | | Radiation dose : | approx. 5 $J/cm^2$ |
| | for UV-B: | Philips TL 20W/12 | |
| | | Main spectrum: | 290-320 nm |
| | | Irradiance: | approx. 0.083 $mW/cm^2$ |
| | | Radiation dose: | approx. 0.05 $J/cm^2$ |

## Determination of phototoxicity

For this study the Neutral Red uptake (NRU) assay of Borenfreund and Puerner (Borenfreund, E, Puerner JA. Toxicity determined in vitro by morphological alterations and Neutral Red absorption. Toxicology Lett. 1985; 24:119-124.) modified according to INVITTOX protocol No 78 (ERGATT/FRAME data bank of in vitro techniques in toxicology. INVITTOX PROTOCOL No 78. 3T3 NRU Phototoxicity Assay. March 1994) has been adapted to examine a possible phototoxic potential of the test item. This assay is based on the active uptake of the Neutral Red dye into the lysosomes of cultured murine fibroblasts. Because lysosomal membranes are known to be a site of action of many phototoxic compounds, this assay can provide a measure of potential for phototoxic injury.

## Preparation of cell culture

A murine fibroblasts clone A 31 (ATCC no. CCL 163 - passage No. 108) were cultured in 175 $cm^2$ tissue culture grade flasks, containing sDMEM (Dulbecco's Minimal Essential Medium, supplemented with 10% fetal calf serum, 2 mM L-glutamine, 100 units/ml Penicillin and 100 $\mu$g/ml streptomycin) at 37°C in a humidified atmosphere of 6% $CO_2$. Before cells approach confluence they were removed from flasks by trypsinisation. Prior to use in an assay, the cells were transferred to 96-well microtiter plates at a concentration of $1\times 10^4$ cells/well in 100 $\mu$l volumes of sDMEM and allowed to attach for 24 h.

## Exposure to test item

For incubation with murine fibroblasts, the test item was diluted in PBS / 3% DMSO (detailed concentrations see in results).

Culture medium (Dulbecco's Modified Eagle Medium(DMEM), GlutaMAX (Gibco Ref 21885-025), 10% Fetal Bovine Serum (FBS) (Gibco Ref 10270-106), 100IU/ml Penicillin and 100 $\mu$g/ml Streptomycin (Gibco Ref 15140-122)) was removed from the wells and murine fibroblasts were washed with PBS. Afterwards 100 $\mu$L of PBS / 3% DMSO containing the test item was added and target cells were incubated for 1 h at 37°C with 6% $CO_2$.

## UV exposure

For each test item the microtiter plates were prepared according to Table 4. "UVA plates" were exposed to approx. 5 $J/cm^2$ UVA light, the "Dark plates" were kept in the dark and served as cytotoxicity control. Plates with chlorpromazine hydrochloride served as positive control. UV flux was measured with a UV-meter (Dr. Gröbel RM21).

Following UV irradiation, the test item was removed from the wells (one washing step with PBS) and replaced with sDMEM. Target cells were then incubated overnight at 37°C in 6% $CO_2$.

**Table 4. 96-well microtiter plate setup**

<table>
<tr><th></th><th>1</th><th>2</th><th>3</th><th>4</th><th>5</th><th>6</th><th>7</th><th>8</th><th>9</th><th>10</th><th>11</th><th>12</th></tr>
<tr><td>A</td><td>S1</td><td>S2</td><td rowspan="8">U01</td><td rowspan="8">U02</td><td rowspan="8">U03</td><td rowspan="8">U04</td><td rowspan="8">U05</td><td rowspan="8">U06</td><td rowspan="8">U07</td><td rowspan="8">U08</td><td>S2</td><td>S1</td></tr>
<tr><td>B</td><td>S1</td><td>S2</td><td>S2</td><td>S1</td></tr>
<tr><td>C</td><td>S1</td><td>S2</td><td>S2</td><td>S1</td></tr>
<tr><td>D</td><td>S1</td><td>S2</td><td>S2</td><td>S1</td></tr>
<tr><td>E</td><td>S1</td><td>S2</td><td>S2</td><td>S1</td></tr>
<tr><td>F</td><td>S1</td><td>S2</td><td>S2</td><td>S1</td></tr>
<tr><td>G</td><td>S1</td><td>S2</td><td>S2</td><td>S1</td></tr>
<tr><td>H</td><td>S1</td><td>S2</td><td>S2</td><td>S1</td></tr>
</table>

96-well microtiter plates were prepared as follows:

Each plate contained wells with cells and solvent but without test item which were either not incubated with Neutral Red solution (0% standard - S1) or were stained with Neutral Red (100% standard -S2) for calculation of the standard cell viability curve. Wells labeled with U01-U08 contained the different test item concentrations.

**Neutral Red uptake**

The ready to use Neutral Red (NR) staining solution was freshly prepared as follows:

- 0.4% aqueous stock solution was shielded from light and filtered before use to remove NR crystals.
- 1:40 dilution of the stock solution was then prepared in sDMEM and added to the cells.

After the incubation the wells to be assayed were filled with 100 µL of the sDMEM containing Neutral Red. The target cells were incubated with the NR for 3 h at 37°C in 6% $CO_2$.

**Measurement of Neutral Red uptake**

Unincorporated Neutral Red was removed from the target cells and the wells washed with at least 100 µL of PBS. 150 µL of Neutral Red desorb solution (1% glacial acetic acid, 50% ethanol in aqua bidest) was then added to quantitatively extract the incorporated dye. After at least 10 mins of vigorous shaking of the plates on a microtiter plate shaker until Neutral Red has been extracted from the cells and formed a homogeneous solution, the absorption of the resulting colored solution was measured with a SPECTRAmax PLUS microtiter plate reader (Molecular Devices) at 540 nm.

**Calculation of cell viability**

Cell viability was calculated with the SOFTmax Pro software package (Molecular Devices). First a two-point standard curve (0% and 100% viability) was calculated with the linear curve fit option of the program based on the following formula:

$$Y = A + ( B \times X )$$

(A = y-intercept of the line; B = slope of the line;
0% cell viability = cells with solvent, but without test item and Neutral Red;
100% cell viability = cells with solvent and Neutral Red, but without test item)

By this means the viability of the cells incubated with increasing concentrations of the test chemical was calculated. Chlorpromazine (HCl) served as positive control in the experiment.

**Calculation of $IC_{50}$ values**

All calculations were performed with the SOFTmax Pro analysis software package (Molecular Devices - for details see:
http://www.mbl.edu/jbpc/files/2014/05/SoftMax_Pro_User_Guide.pdf)

**Calculation of discrimination factor for phototoxicity**

For evaluation of phototoxic potential, the $IC_{50}$ values determined with and without UV exposure were compared.

$$\text{Factor} = IC_{50}\ (\text{-UV}) / IC_{50}\ (\text{+UV})$$

For discrimination between phototoxic and non-phototoxic test chemicals a cut-off factor of >5 was applied (Liebsch M, Spielmann H, Balls M, Brand M, Döring B, Dupuis J, Holzhüter HG, Klecak G, L.Eplattenier H, Lovell W, Maurer T, Moldenhauer F, Moore L, Pape W, Pfannenbecker U, Potthast JM, De Silva O, Steiling W, Willshaw A. First results of the EC/COLIPA Validation Project. In Vitro Phototoxicity Testing. In: In Vitro Skin Toxicology: Irritation, Phototoxicity, Sensitization; Vol. 10. Alternative Methods in Toxicology,-Eds. Rougier A, Maibach HI, Goldberg AM; Mary Ann Liebert Publ.: New York, USA 1994, pp. 243-251).

Test items which are not cytotoxic to murine fibroblasts even at the highest concentrations tested, but show a strong dose dependent decrease in cell viability after UV exposure are considered also phototoxic (Spielmann H, Balls M, Dupuis J, Pape WJW, Pechovitch G, Silva DeO, Holzhütter, HG, Clothier R, Desolle P, Gerberick F, Liebsch M, Lowell WW, Maurer T, Pfannenbecker U, Potthast JM, Csato M, Sladowski D, Steiling W, Brantom P. The international

EU/COLIPA in vitro phototoxicity validation study: Results of phase II (blind trial). Part 1: The 3T3 NRU phototoxicity test. Toxicology in Vitro 1998, 12: 305-327).

The test results were shown below, the compounds of this invention showed very good phototoxicity profile.

**Table 5. The 3T3 test results for the compound of this invention**

| Example No | Phototoxicity factor | $IC_{50}$ (UV-A) (μg/mL) |
|---|---|---|
| 5 | >1.39 | 23.3 |
| 9 | >3.9 | 26.3 |
| 12 | >2.53 | - |
| 68 | 1.53 | 65.9 |
| 77 | >1.9 | 52.5 |
| 106 | >1.67 | 60.25 |

**Example 129**

**Agonist induced mouse lupus nephritis disease model**

To assess compound efficacy in vivo against lupus nephritis, we utilized a murine model of TLR7 agonist-induced lupus-like disease, in which TLR7 activation leads to the development of systemic autoimmune symptoms with elevated levels of autoantibodies to double-stranded DNA (anti-dsDNA), inflammatory cytokines such as IP10, as well as multiple organs involvement especially in kidney and spleen.

BALB/c mice were purchased from Vital River Laboratories Co., Ltd., Beijing, China. All mice were 7 to 8-week old female. To induce disease, animals were topically treated on the right ear with 100μg of Resiquimod (R848) solved in 20μL of acetone for three times per week. Compound or vehicle treatment was administrated orally once daily and 30 minutes prior to the epicutanenous R848 treatment if they were on the same day.

Blood samples were collected once weekly to gauge the level of autoantibody against double stranded DNA and multiple cytokines in serum. The total anti-dsDNA Immunoglobulins in serum were measured according to the manufacturer's instruction with a commercially available ELISA assay (Cat# 5110, Alpha Diagnostic Inti Inc.). Cytokine levels in serum were measured with a ProcartaPlex immunoassay Kit (Cat# PPX-08-MXNKR2Z, Thermo Fisher). Specifically, 10 μL of premixed magnetic capture beads were added to a Drop Array DA-bead plate (Cat# 969-CC-BD-05, Curiox). After washing, 10 μL of diluted sera (1:5) was incubated on the DA-bead plate overnight at 4°C. After washing for three times, 5μL of premixed detection antibody was added to the plate with an incubation for 60 minutes at room temperature. After washing, 10 μL of Streptavidin-PE was added with an incubation for 30 minutes at room temperature. The beads were then re-suspended in 55 μL Reading Buffer. Samples were analyzed with a Luminex 200 (Millipore).

Urine samples were collected once weekly with the animals housed in metabolic cages for 24 hours, and were subjected to the measurements of urinary albumin (UALB), urinary creatinine (UCR) and urinary total protein (UTP) with a Roche Cobas 6000 Chemistry Analyzer (Roche Diagnostics, Mannheim, Germany).

To evaluate kidney histopathology, kidney samples were fixed with neutral buffered formalin and embedded in paraffin. The sliced sections were stained with hematoxylin and eosin, and with periodic acid-Schiff. A pathologist then examined the samples in a blinded manner and graded glomerular lesions semi-quantitatively with a total glomerulonephritis score, the sum of glomerular score, inflammation score, PAS score, and tubular protein score.

Specifically, glomerular scores of 0 to 6 were based on assessment of the glomeruli in the outer one-half of the cortex, and on the most frequent grade encountered in this region. Grade 1: Minimally increased cellularity and/or mesangial expansion; Grade 2: Mildly increased cellularity and mesangial expansion; Grade 3: Moderately increased cellularity and some areas of prominent mesangial expansion and/or capillary proliferation in most affected glomeruli; Grade 4: Markedly increased cellularity and some areas of prominent mesangial expansion and/or capillary proliferation in most affected glomeruli; rare sclerotic glomeruli; may have hypertrophy of parietal cells; Grade 5: Above with <25% of glomeruli sclerotic and/or capillary proliferation in most affected glomeruli; Grade 6: Above with >25% of glomeruli sclerotic - characterized in part by decreased tuft cellularity +/- peri glomerular fibrosis +/- hypertrophy of parietal cells.

Inflammation scores of 0 to 3 were based on both the number and size of inflamed area.

PAS scores of 0 to 3 were based upon the presence of increased staining of the glomerular mesangial matrix in the outer one-half of the cortex. Grade 1: Minimally increased mesangial staining of scattered glomeruli; Grade 2: More extensive expansion (and therefore PAS staining) of the mesangium affecting more of the glomeruli; Grade 3: Pronounced expansion of the mesangium in most of the glomeruli.

Tubular protein scores of 0 to 3 were based on the percentage of tubules containing proteinaceous fluid. Grade 1: <25% of tubules affected; Grade 2: 25 to 50% of tubules affected; Grade 3: >50% of tubules affected. Spleens were harvested and weighed upon study termination to evaluate splenomegaly.

Key findings for Example 106 in the R848 agonist induced mouse lupus nephritis disease model are as follows:

a. Example 106 at 10 mg/kg inhibited the production of Interferon gamma-induced protein 10 (IP-10) in serum after 1 week of treatment (see FIG. 3A);
b. Example 106 at 10 mg/kg significantly reduces the level of anti-dsDNA autoantibodies in serum after 2 weeks of treatment (see FIG. 3B);
c. Example 106 at 10 mg/kg demonstrated benefits in preventing kidney damages with clear evidence of no R848-induced proteinuria increase after 5 weeks of treatment (see FIG. 3C);
d. Example 106 at 10 mg/kg suppressed immune cell hyper-activation in spleen, and therefore prevented splenomegaly in this model with spleens of normal weight as compared to those of healthy animals after 6 weeks of treatment (see FIG. 3D).

Taken together, the aforementioned experiment findings have demonstrate a good therapeutic potential of Example 106, and potentially the compounds of this invention, in treating systemic lupus erythematosus (SLE), lupus nephritis (LN).

## Claims

1. A compound of formula (I),

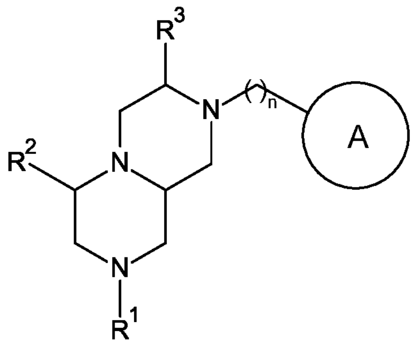

(I),

wherein

$R^1$ is

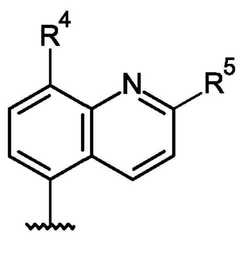
,
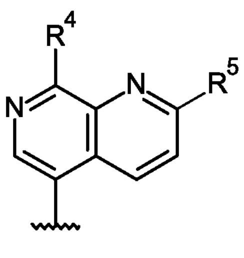
,
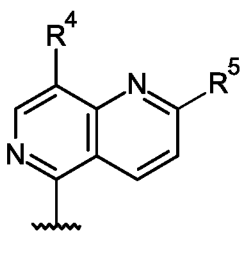
,
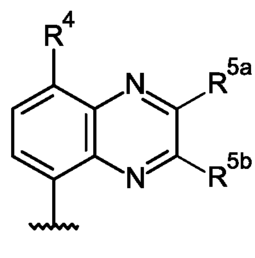
,

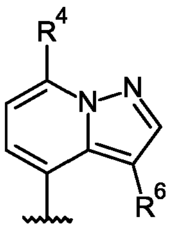 , 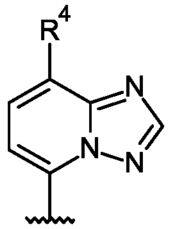 , 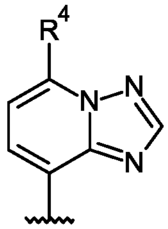 , 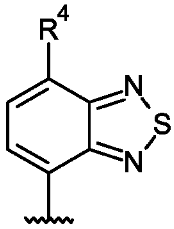 , 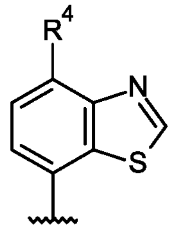 ,

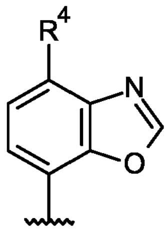 , 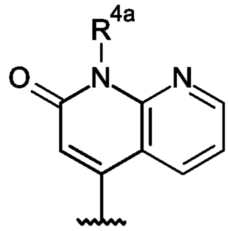 or 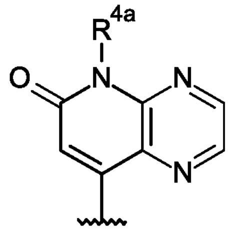 ;

wherein $R^4$ is $C_{1-6}$alkyl, $C_{1-6}$alkoxy, halo$C_{1-6}$alkyl, halogen, nitro or cyano; $R^{4a}$ is $C_{1-6}$alkyl or $C_{3-7}$cycloalkyl; $R^5$, $R^{5a}$ and $R^{5b}$ are independently selected from H and deuterium; $R^6$ is H or halogen;
$R^2$ is H or $C_{1-6}$alkyl;
$R^3$ is H;
Ring A is an unsubstituted or substituted 5-7 membered monocyclic aryl or heteroaryl; or an unsubstituted or substituted 7-12 membered bicyclic heterocyclyl;
n is 0, 1 or 2;

or a pharmaceutically acceptable salt thereof.

**2.** A compound according to claim 1, wherein
Ring A is 1,2,3,4-tetrahydroisoquinolinyl;

5,6,7,8-tetrahydro-1,6-naphthyridinyl;
5,6,7,8-tetrahydro-2,6-naphthyridinyl;
5,6,7,8-tetrahydro-2,7-naphthyridinyl;
isoindolinyl;
phenyl substituted by amino($C_{1-6}$alkoxy)pyrrolidinyl, 5-oxa-2,8-diazaspiro[3.5]nonanyl, morpholinyl or piperazinyl;
pyridinyl substituted once or twice by substituents independently selected from (halopyrrolidinyl)amino; 1,4-diazepanyl; 2,5-diazabicyclo[2.2.1]heptanyl; 3,4,4a,5,7,7a-hexahydro-2H-pyrrolo[3,4-b][1,4]oxazinyl; 3,6-diazabicyclo[3.1.1]heptanyl; 3,8-diazabicyclo[3.2.1]octanyl; 5-oxa-2,8-diazaspiro[3.5]nonanyl; 9-oxa-3,7-diazabicyclo[3.3.1]nonanyl; amino($C_{1-6}$alkoxy)pyrrolidinyl; amino($C_{1-6}$alkyl)azetidinyl; amino($C_{1-6}$alkyl)pyrrolidinyl; amino-1,4-oxazepanyl; amino-2-azaspiro[3.3]heptanyl; aminoazetidinyl; aminohalopyrrolidinyl; $C_{1-6}$alkyl; $C_{1-6}$alkyl-2,6-diazaspiro[3.3]heptanecarbonyl; $C_{1-6}$alkyl-2,6-diazaspiro[3.3]heptanyl; $C_{1-6}$alkylpiperazinyl and piperazinyl; or
pyrimidinyl substituted once or twice by substituents independently selected from amino($C_{1-6}$alkoxy)pyrrolidinyl, amino($C_{1-6}$alkyl)azetidinyl, aminoazetidinyl, $C_{1-6}$alkyl and piperazinyl.

**3.** A compound according to claim 1, of formula (Ib), wherein

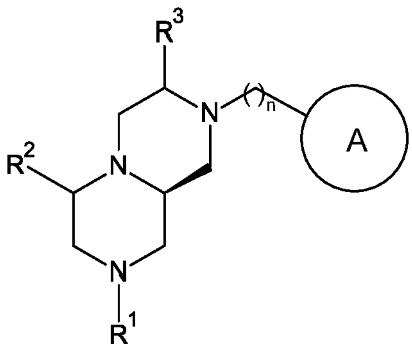

(Ib),

$R^1$ is

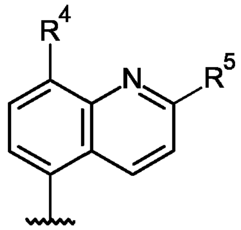, 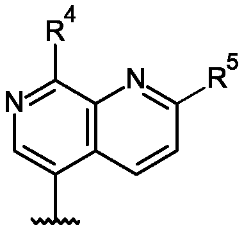, 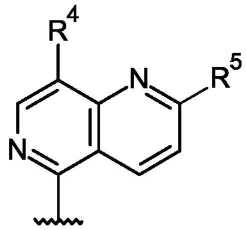, 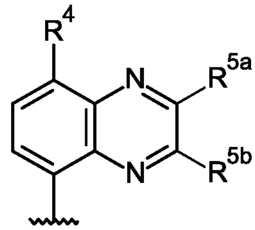,

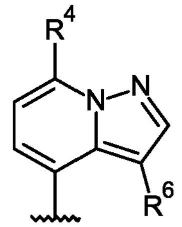, 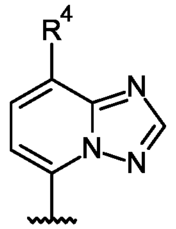, 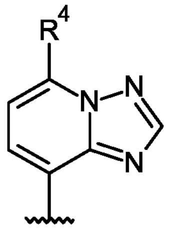, 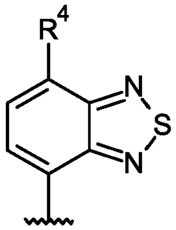, 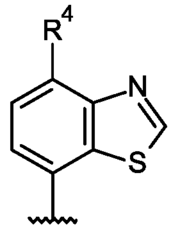,

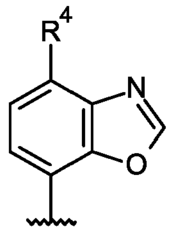, 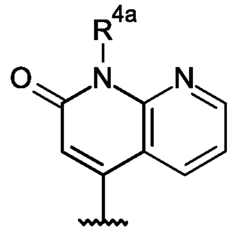 or 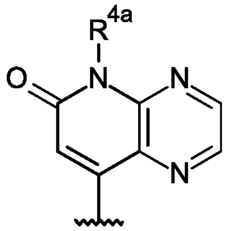;

wherein $R^4$ is $C_{1-6}$alkyl, $C_{1-6}$alkoxy, halo$C_{1-6}$alkyl, halogen, nitro or cyano; $R^{4a}$ is $C_{1-6}$alkyl or $C_{3-7}$cycloalkyl; $R^5$, $R^{5a}$ and $R^{5b}$ are independently selected from H and deuterium; $R^6$ is H or halogen;
$R^2$ is H or $C_{1-6}$alkyl;
$R^3$ is H;
Ring A is 1,2,3,4-tetrahydroisoquinolinyl;

5,6,7,8-tetrahydro-1,6-naphthyridinyl;
5,6,7,8-tetrahydro-2,6-naphthyridinyl;
5,6,7,8-tetrahydro-2,7-naphthyridinyl;
isoindolinyl;
phenyl substituted by amino($C_{1-6}$alkoxy)pyrrolidinyl, 5-oxa-2,8-diazaspiro[3.5]nonanyl, morpholinyl or piperazinyl;
pyridinyl substituted once or twice by substituents independently selected from (halopyrrolidinyl)amino; 1,4-diazepanyl; 2,5-diazabicyclo[2.2.1]heptanyl; 3,4,4a,5,7,7a-hexahydro-2H-pyrrolo[3,4-b][1,4]oxazinyl; 3,6-diazabicyclo[3.1.1]heptanyl; 3,8-diazabicyclo[3.2.1]octanyl; 5-oxa-2,8-diazaspiro[3.5]nonanyl; 9-oxa-3,7-diazabicyclo[3.3.1]nonanyl; amino($C_{1-6}$alkoxy)pyrrolidinyl; amino($C_{1-6}$alkyl)azetidinyl; amino($C_{1-6}$alkyl)pyrrolidinyl; amino-1,4-oxazepanyl; amino-2-azaspiro[3.3]heptanyl; aminoazetidinyl; aminohalopyrrolidinyl; $C_{1-6}$alkyl; $C_{1-6}$alkyl-2,6-diazaspiro[3.3]heptanecarbonyl; $C_{1-6}$alkyl-2,6-diazaspiro[3.3]heptanyl; $C_{1-6}$alkylpiperazinyl and piperazinyl; or

pyrimidinyl substituted once or twice by substituents independently selected from amino($C_{1-6}$alkoxy)pyrrolidinyl, amino($C_{1-6}$alkyl)azetidinyl, aminoazetidinyl, $C_{1-6}$alkyl and piperazinyl;

n is 0, 1 or 2;

or a pharmaceutically acceptable salt thereof.

**4.** A compound according to claim 2 or 3, wherein $R^1$ is

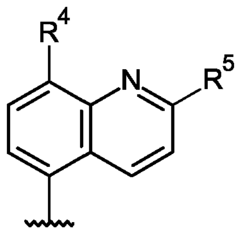
,
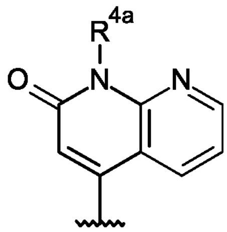
or
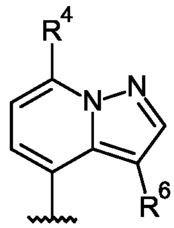
;

wherein $R^4$ is cyano; $R^{4a}$ is $C_{1-6}$alkyl; $R^5$ is H or deuterium; $R^6$ is H.

**5.** A compound according to claim 4, wherein $R^2$ is $C_{1-6}$alkyl.

**6.** A compound according to claim 5, wherein

$R^1$ is

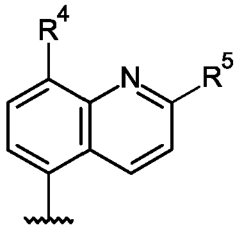
,
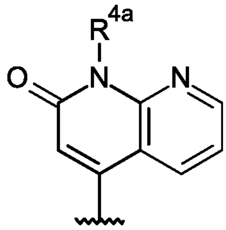
or
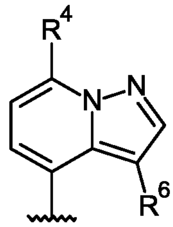
;

wherein $R^4$ is cyano; $R^{4a}$ is $C_{1-6}$alkyl; $R^5$ is H or deuterium; $R^6$ is H;

$R^2$ is methyl;

$R^3$ is H;

Ring A is 1,2,3,4-tetrahydroisoquinolin-7-yl; 1,2,3,4-tetrahydroisoquinolin-5-yl; 1,2,3,4-tetrahydroisoquinolin-6-yl; 1,2,3,4-tetrahydroisoquinolin-7-yl; 1,2,3,4-tetrahydroisoquinolin-8-yl; 5,6,7,8-tetrahydro-1,6-naphthyridin-2-yl; 5,6,7,8-tetrahydro-1,6-naphthyridin-3-yl; 5,6,7,8-tetrahydro-2,6-naphthyridin-1-yl; 5,6,7,8-tetrahydro-2,7-naphthyridin-4-yl; isoindolin-4-yl; 3-amino-4-methoxy-pyrrolidin-1-ylphenyl; 5-oxa-2,8-diazaspiro[3.5]nonan-2-ylphenyl; morpholin-2-ylphenyl; piperazin-1-ylphenyl; (4-fluoropyrrolidin-3-yl)amino(methyl)pyridinyl; 1,4-diazepan-1-ylpyridinyl; 2,5-diazabicyclo[2.2.1]heptan-2-yl(methyl)pyridinyl; 2,5-diazabicyclo[2.2.1]heptan-2-ylpyridinyl; 2-methylpiperazin-1-yl(methyl)pyridinyl; 3,4,4a,5,7,7a-hexahydro-2H-pyrrolo[3,4-b][1,4]oxazin-6-yl(methyl)pyridinyl; (3,4,4a,5,7,7a-hexahydro-2H-pyrrolo[3,4-b][1,4]oxazin-6-yl)pyridinyl; 3,6-diazabicyclo[3.1.1]heptan-3-yl(methyl)pyridinyl; 3,8-diazabicyclo[3.2.1]octan-3-yl(methyl)pyridinyl; 3,8-diazabicyclo[3.2.1]octan-3-ylpyridinyl; 3-amino-3-methyl-azetidin-1-yl(methyl)pyridinyl; 3-amino-3-methyl-azetidin-1-ylpyridinyl; 3-amino-3-methyl-pyrrolidin-1-yl(methyl)pyridinyl; 3-amino-3-methyl-pyrrolidin-1-ylpyridinyl; 3-amino-4-fluoro-pyrrolidin-1-ylpyridinyl; 3-amino-4-methoxy-pyrrolidin-1-yl(methyl)pyridinyl; 3-amino-4-methoxy-pyrrolidin-1 -ylpyridinyl; 3-aminoazetidin-1-ylpyridinyl; 3-methylpiperazin-1-yl(methyl)pyridinyl; 3-methylpiperazin-1-ylpyridinyl; 5-oxa-2,8-diazaspiro[3.5]nonan-2-ylpyridinyl; 6-amino-1,4-oxazepan-4-yl(methyl)pyridinyl; 6-amino-1,4-oxazepan-4-ylpyridinyl; 6-amino-2-azaspiro[3.3]heptan-2-yl(methyl)pyridinyl; 6-methyl-2,6-diazaspiro[3.3]heptan-2-yl(methyl)pyridinyl; 6-methyl-2,6-diazaspiro[3.3]heptan-2-ylpyridinyl; 6-methyl-2,6-diazaspiro[3.3]heptane-2-carbonyl(methyl)pyridinyl; 9-oxa-3,7-diazabicyclo[3.3.1]nonan-3-yl(methyl)pyridinyl; piperazin-1-yl(methyl)pyridinyl; piperazin-1-ylpyridinyl; 3-amino-3-methyl-azetidin-1-yl(methyl)pyrimidinyl; 3-amino-4-methoxy-pyrrolidin-1-yl(methyl)pyrimidinyl; 3-amino-4-methoxy-pyrrolidin-1-ylpyrimidinyl; 3-aminoazetidin-1-ylpyrimidinyl; piperazin-1-yl(methyl)pyrimidinyl or piperazin-1-ylpyrimidinyl;
n is 0, 1 or 2;

or a pharmaceutically acceptable salt thereof.

**7.** A compound according to claim 5, wherein $R^1$ is

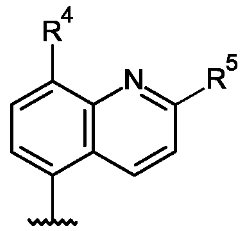

;

wherein $R^4$ is cyano; $R^5$ is H or deuterium.

**8.** A compound according to claim 7, wherein Ring A is 1,2,3,4-tetrahydroisoquinolinyl;

phenyl substituted by morpholinyl;
pyridinyl substituted once or twice by substituents independently selected from 3,4,4a,5,7,7a-hexahydro-2H-pyrrolo[3,4-b][1,4]oxazinyl; amino($C_{1-6}$alkoxy)pyrrolidinyl; amino($C_{1-6}$alkyl)azetidinyl; aminohalopyrrolidinyl; $C_{1-6}$alkyl; $C_{1-6}$alkyl-2,6-diazaspiro[3.3]heptanyl and piperazinyl;
pyrimidinyl substituted twice by substituents independently selected from amino($C_{1-6}$alkoxy)pyrrolidinyl; amino($C_{1-6}$alkyl)azetidinyl and $C_{1-6}$alkyl.

**9.** A compound according to claim 8, wherein ring A is 1,2,3,4-tetrahydroisoquinolinyl; morpholinylphenyl; piperazinylpyridinyl; (amino($C_{1-6}$alkoxy)pyrrolidinyl)pyridinyl; (aminohalopyrrolidinyl)pyridinyl; piperazinyl($C_{1-6}$alkyl)pyridinyl; (amino($C_{1-6}$alkoxy)pyrrolidinyl)pyridinyl; (aminohalopyrrolidinyl)pyridinyl; ($C_{1-6}$alkyl-2,6-diazaspiro[3.3]heptanyl)pyridinyl; (amino($C_{1-6}$alkyl)azetidinyl)pyridinyl; $C_{1-6}$alkyl(amino($C_{1-6}$alkyl)azetidinyl)pyridinyl; 3,4,4a,5,7,7a-hexahydro-2H-pyrrolo[3,4-b][1,4]oxazinyl($C_{1-6}$alkyl)pyridinyl; (3,4,4a,5,7,7a-hexahydro-2H-pyrrolo[3,4-b][1,4]oxazinyl)pyridinyl; $C_{1-6}$alkyl(amino($C_{1-6}$alkyl)azetidinyl)pyrimidinyl or $C_{1-6}$alkyl(amino($C_{1-6}$alkoxy)pyrrolidinyl)pyrimidinyl.

**10.** A compound according to claim 9, wherein ring A is 1,2,3,4-tetrahydroisoquinolin-7-yl; morpholin-2-ylphenyl; (3,4,4a,5,7,7a-hexahydro-2H-pyrrolo[3,4-b][1,4]oxazin-6-yl)-2-methyl-3-pyridinyl; (3,4,4a,5,7,7a-hexahydro-2H-pyrrolo[3,4-b][1,4]oxazin-6-yl)-3-pyridinyl; (3-amino-4-fluoro-pyrrolidin-1-yl)-3-pyridinyl; (3-amino-4-fluoro-pyrrolidin-1-yl)-3-pyridinyl; (3-amino-4-methoxy-pyrrolidin-1-yl)-3-pyridinyl; (3-amino-4-methoxy-pyrrolidin-1-yl)-3-pyridinyl; 2-methyl-6-piperazin-1-yl-3-pyridinyl; 6-(3-amino-3-methyl-azetidin-1-yl)-2-methyl-3-pyridinyl; 6-(3-amino-3-methyl-azetidin-1-yl)-3-pyridinyl; 6-(6-methyl-2,6-diazaspiro[3.3]heptan-2-yl)-3-pyridinyl; 6-piperazin-1-yl-3 -pyridinyl; (3 -amino-4-methoxy-pyrrolidin-1 -yl)-6-methyl-pyrimidin-4-yl or (3-amino-3-methyl-azetidin-1-yl)-6-methyl-pyrimidin-4-yl.

**11.** A compound according to claim 2 or 3, wherein

$R^1$ is

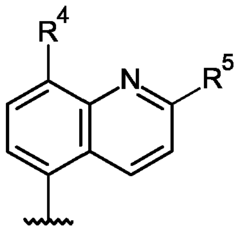

;

wherein $R^4$ is cyano; $R^5$ is H or deuterium;
$R^2$ is $C_{1-6}$alkyl;
$R^3$ is H;
Ring A is 1,2,3,4-tetrahydroisoquinolinyl;

phenyl substituted by morpholinyl;
pyridinyl substituted once or twice by substituents independently selected from 3,4,4a,5,7,7a-hexahydro-2H-pyrrolo[3,4-b][1,4]oxazinyl; amino($C_{1-6}$alkoxy)pyrrolidinyl; amino($C_{1-6}$alkyl)azetidinyl; aminohalopyrrolidinyl; $C_{1-6}$alkyl; $C_{1-6}$alkyl-2,6-diazaspiro[3.3]heptanyl and piperazinyl;
pyrimidinyl substituted twice by substituents independently selected from amino($C_{1-6}$alkoxy)pyrrolidinyl; amino($C_{1-6}$alkyl)azetidinyl and $C_{1-6}$alkyl;

n is 0, 1 or 2;

or a pharmaceutically acceptable salt thereof.

**12.** A compound according to claim 11, wherein

$R^1$ is

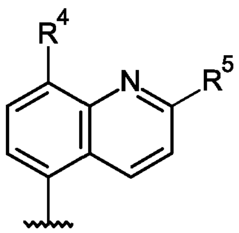

;

wherein $R^4$ is cyano; $R^5$ is H or deuterium;
$R^2$ is $C_{1-6}$alkyl;
$R^3$ is H;
Ring A is 1,2,3,4-tetrahydroisoquinolinyl; morpholinylphenyl; piperazinylpyridinyl; (amino($C_{1-6}$alkoxy)pyrrolidinyl)pyridinyl; (aminohalopyrrolidinyl)pyridinyl; piperazinyl($C_{1-6}$alkyl)pyridinyl; (amino($C_{1-6}$alkoxy)pyrrolidinyl)pyridinyl; (aminohalopyrrolidinyl)pyridinyl; ($C_{1-6}$alkyl-2,6-diazaspiro[3.3]heptanyl)pyridinyl; (amino($C_{1-6}$alkyl)azetidinyl)pyridinyl; $C_{1-6}$alkyl(amino($C_{1-6}$alkyl)azetidinyl)pyridinyl; 3,4,4a,5,7,7a-hexahydro-2H-pyrrolo[3,4-b][1,4]oxazinyl($C_{1-6}$alkyl)pyridinyl; (3,4,4a,5,7,7a-hexahydro-2H-pyrrolo[3,4-b][1,4]oxazinyl)pyridinyl; $C_{1-6}$alkyl(amino($C_{1-6}$alkyl)azetidinyl)pyrimidinyl or $C_{1-6}$alkyl(amino($C_{1-6}$alkoxy)pyrrolidinyl)pyrimidinyl;
n is 0, 1 or 2;

or a pharmaceutically acceptable salt thereof.

**13.** A compound according to claim 11, wherein

$R^1$ is

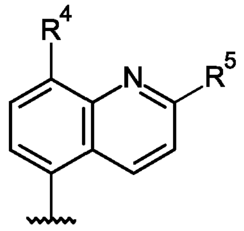

;

wherein $R^4$ is cyano; $R^5$ is H or deuterium;
$R^2$ is methyl;
$R^3$ is H;
Ring A is 1,2,3,4-tetrahydroisoquinolin-7-yl; 4-(morpholin-2-yl)phenyl; (3,4,4a,5,7,7a-hexahydro-2H-pyrrolo[3,4-b][1,4]oxazin-6-yl)-2-methyl-3-pyridinyl; (3,4,4a,5,7,7a-hexahydro-2H-pyrrolo[3,4-b][1,4]oxazin-6-yl)-3-pyridinyl; (3-amino-4-fluoro-pyrrolidin-1-yl)-3-pyridinyl; (3-amino-4-fluoro-pyrrolidin-1-yl)-3-pyridinyl; (3 -amino-4-methoxy-pyrrolidin-1-yl)-3 -pyridinyl; (3-amino-4-methoxy-pyrrolidin-1-yl)-3 -pyridinyl; 2-methyl-6-piperazin-1-yl-3-pyridinyl; 6-(3-amino-3-methyl-azetidin-1-yl)-2-methyl-3-pyridinyl; 6-(3-amino-3-methyl-azetidin-1-yl)-3-pyridinyl; 6-(6-methyl-2,6-diazaspiro[3.3]heptan-2-yl)-3-pyridinyl; 6-piperazin-1-yl-3-pyridinyl; (3-amino-4-methoxy-pyrrolidin-1-yl)-6-methyl-pyrimidin-4-yl or (3-amino-3-methyl-azetidin-1-yl)-6-methyl-pyrimidin-4-yl;
n is 0, 1 or 2;

or a pharmaceutically acceptable salt thereof.

**14.** A compound according to any one of claims 1 to 13, wherein the compound is selected from:

5-[*cis*-4-methyl-8-(1,2,3,4-tetrahydroisoquinolin-7-ylmethyl)-3,4,6,7,9,9a-hexahydro-*1H*-pyrazino[1,2-a]pyrazin-2-yl]quinoline-8-carbonitrile;
5-[*cis*-4-methyl-8-(1,2,3,4-tetrahydroisoquinolin-5-yl)-3,4,6,7,9,9a-hexahydro-*1H*-pyrazino[1,2-a]pyrazin-2-yl]quinoline-8-carbonitrile;
5-[*cis*-4-methyl-8-[(2-piperazin-1-yl-4-pyridyl)methyl]-3,4,6,7,9,9a-hexahydro-*1H*-pyrazino[1,2-a]pyrazin-2-yl]quinoline-8-carbonitrile;
5-[*cis*-8-isoindolin-4-yl-4-methyl-3,4,6,7,9,9a-hexahydro-*1H*-pyrazino[1,2-a]pyrazin-2-yl] quinoline-8-carbonitrile;
5-[(*4R*,*9aS*)-4-methyl-8-(1,2,3,4-tetrahydroisoquinolin-7-ylmethyl)-3,4,6,7,9,9a-hexahydro-*1H*-pyrazino[1,2-a]pyrazin-2-yl]quinoline-8-carbonitrile;
5-[(*4R*,*9aS*)-4-methyl-8-(5,6,7,8-tetrahydro-1,6-naphthyridin-3-ylmethyl)-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-(4-piperazin-1-ylphenyl)ethyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[(6-piperazin-1-yl-3-pyridyl)methyl]-3,4,6,7,9,9a-hexahydro-*1H*-pyrazino[1,2-a]pyrazin-2-yl]quinoline-8-carbonitrile;
5-[(*4R*,*9aS*)-8-isoindolin-4-yl-4-methyl-3,4,6,7,9,9a-hexahydro-*1H*-pyrazino[1,2-a]pyrazin-2-yl]quinoline-8-carbonitrile;
5-[(*4R*,*9aS*)-4-methyl-8-(1,2,3,4-tetrahydroisoquinolin-6-ylmethyl)-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-(6-piperazin-1-yl-3-pyridyl)ethyl]-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3R*,*4R*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[5-[(*3R*,*4R*)-3-amino-4-methoxy-pyrrolidin-1-yl]-2-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[[6-(1,4-diazepan-1-yl)-3-pyridyl]methyl]-4-methyl-3,4,6,7,9,9a-hexahydro-*1H*-pyrazino[1,2-a]pyrazin-2-yl]quinoline-8-carbonitrile;
5-[(*4R*,*9aR*)-4-methyl-8-(5,6,7,8-tetrahydro-2,6-naphthyridin-1-yl)-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3R*,*4S*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3R*,*4R*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[5-[(*3R*,*4S*)-3-amino-4-fluoro-pyrrolidin-1-yl]-2-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[5-[(*3S*)-3-methylpiperazin-1-yl]-2-pyridyl]ethyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[6-[(*3S*)-3-methylpiperazin-1-yl]-2-pyridyl]ethyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[6-(6-methyl-2,6-diazaspiro[3.3]heptan-2-yl)-3-pyridyl]ethyl]-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3R*,*4S*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*3S*,*4S*)-3-amino-4-methoxy-pyrrolidin-1-yl]-3 -pyridyl]ethyl] -4-methyl-3,4,6,7,9,9a-hexahydro-*1H*-pyrazino[1,2-a]pyrazin-2-yl]quinoline-8-carbonitrile;
5-[(*4R*,*9aS)*-8-[2-[4-[(*3R*,*4R*)-3-amino-4-methoxy-pyrrolidin-1-yl]phenyl]ethyl]-4-methyl-3,4,6,7,9,9a-hexahydro-*1H*-pyrazino[1,2-a]pyrazin-2-yl]quinoline-8-carbonitrile;
5-[(*4R*,*9aS*)-4-methyl-8-[(6-piperazin-1-yl-2-pyridyl)methyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[4-(5-oxa-2,8-diazaspiro[3.5]nonan-2-yl)phenyl]ethyl]-3,4,6,7,9,9a-hexahydro-*1H*-pyrazino[1,2-a]pyrazin-2-yl]quinoline-8-carbonitrile;
5-[(*4R*,*9aR*)-4-methyl-8-(4-piperazin-1-ylpyrimidin-2-yl)-3,4,6,7,9,9a-hexahydro-*1H*-pyrazino[1,2-a]pyrazin-2-yl]quinoline-8-carbonitrile;
5-[(*4R*,*9aR*)-4-methyl-8-(6-methyl-2-piperazin-1-yl-pyrimidin-4-yl)-3,4,6,7,9,9a-hexahydro-1*H*-pyrazino[1,2-a]pyrazin-2-yl]quinoline-8-carbonitrile;
5-[(*4R*,*9aS*)-4-methyl-8-(2-piperazin-1-yl-4-pyridyl)-3,4,6,7,9,9a-hexahydro-1*H*-pyrazino[1,2-a]pyrazin-2-yl]quinoline-8-carbonitrile;
5-[(*4R*,*9aR*)-4-methyl-8-(2-piperazin-1-ylpyrimidin-4-yl)-3,4,6,7,9,9a-hexahydro-1*H*-pyrazino[1,2-a]pyrazin-2-yl]quinoline-8-carbonitrile;
5-[(*4R*,*9aR*)-4-methyl-8-(4-piperazin-1-yl-2-pyridyl)-3,4,6,7,9,9a-hexahydro-1*H*-pyrazino[1,2-a]pyrazin-2-yl]quinoline-8-carbonitrile;
5-(8-isoindolin-4-yl-3,4,6,7,9,9a-hexahydro-1*H*-pyrazino[1,2-a]pyrazin-2-yl)quinoline-8-carbonitrile;
5-[(*4R*,*9aR*)-8-[2-[(*3R*,*4R*)-3-amino-4-methoxy-pyrrolidin-1-yl]pyrimidin-4-yl]-4-methyl-3,4,6,7,9,9a-hexahydro-1*H*-pyrazino[1,2-a]pyrazin-2-yl]quinoline-8-carbonitrile;
5-[(*4R*,*9aR*)-8-[4-[(*3R*,*4R*)-3-amino-4-methoxy-pyrrolidin-1-yl]pyrimidin-2-yl]-4-methyl-3,4,6,7,9,9a-hexahydro-1*H*-pyrazino[1,2-a]pyrazin-2-yl]quinoline-8-carbonitrile;
5-[2-(4-methyl-6-piperazin-1-yl-3-pyridyl)-3,4,6,7,9,9a-hexahydro-1*H*-pyrazino[1,2-a]pyrazin-8-yl]quinoline-8-carbonitrile;
5-[(*4R*,*9aR*)-8-[4-(3-aminoazetidin-1-yl)pyrimidin-2-yl]-4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[(*3S*,*4R*)-3-amino-4-fluoro-pyrrolidin-1-yl]-4-pyridyl]-4-methyl-3,4,6,7,9,9a-hexahydro-1*H*-pyrazino[1,2-a]pyrazin-2-yl]quinoline-8-carbonitrile;
5-[(*4R*,*9aS*)-8-[2-(3-aminoazetidin-1-yl)-4-pyridyl]-4-methyl-3,4,6,7,9,9a-hexahydro-1*H*-pyrazino[1,2-a]pyrazin-2-yl]quinoline-8-carbonitrile;
5-[(*4R*,*9aS*)-4-methyl-8-(5,6,7,8-tetrahydro-2,7-naphthyridin-4-yl)-3,4,6,7,9,9a-hexahydro-1*H*-pyrazino[1,2-a]pyrazin-2-yl]quinoline-8-carbonitrile;
5-[(*4R*,*9aR*)-4-methyl-8-(5-methyl-4-piperazin-1-yl-pyrimidin-2-yl)-3,4,6,7,9,9a-hexahydro-1*H*-pyrazino[1,2-a]pyrazin-2-yl]quinoline-8-carbonitrile;
5-[(*4R*,*9aR*)-4-methyl-8-(5,6,7,8-tetrahydro-2,7-naphthyridin-4-yl)-3,4,6,7,9,9a-hexahydro-1*H*-pyrazino[1,2-a]pyrazin-2-yl]quinoline-8-carbonitrile;
5-[(*4R*,*9aR*)-8-[2-[(*3R*,*4R*)-3-amino-4-methoxy-pyrrolidin-1-yl]-6-methyl-pyrimidin-4-yl]-4-methyl-3,4,6,7,9,9a-hexahydro-1*H*-pyrazino[1,2-a]pyrazin-2-yl]quinoline-8-carbonitrile;
5-[2-[6-[(*6R*)-6-amino-1,4-oxazepan-4-yl]-4-methyl-3-pyridyl]-3,4,6,7,9,9a-hexahydro-1*H*-pyrazino[1,2-a]pyrazin-8-yl]quinoline-8-carbonitrile;
5-[*trans*-4-methyl-8-(1,2,3,4-tetrahydroisoquinolin-5-yl)-3,4,6,7,9,9a-hexahydro-1*H*-pyrazino[1,2-a]pyrazin-2-yl]quinoline-8-carbonitrile;
5-[(*4R*,*9aS*)-8-[2-[(*6S*)-6-amino-1,4-oxazepan-4-yl]-4-pyridyl]-4-methyl-3,4,6,7,9,9a-hexahydro-1*H*-pyrazino[1,2-a]pyrazin-2-yl]quinoline-8-carbonitrile;
5-[(*4R*,*9aS*)-8-[2-[(*6R*)-6-amino-1,4-oxazepan-4-yl]-4-pyridyl]-4-methyl-3,4,6,7,9,9a-hexahydro-1*H*-pyrazino[1,2-a]pyrazin-2-yl]quinoline-8-carbonitrile;

5-[(*4R*,*9aR*)-8-[2-(3-amino-3 -methyl-azetidin-1-yl)-6-methyl-pyrimidin-4-yl]-4-methyl-3,4,6,7,9,9a-hexahydro-1*H*-pyrazino[1,2-a]pyrazin-2-yl]quinoline-8-carbonitrile;
5-[(*4R*,*9aS*)-8-[2-( -amino-3 -methyl-azetidin-1-yl)-4-pyridyl]-4-methyl-3,4,6,7,9,9a-hexahydro-1*H*-pyrazino[1,2-a]pyrazin-2-yl]quinoline-8-carbonitrile;
5-[(*4R*,*9aS*)-4-methyl-8-[(5-piperazin-1-yl-3 -pyridyl)methyl] -3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[6-[(*3R*)-3-methylpiperazin-1-yl]-2-pyridyl]ethyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[2-[6-(5-oxa-2,8-diazaspiro[3.5]nonan-2-yl)-3-pyridyl]ethyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[(2-methyl-6-piperazin-1-yl-3 -pyridyl)methyl] -3,4,6,7,9,9a-hexahydro-*1H*-pyrazino[1,2-a]pyrazin-2-yl]quinoline-8-carbonitrile;
5-[(*4R*,*9aS*)-4-methyl-8-(1,2,3,4-tetrahydroisoquinolin-7-ylmethyl)-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[6-[(*3R*,*4R*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[6-[(*3R*,4*S*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[2-[6-(6-methyl-2,6-diazaspiro[3.3]heptan-2-yl)-3-pyridyl]ethyl]-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[6-(3-amino-3 -methyl-azetidin-1-yl)-3 -pyridyl] ethyl] -4-methyl-3,4,6,7,9,9a-hexahydro-*1H*-pyrazino[1,2-a]pyrazin-2-yl]quinoline-8-carbonitrile;
4-[(*4R*,*9aS*)-8-[2-[4-[(*3R*,*4R*)-3-amino-4-methoxy-pyrrolidin-1-yl]phenyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile;
4-[(*4R*,*9aS*)-8-[2-[6-[(*3R*,*4R*)-3-amino-4-methoxy-pyrrolidin-1-yl]-3-pyridyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile;
4-[(*4R*,*9aS*)-8-[2-[4-[(*3R*,*4R*)-3-amino-4-methoxy-pyrrolidin-1-yl]phenyl]ethyl]-4-methyl-3,4,6,7,9,9a-hexahydro-*1H*-pyrazino[1,2-a]pyrazin-2-yl]pyrazolo[1,5-a]pyridine-7-carbonitrile;
4-[(*4R*,*9aS*)-4-methyl-8-(1,2,3,4-tetrahydroisoquinolin-7-ylmethyl)-3,4,6,7,9,9a-hexahydro-*1H*-pyrazino[1,2-a]pyrazin-2-yl]-3-fluoro-pyrazolo[1,5-a]pyridine-7-carbonitrile;
5-[(*4R*,*9aS*)-4-methyl-8-(1,2,3,4-tetrahydroisoquinolin-5-ylmethyl)-3,4,6,7,9,9a-hexahydro-*1H*-pyrazino[1,2-a]pyrazin-2-yl]quinoline-8-carbonitrile;
5-[(*4R*,*9aS*)-4-methyl-8-(1,2,3,4-tetrahydroisoquinolin-8-ylmethyl)-3,4,6,7,9,9a-hexahydro-1H-pyrazino[1,2-a]pyrazin-2-y1]quinoline-8-carbonitrile;
5-[(*4R*,*9aS*)-8-(isoindolin-4-ylmethyl)-4-methy1-3,4,6,7,9,9a-hexahydro-*1H*-pyrazino[1,2-a]pyrazin-2-yl]quinoline-8-carbonitrile;
5-[(*4R*,*9aS*)-8-[2-(3-amino-3-methyl-azetidin-1-yl)-6-methyl-4-pyridyl]-4-methyl-3,4,6,7,9,9a-hexahydro-1*H*-pyrazino[1,2-a]pyrazin-2-yl]quinoline-8-carbonitrile;
4-[(*4R*,*9aS*)-8-[2-[6-(3-amino-3 -methyl-azetidin-1 -yl)-3 -pyridyl] ethyl] -4-methyl-3,4,6,7,9,9a-hexahydro-*1H*-pyrazino[1,2-a]pyrazin-2-yl]-3-fluoro-pyrazolo[1,5-a]pyridine-7-carbonitrile;
4-[(*4R*,*9aS*)-8-[2-[6-[(*3R*,*4S*)-3-amino-4-fluoro-pyrrolidin-1-yl]-3-pyridyl]ethyl]-4-methyl-3,4,6,7,9,9a-hexahydro-*1H*-pyrazino[1,2-a]pyrazin-2-yl]-3-fluoro-pyrazolo[1,5-a]pyridine-7-carbonitrile;
5-[(*4R*,*9aS*)-8-[[6-(3 -amino-3 -methyl-azetidin-1-yl)-2-methyl-3 -pyridyl] methyl] -4-methyl-3,4,6,7,9,9a-hexahydro-*1H*-pyrazino[1,2-a]pyrazin-2-yl]quinoline-8-carbonitrile;
5-[(*4R*,*9aS*)-8-[2-[6-[(*4a*R,*7aR*)-3,4,4a,5,7,7a-hexahydro-*2H*-pyrrolo[3,4-b][1,4]oxazin-6-yl] -3 -pyridyl] ethyl] -4-methyl-3 ,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[(6-piperazin-1-yl-3 -pyridyl)methyl] -3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-(3 -amino-3 -methyl-azetidin-1-yl)-2-methyl-3 -pyridyl] methyl] -4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[[2-methyl-6-(6-methyl-2,6-diazaspiro[3.3 heptan-2-yl)-3-pyridyl]methyl]-3,4,6,7,9,9a-hexahydro-1*H*-pyrazino[1,2-a]pyrazin-2-yl]-2-deuterio-quinoline-8-carbonitrile;
5-[(*4R*,*9aS*)-8-[[6-[(3R)-3-amino-3 -methyl-pyrrolidin-1-yl] -2-methyl-3 -pyridyl]methyl] -4-methyl-3,4,6,7,9,9a-hexahydro-1*H*-pyrazino[1,2-a]pyrazin-2-yl]-2-deuterio-quinoline-8-carbonitrile;
5-[(*4R*,*9aS*)-8-[[6-[[(*3S*,*4R*)-4-fluoropyrrolidin-3 -yl]amino]-2-methyl-3-pyridyl] methyl] -4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[[2-methyl-6-(9-oxa-3,7-diazabicyclo[3.3.1]nonan-3-yl)-3-pyridyl]methyl]-3,4,6,7,9,9a-hexahydro-*1H*-pyrazino[1,2-a]pyrazin-2-yl]-2-deuterio-quinoline-8-carbonitrile;

5-[(*4R*,*9aS*)-8-[2-[6-[(3*R*)-3-amino-3 -methyl-pyrrolidin-1-yl] -3 -pyridyl] ethyl]-4-methyl-3,4,6,7,9,9a-hexahydro-1H-pyrazino[1,2-a]pyrazin-2-yl]-2-deuterio-quinoline-8-carbonitrile;
5-[(*4R*,*9aS*)-4-methyl-8-[[2-methyl-6-[(*2S*)-2-methylpiperazin-1-yl]-3-pyridyl]methyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[[2-methyl-6-[(*3R*)-3-methylpiperazin-1-yl]-3-pyridyl]methyl]-3,4,6,7,9,9a-hexahydro-*1H*-pyrazino[1,2-a]pyrazin-2-yl]quinoline-8-carbonitrile;
5-[(*4R*,*9aS*)-4-methyl-8-[(4-methyl-6-piperazin-1-yl-3-pyridyl)methyl]-3,4,6,7,9,9a-hexahydro-*1H*-pyrazino[1,2-a]pyrazin-2-yl]-2-deuterio-quinoline-8-carbonitrile;
5-[(*4R*,*9aS*)-4-methyl-8-[(3-methyl-6-piperazin-1-yl-2-pyridyl)methyl]-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[2-[(*3R*,*4S*)-3 -amino-4-fluoro-pyrrolidin-1-yl]-4-pyridyl]ethyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-(3,6-diazabicyclo[3.1.1]heptan-3 -yl)-2-methyl-3 -pyridyl]methyl] -4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-(3,8-diazabicyclo[3.2.1]octan-3-yl)-2-methyl-3-pyridyl]methyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-[(*1S*,*4S*)-2,5-diazabicyclo[2.2.1]heptan-2-yl]-2-methyl-3-pyridyl] methyl] -4-methyl-3 ,4,6,7,9,9a-hexahydro-*1H*-pyrazino[1,2-a]pyrazin-2-yl]-2-deuterio-quinoline-8-carbonitrile;
4-[(*4R*,*9aR*)-4-methyl-8-(3-methyl-5-piperazin-1-yl-2-pyridyl)-3,4,6,7,9,9a-hexahydro-*1H*-pyrazino[1,2-a]pyrazin-2-yl]-1-methyl-1,8-naphthyridin-2-one;
5-[(*4R*,*9aS*)-8-[[6-[(*1S*,*4S*)-2,5-diazabicyclo[2.2.1]heptan-2-yl]-4-methyl-3-pyridyl] methyl] -4-methyl-3 ,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-[(*1S*,*4S*)-2,5-diazabicyclo[2.2.1]heptan-2-yl]-3-methyl-2-pyridyl]-4-methyl-3,4,6,7,9,9a-hexahydro-1H-pyrazino[1,2-a]pyrazin-2-yl]-2-deuterio-quinoline-8-carbonitrile;
5-[(*4R*,*9aR*)-8-[5-[(*3R*,*4S*)-3-amino-4-methoxy-pyrrolidin-1-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-[(*3R*)-3-amino-3 -methyl-pyrrolidin-1-yl]-3 -methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aS*)-8-[[6-[(*4aR*,*7aR*)-3,4,4a,5,7,7a-hexahydro-*2H*-pyrrolo[3,4-b][1,4]oxazin-6-yl] -2-methyl-3 -pyridyl] methyl] -4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-[(*3R*,*4R*)-3-amino-4-methoxy-pyrrolidin-1-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-[(*4aR*,*7aR*)-3,4,4a,5,7,7a-hexahydro-*2H*-pyrrolo[3,4-b][1,4]oxazin-6-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-(6-amino-2-azaspiro[3.3]heptan-2-yl)-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-4-methyl-8-[3-methyl-5-(6-methyl-2,6-diazaspiro[3.3]heptane-2-carbonyl)-2-pyridyl] -3,4,6,7,9,9a-hexahydro-*1H*-pyrazino[1,2-a]pyrazin-2-yl]-2-deuterio-quinoline-8-carbonitrile;
5-[(*4S*,*9aR*)-4-methyl-8-[3-methyl-5-(6-methyl-2,6-diazaspiro[3.3]heptane-2-carbonyl)-2-pyridyl] -3,4,6,7,9,9a-hexahydro-*1H*-pyrazino[1,2-a]pyrazin-2-yl]-2-deuterio-quinoline-8-carbonitrile;
5-[(*4R*,*9aS*)-8-[2-[(*3R*)-3-amino-3-methyl-pyrrolidin-1-yl]-4-pyridyl] -4-methyl-3,4,6,7,9,9a-hexahydro-*1H*-pyrazino[1,2-a]pyrazin-2-yl]quinoline-8-carbonitrile;
5-[(*4S*,*9aS*)-4-methyl-8-[4-[(*2R*)-morpholin-2-yl]phenyl]-3,4,6,7,9,9a-hexahydro-*1H*-pyrazino[1,2-a]pyrazin-2-yl]-2-deuterio-quinoline-8-carbonitrile;
5-[(*4S*,*9aS*)-4-methyl-8-[4-[(*2S*)-morpholin-2-yl]phenyl]-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[5-(3,6-diazabicyclo[3.1.1]heptan-3 -yl)-3 -methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4R*,*9aR*)-8-[5-[2-[(*3S*,*4S*)-3-amino-4-methoxy-pyrrolidin-1-yl]ethyl]-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[5-[(*6R*)-6-amino-1,4-oxazepan-4-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[5-[(*1S*,*4S*)-2,5-diazabicyclo[2.2.1]heptan-2-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[6-(3,8-diazabicyclo[3.2.1]octan-3-yl)-3-pyridyl]-4-methyl-3,4,6,7,9,9a-hexahydro-*1H*-pyrazino[1,2-a]pyrazin-2-yl]-2-deuterio-quinoline-8-carbonitrile;
4-[(*4S*,*9aR*)-4-methyl-8-[3-methyl-5-(6-methyl-2,6-diazaspiro[3.3]heptan-2-yl)-2-pyridyl]-3,4,6,7,9,9a-hexahydro-*1H*-pyrazino[1,2-a]pyrazin-2-yl]-1-methyl-1,8-naphthyridin-2-one;

5-[(*4S*,*9aR*)-8-[5-[(*4aR*,*7aR*)-3,4,4a,5,7,7a-hexahydro-*2H*-pyrrolo[3,4-b][1,4]oxazin-6-yl]-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[6-[(*3R*,*4R*)-3-amino-4-methoxy-pyrrolidin-1-yl]-3-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-8-[6-[2-[(*3R*,*4S*)-3-amino-4-fluoro-pyrrolidin-1 -yl]ethyl] -3 -pyridyl]-4-methyl-3,4,6,7,9,9a-hexahydro-1*H*-pyrazino[1,2-a]pyrazin-2-yl]-2-deuterio-quinoline-8-carbonitrile;
5-[(*4S*,*9aR*)-8-[5-(3-amino-3-methyl-azetidin-1-yl)-3-methyl-2-pyridyl]-4-methyl-3,4,6,7,9,9a-hexahydro-*1H*-pyrazino[1,2-a]pyrazin-2-yl]-2-deuterio-quinoline-8-carbonitrile;
5-[(*4S*,*9aR*)-4-methyl-8-(5,6,7,8-tetrahydro-1,6-naphthyridin-2-yl)-3,4,6,7,9,9a-hexahydro-1*H*-pyrazino[1,2-a]pyrazin-2-yl]-2-deuterio-quinoline-8-carbonitrile;
5-[(4*S*,9*aR*)-8-[5-[2-[(3*S*,4*S*)-3-amino-4-methoxy-pyrrolidin-1-yl]ethyl]-2-pyridyl]-4-methy1-3,4,6,7,9,9a-hexahydro-1*H*-pyrazino[1,2-a]pyrazin-2-yl]-2-deuterio-quinoline-8-carbonitrile;
5-[(4*S*,9*aR*)-8-[5-[[(3*R*,4*R*)-3-amino-4-methoxy-pyrrolidin-1-yl]methyl]-6-methyl-2-pyridyl]-4-methyl-3,4,6,7,9,9a-hexahydro-1*H*-pyrazino[l,2-a]pyrazin-2-yl]-2-deuterio-quinoline-8-carbonitrile;
5-[(*4R*,*9aS*)-8-[2-[6-[(*4aR*,*7aR*)-3,4,4a,5,7,7a-hexahydro-2*H*-pyrrolo[3,4-b][1,4]oxazin-6-yl] -3 -pyridyl] ethyl] -4-methyl-3 ,4,6,7,9,9a-hexahydro-1*H*-pyrazino[1,2-a]pyrazin-2-yl]quinoline-8-carbonitrile; and
5-[(*4S*,*9aS*)-4-methyl-8-[4-[(*2R*)-morpholin-2-yl]phenyl]-3,4,6,7,9,9a-hexahydro-*1H*-pyrazino[1,2-a]pyrazin-2-yl]quinoline-8-carbonitrile;
or a pharmaceutically acceptable salt thereof.

**15.** A process for the preparation of a compound according to any one of claims 1 to 14 comprising any one of the following steps:

a) Buchwald-Hartwig amination reaction or nucleophilic substitution between compound of formula (IX),

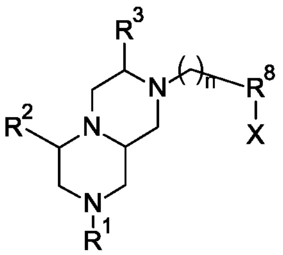

(IX),

and amine (X),

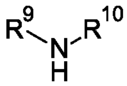

(X);

b) Buchwald-Hartwig amination reaction or nucleophilic substitution between compound of formula (V),

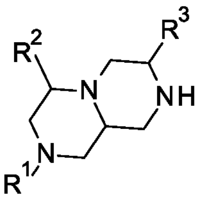

(V),

and compound of formula (VI),

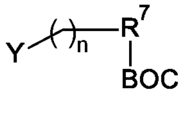

(VI);

wherein n is 0, 1 or 2; X is halogen; Y is halogen or methanesulfonate; $R^7$ and $R^8$ is aryl or heteroaryl; $R^9$ and $R^{10}$ together with the nitrogen atom they are attached to form a heterocyclyl.

**16.** A compound or pharmaceutically acceptable salt according to any one of claims 1 to 14 for use as therapeutically active substance.

**17.** A pharmaceutical composition comprising a compound in accordance with any one of claims 1 to 14 and a therapeutically inert carrier.

**18.** A compound or pharmaceutically acceptable salt, enantiomer or diastereomer according to any one of claims 1 to 14 for use in the treatment or prophylaxis of systemic lupus erythematosus or lupus nephritis.

## Patentansprüche

**1.** Verbindung der Formel (I)

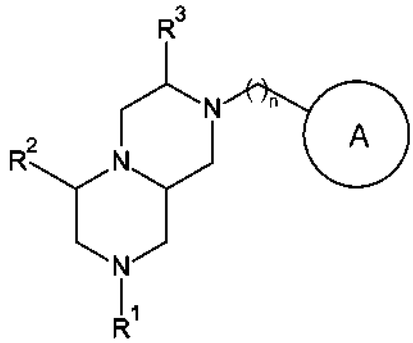

(I),

wobei

$R^1$

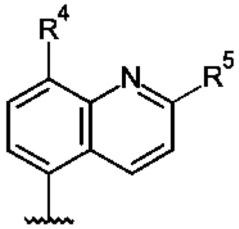

,

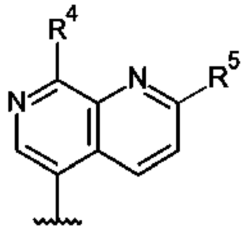

,

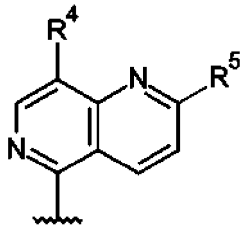

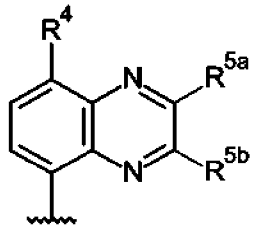

,

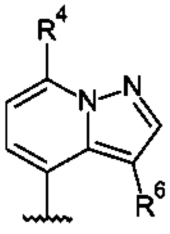

,

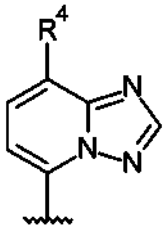

,

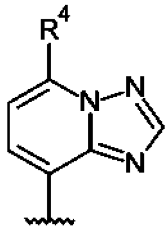

,

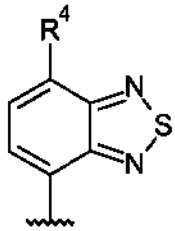

,

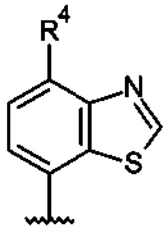

,

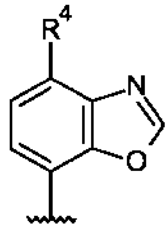

,

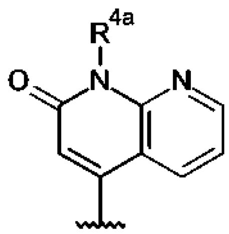

oder

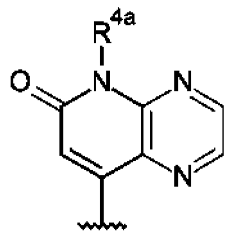

ist; wobei $R^4$ $C_{1-6}$-Alkyl, $C_{1-6}$-Alkoxy, Halogen-$C_{1-6}$-alkyl, Halogen, Nitro oder Cyano ist; $R^{4a}$ $C_{1-6}$-Alkyl oder $C_{3-7}$-Cycloalkyl ist; $R^5$, $R^{5a}$ und $R^{5b}$ unabhängig voneinander aus H und Deuterium ausgewählt sind; $R^6$ H oder Halogen ist;
$R^2$ H oder $C_{1-6}$-Alkyl ist;
$R^3$ H ist;
Ring A ein unsubstituiertes oder substituiertes 5- bis 7-gliedriges monocyclisches Aryl oder Heteroaryl oder ein unsubstituiertes oder substituiertes 7- bis 12-gliedriges bicyclisches Heterocyclyl ist;
n 0, 1 oder 2 ist;

oder ein pharmazeutisch unbedenkliches Salz davon.

**2.** Verbindung nach Anspruch 1, wobei
Ring A 1,2,3,4-Tetrahydroisochinolinyl;

5, 6,7, 8-Tetrahydro-1,6-naphthyridinyl;
5,6,7, 8-Tetrahydro-2,6-naphthyridinyl;
5,6,7,8-Tetrahydro-2,7-naphthyridinyl;
Isoindolinyl;
Phenyl, das mit Amino-($C_{1-6}$-alkoxy)pyrrolidinyl, 5-Oxa-2,8-diazaspiro[3.5]nonanyl, Morpholinyl oder Piperazinyl substituiert ist;
Pyridinyl, das einmal oder zweimal mit Substituenten substituiert ist, die unabhängig voneinander aus (Halogenpyrrolidinyl)amino; 1,4-Diazepanyl; 2,5-Diazabicyclo[2.2.1]heptanyl; 3,4,4a,5,7,7a-Hexahydro-2H-pyrrolo[3,4-b][1,4]oxazinyl; 3,6-Diazabicyclo[3.1.1]heptanyl; 3,8-Diazabicyclo[3.2.1]octanyl; 5-Oxa-2,8-diazaspiro[3.5]nonanyl; 9-Oxa-3,7-diazabicyclo[3.3.1]nonanyl; Amino-($C_{1-6}$-alkoxy)pyrrolidinyl; Amino-($C_{1-6}$-alkyl)azetidinyl; Amino-($C_{1-6}$-alkyl)pyrrolidinyl; Amino-1,4-oxazepanyl; Amino-2-azaspiro[3.3]heptanyl; Aminoazetidinyl; Aminohalogenpyrrolidinyl; $C_{1-6}$-Alkyl; $C_{1-6}$-Alkyl-2,6-diazaspiro[3.3]heptancarbonyl; $C_{1-6}$-Alkyl-2,6-diazaspiro[3.3]heptanyl; $C_{1-6}$-Alkylpiperazinyl und Piperazinyl ausgewählt sind; oder
Pyrimidinyl, das einmal oder zweimal mit Substituenten substituiert ist, die unabhängig voneinander aus Amino-($C_{1-6}$-alkoxy)pyrrolidinyl, Amino-($C_{1-6}$-alkyl)azetidinyl, Aminoazetidinyl, $C_{1-6}$-Alkyl und Piperazinyl ausgewählt sind, ist.

**3.** Verbindung nach Anspruch 1 der Formel (Ib)

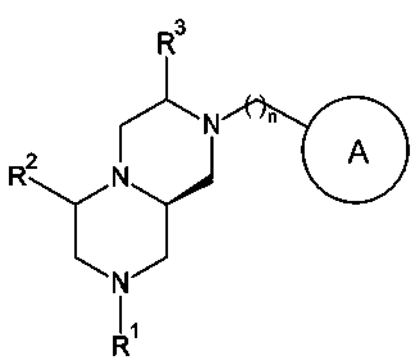

(Ib),

wobei

$R^1$

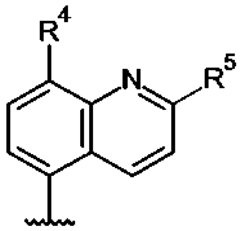 , 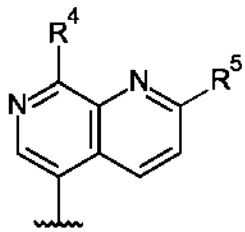 , 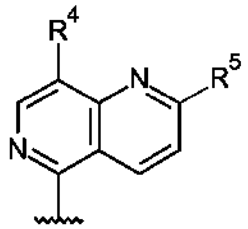 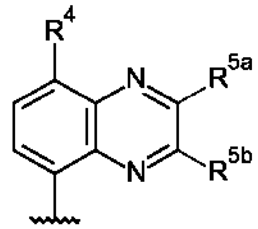 ,

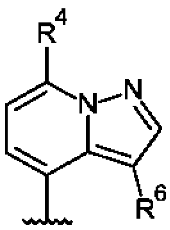 , 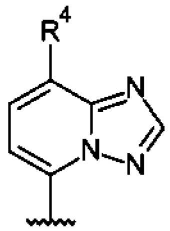 , 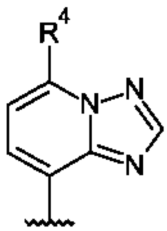 , 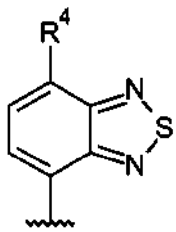 , 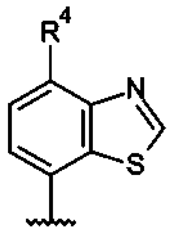 , 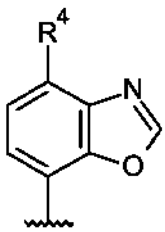 ,

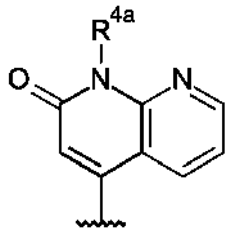 oder 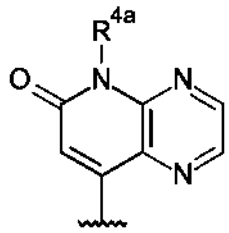

ist; wobei $R^4$ $C_1$-$_6$-Alkyl, $C_1$-$_6$-Alkoxy, Halogen-$C_1$-$_6$-alkyl, Halogen, Nitro oder Cyano ist; $R^{4a}$ $C_1$-$_6$-Alkyl oder $C_3$-$_7$-Cycloalkyl ist; $R^5$, $R^{5a}$ und $R^{5b}$ unabhängig voneinander aus H und Deuterium ausgewählt sind; $R^6$ H oder Halogen ist;
$R^2$ H oder $C_1$-$_6$-Alkyl ist;
$R^3$ H ist;
Ring A 1,2,3,4-Tetrahydroisochinolinyl;

5,6,7,8-Tetrahydro-1,6-naphthyridinyl,
5,6,7,8-Tetrahydro-2,6-naphthyridinyl;
5,6,7,8-Tetrahydro-2,7-naphthyridinyl;
Isoindolinyl;
Phenyl, das mit Amino-($C_1$-$_6$-alkoxy)pyrrolidinyl, 5-Oxa-2,8-diazaspiro[3.5]nonanyl, Morpholinyl oder Piperazinyl substituiert ist;
Pyridinyl, das einmal oder zweimal mit Substituenten substituiert ist, die unabhängig voneinander aus (Halogenpyrrolidinyl)amino; 1,4-Diazepanyl; 2,5-Diazabicyclo[2.2.1]heptanyl; 3,4,4a,5,7,7a-Hexahydro-2H-pyrrolo[3,4-b][1,4]oxazinyl; 3,6-Diazabicyclo[3.1.1]heptanyl; 3,8-Diazabicyclo[3.2.1]octanyl; 5-Oxa-2,8-diazaspiro[3.5]nonanyl; 9-Oxa-3,7-diazabicyclo[3.3.1]nonanyl; Amino-($C_1$-$_6$-alkoxy)pyrrolidinyl; Amino-($C_1$-$_6$-alkyl)azetidinyl; Amino-($C_1$-$_6$-alkyl)pyrrolidinyl; Amino-1,4-oxazepanyl; Amino-2-azaspiro[3.3]heptanyl; Aminoazetidinyl; Aminohalogenpyrrolidinyl; $C_1$-$_6$-Alkyl; $C_1$-$_6$-Alkyl-2,6-diazaspiro[3.3]heptancarbonyl; $C_1$-$_6$-Alkyl-2,6-diazaspiro[3.3]heptanyl; $C_1$-$_6$-Alkylpiperazinyl und Piperazinyl ausgewählt sind; oder
Pyrimidinyl, das einmal oder zweimal mit Substituenten substituiert ist, die unabhängig voneinander aus Amino-($C_1$-$_6$-alkoxy)pyrrolidinyl, Amino-($C_1$-$_6$-alkyl)azetidinyl, Aminoazetidinyl, $C_1$-$_6$-Alkyl und Piperazinyl ausgewählt sind, ist;

n 0, 1 oder 2 ist;

oder ein pharmazeutisch unbedenkliches Salz davon.

**4.** Verbindung nach Anspruch 2 oder 3, wobei $R^1$

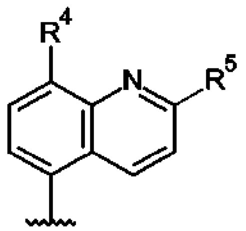
,
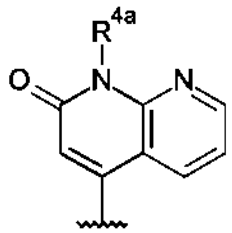
oder
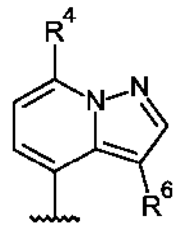

ist; wobei $R^4$ Cyano ist; $R^{4a}$ $C_{1}$-$_{6}$-Alkyl ist; $R^5$ H oder Deuterium ist; $R^6$ H ist.

**5.** Verbindung nach Anspruch 4, wobei $R^2$ $C_{1}$-$_{6}$-Alkyl ist.

**6.** Verbindung nach Anspruch 5, wobei

R1

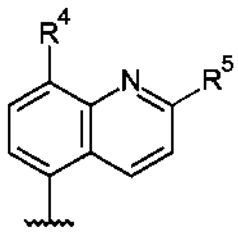
,
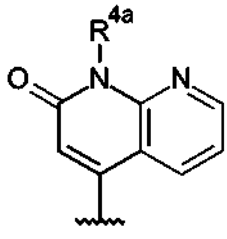
oder
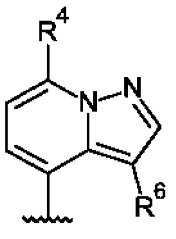

ist; wobei $R^4$ Cyano ist; $R^{4a}$ $C_{1}$-$_{6}$-Alkyl ist; $R^5$ H oder Deuterium ist; $R^6$ H ist;
$R^2$ Methyl ist;
$R^3$ H ist;
Ring A 1,2,3,4-Tetrahydroisochinolin-7-yl, 1,2,3,4-Tetrahydroisochinolin-5-yl; 1,2,3,4-Tetrahydroisochinolin-6-yl; 1,2,3,4-Tetrahydroisochinolin-7-yl, 1,2,3,4-Tetrahydroisochinolin-8-yl; 5,6,7,8-Tetrahydro-1,6-naphthyridin-2-yl; 5,6,7,8-Tetrahydro-1,6-naphthyridin-3-yl; 5,6,7,8-Tetrahydro-2,6-naphthyridin-1-yl; 5,6,7,8-Tetrahydro-2,7-naphthyridin-4-yl; Isoindolin-4-yl; 3-Amino-4-methoxypyrrolidin-1-ylphenyl; 5-Oxa-2,8-diazaspiro[3.5]nonan-2-ylphenyl; Morpholin-2-ylphenyl; Piperazin-1-ylphenyl; (4-Fluorpyrrolidin-3-yl)amino-(methyl)pyridinyl; 1,4-Diazepan-1-ylpyridinyl; 2,5-Diazabicyclo[2.2.1]heptan-2-yl-(methyl)pyridinyl; 2,5-Diazabicyclo[2.2.1]heptan-2-ylpyridinyl; 2-Methylpiperazin-1-yl-(methyl)pyridinyl, 3,4,4a,5,7,7a-Hexahydro-2H-pyrrolo[3,4-b][1,4]oxazin-6-yl-(methyl)pyridinyl, (3,4,4a,5,7,7a-Hexahydro-2H-pyrrolo[3,4-b][1,4]oxazin-6-yl)pyridinyl; 3,6-Diazabicyclo[3.1.1]heptan-3-yl-(methyl)pyridinyl; 3,8-Diazabicyclo[3.2.1]octan-3-yl-(methyl)pyridinyl; 3,8-Diazabicyclo[3.2.1]octan-3-ylpyridinyl; 3-Amino-3-methylazetidin-1-yl-(methyl)pyridinyl, 3-Amino-3-methylazetidin-1-ylpyridinyl, 3-Amino-3-methylpyrrolidin-1-yl-(methyl)pyridinyl, 3-Amino-3-methylpyrrolidin-1-ylpyridinyl; 3-Amino-4-fluorpyrrolidin-1-ylpyridinyl, 3-Amino-4-methoxypyrrolidin-1-yl-(methyl)pyridinyl; 3-Amino-4-methoxypyrrolidin-1-ylpyridinyl, 3-Aminoazetidin-1-ylpyridinyl; 3-Methylpiperazin-1-yl-(methyl)pyridinyl; 3-Methylpiperazin-1-ylpyridinyl; 5-Oxa-2,8-diazaspiro[3.5]nonan-2-ylpyridinyl; 6-Amino-1,4-oxazepan-4-yl-(methyl)pyridinyl; 6-Amino-1,4-oxazepan-4-ylpyridinyl, 6-Amino-2-azaspiro[3.3]heptan-2-yl-(methyl)pyridinyl; 6-Methyl-2,6-diazaspiro[3.3]heptan-2-yl-(methyl)pyridinyl; 6-Methyl-2,6-diazaspiro[3.3]heptan-2-ylpyridinyl; 6-Methyl-2,6-diazaspiro[3.3]heptan-2-carbonyl-(methyl)pyridinyl; 9-Oxa-3,7-diazabicyclo[3.3.1]nonan-3-yl-(methyl)pyridinyl; Piperazin-1-yl-(methyl)pyridinyl, Piperazin-1-ylpyridinyl; 3-Amino-3-methylazetidin-1-yl-(methyl)pyrimidinyl, 3-Amino-4-methoxypyrrolidin-1-yl-(methyl)pyrimidinyl; 3 -Amino-4-methoxypyrrolidin-1-ylpyrimidinyl; 3-Aminoazetidin-1-ylpyrimidinyl, Piperazin-1-yl-(methyl)pyrimidinyl oder Piperazin-1-ylpyrimidinyl ist;
n 0, 1 oder 2 ist;

oder ein pharmazeutisch unbedenkliches Salz davon.

**7.** Verbindung nach Anspruch 5, wobei $R^1$

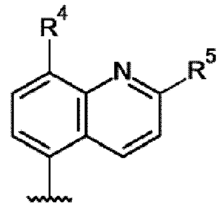

ist; wobei $R^4$ Cyano ist; $R^5$ H oder Deuterium ist.

**8.** Verbindung nach Anspruch 7, wobei
Ring A 1,2,3,4-Tetrahydroisochinolinyl;

mit Morpholinyl substituiertes Phenyl;
Pyridinyl, das einmal oder zweimal mit Substituenten substituiert ist, die unabhängig voneinander aus 3,4,4a,5,7,7a-Hexahydro-2H-pyrrolo[3,4-b][1,4]oxazinyl; Amino-($C_1$-$_6$-alkoxy)pyrrolidinyl; Amino-($C_1$-$_6$-alkyl)azetidinyl; Aminohalogenpyrrolidinyl; $C_1$-$_6$-Alkyl; $C_1$-$_6$-Alkyl-2,6-diazaspiro[3.3]heptanyl und Piperazinyl ausgewählt sind;
Pyrimidinyl, das zweimal mit Substituenten substituiert ist, die unabhängig voneinander aus Amino-($C_1$-$_6$-alkoxy)pyrrolidinyl; Amino-($C_1$-$_6$-alkyl)-azetidinyl und $C_1$-$_6$-Alkyl ausgewählt sind.

**9.** Verbindung nach Anspruch 8, wobei Ring A 1,2,3,4-Tetrahydroisochinolinyl; Morpholinylphenyl; Piperazinylpyridinyl; (Amino-($C_1$-$_6$-alkoxy)pyrrolidinyl)pyridinyl; (Aminohalogenpyrrolidinyl)pyridinyl; Piperazinyl-($C_1$-$_6$-alkyl)pyridinyl; (Amino-($C_1$-$_6$-alkoxy)pyrrolidinyl)pyridinyl; (Aminohalogenpyrrolidinyl)pyridinyl; ($C_1$-$_6$-Alkyl-2,6-diazaspiro[3.3]heptanyl)pyridinyl; (Amino-($C_1$-$_6$-alkyl)azetidinyl)pyridinyl; $C_1$-$_6$-Alkyl-(amino-($C_1$-$_6$-alkyl)azetidinyl)pyridinyl; 3,4,4a,5,7,7a-Hexahydro-2H-pyrrolo[3,4-b][1,4]oxazinyl-($C_1$-$_6$-alkyl)pyridinyl; (3,4,4a,5,7,7a-Hexahydro-2H-pyrrolo[3,4-b][1,4]oxazinyl)pyridinyl; $C_1$-$_6$-Alkyl-(amino-($C_1$-$_6$-alkyl)azetidinyl)pyrimidinyl oder $C_1$-$_6$-Alkyl-(amino-($C_1$-$_6$-alkoxy)pyrrolidinyl)pyrimidinyl ist.

**10.** Verbindung nach Anspruch 9, wobei Ring A 1,2,3,4-Tetrahydroisochinolin-7-yl; Morpholin-2-ylphenyl; (3,4,4a,5,7,7a-Hexahydro-2H-pyrrolo[3,4-b][1,4]oxazin-6-yl)-2-methyl-3-pyridinyl; (3,4,4a,5,7,7a-Hexahydro-2H-pyrrolo[3,4-b][1,4]oxazin-6-yl)-3-pyridinyl; (3-Amino-4-fluorpyrrolidin-1-yl)-3-pyridinyl; (3-Amino-4-fluorpyrrolidin-1-yl)-3-pyridinyl; (3-Amino-4-methoxypyrrolidin-1-yl)-3-pyridinyl, (3-Amino-4-methoxypyrrolidin-1-yl)-3-pyridinyl; 2-Methyl-6-piperazin-1-yl-3-pyridinyl, 6-(3-Amino-3-methylazetidin-1-yl)-2-methyl-3-pyridinyl; 6-(3-Amino-3-methylazetidin-1-yl)-3-pyridinyl; 6-(6-Methyl-2,6-diazaspiro[3.3]heptan-2-yl)-3-pyridinyl; 6-Piperazin-1-yl-3-pyridinyl; (3-Amino-4-methoxypyrrolidin-1-yl)-6-methylpyrimidin-4-yl oder (3-Amino-3-methylazetidin-1-yl)-6-methylpyrimidin-4-yl ist.

**11.** Verbindung nach Anspruch 2 oder 3, wobei

$R^1$

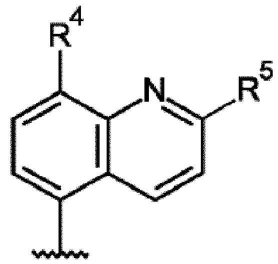

ist; wobei $R^4$ Cyano ist; $R^5$ H oder Deuterium ist;
$R^2$ $C_1$-$_6$-Alkyl ist;
$R^3$ H ist;
Ring A 1,2,3,4-Tetrahydroisochinolinyl;

mit Morpholinyl substituiertes Phenyl;
Pyridinyl, das einmal oder zweimal mit Substituenten substituiert ist, die unabhängig voneinander aus 3,4,4a,5,7,7a-Hexahydro-2H-pyrrolo[3,4-b][1,4]oxazinyl; Amino-($C_{1}$-$_{6}$-alkoxy)pyrrolidinyl; Amino-($C_{1}$-$_{6}$-alkyl)azetidinyl; Aminohalogenpyrrolidinyl; $C_{1}$-$_{6}$-Alkyl; $C_{1}$-$_{6}$-Alkyl-2,6-diazaspiro[3.3]heptanyl und Piperazinyl ausgewählt sind;
Pyrimidinyl, das zweimal mit Substituenten substituiert ist, die unabhängig voneinander aus Amino-($C_{1}$-$_{6}$-alkoxy)pyrrolidinyl; Amino-($C_{1}$-$_{6}$-alkyl)azetidinyl und $C_{1}$-$_{6}$-Alkyl ausgewählt sind, ist;

n 0, 1 oder 2 ist;

oder ein pharmazeutisch unbedenkliches Salz davon.

**12.** Verbindung nach Anspruch 11, wobei

$R^1$

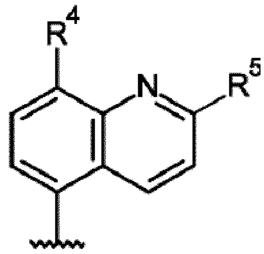

ist; wobei $R^4$ Cyano ist; $R^5$ H oder Deuterium ist;
$R^2$ $C_{1}$-$_{6}$-Alkyl ist;
$R^3$ H ist;
Ring A 1,2,3,4-Tetrahydroisochinolinyl; Morpholinylphenyl; Piperazinylpyridinyl; (Amino-($C_{1}$-$_{6}$-alkoxy)pyrrolidinyl)pyridinyl; (Aminohalogenpyrrolidinyl)pyridinyl; Piperazinyl-($C_{1}$-$_{6}$-alkyl)pyridinyl; (Amino-($C_{1}$-$_{6}$-alkoxy)pyrrolidinyl)pyridinyl; (Aminohalogenpyrrolidinyl)pyridinyl; ($C_{1}$-$_{6}$-Alkyl-2, 6-diazaspiro[3.3]heptanyl)pyridinyl; (Amino-($C_{1}$-$_{6}$-alkyl)azetidinyl)pyridinyl; $C_{1}$-$_{6}$-Alkyl-(amino-($C_{1}$-$_{6}$-alkyl)azetidinyl)pyridinyl; 3,4,4a,5,7,7a-Hexahydro-2H-pyrrolo[3,4-b][1,4]oxazinyl-($C_{1}$-$_{6}$-alkyl)pyridinyl; (3,4,4a,5,7,7a-Hexahydro-2H-pyrrolo[3,4-b][1,4]oxazinyl)pyridinyl, $C_{1}$-$_{6}$-Alkyl-(amino-($C_{1}$-$_{6}$-alkyl)azetidinyl)pyrimidinyl oder $C_{1}$-$_{6}$-Alkyl-(amino-($C_{1}$-$_{6}$-alkoxy)pyrrolidinyl)pyrimidinyl ist;
n 0, 1 oder 2 ist;

oder ein pharmazeutisch unbedenkliches Salz davon.

**13.** Verbindung nach Anspruch 11, wobei

$R^1$

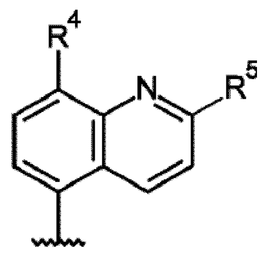

ist; wobei $R^4$ Cyano ist; $R^5$ H oder Deuterium ist;
$R^2$ Methyl ist;
$R^3$ H ist;
Ring A 1,2,3,4-Tetrahydroisochinolin-7-yl; 4-(Morpholin-2-yl)phenyl; (3,4,4a,5,7,7a-Hexahydro-2H-pyrrolo[3,4-b][1,4]oxazin-6-yl)-2-methyl-3-pyridinyl; (3,4,4a,5,7,7a-Hexahydro-2H-pyrrolo[3,4-b][1,4]oxazin-6-yl)-3-pyridinyl; (3-Amino-4-fluorpyrrolidin-1-yl)-3-pyridinyl, (3-Amino-4-fluorpyrrolidin-1-yl)-3-pyridinyl; (3-Amino-4-methoxypyrrolidin-1-yl)-3-pyridinyl; (3-Amino-4-methoxypyrrolidin-1-yl)-3-pyridinyl; 2-Methyl-6-piperazin-1-yl-3-pyridinyl; 6-(3-Amino-3-methylazetidin-1-yl)-2-methyl-3-pyridinyl; 6-(3-Amino-3-methylazetidin-1-yl)-3-pyridinyl; 6-(6-Methyl-2,6-diazaspiro[3.3]heptan-2-yl)-3-pyridinyl; 6-Piperazin-1-yl-3-pyridinyl, (3-Amino-4-methoxypyrrolidin-1-yl)-6-methylpyrimidin-4-yl oder (3-Amino-3-methylazetidin-1-yl)-6-methylpyrimidin-4-yl ist;
n 0, 1 oder 2 ist;

oder ein pharmazeutisch unbedenkliches Salz davon.

**14.** Verbindung nach einem der Ansprüche 1 bis 13, wobei die Verbindung aus Folgenden ausgewählt ist:

5-[cis-4-Methyl-8-(1,2,3,4-tetrahydroisochinolin-7-ylmethyl)-3,4,6,7,9,9a-hexahydro-*1H*-pyrazino[1,2-a]pyrazin-2-yl]chinolin-8-carbonitril;
5-[*cis*-4-Methyl-8-(1,2,3,4-tetrahydroisochinolin-5-yl)-3,4,6,7,9,9a-hexahydro-*1H*-pyrazino[1,2-a]pyrazin-2-yl]chinolin-8-carbonitril;
5-[*cis*-4-Methyl-8-[(2-piperazin-1-yl-4-pyridyl)methyl]-3,4,6,7,9,9a-hexahydro-*1H*-pyrazino[1,2-a]pyrazin-2-yl]chinolin-8-carbonitril;
5-[*cis*-8-Isoindolin-4-yl-4-methyl-3,4,6,7,9,9a-hexahydro-*1H*-pyrazino[1,2-a]pyrazin-2-yl]chinolin-8-carbonitril;
5-[(*4R*,*9aS*)-4-Methyl-8-(1,2,3,4-tetrahydroisochinolin-7-ylmethyl)-3,4,6,7,9,9a-hexahydro-*1H*-pyrazino[1,2-a]pyrazin-2-yl]chinolin-8-carbonitril;
5-[(*4R*,*9aS*)-4-Methyl-8-(5,6,7,8-tetrahydro-1,6-naphthyridin-3-ylmethyl)-3,4,6,7,9,9a-hexahydro-*1H*-pyrazino[1,2-a]pyrazin-2-yl]chinolin-8-carbonitril;
5-[(*4R*,*9aS*)-4-Methyl-8-[2-(4-piperazin-1-ylphenyl)ethyl]-3,4,6,7,9,9a-hexahydro-*1H*-pyrazino[1,2-a]pyrazin-2-yl]chinolin-8-carbonitril;
5-[(*4R*,*9aS*)-4-Methyl-8-[(6-piperazin-1-yl-3-pyridyl)methyl]-3,4,6,7,9,9a-hexahydro-*1H*-pyrazino[1,2-a]pyrazin-2-yl]chinolin-8-carbonitril;
5-[(*4R*,*9aS*)-8-Isoindolin-4-yl-4-methyl-3,4,6,7,9,9a-hexahydro-*1H*-pyrazino[1,2-a]pyrazin-2-yl]chinolin-8-carbonitril;
5-[(*4R*,*9aS*)-4-Methyl-8-(1,2,3,4-tetrahydroisochinolin-6-ylmethyl)-3,4,6,7,9,9a-hexahydro-*1H*-pyrazino[1,2-a]pyrazin-2-yl]chinolin-8-carbonitril;
5-[(*4R*,*9aS*)-4-Methyl-8-[2-(6-piperazin-1-yl-3-pyridyl)ethyl]-3,4,6,7,9,9a-hexahydro-*1H*-pyrazino[1,2-a]pyrazin-2-yl]chinolin-8-carbonitril;
5-[(*4R*,*9aS*)-8-[2-[6-[(*3R*,*4R*)-3-Amino-4-methoxypyrrolidin-1-yl]-3-pyridyl]ethyl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-[5-[(*3R*,*4R*)-3-Amino-4-methoxypyrrolidin-1-yl]-2-pyridyl]ethyl]-4-methyl-3,4,6,7,9,9a-hexahydro-*1H*-pyrazino[1,2-a]pyrazin-2-yl]chinolin-8-carbonitril;
5-[(*4R*,*9aS*)-8-[[6-(1,4-Diazepan-1-yl)-3-pyridyl]methyl]-4-methyl-3,4,6,7,9,9a-hexahydro-*1H*-pyrazino[1,2-a]pyrazin-2-yl]chinolin-8-carbonitril;
5-[(*4R*,*9aR*)-4-Methyl-8-(5,6,7,8-tetrahydro-2,6-naphthylidin-1-yl)-3,4,6,7,9,9a-hexahydro-*1H*-pyrazino[1,2-a]pyrazin-2-yl]chinolin-8-carbonitril;
5-[(*4R*,*9aS*)-8-[2-[6-[(*3R*,*4S*)-3-Amino-4-fluorpyrrolidin-1-yl]-3-pyridyl]ethyl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-[6-[(*3R*,*4R*)-3-Amino-4-fluorpyrrolidin-1-yl]-3-pyridyl]ethyl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-[5-[(*3R*,*4S*)-3-Amino-4-fluorpyrrolidin-1-yl]-2-pyridyl]ethyl]-4-methyl-3,4,6,7,9,9a-hexahydro-*1H*-pyrazino[1,2-a]pyrazin-2-yl]chinolin-8-carbonitril;
5-[(*4R*,*9aS*)-4-Methyl-8-[2-[5-[(*3S*)-3-methylpiperazin-1-yl]-2-pyridyl]ethyl]-3,4,6,7,9,9a-hexahydro-*1H*-pyrazino[1,2-a]pyrazin-2-yl]chinolin-8-carbonitril;
5-[(*4R*,*9aS*)-4-Methyl-8-[2-[6-[(*3S*)-3-methylpiperazin-1-yl]-2-pyridyl]ethyl]-3,4,6,7,9,9a-hexahydro-*1H*-pyrazino[1,2-a]pyrazin-2-yl]chinolin-8-carbonitril;
5-[(*4R*,*9aS*)-4-Methyl-8-[2-[6-(6-methyl-2,6-diazaspiro[3.3]heptan-2-yl)-3-pyridyl]ethyl]-3,4,6,7,9,9a-hexahydro-*1H*-pyrazino[1,2-a]pyrazin-2-yl]chinolin-8-carbonitril;
5-[(*4R*,*9aS*)-8-[2-[6-[(*3R*,*4S*)-3-Amino-4-methoxypyrrolidin-1-yl]-3-pyridyl]ethyl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-[6-[(*3S*,*4S*)-3-Amino-4-methoxypyrrolidin-1-yl]-3-pyridyl]ethyl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-[4-[(*3R*,*4R*)-3-Amino-4-methoxypyrrolidin-1-yl]phenyl]ethyl]-4-methyl-3,4,6,7,9,9a-hexahydro-*1H*-pyrazino[1,2-a]pyrazin-2-yl]chinolin-8-carbonitril;

5-[(*4R*,*9aS*)-4-Methyl-8-[(6-piperazin-1-yl-2-pyridyl)methyl]-3,4,6,7,9,9a-hexahydro-*1H*-pyrazino[1,2-a]pyrazin-2-yl]chinolin-8-carbonitril;
5-[(*4R*,*9aS*)-4-Methyl-8-[2-[4-(5-oxa-2,8-diazaspiro[3.5]nonan-2-yl)phenyl]ethyl]-3,4,6,7,9,9a-hexahydro-*1H*-pyrazino[1,2-a]pyrazin-2-yl]chinolin-8-carbonitril;
5-[(*4R*,*9aR*)-4-Methyl-8-(4-piperazin-1-ylpyrimidin-2-yl)-3,4,6,7,9,9a-hexahydro-*1H*-pyrazino[1,2-a]pyrazin-2-yl]chinolin-8-carbonitril;
5-[(*4R*,*9aR*)-4-Methyl-8-(6-methyl-2-piperazin-1-yl-pyrimidin-4-yl)-3,4,6,7,9,9a-hexahydro-*1H*-pyrazino[1,2-a]pyrazin-2-yl]chinolin-8-carbonitril;
5-[(*4R*,*9aS*)-4-Methyl-8-(2-piperazin-1-yl-4-pyridyl)-3,4,6,7,9,9a-hexahydro-*1H*-pyrazino[1,2-a]pyrazin-2-yl]chinolin-8-carbonitril;
5-[(*4R*,*9aR*)-4-Methyl-8-(2-piperazin-1-ylpyrimidin-4-yl)-3,4,6,7,9,9a-hexahydro-*1H*-pyrazino[1,2-a]pyrazin-2-yl]chinolin-8-carbonitril;
5-[(*4R*,*9aR*)-4-Methyl-8-(4-piperazin-1-yl-2-pyridyl)-3,4,6,7,9,9a-hexahydro-*1H*-pyrazino[1,2-a]pyrazin-2-yl]chinolin-8-carbonitril;
5-(8-Isoindolin-4-yl-3,4,6,7,9,9a-hexahydro-*1H*-pyrazino[1,2-a]pyrazin-2-yl)chinolin-8-carbonitril,
5-[(*4R*,*9aR*)-8-[2-[(*3R,4R*)-3-Amino-4-methoxypyrrolidin-1-yl]pyrimidin-4-yl]-4-methyl-3,4,6,7,9,9a-hexahydro-*1H*-pyrazino[1,2-a]pyrazin-2-yl]chinolin-8-carbonitril;
5-[(*4R*,*9aR*)-8-[4-[(*3R*,*4R*)-3-Amino-4-methoxypyrrolidin-1-yl]pyrimidin-2-yl]-4-methyl-3,4,6,7,9,9a-hexahydro-*1H*-pyrazino[1,2-a]pyrazin-2-yl]chinolin-8-carbonitril;
5-[2-(4-Methyl-6-piperazin-1-yl-3-pyridyl)-3,4,6,7,9,9a-hexahydro-*1H*-pyrazino[1,2-a]pyrazin-8-yl]chinolin-8-carbonitril;
5-[(*4R*,*9aR*)-8-[4-(3-Aminoazetidin-1-yl)pyrimidin-2-yl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-[(*3S*,*4R*)-3-Amino-4-fluorpyrrolidin-1-yl]-4-pyridyl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-(3-Aminoazetidin-1-yl)-4-pyridyl]-4-methyl-3,4,6,7,9,9a-hexahydro-*1H*-pyrazino[1,2-a]pyrazin-2-yl]chinolin-8-carbonitril;
5-[(*4R*,*9aS*)-4-Methyl-8-(5,6,7,8-tetrahydro-2,7-naphthyridin-4-yl)-3,4,6,7,9,9a-hexahydro-*1H*-pyrazino[1,2-a]pyrazin-2-yl]chinolin-8-carbonitril;
5-[(*4R*,*9aR*)-4-Methyl-8-(5-methyl-4-piperazin-1-yl-pyrimidin-2-yl)-3,4,6,7,9,9a-hexahydro-*1H*-pyrazino[1,2-a]pyrazin-2-yl]chinolin-8-carbonitril;
5-[(*4R*,*9aR*)-4-Methyl-8-(5,6,7,8-tetrahydro-2,7-naphthyridin-4-yl)-3,4,6,7,9,9a-hexahydro-*1H*-pyrazino[1,2-a]pyrazin-2-yl]chinolin-8-carbonitril;
5-[(*4R*,*9aR*)-8-[2-[(*3R*,*4R*)-3-Amino-4-methoxypyrrolidin-1-yl]-6-methylpyrimidin-4-yl]-4-methyl-3,4,6,7,9,9a-hexahydro-*1H*-pyrazino[1,2-a]pyrazin-2-yl]chinolin-8-carbonitril;
5-[2-[6-[(*6R*)-6-Amino-1,4-oxazepan-4-yl]-4-methyl-3-pyridyl]-3,4,6,7,9,9a-hexahydro-*1H*-pyrazino[1,2-a]pyrazin-8-yl]chinolin-8-carbonitril;
5-[*trans*-4-Methyl-8-(1,2,3,4-tetrahydroisochinolin-5-yl)-3,4,6,7,9,9a-hexahydro-*1H*-pyrazino[1,2-a]pyrazin-2-yl]chinolin-8-carbonitril;
5-[(*4R*,*9aS*)-8-[2-[(*6S*)-6-Amino-1,4-oxazepan-4-yl]-4-pyridyl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-[(*6R*)-6-Amino-1,4-oxazepan-4-yl]-4-pyridyl]-4-methyl-3,4,6,7,9,9a-hexahydro-*1H*-pyrazino[1,2-a]pyrazin-2-yl]chinolin-8-carbonitril;
5-[(*4R*,*9aR*)-8-[2-(3-Amino-3-methylazetidin-1-yl)-6-methylpyrimidin-4-yl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-(3-Amino-3-methylazetidin-1-yl)-4-pyridyl]-4-methyl-3,4,6,7,9,9a-hexahydro-*1H*-pyrazino[1,2-a]pyrazin-2-yl]chinolin-8-carbonitril;
5-[(*4R*,*9aS*)-4-Methyl-8-[(5-piperazin-1-yl-3-pyridyl)methyl]-3,4,6,7,9,9a-hexahydro-*1H*-pyrazino[1,2-a]pyrazin-2-yl]chinolin-8-carbonitril;
5-[(*4R*,*9aS*)-4-Methyl-8-[2-[6-[(*3R*)-3-methylpiperazin-1-yl]-2-pyridyl]ethyl]-3,4,6,7,9,9a-hexahydro-*1H*-pyrazino[1,2-a]pyrazin-2-yl]chinolin-8-carbonitril;
5-[(*4R*,*9aS*)-4-Methyl-8-[2-[6-(5-oxa-2,8-diazaspiro[3.5]nonan-2-yl)-3-pyridyl]ethyl]-3,4,6,7,9,9a-hexahydro-*1H*-pyrazino[1,2-a]pyrazin-2-yl]chinolin-8-carbonitril;
5-[(*4R*,*9aS*)-4-Methyl-8-[(2-methyl-6-piperazin-1-yl-3-pyridyl)methyl]-3,4,6,7,9,9a-hexahydro-*1H*-pyrazino[1,2-a]pyrazin-2-yl]chinolin-8-carbonitril;
5-[(*4R*,*9aS*)-4-Methyl-8-(1,2,3,4-tetrahydroisochinolin-7-ylmethyl)-3,4,6,7,9,9a-hexahydro-*1H*-pyrazino[1,2-a]pyrazin-2-yl]-2-deuteriochinolin-8-carbonitril;
5-[(*4R*,*9aS*)-8-[2-[6-[(*3R*,*4R*)-3-Amino-4-methoxypyrrolidin-1-yl]-3-pyridyl]ethyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-8-[2-[6-[(*3R*,*4S*)-3-Amino-4-fluorpyrrolidin-1-yl]-3-pyridyl]ethyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-4-Methyl-8-[2-[6-(6-methyl-2,6-diazaspiro[3.3]heptan-2-yl)-3-pyridyl]ethyl]-3,4,6,7,9,9a-hexahydro-*1H*-pyrazino[1,2-a]pyrazin-2-yl]-2-deuteriochinolin-8-carbonitril,
5-[(*4R*,*9aS*)-8-[2-[6-(3-Amino-3-methylazetidin-1-yl)-3-pyridyl]ethyl]-4-methyl-3,4,6,7,9,9a-hexahydro-*1H*-pyrazino[1,2-a]pyrazin-2-yl]chinolin-8-carbonitril;
4-[(*4R*,*9aS*)-8-[2-[4-[(*3R*,*4R*)-3-Amino-4-methoxypyrrolidin-1-yl]phenyl]ethyl]-4-methyl-3,4,6,7,9,9a-hexahydro-*1H*-pyrazino[1,2-a]pyrazin-2-yl]-3-fluorpyrazolo[1,5-a]pyridin-7-carbonitril;
4-[(*4R*,*9aS*)-8-[2-[6-[(*3R*,*4R*)-3-Amino-4-methoxypyrrolidin-1-yl]-3-pyridyl]ethyl]-4-methyl-3,4,6,7,9,9a-hexahydro-*1H*-pyrazino[1,2-a]pyrazin-2-yl]-3-fluorpyrazolo[1,5-a]pyridin-7-carbonitril;
4-[(*4R*,*9aS*)-8-[2-[4-[(*3R*,*4R*)-3-Amino-4-methoxypyrrolidin-1-yl]phenyl]ethyl]-4-methyl-3,4,6,7,9,9a-hexahydro-*1H*-pyrazino[1,2-a]pyrazin-2-yl]pyrazolo[1,5-a]pyridin-7-carbonitril,
4-[(*4R*,*9aS*)-4-Methyl-8-(1,2,3,4-tetrahydroisochinolin-7-ylmethyl)-3,4,6,7,9,9a-hexahydro-*1H*-pyrazino[1,2-a]pyrazin-2-yl]-3-fluorpyrazolo[1,5-a]pyridin-7-carbonitril;
5-[(*4R*,*9aS*)-4-Methyl-8-(1,2,3,4-tetrahydroisochinolin-5-ylmethyl)-3,4,6,7,9,9a-hexahydro-*1H*-pyrazino[1,2-a]pyrazin-2-yl]chinolin-8-carbonitril;
5-[(*4R*,*9aS*)-4-Methyl-8-(1,2,3,4-tetrahydroisochinolin-8-ylmethyl)-3,4,6,7,9,9a-hexahydro-1H-pyrazino[1,2-a]pyrazin-2-yl]chinolin-8-carbonitril;
5-[(*4R*,*9aS*)-8-(Isoindolin-4-ylmethyl)-4-methyl-3,4,6,7,9,9a-hexahydro-*1H*-pyrazino[1,2-a]pyrazin-2-yl]chinolin-8-carbonitril;
5-[(*4*R,*9aS*)-8-[2-(3-Amino-3-methylazetidin-1-yl)-6-methyl-4-pyridyl]-4-methyl-3,4,6,7,9,9a-hexahydro-*1H*-pyrazino[1,2-a]pyrazin-2-yl]chinolin-8-carbonitril;
4-[(*4R*,*9aS*)-8-[2-[6-(3-Amino-3-methylazetidin-1-yl)-3-pyridyl]ethyl]-4-methyl-3,4,6,7,9,9a-hexahydro-*1H*-pyrazino[1,2-a]pyrazin-2-yl]-3-fluorpyrazolo[1,5-a]pyridin-7-carbonitril;
4-[(*4R*,*9aS*)-8-[2-[6-[(*3R*,*4S*)-3-Amino-4-fluorpyrrolidin-1-yl]-3-pyridyl]ethyl]-4-methyl-3,4,6,7,9,9a-hexahydro-*1H*-pyrazino[1,2-a]pyrazin-2-yl]-3-fluorpyrazolo[1,5-a]pyridin-7-carbonitril;
5-[(*4R*,*9aS*)-8-[[6-(3-Amino-3-methylazetidin-1-yl)-2-methyl-3-pyridyl]methyl]-4-methyl-3,4,6,7,9,9a-hexahydro-*1H*-pyrazino[1,2-a]pyrazin-2-yl]chinolin-8-carbonitril;
5-[(*4R*,*9aS*)-8-[2-[6-[(*4aR*,*7aR*)-3,4,4a,5,7,7a-Hexahydro-*2H*-pyrrolo[3,4-b][1,4]oxazin-6-yl]-3-pyridyl]ethyl]-4-methyl-3,4,6,7,9,9a-hexahydro-*1H*-pyrazino[1,2-a]pyrazin-2-yl]-2-deuteriochinolin-8-carbonitril;
5-[(*4R*,*9aS*)-4-Methyl-8-[(6-piperazin-1-yl-3-pyridyl)methyl]-3,4,6,7,9,9a-hexahydro-*1H*-pyrazino[1,2-a]pyrazin-2-yl]-2-deuteriochinolin-8-carbonitril;
5-[(*4R*,*9aS*)-8-[[6-(3-Amino-3-methylazetidin-1-yl)-2-methyl-3-pyridyl]methyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-4-Methyl-8-[[2-methyl-6-(6-methyl-2,6-diazaspiro[3.3]heptan-2-yl)-3-pyridyl]methyl]-3,4,6,7,9,9a-hexahydro-*1H*-pyrazino[1,2-a]pyrazin-2-yl]-2-deuteriochinolin-8-carbonitril,
5-[(*4R*,*9aS*)-8-[[6-[(*3R*)-3-Amino-3-methylpyrrolidin-1-yl]-2-methyl-3-pyridyl]methyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-8-[[6-[[(*3S*,*4R*)-4-Fluorpyrrolidin-3-yl]amino]-2-methyl-3-pyridyl]methyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-4-Methyl-8-[[2-methyl-6-(9-oxa-3,7-diazabicyclo[3.3.1]nonan-3-yl)-3-pyridyl]methyl]-3,4,6,7,9,9a-hexahydro-*1H*-pyrazino[1,2-a]pyrazin-2-yl]-2-deuteriochinolin-8-carbonitril,
5-[(*4R*,*9aS*)-8-[2-[6-[(*3R*)-3-Amino-3-methylpyrrolidin-1-yl]-3-pyridyl]ethyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-4-Methyl-8-[[2-methyl-6-[(*2S*)-2-methylpiperazin-1-yl]-3-pyridyl]methyl]-3,4,6,7,9,9a-hexahydro-*1H*-pyrazino[1,2-a]pyrazin-2-yl]chinolin-8-carbonitril;
5-[(*4R*,9a*S*)-4-Methyl-8-[[2-methyl-6-[(*3R*)-3-methylpiperazin-1-yl]-3-pyridyl]methyl]-3,4,6,7,9,9a-hexahydro-*1H*-pyrazino[1,2-a]pyrazin-2-yl]chinolin-8-carbonitril;
5-[(*4R*,*9aS*)-4-Methyl-8-[(4-methyl-6-piperazin-1-yl-3-pyridyl)methyl]-3,4,6,7,9,9a-hexahydro-*1H*-pyrazino[1,2-a]pyrazin-2-yl]-2-deuteriochinolin-8-carbonitril;
5-[(*4R*,*9aS*)-4-Methyl-8-[(3-methyl-6-piperazin-1-yl-2-pyridyl)methyl]-3,4,6,7,9,9a-hexahydro-*1H*-pyrazino[1,2-a]pyrazin-2-yl]-2-deuteriochinolin-8-carbonitril;
5-[(*4R*,*9aS*)-8-[2-[2-[(*3R*,*4S*)-3-Amino-4-fluorpyrrolidin-1-yl]-4-pyridyl]ethyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-8-[[6-(3,6-Diazabicyclo[3.1.1]heptan-3-yl)-2-methyl-3-pyridyl]methyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-8-[[6-(3,8-Diazabicyclo[3.2.1]octan-3-yl)-2-methyl-3-pyridyl]methyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-8-[[6-[(*1S*,*4S*)-2,5-Diazabicyclo[2.2.1]heptan-2-yl]-2-methyl-3-pyridyl]methyl]-4-methyl-3,4,6,7,9,9a-hexahydro-*1H*-pyrazino[1,2-a]pyrazin-2-yl]-2-deuteriochinolin-8-carbonitril;
4-[(*4R*,*9aR*)-4-Methyl-8-(3-methyl-5-piperazin-1-yl-2-pyridyl)-3,4,6,7,9,9a-hexahydro-*1H*-pyrazino[1,2-a]pyrazin-2-yl]-1-methyl-1,8-naphthyridin-2-on;
5-[(*4R*,*9aS*)-8-[[6-[(*1S*,*4S*)-2,5-Diazabicyclo[2.2.1]heptan-2-yl]-4-methyl-3-pyridyl]methyl]-4-methyl-3,4,6,7,9,9a-hexahydro-*1H*-pyrazino[1,2-a]pyrazin-2-yl]-2-deuteriochinolin-8-carbonitril;
5-[(*4R*,*9aR*)-8-[5-[(*1S*,4*S*)-2,5-Diazabicyclo[2.2.1]heptan-2-yl]-3-methyl-2-pyridyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aR*)-8-[5-[(*3R*,*4S*)-3-Amino-4-methoxypyrrolidin-1-yl]-3-methyl-2-pyridyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aR*)-8-[5-[(*3R*)-3-Amino-3-methylpyrrolidin-1-yl]-3-methyl-2-pyridyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aS*)-8-[[6-[(*4aR*,*7aR*)-3,4,4a,5,7,7a-Hexahydro-*2H*-pyrrolo[3,4-b][1,4]oxazin-6-yl]-2-methyl-3-pyridyl]methyl]-4-methyl-3,4,6,7,9,9a-hexahydro-*1H*-pyrazino[1,2-a]pyrazin-2-yl]-2-deuteriochinolin-8-carbonitril;
5-[(*4R*,*9aR*)-8-[5-[(*3R*,*4R*)-3-Amino-4-methoxypyrrolidin-1-yl]-3-methyl-2-pyridyl]-4-methyl-3,4,6,7,9,9a-hexahydro-*1H*-pyrazino[1,2-a]pyrazin-2-yl]-2-deuteriochinolin-8-carbonitril,
5-[(*4R*,*9aR*)-8-[5-[(*4aR*,*7aR*)-3,4,4a,5,7,7a-Hexahydro-*2H*-pyrrolo[3,4-b][1,4]oxazin-6-yl]-3-methyl-2-pyridyl]-4-methyl-3,4,6,7,9,9a-hexahydro-*1H*-pyrazino[1,2-a]pyrazin-2-yl]-2-deuteriochinolin-8-carbonitril;
5-[(*4R*,*9aR*)-8-[5-(6-Amino-2-azaspiro[3.3]heptan-2-yl)-3-methyl-2-pyridyl]-4-methyl-3,4,6,7,9,9a-hexahydro-*1H*-pyrazino[1,2-a]pyrazin-2-yl]-2-deuteriochinolin-8-carbonitril;
5-[(*4R*,*9aR*)-4-Methyl-8-[3-methyl-5-(6-methyl-2,6-diazaspiro[3.3]heptan-2-carbonyl)-2-pyridyl]-3,4,6,7,9,9a-hexahydro-*1H*-pyrazino[1,2-a]pyrazin-2-yl]-2-deuteriochinolin-8-carbonitril,
5-[(*4S*,*9aR*)-4-Methyl-8-[3-methyl-5-(6-methyl-2,6-diazaspiro[3.3]heptan-2-carbonyl)-2-pyridyl]-3,4,6,7,9,9a-hexahydro-*1H*-pyrazino[1,2-a]pyrazin-2-yl]-2-deuteriochinolin-8-carbonitril,
5-[(*4R*,*9aS*)-8-[2-[(*3R*)-3-Amino-3-methylpyrrolidin-1-yl]-4-pyridyl]-4-methyl-3,4,6,7,9,9a-hexahydro-*1H*-pyrazino[1,2-a]pyrazin-2-yl]chinolin-8-carbonitril;
5-[(*4S*,*9aS*)-4-Methyl-8-[4-[(*2R*)-morpholin-2-yl]phenyl]-3,4,6,7,9,9a-hexahydro-*1H*-pyrazino[1,2-a]pyrazin-2-yl]-2-deuteriochinolin-8-carbonitril;
5-[(*4S*,*9aS*)-4-Methyl-8-[4-[(*2S*)-morpholin-2-yl]phenyl]-3,4,6,7,9,9a-hexahydro-*1H*-pyrazino[1,2-a]pyrazin-2-yl]-2-deuteriochinolin-8-carbonitril;
5-[(*4S*,*9aR*)-8-[5-(3,6-Diazabicyclo[3.1.1]heptan-3-yl)-3-methyl-2-pyridyl]-4-methyl-3,4,6,7,9,9a-hexahydro-*1H*-pyrazino[1,2-a]pyrazin-2-yl]-2-deuteriochinolin-8-carbonitril;
5-[(*4R*,*9aR*)-8-[5-[2-[(*3S*,*4S*)-3-Amino-4-methoxypyrrolidin-1-yl]ethyl]-2-pyridyl]-4-methyl-3,4,6,7,9,9a-hexahydro-*1H*-pyrazino[1,2-a]pyrazin-2-yl]-2-deuteriochinolin-8-carbonitril,
5-[(*4S*,*9aR*)-8-[5-[(*6R*)-6-Amino-1,4-oxazepan-4-yl]-3-methyl-2-pyridyl]-4-methyl-3,4,6,7,9,9a-hexahydro-*1H*-pyrazino[1,2-a]pyrazin-2-yl]-2-deuteriochinolin-8-carbonitril;
5-[(*4S*,*9aR*)-8-[5-[(*1S*,*4S*)-2,5-Diazabicyclo[2.2.1]heptan-2-yl]-3-methyl-2-pyridyl]-4-methyl-3,4,6,7,9,9a-hexahydro-*1H*-pyrazino[1,2-a]pyrazin-2-yl]-2-deuteriochinolin-8-carbonitril,
5-[(*4S*,*9aR*)-8-[6-(3,8-Diazabicyclo[3.2.1]octan-3-yl)-3-pyridyl]-4-methyl-3,4,6,7,9,9a-hexahydro-*1H*-pyrazino[1,2-a]pyrazin-2-yl]-2-deuteriochinolin-8-carbonitril;
4-[(*4S*,*9aR*)-4-Methyl-8-[3-methyl-5-(6-methyl-2,6-diazaspiro[3.3]heptan-2-yl)-2-pyridyl]-3,4,6,7,9,9a-hexahydro-*1H*-pyrazino[1,2-a]pyrazin-2-yl]-1-methyl-1,8-naphthyridin-2-on;
5-[(*4S*,*9aR*)-8-[5-[(4*aR*,*7aR*)-3,4,4a,5,7,7a-Hexahydro-2*H*-pyrrolo[3,4-b][1,4]oxazin-6-yl]-3-methyl-2-pyridyl]-4-methyl-3,4,6,7,9,9a-hexahydro-*1H*-pyrazino[1,2-a]pyrazin-2-yl]-2-deuteriochinolin-8-carbonitril;
5-[(*4S*,*9aR*)-8-[6-[(*3R*,*4R*)-3-Amino-4-methoxypyrrolidin-1-yl]-3-pyridyl]-4-methyl-3,4,6,7,9,9a-hexahydro-*1H*-pyrazino[1,2-a]pyrazin-2-yl]-2-deuteriochinolin-8-carbonitril;
5-[(*4S*,*9aR*)-8-[6-[2-[(*3R*,*4S*)-3-Amino-4-fluorpyrrolidin-1-yl]ethyl]-3-pyridyl]-4-methyl-3,4,6,7,9,9a-hexahydro-*1H*-pyrazino[1,2-a]pyrazin-2-yl]-2-deuteriochinolin-8-carbonitril;
5-[(*4S*,*9aR*)-8-[5-(3-Amino-3-methylazetidin-1-yl)-3-methyl-2-pyridyl]-4-methyl-3,4,6,7,9,9a-hexahydro-*1H*-pyrazino[1,2-a]pyrazin-2-yl]-2-deuteriochinolin-8-carbonitril;
5-[(*4S*,*9aR*)-4-Methyl-8-(5,6,7,8-tetrahydro-1,6-naphthyridin-2-yl)-3,4,6,7,9,9a-hexahydro-*1H*-pyrazino[1,2-a]pyrazin-2-yl]-2-deuteriochinolin-8-carbonitril;
5-[(*4S*,*9aR*)-8-[5-[2-[(*3S*,*4S*)-3-Amino-4-methoxypyrrolidin-1-yl]ethyl]-2-pyridyl]-4-methyl-3,4,6,7,9,9a-hexahydro-*1H*-pyrazino[1,2-a]pyrazin-2-yl]-2-deuteriochinolin-8-carbonitril;
5-[(*4S*,*9aR*)-8-[5-[[(*3R*,*4R*)-3-Amino-4-methoxypyrrolidin-1-yl]methyl]-6-methyl-2-pyridyl]-4-methyl-3,4,6,7,9,9a-hexahydro-*1H*-pyrazino[1,2-a]pyrazin-2-yl]-2-deuteriochinolin-8-carbonitril;
5-[(*4R*,*9aS*)-8-[2-[6-[(*4aR*,*7aR*)-3,4,4a,5,7,7a-Hexahydro-*2H*-pyrrolo[3,4-b][1,4]oxazin-6-yl]-3-pyridyl]ethyl]-4-methyl-3,4,6,7,9,9a-hexahydro-*1H*-pyrazino[1,2-a]pyrazin-2-yl]chinolin-8-carbonitril; und
5-[(*4S*,*9aS*)-4-Methyl-8-[4-[(*2R*)-morpholin-2-yl]phenyl]-3,4,6,7,9,9a-hexahydro-*1H*-pyrazino[1,2-a]pyrazin-2-yl]chinolin-8-carbonitril;
oder ein pharmazeutisch unbedenkliches Salz davon.

**15.** Verfahren zur Herstellung einer Verbindung nach einem der Ansprüche 1 bis 14, umfassend einen der folgenden Schritte:

a) Buchwald-Hartwig-Aminierungsreaktion oder nukleophile Substitution zwischen einer Verbindung der Formel (IX)

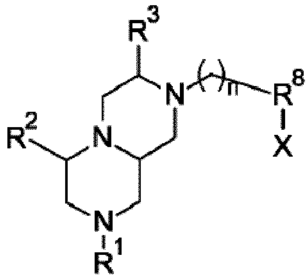
(IX)

und einem Amin (X)

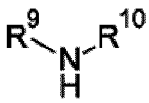
(X);

b) Buchwald-Hartwig-Aminierungsreaktion oder nukleophile Substitution zwischen einer Verbindung der Formel (V)

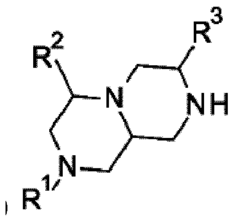
(V)

und einer Verbindung der Formel (VI)

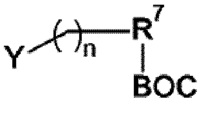
(VI);

wobei n 0, 1 oder 2 ist; X Halogen ist; Y Halogen oder Methansulfonat ist; $R^7$ und $R^8$ Aryl oder Heteroaryl ist; $R^9$ und $R^{10}$ zusammen mit dem Stickstoffatom, an das sie gebunden sind, ein Heterocyclyl bilden.

**16.** Verbindung oder pharmazeutisch unbedenkliches Salz nach einem der Ansprüche 1 bis 14 zur Verwendung als therapeutisch wirksame Substanz.

**17.** Pharmazeutische Zusammensetzung, umfassend eine Verbindung nach einem der Ansprüche 1 bis 14 und einen therapeutisch inerten Träger.

**18.** Verbindung oder pharmazeutisch unbedenkliches Salz, Enantiomer oder Diastereomer nach einem der Ansprüche 1 bis 14 zur Verwendung bei der Behandlung oder Prophylaxe von systemischem Lupus erythematodes oder Lupusnephritis.

## Revendications

**1.** Composé de formule (I),

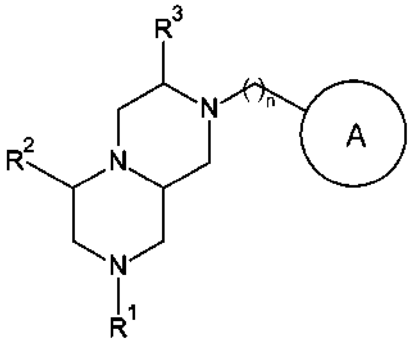

(I),

dans lequel

$R^1$ représente

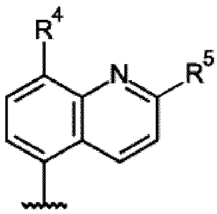 , 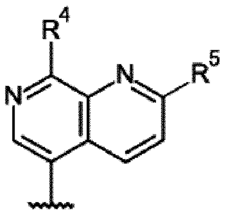 , 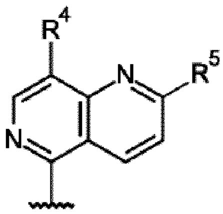 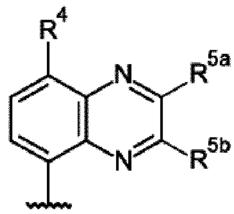 ,

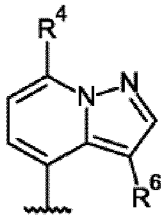 , 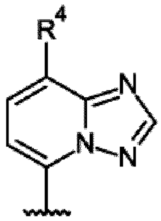 , 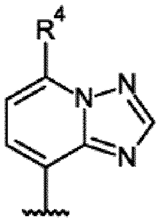 , 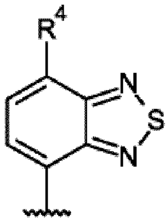 , 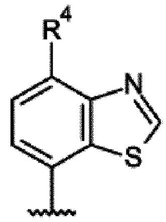 ,

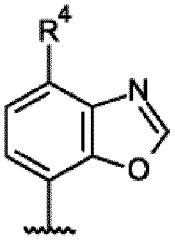 , 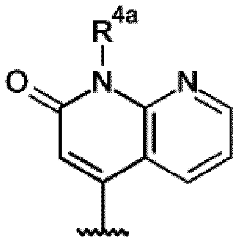 ou 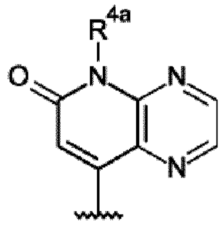 ;

dans lequel $R^4$ représente un alkyle en $C_{1-6}$, un alcoxy en $C_{1-6}$, un halogénoalkyle en $C_{1-6}$, un halogène, un nitro ou un cyano ; $R^{4a}$ représente un alkyle en $C_{1-6}$ ou un cycloalkyle en $C_{3-7}$ ; $R^5$, $R^{5a}$ et $R^{5b}$ sont indépendamment choisis parmi H et un deutérium ; $R^6$ représente H ou un halogène ;

$R^2$ représente H ou un alkyle en $C_{1-6}$ ;
$R^3$ représente H ;
le cycle A représente un aryle ou un hétéroaryle monocyclique à 5 à 7 chaînons non substitué ou substitué ; ou un hétérocyclyle bicyclique à 7 à 12 chaînons non substitué ou substitué ;
n représente 0, 1 ou 2 ;

ou un sel pharmaceutiquement acceptable de celui-ci.

**2.** Composé selon la revendication 1, dans lequel le cycle A représente un 1,2,3,4-tétrahydroisoquinoléinyle ;

un 5,6,7,8-tétrahydro-1,6-naphtyridinyle ;
un 5,6,7,8-tétrahydro-2,6-naphtyridinyle ;
un 5,6,7,8-tétrahydro-2,7-naphtyridinyle ;
un isoindolinyle ;
un phényle substitué par un amino(alcoxy en $C_{1-6}$)pyrrolidinyle, un 5-oxa-2,8-diazaspiro[3.5]nonanyle, un morpholinyle ou un pipérazinyle ;
un pyridinyle substitué une ou deux fois par des substituants indépendamment choisis parmi un (halogénopyrrolidinyl)amino ; un 1,4-diazépanyle ; un 2,5-diazabicyclo[2.2.1]heptanyle ; un 3,4,4a,5,7,7a-hexahydro-2H-pyrrolo[3,4-b][1,4]oxazinyle ; un 3,6-diazabicyclo[3.1.1]heptanyle ; un 3,8-diazabicyclo[3.2.1]octanyle ; un 5-oxa-2,8-diazaspiro[3.5]nonanyle ; un 9-oxa-3,7-diazabicyclo[3.3.1]nonanyle ; un amino(alcoxy en $C_{1-6}$)pyrrolidinyle ; un amino(alkyle en $C_{1-6}$)azétidinyle ; un amino(alkyle en $C_{1-6}$)pyrrolidinyle ; un amino-1,4-oxazépanyle ; un amino-2-azaspiro[3.3]heptanyle ; un aminoazétidinyle ; un aminohalogénopyrrolidinyle ; un alkyle en $C_{1-6}$ ; un alkyle en $C_{1-6}$-2,6-diazaspiro[3.3]heptanecarbonyle ; un alkyle en $C_{1-6}$-2,6-diazaspiro[3.3]heptanyle ; un alkyle en $C_{1-6}$-pipérazinyle et un pipérazinyle ; ou
un pyrimidinyle substitué une ou deux fois par des substituants indépendamment choisis parmi un amino(alcoxy en $C_{1-6}$)pyrrolidinyle, un amino(alkyle en $C_{1-6}$)azétidinyle, un aminoazétidinyle, un alkyle en $C_{1-6}$ et un pipérazinyle.

**3.** Composé selon la revendication 1, de formule (Ib),

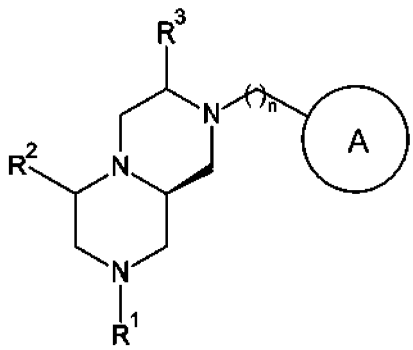
(Ib),

dans lequel

$R^1$ représente

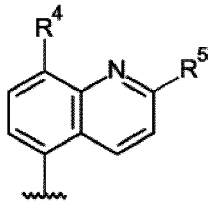
,
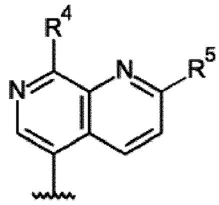
,
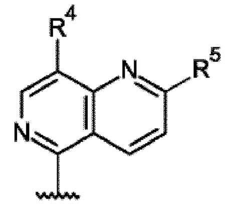
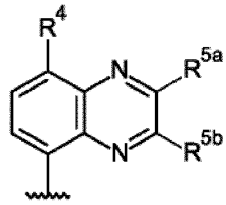
,

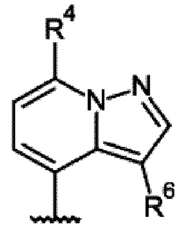,
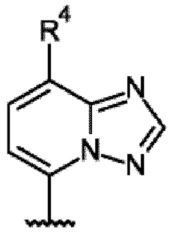,
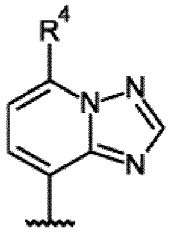,
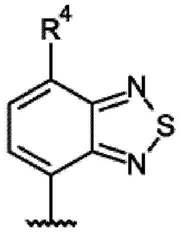,
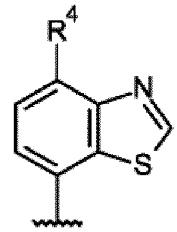

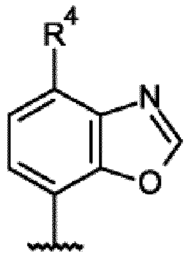,
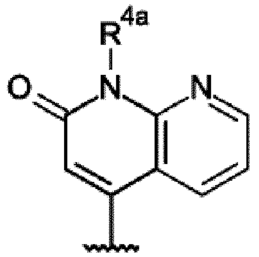
ou
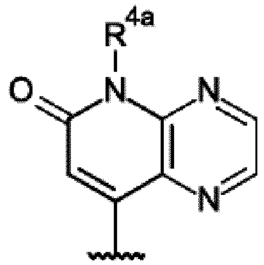
;

dans lequel $R^4$ représente un alkyle en $C_{1-6}$, un alcoxy en $C_{1-6}$, un halogénoalkyle en $C_{1-6}$, un halogène, un nitro ou un cyano ; $R^{4a}$ représente un alkyle en $C_{1-6}$ ou un cycloalkyle en $C_{3-7}$ ; $R^5$, $R^{5a}$ et $R^{5b}$ sont indépendamment choisis parmi H et un deutérium ; $R^6$ représente H ou un halogène ;
$R^2$ représente H ou un alkyle en $C_{1-6}$ ;
$R^3$ représente H ;
le cycle A représente un 1,2,3,4-tétrahydroisoquinoléinyle ;

un 5,6,7,8-tétrahydro-1,6-naphtyridinyle ;
un 5,6,7,8-tétrahydro-2,6-naphtyridinyle ;
un 5,6,7,8-tétrahydro-2,7-naphtyridinyle ;
un isoindolinyle ;
un phényle substitué par un amino(alcoxy en $C_{1-6}$)pyrrolidinyle, un 5-oxa-2,8-diazaspiro[3.5]nonanyle, un morpholinyle ou un pipérazinyle ;
un pyridinyle substitué une ou deux fois par des substituants indépendamment choisis parmi un (halogénopyrrolidinyl)amino ; un 1,4-diazépanyle ; un 2,5-diazabicyclo[2.2.1]heptanyle ; un 3,4,4a,5,7,7a-hexahydro-2H-pyrrolo[3,4-b][1,4]oxazinyle ; un 3,6-diazabicyclo[3.1.1]heptanyle ; un 3,8-diazabicyclo[3.2.1]octanyle ; un 5-oxa-2,8-diazaspiro[3.5]nonanyle ; un 9-oxa-3,7-diazabicyclo[3.3.1]nonanyle ; un amino(alcoxy en $C_{1-6}$)pyrrolidinyle ; un amino(alkyle en $C_{1-6}$)azétidinyle ; un amino(alkyle en $C_{1-6}$)pyrrolidinyle ; un amino-1,4-oxazépanyle ; un amino-2-azaspiro[3.3]heptanyle ; un aminoazétidinyle ; un aminohalogénopyrrolidinyle ; un alkyle en $C_{1-6}$ ; un alkyle en $C_{1-6}$-2,6-diazaspiro[3.3]heptanecarbonyle ; un alkyle en $C_{1-6}$-2,6-diazaspiro[3.3]heptanyle ; un alkyle en $C_{1-6}$-pipérazinyle et un pipérazinyle ; ou
un pyrimidinyle substitué une ou deux fois par des substituants indépendamment choisis parmi un amino(alcoxy en $C_{1-6}$)pyrrolidinyle, un amino(alkyle en $C_{1-6}$)azétidinyle, un aminoazétidinyle, un alkyle en $C_{1-6}$ et un pipérazinyle ;

n représente 0, 1 ou 2 ;

ou un sel pharmaceutiquement acceptable de celui-ci.

**4.** Composé selon la revendication 2 ou 3, dans lequel $R^1$ représente

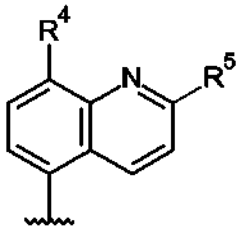 , 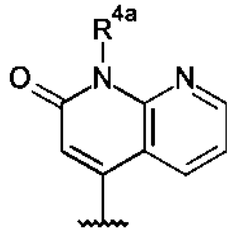 ou 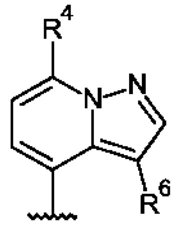 ;

dans lequel $R^4$ représente un cyano ; $R^{4a}$ représente un alkyle en $C_{1-6}$ ; $R^5$ représente H ou un deutérium ; $R^6$ représente H.

**5.** Composé selon la revendication 4, dans lequel $R^2$ représente un alkyle en $C_{1-6}$.

**6.** Composé selon la revendication 5, dans lequel

$R^1$ représente

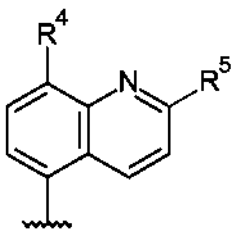 , 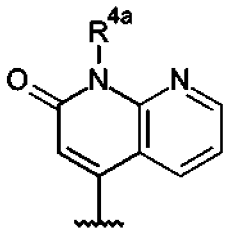 ou 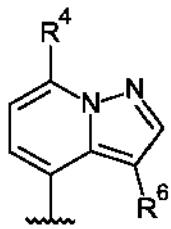 ;

dans lequel $R^4$ représente un cyano ; $R^{4a}$ représente un alkyle en $C_{1-6}$ ; $R^5$ représente H ou un deutérium ; $R^6$ représente H ;
$R^2$ représente un méthyle ;
$R^3$ représente H ;
le cycle A représente un 1,2,3,4-tétrahydroisoquinoléin-7-yle ; un 1,2,3,4-tétrahydroisoquinoléin-5-yle ; un 1,2,3,4-tétrahydroisoquinoléin-6-yle ; un 1,2,3,4-tétrahydroisoquinoléin-7-yle ; un 1,2,3,4-tétrahydroisoquinoléin-8-yle ; un 5,6,7,8-tétrahydro-1,6-naphtyridin-2-yle ; un 5,6,7,8-tétrahydro-1,6-naphtyridin-3-yle ; un 5,6,7,8-tétrahydro-2,6-naphtyridin-1-yle ; un 5,6,7,8-tétrahydro-2,7-naphtyridin-4-yle ; un isoindolin-4-yle ; un 3-amino-4-méthoxy-pyrrolidin-1-ylphényle ; un 5-oxa-2,8-diazaspiro[3.5]nonan-2-ylphényle ; un morpholin-2-ylphényle ; un pipérazin-1-ylphényle ; un (4-fluoropyrrolidin-3-yl)amino(méthyl)pyridinyle ; un 1,4-diazépan-1-ylpyridinyle ; un 2,5-diazabicyclo[2.2.1]heptan-2-yl(méthyl)pyridinyle ; un 2,5-diazabicyclo[2.2.1]heptan-2-ylpyridinyle ; un 2-méthylpipérazin-1-yl(méthyl)pyridinyle ; un 3,4,4a,5,7,7a-hexahydro-2H-pyrrolo[3,4-b][1,4]oxazin-6-yl(méthyl)pyridinyle ; un (3,4,4a,5,7,7a-hexahydro-2H-pyrrolo[3,4-b][1,4]oxazin-6-yl)pyridinyle ; un 3,6-diazabicyclo[3.1.1]heptan-3-yl(méthyl)pyridinyle ; un 3,8-diazabicyclo[3.2.1]octan-3-yl(méthyl)pyridinyle ; un 3,8-diazabicyclo[3.2.1]octan-3-ylpyridinyle ; un 3-amino-3-méthyl-azétidin-1-yl(méthyl)pyridinyle ; un 3-amino-3-méthyl-azétidin-1-ylpyridinyle ; un 3-amino-3-méthyl-pyrrolidin-1-yl(méthyl)pyridinyle ; un 3-amino-3-méthyl-pyrrolidin-1-ylpyridinyle ; un 3-amino-4-fluoro-pyrrolidin-1-ylpyridinyle ; un 3-amino-4-méthoxy-pyrrolidin-1-yl(méthyl)pyridinyle ; un 3-amino-4-méthoxy-pyrrolidin-1-ylpyridinyle ; un 3-aminoazétidin-1-ylpyridinyle ; un 3-méthylpipérazin-1-yl(méthyl)pyridinyle ; un 3-méthylpipérazin-1-ylpyridinyle ; un 5-oxa-2,8-diazaspiro[3.5]nonan-2-ylpyridinyle ; un 6-amino-1,4-oxazépan-4-yl(méthyl)pyridinyle ; un 6-amino-1,4-oxazépan-4-ylpyridinyle ; un 6-amino-2-azaspiro[3.3]heptan-2-yl(méthyl)pyridinyle ; un 6-méthyl-2,6-diazaspiro[3.3]heptan-2-yl(méthyl)pyridinyle ; un 6-méthyl-2,6-diazaspiro[3.3]heptan-2-ylpyridinyle ; un 6-méthyl-2,6-diazaspiro[3.3]heptane-2-carbonyl(méthyl)pyridinyle ; un 9-oxa-3,7-diazabicyclo[3.3.1]nonan-3-yl(méthyl)pyridinyle ; un pipérazin-1-yl(méthyl)pyridinyle ; un pipérazin-1-ylpyridinyle ; un 3-amino-3-méthyl-azétidin-1-yl(méthyl)pyrimidinyle ; un 3-amino-4-méthoxy-pyrrolidin-1-yl(méthyl)pyrimidinyle ; un 3-amino-4-méthoxy-pyrrolidin-1-ylpyrimidinyle ; un 3-aminoazétidin-1-ylpyrimidinyle ; un pipérazin-1-yl(méthyl)pyrimidinyle ou un pipérazin-1-ylpyrimidinyle ;
n représente 0, 1 ou 2 ;

ou un sel pharmaceutiquement acceptable de celui-ci.

**7.** Composé selon la revendication 5, dans lequel $R^1$ représente

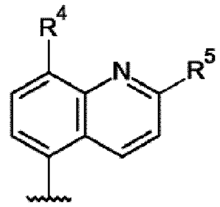

;

dans lequel $R^4$ représente un cyano ; $R^5$ représente H ou un deutérium.

**8.** Composé selon la revendication 7, dans lequel
le cycle A représente un 1,2,3,4-tétrahydroisoquinoléinyle ;

un phényle substitué par un morpholinyle ;
un pyridinyle substitué une ou deux fois par des substituants indépendamment choisis parmi un 3,4,4a,5,7,7a-hexahydro-2H-pyrrolo[3,4-b][1,4]oxazinyle ; un amino(alcoxy en $C_{1-6}$)pyrrolidinyle ; un amino(alkyle en $C_{1-6}$)azétidinyle ; un aminohalogénopyrrolidinyle ; un alkyle en $C_{1-6}$ ; un alkyle en $C_{1-6}$-2,6-diazaspiro[3.3]heptanyle et un pipérazinyle ;
un pyrimidinyle substitué deux fois par des substituants indépendamment choisis parmi un amino(alcoxy en $C_{1-6}$)pyrrolidinyle ; un amino(alkyle en $C_{1-6}$)azétidinyle et un alkyle en $C_{1-6}$.

**9.** Composé selon la revendication 8, dans lequel le cycle A représente un 1,2,3,4-tétrahydroisoquinoléinyle ; un morpholinylphényle ; un pipérazinylpyridinyle ; un (amino(alcoxy en $C_{1-6}$)pyrrolidinyl)pyridinyle ; un (aminohalogénopyrrolidinyl)pyridinyle ; un pipérazinyl(alkyle en $C_{1-6}$)pyridinyle ; un (amino(alcoxy en $C_{1-6}$)pyrrolidinyl)pyridinyle ; un (aminohalogénopyrrolidinyl)pyridinyle ; un (alkyle en $C_{1-6}$-2,6-diazaspiro[3.3]heptanyl)pyridinyle ; un (amino(alkyle en $C_{1-6}$)azétidinyl)pyridinyle ; un alkyle en $C_{1-6}$(amino(alkyle en $C_{1-6}$)azétidinyl)pyridinyle ; un 3,4,4a,5,7,7a-hexahydro-2H-pyrrolo[3,4-b][1,4]oxazinyl(alkyle en $C_{1-6}$)pyridinyle ; un (3,4,4a,5,7,7a-hexahydro-2H-pyrrolo[3,4-b][1,4]oxazinyl)pyridinyle ; un alkyle en $C_{1-6}$(amino(alkyle en $C_{1-6}$)azétidinyl)pyrimidinyle ou un alkyle en $C_{1-6}$(amino(alcoxy en $C_{1-6}$)pyrrolidinyl)pyrimidinyle.

**10.** Composé selon la revendication 9, dans lequel le cycle A représente un 1,2,3,4-tétrahydroisoquinoléin-7-yle ; un morpholin-2-ylphényle ; un (3,4,4a,5,7,7a-hexahydro-2H-pyrrolo[3,4-b][1,4]oxazin-6-yl)-2-méthyl-3-pyridinyle ; un (3,4,4a,5,7,7a-hexahydro-2H-pyrrolo[3,4-b][1,4]oxazin-6-yl)-3-pyridinyle ; un (3-amino-4-fluoro-pyrrolidin-1-yl)-3-pyridinyle ; un (3-amino-4-fluoro-pyrrolidin-1-yl)-3-pyridinyle ; un (3-amino-4-méthoxy-pyrrolidin-1-yl)-3-pyridinyle ; un (3-amino-4-méthoxy-pyrrolidin-1-yl)-3-pyridinyle ; un 2-méthyl-6-pipérazin-1-yl-3-pyridinyle ; un 6-(3-amino-3-méthyl-azétidin-1-yl)-2-méthyl-3-pyridinyle ; un 6-(3-amino-3-méthyl-azétidin-1-yl)-3-pyridinyle ; un 6-(6-méthyl-2,6-diazaspiro[3.3]heptan-2-yl)-3-pyridinyle ; un 6-pipérazin-1-yl-3-pyridinyle ; un (3-amino-4-méthoxy-pyrrolidin-1-yl)-6-méthyl-pyrimidin-4-yle ou un (3-amino-3-méthyl-azétidin-1-yl)-6-méthyl-pyrimidin-4-yle.

**11.** Composé selon la revendication 2 ou 3, dans lequel

$R^1$ représente

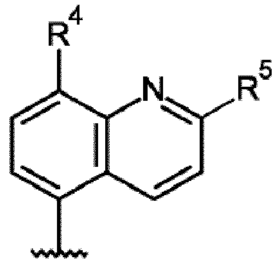

;

dans lequel $R^4$ représente un cyano ; $R^5$ représente H ou un deutérium ;
$R^2$ représente un alkyle en $C_{1-6}$ ;
$R^3$ représente H ;
le cycle A représente un 1,2,3,4-tétrahydroisoquinoléinyle ;

un phényle substitué par un morpholinyle ;
un pyridinyle substitué une ou deux fois par des substituants indépendamment choisis parmi un 3,4,4a,5,7,7a-hexahydro-2H-pyrrolo[3,4-b][1,4]oxazinyle ; un amino(alcoxy en $C_{1-6}$)pyrrolidinyle ; un amino(alkyle en $C_{1-6}$)azétidinyle ; un aminohalogénopyrrolidinyle ; un alkyle en $C_{1-6}$ ; un alkyle en $C_{1-6}$-2,6-diazaspiro[3.3]heptanyle et un pipérazinyle ;
un pyrimidinyle substitué deux fois par des substituants indépendamment choisis parmi un amino(alcoxy en $C_{1-6}$)pyrrolidinyle ; un amino(alkyle en $C_{1-6}$)azétidinyle et un alkyle en $C_{1-6}$ ;

n représente 0, 1 ou 2 ;

ou un sel pharmaceutiquement acceptable de celui-ci.

**12.** Composé selon la revendication 11, dans lequel

$R^1$ représente

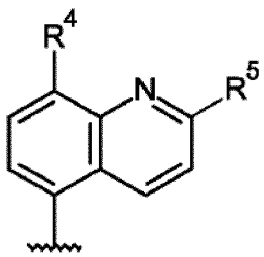

;

dans lequel $R^4$ représente un cyano ; $R^5$ représente H ou un deutérium ;
$R^2$ représente un alkyle en $C_{1-6}$ ;
$R^3$ représente H ;
le cycle A représente un 1,2,3,4-tétrahydroisoquinoléinyle ; un morpholinylphényle ; un pipérazinylpyridinyle ; un (amino(alcoxy en $C_{1-6}$)pyrrolidinyl)pyridinyle ; un (aminohalogénopyrrolidinyl)pyridinyle ; un pipérazinyl(alkyle en $C_{1-6}$)pyridinyle ; un (amino(alcoxy en $C_{1-6}$)pyrrolidinyl)pyridinyle ; un (aminohalogénopyrrolidinyl)pyridinyle ; un (alkyle en $C_{1-6}$-2,6-diazaspiro[3.3]heptanyl)pyridinyle ; un (amino(alkyle en $C_{1-6}$)azétidinyl)pyridinyle ; un alkyle en $C_{1-6}$(amino(alkyle en $C_{1-6}$)azétidinyl)pyridinyle ; un 3,4,4a,5,7,7a-hexahydro-2H-pyrrolo[3,4-b][1,4]oxazinyl(alkyle en $C_{1-6}$)pyridinyle ; un (3,4,4a,5,7,7a-hexahydro-2H-pyrrolo[3,4-b][1,4]oxazinyl)pyridinyle ; un alkyle en $C_{1-6}$(amino(alkyle en $C_{1-6}$)azétidinyl)pyrimidinyle ou un alkyle en $C_{1-6}$(amino(alcoxy en $C_{1-6}$)pyrrolidinyl)pyrimidinyle ;
n représente 0, 1 ou 2 ;

ou un sel pharmaceutiquement acceptable de celui-ci.

**13.** Composé selon la revendication 11, dans lequel

$R^1$ représente

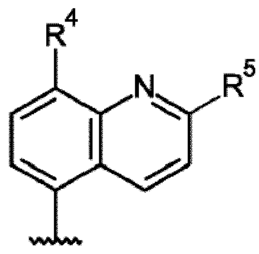

;

dans lequel $R^4$ représente un cyano ; $R^5$ représente H ou un deutérium ;
$R^2$ représente un méthyle ;
$R^3$ représente H ;
le cycle A représente un 1,2,3,4-tétrahydroisoquinoléin-7-yle ; un 4-(morpholin-2-yl)phényle ; un (3,4,4a,5,7,7a-hexahydro-2H-pyrrolo[3,4-b][1,4]oxazin-6-yl)-2-méthyl-3-pyridinyle ; un (3,4,4a,5,7,7a-hexahydro-2H-pyrrolo[3,4-b][1,4]oxazin-6-yl)-3-pyridinyle ; un (3-amino-4-fluoro-pyrrolidin-1-yl)-3-pyridinyle ; un (3-amino-4-fluoro-pyrrolidin-1-yl)-3-pyridinyle ; un (3-amino-4-méthoxy-pyrrolidin-1-yl)-3-pyridinyle ; un (3-amino-4-méthoxy-pyrrolidin-1-yl)-3-pyridinyle ; un 2-méthyl-6-pipérazin-1-yl-3-pyridinyle ; un 6-(3-amino-3-méthyl-azétidin-1-yl)-2-méthyl-3-pyridinyle ; un 6-(3-amino-3-méthyl-azétidin-1-yl)-3-pyridinyle ; un 6-(6-méthyl-2,6-diazaspiro[3.3]heptan-2-yl)-3-pyridinyle ; un 6-pipérazin-1-yl-3-pyridinyle ; un (3-amino-4-méthoxy-pyrrolidin-1-yl)-6-méthyl-pyrimidin-4-yle ou un (3-amino-3-méthyl-azétidin-1-yl)-6-méthyl-pyrimidin-4-yle ;
n représente 0, 1 ou 2 ;

ou un sel pharmaceutiquement acceptable de celui-ci.

**14.** Composé selon l'une quelconque des revendications 1 à 13, dans lequel le composé est choisi parmi :

le 5-[*cis*-4-méthyl-8-(1,2,3,4-tétrahydroisoquinoléin-7-ylméthyl)-3,4,6,7,9,9a-hexahydro-*1H*-pyrazino[1,2-a]pyrazin-2-yl]quinoléine-8-carbonitrile ;
le 5-[*cis*-4-méthyl-8-(1,2,3,4-tétrahydroisoquinoléin-5-yl)-3,4,6,7,9,9a-hexahydro-*1H*-pyrazino[1,2-a]pyrazin-2-yl]quinoléine-8-carbonitrile ;
le 5-[*cis*-4-méthyl-8-[(2-pipérazin-1-yl-4-pyridyl)méthyl]-3,4,6,7,9,9a-hexahydro-*1H*-pyrazino[1,2-a]pyrazin-2-yl]quinoléine-8-carbonitrile ;
le 5-[*cis*-8-isoindolin-4-yl-4-méthyl-3,4,6,7,9,9a-hexahydro-*1H*-pyrazino[1,2-a]pyrazin-2-yl]quinoléine-8-carbonitrile ;
le 5-[(*4R*,*9aS*)-4-méthyl-8-(1,2,3,4-tétrahydroisoquinoléin-7-ylméthyl)-3,4,6,7,9,9a-hexahydro-*1H*-pyrazino[1,2-a]pyrazin-2-yl]quinoléine-8-carbonitrile ;
le 5-[(*4R*,*9aS*)-4-méthyl-8-(5,6,7,8-tétrahydro-1,6-naphtyridin-3-ylméthyl)-3,4,6,7,9,9a-hexahydro-*1H*-pyrazino[1,2-a]pyrazin-2-yl]quinoléine-8-carbonitrile;
le 5-[(*4R*,*9aS*)-4-méthyl-8-[2-(4-pipérazin-1-ylphényl)éthyl]-3,4,6,7,9,9a-hexahydro-*1H*-pyrazino[1,2-a]pyrazin-2-yl]quinoléine-8-carbonitrile,
le 5-[(*4R*,*9aS*)-4-méthyl-8-[(6-pipérazin-1-yl-3-pyridyl)méthyl]-3,4,6,7,9,9a-hexahydro-*1H*-pyrazino[1,2-a]pyrazin-2-yl]quinoléine-8-carbonitrile,
le 5-[(*4R*,*9aS*)-8-isoindolin-4-yl-4-méthyl-3,4,6,7,9,9a-hexahydro-*1H*-pyrazino[1,2-a]pyrazin-2-yl]quinoléine-8-carbonitrile ;
le 5-[(*4R*,*9aS*)-4-méthyl-8-(1,2,3,4-tétrahydroisoquinoléin-6-ylméthyl)-3,4,6,7,9,9a-hexahydro-*1H*-pyrazino[1,2-a]pyrazin-2-yl]quinoléine-8-carbonitrile ;
le 5-[(*4R*,*9aS*)-4-méthyl-8-[2-(6-pipérazin-1-yl-3-pyridyl)éthyl]-3,4,6,7,9,9a-hexahydro-*1H*-pyrazino[1,2-a]pyrazin-2-yl]quinoléine-8-carbonitrile,
le 5-[(*4R*,*9aS*)-8-[2-[6-[(*3R*,*4R*)-3-amino-4-méthoxy-pyrrolidin-1-yl]-3-pyridyl]éthyl]-4-méthyl-3,4,6,7,9,9a-hexahydro-*1H*-pyrazino[1,2-a]pyrazin-2-yl]quinoléine-8-carbonitrile,
le 5-[(*4R*,*9aS*)-8-[2-[5-[(*3R*,*4R*)-3-amino-4-méthoxy-pyrrolidin-1-yl]-2-pyridyl]éthyl]-4-méthyl-3,4,6,7,9,9a-hexahydro-*1H*-pyrazino[1,2-a]pyrazin-2-yl]quinoléine-8-carbonitrile,
le 5-[(*4R*,*9aS*)-8-[[6-(1,4-diazépan-1-yl)-3-pyridyl]méthyl]-4-méthyl-3,4,6,7,9,9a-hexahydro-*1H*-pyrazino[1,2-a]pyrazin-2-yl]quinoléine-8-carbonitrile ;
le 5-[(*4R*,*9a*R)-4-méthyl-8-(5,6,7,8-tétrahydro-2,6-naphtyridin-1-yl)-3,4,6,7,9,9a-hexahydro-*1H*-pyrazino[1,2-a]pyrazin-2-yl]quinoléine-8-carbonitrile ;
le 5-[(*4R*,*9aS*)-8-[2-[6-[(*3R*,*4S*)-3-amino-4-fluoro-pyrrolidin-1-yl]-3-pyridyl]éthyl]-4-méthyl-3,4,6,7,9,9a-hexahydro-*1H*-pyrazino[1,2-a]pyrazin-2-yl]quinoléine-8-carbonitrile,

le 5-[(*4R*,*9aS*)-8-[2-[6-[(*3R*,*4R*)-3-amino-4-fluoro-pyrrolidin-1-yl]-3-pyridyl]éthyl]-4-méthyl-3,4,6,7,9,9a-hexahydro-*1H*-pyrazino[1,2-a]pyrazin-2-yl]quinoléine-8-carbonitrile,

le 5-[(*4R*,*9aS*)-8-[2-[5-[(*3R*,*4S*)-3-amino-4-fluoro-pyrrolidin-1-yl]-2-pyridyl]éthyl]-4-méthyl-3,4,6,7,9,9a-hexahydro-*1H*-pyrazino[1,2-a]pyrazin-2-yl]quinoléine-8-carbonitrile,

le 5-[(*4R*,*9aS*)-4-méthyl-8-[2-[5-[(*3S*)-3-méthylpipérazin-1-yl]-2-pyridyl]éthyl]-3,4,6,7,9,9a-hexahydro-*1H*-pyrazino[1,2-a]pyrazin-2-yl]quinoléine-8-carbonitrile ;

le 5-[(*4R*,*9aS*)-4-méthyl-8-[2-[6-[(*3S*)-3-méthylpipérazin-1-yl]-2-pyridyl]éthyl]-3,4,6,7,9,9a-hexahydro-*1H*-pyrazino[1,2-a]pyrazin-2-yl]quinoléine-8-carbonitrile ;

le 5-[(*4R*,*9aS*)-4-méthyl-8-[2-[6-(6-méthyl-2,6-diazaspiro[3.3]heptan-2-yl)-3-pyridyl]éthyl]-3,4,6,7,9,9a-hexahydro-*1H*-pyrazino[1,2-a]pyrazin-2-yl]quinoléine-8-carbonitrile ;

le 5-[(*4R*,*9aS*)-8-[2-[6-[(*3R*,*4S*)-3-amino-4-méthoxy-pyrrolidin-1-yl]-3-pyridyl]éthyl]-4-méthyl-3,4,6,7,9,9a-hexahydro-*1H*-pyrazino[1,2-a]pyrazin-2-yl]quinoléine-8-carbonitrile ;

le 5-[(*4R*,*9aS*)-8-[2-[6-[(*3S*,*4S*)-3-amino-4-méthoxy-pyrrolidin-1-yl]-3-pyridyl]éthyl]-4-méthyl-3,4,6,7,9,9a-hexahydro-*1H*-pyrazino[1,2-a]pyrazin-2-yl]quinoléine-8-carbonitrile ;

le 5-[(*4R*,*9aS*)-8-[2-[4-[(*3R*,*4R*)-3-amino-4-méthoxy-pyrrolidin-1-yl]phényl]éthyl]-4-méthyl-3,4,6,7,9,9a-hexahydro-*1H*-pyrazino[1,2-a]pyrazin-2-yl]quinoléine-8-carbonitrile ;

le 5-[(*4R*,*9aS*)-4-méthyl-8-[(6-pipérazin-1-yl-2-pyridyl)méthyl]-3,4,6,7,9,9a-hexahydro-*1H*-pyrazino[1,2-a]pyrazin-2-yl]quinoléine-8-carbonitrile ;

le 5-[(*4R*,*9aS*)-4-méthyl-8-[2-[4-(5-oxa-2,8-diazaspiro[3.5]nonan-2-yl)phényl]éthyl]-3,4,6,7,9,9a-hexahydro-*1H*-pyrazino[1,2-a]pyrazin-2-yl]quinoléine-8-carbonitrile ;

le 5-[(*4R*,*9aR*)-4-méthyl-8-(4-pipérazin-1-ylpyrimidin-2-yl)-3,4,6,7,9,9a-hexahydro-*1H*-pyrazino[1,2-a]pyrazin-2-yl]quinoléine-8-carbonitrile ;

le 5-[(*4R*,*9aR*)-4-méthyl-8-(6-méthyl-2-pipérazin-1-yl-pyrimidin-4-yl)-3,4,6,7,9,9a-hexahydro-1*H*-pyrazino[1,2-a]pyrazin-2-yl]quinoléine-8-carbonitrile ;

le 5-[(*4R*,*9aS*)-4-méthyl-8-(2-pipérazin-1-yl-4-pyridyl)-3,4,6,7,9,9a-hexahydro-1*H*-pyrazino[1,2-a]pyrazin-2-yl]quinoléine-8-carbonitrile ;

le 5-[(*4R*,*9aR*)-4-méthyl-8-(2-pipérazin-1-ylpyrimidin-4-yl)-3,4,6,7,9,9a-hexahydro-1*H*-pyrazino[1,2-a]pyrazin-2-yl]quinoléine-8-carbonitrile ;

le 5-[(*4R*,*9aR*)-4-méthyl-8-(4-pipérazin-1-yl-2-pyridyl)-3,4,6,7,9,9a-hexahydro-1*H*-pyrazino[1,2-a]pyrazin-2-yl]quinoléine-8-carbonitrile ;

le 5-(8-isoindolin-4-yl-3,4,6,7,9,9a-hexahydro-1*H*-pyrazino[1,2-a]pyrazin-2-yl)quinoléine-8-carbonitrile ;

le 5-[(*4R*,*9aR*)-8-[2-[(*3R*,*4R*)-3-amino-4-méthoxy-pyrrolidin-1-yl]pyrimidin-4-yl]-4-méthyl-3,4,6,7,9,9a-hexahydro-1*H*-pyrazino[1,2-a]pyrazin-2-yl]quinoléine-8-carbonitrile ;

le 5-[(*4R*,*9aR*)-8-[4-[(*3R*,*4R*)-3-amino-4-méthoxy-pyrrolidin-1-yl]pyrimidin-2-yl]-4-méthyl-3,4,6,7,9,9a-hexahydro-1*H*-pyrazino[1,2-a]pyrazin-2-yl]quinoléine-8-carbonitrile ;

le 5-[2-(4-méthyl-6-pipérazin-1-yl-3-pyridyl)-3,4,6,7,9,9a-hexahydro-1*H*-pyrazino[1,2-a]pyrazin-8-yl]quinoléine-8-carbonitrile ;

le 5-[(*4R*,*9aR*)-8-[4-(3-aminoazétidin-1-yl)pyrimidin-2-yl]-4-méthyl-3,4,6,7,9,9a-hexahydro-*1H*-pyrazino[1,2-a]pyrazin-2-yl]quinoléine-8-carbonitrile;

le 5-[(*4R*,*9aS*)-8-[2-[(*3S*,*4R*)-3-amino-4-fluoro-pyrrolidin-1-yl]-4-pyridyl]-4-méthyl-3,4,6,7,9,9a-hexahydro-1*H*-pyrazino[1,2-a]pyrazin-2-yl]quinoléine-8-carbonitrile ;

le 5-[(*4R*,*9aS*)-8-[2-(3-aminoazétidin-1-yl)-4-pyridyl]-4-méthyl-3,4,6,7,9,9a-hexahydro-1*H*-pyrazino[1,2-a]pyrazin-2-yl]quinoléine-8-carbonitrile ;

le 5-[(*4R*,*9aS*)-4-méthyl-8-(5,6,7,8-tétrahydro-2,7-naphtyridin-4-yl)-3,4,6,7,9,9a-hexahydro-1*H*-pyrazino[1,2-a]pyrazin-2-yl]quinoléine-8-carbonitrile ;

le 5-[(*4R*,*9aR*)-4-méthyl-8-(5-méthyl-4-pipérazin-1-yl-pyrimidin-2-yl)-3,4,6,7,9,9a-hexahydro-1*H*-pyrazino[1,2-a]pyrazin-2-yl]quinoléine-8-carbonitrile ;

le 5-[(*4R*,*9aR*)-4-méthyl-8-(5,6,7,8-tétrahydro-2,7-naphtyridin-4-yl)-3,4,6,7,9,9a-hexahydro-1*H*-pyrazino[1,2-a]pyrazin-2-yl]quinoléine-8-carbonitrile ;

le 5-[(*4R*,*9aR*)-8-[2-[(*3R*,*4R*)-3-amino-4-méthoxy-pyrrolidin-1-yl]-6-méthyl-pyrimidin-4-yl]-4-méthyl-3,4,6,7,9,9a-hexahydro-1*H*-pyrazino[1,2-a]pyrazin-2-yl]quinoléine-8-carbonitrile ;

le 5-[2-[6-[(*6R*)-6-amino-1,4-oxazépan-4-yl]-4-méthyl-3-pyridyl]-3,4,6,7,9,9a-hexahydro-1*H*-pyrazino[1,2-a]pyrazin-8-yl]quinoléine-8-carbonitrile ;

le 5-[*trans*-4-méthyl-8-(1,2,3,4-tétrahydroisoquinoléin-5-yl)-3,4,6,7,9,9a-hexahydro-1*H*-pyrazino[1,2-a]pyrazin-2-yl]quinoléine-8-carbonitrile ;

le 5-[(*4R*,*9aS*)-8-[2-[(*6S*)-6-amino-1,4-oxazépan-4-yl]-4-pyridyl]-4-méthyl-3,4,6,7,9,9a-hexahydro-1*H*-pyrazino[1,2-a]pyrazin-2-yl]quinoléine-8-carbonitrile ;

le 5-[(*4R*,*9aS*)-8-[2-[(*6R*)-6-amino-1,4-oxazépan-4-yl]-4-pyridyl]-4-méthyl-3,4,6,7,9,9a-hexahydro-1*H*-pyrazino[1,2-a]pyrazin-2-yl]quinoléine-8-carbonitrile ;

le 5-[(*4R*,*9aR*)-8-[2-(3-amino-3-méthyl-azétidin-1-yl)-6-méthyl-pyrimidin-4-yl]-4-méthyl-3,4,6,7,9,9a-hexahydro-1*H*-pyrazino[1,2-a]pyrazin-2-yl]quinoléine-8-carbonitrile ;

le 5-[(*4R*,*9aS*)-8-[2-(3-amino-3-méthyl-azétidin-1-yl)-4-pyridyl]-4-méthyl-3,4,6,7,9,9a-hexahydro-1*H*-pyrazino[1,2-a]pyrazin-2-yl]quinoléine-8-carbonitrile ;

le 5-[(*4R*,*9aS*)-4-méthyl-8-[(5-pipérazin-1-yl-3-pyridyl)méthyl]-3,4,6,7,9,9a-hexahydro-*1H*-pyrazino[1,2-a]pyrazin-2-yl]quinoléine-8-carbonitrile ;

le 5-[(*4R*,*9aS*)-4-méthyl-8-[2-[6-[(*3R*)-3-méthylpipérazin-1-yl]-2-pyridyl]éthyl]-3,4,6,7,9,9a-hexahydro-*1H*-pyrazino[1,2-a]pyrazin-2-yl]quinoléine-8-carbonitrile ;

le 5-[(*4R*,*9aS*)-4-méthyl-8-[2-[6-(5-oxa-2,8-diazaspiro[3.5]nonan-2-yl)-3-pyridyl]éthyl]-3,4,6,7,9,9a-hexahydro-*1H*-pyrazino[1,2-a]pyrazin-2-yl]quinoléine-8-carbonitrile ;

le 5-[(*4R*,*9aS*)-4-méthyl-8-[(2-méthyl-6-pipérazin-1-yl-3-pyridyl)méthyl]-3,4,6,7,9,9a-hexahydro-*1H*-pyrazino[1,2-a]pyrazin-2-yl]quinoléine-8-carbonitrile ;

le 5-[(*4R*,*9aS*)-4-méthyl-8-(1,2,3,4-tétrahydroisoquinoléin-7-ylméthyl)-3,4,6,7,9,9a-hexahydro-*1H*-pyrazino[1,2-a]pyrazin-2-yl]-2-deutério-quinoléine-8-carbonitrile ;

le 5-[(*4R*,*9aS*)-8-[2-[6-[(*3R*,*4R*)-3-amino-4-méthoxy-pyrrolidin-1-yl]-3-pyridyl]éthyl]-4-méthyl-3,4,6,7,9,9a-hexahydro-*1H*-pyrazino[1,2-a]pyrazin-2-yl]-2-deutério-quinoléine-8-carbonitrile ;

le 5-[(*4R*,*9aS*)-8-[2-[6-[(*3R*,*4S*)-3-amino-4-fluoro-pyrrolidin-1-yl]-3-pyridyl]éthyl]-4-méthyl-3,4,6,7,9,9a-hexahydro-*1H*-pyrazino[1,2-a]pyrazin-2-yl]-2-deutério-quinoléine-8-carbonitrile ;

le 5-[(*4R*,*9aS*)-4-méthyl-8-[2-[6-(6-méthyl-2,6-diazaspiro[3.3]heptan-2-yl)-3-pyridyl]éthyl]-3,4,6,7,9,9a-hexahydro-*1H*-pyrazino[1,2-a]pyrazin-2-yl]-2-deutério-quinoléine-8-carbonitrile ;

le 5-[(*4R*,*9aS*)-8-[2-[6-(3-amino-3-méthyl-azétidin-1-yl)-3-pyridyl]éthyl]-4-méthyl-3,4,6,7,9,9a-hexahydro-*1H*-pyrazino[1,2-a]pyrazin-2-yl]quinoléine-8-carbonitrile ;

le 4-[(*4R*,*9aS*)-8-[2-[4-[(*3R*,*4R*)-3-amino-4-méthoxy-pyrrolidin-1-yl]phényl]éthyl]-4-méthyl-3,4,6,7,9,9a-hexahydro-*1H*-pyrazino[1,2-a]pyrazin-2-yl]-3-fluoro-pyrazolo[1,5-a]pyridine-7-carbonitrile ;

le 4-[(*4R*,*9aS*)-8-[2-[6-[(*3R*,*4R*)-3-amino-4-méthoxy-pyrrolidin-1-yl]-3-pyridyl]éthyl]-4-méthyl-3,4,6,7,9,9a-hexahydro-*1H*-pyrazino[1,2-a]pyrazin-2-yl]-3-fluoro-pyrazolo[1,5-a]pyridine-7-carbonitrile ;

le 4-[(*4R*,*9aS*)-8-[2-[4-[(*3R*,*4R*)-3-amino-4-méthoxy-pyrrolidin-1-yl]phényl]éthyl]-4-méthyl-3,4,6,7,9,9a-hexahydro-*1H*-pyrazino[1,2-a]pyrazin-2-yl]pyrazolo[1,5-a]pyridine-7-carbonitrile ;

le 4-[(*4R*,*9aS*)-4-méthyl-8-(1,2,3,4-tétrahydroisoquinoléin-7-ylméthyl)-3,4,6,7,9,9a-hexahydro-*1H*-pyrazino[1,2-a]pyrazin-2-yl]-3-fluoro-pyrazolo[1,5-a]pyridine-7-carbonitrile ;

le 5-[(*4R*,*9aS*)-4-méthyl-8-(1,2,3,4-tétrahydroisoquinoléin-5-ylméthyl)-3,4,6,7,9,9a-hexahydro-*1H*-pyrazino[1,2-a]pyrazin-2-yl]quinoléine-8-carbonitrile ;

le 5-[(*4R*,*9aS*)-4-méthyl-8-(1,2,3,4-tétrahydroisoquinoléin-8-ylméthyl)-3,4,6,7,9,9a-hexahydro-1H-pyrazino[1,2-a]pyrazin-2-yl]quinoléine-8-carbonitrile ;

le 5-[(*4R*,*9aS*)-8-(isoindolin-4-ylméthyl)-4-méthyl-3,4,6,7,9,9a-hexahydro-*1H*-pyrazino[1,2-a]pyrazin-2-yl]quinoléine-8-carbonitrile ;

le 5-[(*4R*,*9aS*)-8-[2-(3-amino-3-méthyl-azétidin-1-yl)-6-méthyl-4-pyridyl]-4-méthyl-3,4,6,7,9,9a-hexahydro-1*H*-pyrazino[1,2-a]pyrazin-2-yl]quinoléine-8-carbonitrile ;

le 4-[(*4R*,*9aS*)-8-[2-[6-(3-amino-3-méthyl-azétidin-1-yl)-3-pyridyl]éthyl]-4-méthyl-3,4,6,7,9,9a-hexahydro-*1H*-pyrazino[1,2-a]pyrazin-2-yl]-3-fluoro-pyrazolo[1,5-a]pyridine-7-carbonitrile ;

le 4-[(*4R*,*9aS*)-8-[2-[6-[(*3R*,*4S*)-3-amino-4-fluoro-pyrrolidin-1-yl]-3-pyridyl]éthyl]-4-méthyl-3,4,6,7,9,9a-hexahydro-*1H*-pyrazino[1,2-a]pyrazin-2-yl]-3-fluoro-pyrazolo[1,5-a]pyridine-7-carbonitrile ;

le 5-[(*4R*,*9aS*)-8-[[6-(3-amino-3-méthyl-azétidin-1-yl)-2-méthyl-3-pyridyl]méthyl]-4-méthyl-3,4,6,7,9,9a-hexahydro-*1H*-pyrazino[1,2-a]pyrazin-2-yl]quinoléine-8-carbonitrile ;

le 5-[(*4R*,*9aS*)-8-[2-[6-[(*4aR*,*7aR*)-3,4,4a,5,7,7a-hexahydro-*2H*-pyrrolo[3,4-b][1,4]oxazin-6-yl]-3-pyridyl]éthyl]-4-méthyl-3,4,6,7,9,9a-hexahydro-*1H*-pyrazino[1,2-a]pyrazin-2-yl]-2-deutério-quinoléine-8-carbonitrile ;

le 5-[(*4R*,*9aS*)-4-méthyl-8-[(6-pipérazin-1-yl-3-pyridyl)méthyl]-3,4,6,7,9,9a-hexahydro-*1H*-pyrazino[1,2-a]pyrazin-2-yl]-2-deutério-quinoléine-8-carbonitrile ;

le 5-[(*4R*,*9aS*)-8-[[6-(3-amino-3-méthyl-azétidin-1-yl)-2-méthyl-3-pyridyl]méthyl]-4-méthyl-3,4,6,7,9,9a-hexahydro-*1H*-pyrazino[1,2-a]pyrazin-2-yl]-2-deutério-quinoléine-8-carbonitrile ;

le 5-[(*4R*,*9aS*)-4-méthyl-8-[[2-méthyl-6-(6-méthyl-2,6-diazaspiro[3.3]heptan-2-yl)-3-pyridyl]méthyl]-3,4,6,7,9,9a-hexahydro-1*H*-pyrazino[1,2-a]pyrazin-2-yl]-2-deutério-quinoléine-8-carbonitrile ;

le 5-[(*4R*,*9aS*)-8-[[6-[(*3R*)-3-amino-3-méthyl-pyrrolidin-1-yl]-2-méthyl-3-pyridyl]méthyl]-4-méthyl-3,4,6,7,9,9a-hexahydro-1*H*-pyrazino[1,2-a]pyrazin-2-yl]-2-deutério-quinoléine-8-carbonitrile ;

le 5-[(*4R*,*9aS*)-8-[[6-[[(*3S*,*4R*)-4-fluoropyrrolidin-3-yl]amino]-2-méthyl-3-pyridyl]méthyl]-4-méthyl-3,4,6,7,9,9a-hexahydro-*1H*-pyrazino[1,2-a]pyrazin-2-yl]-2-deutério-quinoléine-8-carbonitrile ;

le 5-[(*4R*,*9aS*)-4-méthyl-8-[[2-méthyl-6-(9-oxa-3,7-diazabicyclo[3.3.1]nonan-3-yl)-3-pyridyl]méthyl]-3,4,6,7,9,9a-hexahydro-*1H*-pyrazino[1,2-a]pyrazin-2-yl]-2-deutério-quinoléine-8-carbonitrile ;
le 5-[(*4R*,*9aS*)-8-[2-[6-[(*3R*)-3-amino-3-méthyl-pyrrolidin-1-yl]-3-pyridyl]éthyl]-4-méthyl-3,4,6,7,9,9a-hexahydro-1H-pyrazino[1,2-a]pyrazin-2-yl]-2-deutério-quinoléine-8-carbonitrile ;
le 5-[(*4R*,*9aS*)-4-méthyl-8-[[2-méthyl-6-[(*2S*)-2-méthylpipérazin-1-yl]-3-pyridyl]méthyl]-3,4,6,7,9,9a-hexahydro-*1H*-pyrazino[1,2-a]pyrazin-2-yl]quinoléine-8-carbonitrile ;
le 5-[(*4R*,*9aS*)-4-méthyl-8-[[2-méthyl-6-[(*3R*)-3-méthylpipérazin-1-yl]-3-pyridyl]méthyl]-3,4,6,7,9,9a-hexahydro-*1H*-pyrazino[1,2-a]pyrazin-2-yl]quinoléine-8-carbonitrile ;
le 5-[(*4R*,*9aS*)-4-méthyl-8-[(4-méthyl-6-pipérazin-1-yl-3-pyridyl)méthyl]-3,4,6,7,9,9a-hexahydro-*1H*-pyrazino[1,2-a]pyrazin-2-yl]-2-deutério-quinoléine-8-carbonitrile ;
le 5-[(*4R*,*9aS*)-4-méthyl-8-[(3-méthyl-6-pipérazin-1-yl-2-pyridyl)méthyl]-3,4,6,7,9,9a-hexahydro-*1H*-pyrazino[1,2-a]pyrazin-2-yl]-2-deutério-quinoléine-8-carbonitrile ;
le 5-[(*4R*,*9aS*)-8-[2-[2-[(*3R*,*4S*)-3-amino-4-fluoro-pyrrolidin-1-yl]-4-pyridyl]éthyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aS*)-8-[[6-(3,6-diazabicyclo[3.1.1]heptan-3-yl)-2-méthyl-3-pyridyl]méthyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aS*)-8-[[6-(3,8-diazabicyclo[3.2.1]octan-3-yl)-2-méthyl-3-pyridyl]méthyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aS*)-8-[[6-[(*1S*,*4S*)-2,5-diazabicyclo[2.2.1]heptan-2-yl]-2-méthyl-3-pyridyl]méthyl]-4-méthyl-3,4,6,7,9,9a-hexahydro-*1H*-pyrazino[1,2-a]pyrazin-2-yl]-2-deutério-quinoléine-8-carbonitrile ;
la 4-[(*4R*,*9aR*)-4-méthyl-8-(3-méthyl-5-pipérazin-1-yl-2-pyridyl)-3,4,6,7,9,9a-hexahydro-*1H*-pyrazino[1,2-a]pyrazin-2-yl]-1-méthyl-1,8-naphtyridin-2-one ;
le 5-[(*4R*,*9aS*)-8-[[6-[(*1S*,*4S*)-2,5-diazabicyclo[2.2.1]heptan-2-yl]-4-méthyl-3-pyridyl]méthyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aR*)-8-[5-[(*1S*,*4S*)-2,5-diazabicyclo[2.2.1]heptan-2-yl]-3-méthyl-2-pyridyl]-4-méthyl-3,4,6,7,9,9a-hexahydro-1H-pyrazino[1,2-a]pyrazin-2-yl]-2-deutério-quinoléine-8-carbonitrile ;
le 5-[(*4R*,*9aR*)-8-[5-[(*3R*,*4S*)-3-amino-4-méthoxy-pyrrolidin-1-yl]-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aR*)-8-[5-[(*3R*)-3-amino-3-méthyl-pyrrolidin-1-yl]-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aS*)-8-[[6-[(*4aR*,*7aR*)-3,4,4a,5,7,7a-hexahydro-*2H*-pyrrolo[3,4-b][1,4]oxazin-6-yl]-2-méthyl-3-pyridyl]méthyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aR*)-8-[5-[(*3R*,*4R*)-3-amino-4-méthoxy-pyrrolidin-1-yl]-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aR*)-8-[5-[(*4aR*,*7aR*)-3,4,4a,5,7,7a-hexahydro-*2H*-pyrrolo[3,4-b][1,4]oxazin-6-yl]-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aR*)-8-[5-(6-amino-2-azaspiro[3.3]heptan-2-yl)-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aR*)-4-méthyl-8-[3-méthyl-5-(6-méthyl-2,6-diazaspiro[3.3]heptane-2-carbonyl)-2-pyridyl]-3,4,6,7,9,9a-hexahydro-*1H*-pyrazino[1,2-a]pyrazin-2-yl]-2-deutério-quinoléine-8-carbonitrile ;
le 5-[(*4S*,*9aR*)-4-méthyl-8-[3-méthyl-5-(6-méthyl-2,6-diazaspiro[3.3]heptane-2-carbonyl)-2-pyridyl]-3,4,6,7,9,9a-hexahydro-*1H*-pyrazino[1,2-a]pyrazin-2-yl]-2-deutério-quinoléine-8-carbonitrile ;
le 5-[(*4R*,*9aS*)-8-[2-[(*3R*)-3-amino-3-méthyl-pyrrolidin-1-yl]-4-pyridyl]-4-méthyl-3,4,6,7,9,9a-hexahydro-*1H*-pyrazino[1,2-a]pyrazin-2-yl]quinoléine-8-carbonitrile ;
le 5-[(*4S*,*9aS*)-4-méthyl-8-[4-[(*2R*)-morpholin-2-yl]phényl]-3,4,6,7,9,9a-hexahydro-*1H*-pyrazino[1,2-a]pyrazin-2-yl]-2-deutério-quinoléine-8-carbonitrile ;
le 5-[(*4S*,*9aS*)-4-méthyl-8-[4-[(*2S*)-morpholin-2-yl]phényl]-3,4,6,7,9,9a-hexahydro-*1H*-pyrazino[1,2-a]pyrazin-2-yl]-2-deutério-quinoléine-8-carbonitrile ;
le 5-[(*4S*,*9aR*)-8-[5-(3,6-diazabicyclo[3.1.1]heptan-3-yl)-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4R*,*9aR*)-8-[5-[2-[(*3S*,*4S*)-3-amino-4-méthoxy-pyrrolidin-1-yl]éthyl]-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4S*,*9aR*)-8-[5-[(*6R*)-6-amino-1,4-oxazépan-4-yl]-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4S*,*9aR*)-8-[5-[(*1S*,*4S*)-2,5-diazabicyclo[2.2.1]heptan-2-yl]-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4S*,*9aR*)-8-[6-(3,8-diazabicyclo[3.2.1]octan-3-yl)-3-pyridyl]-4-méthyl-3,4,6,7,9,9a-hexahydro-*1H*-pyrazino[1,2-a]pyrazin-2-yl]-2-deutério-quinoléine-8-carbonitrile ;
la 4-[(*4S*,*9aR*)-4-méthyl-8-[3-méthyl-5-(6-méthyl-2,6-diazaspiro[3.3]heptan-2-yl)-2-pyridyl]-3,4,6,7,9,9a-hexahydro-1*H*-pyrazino[1,2-a]pyrazin-2-yl]-1-méthyl-1,8-naphtyridin-2-one ;
le 5-[(*4S*,*9aR*)-8-[5-[(*4aR*,*7aR*)-3,4,4a,5,7,7a-hexahydro-*2H*-pyrrolo[3,4-b][1,4]oxazin-6-yl]-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4S,9aR*)-8-[6-[(*3R,4R*)-3-amino-4-méthoxy-pyrrolidin-1-yl]-3-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4S,9aR*)-8-[6-[2-[(*3R,4S*)-3-amino-4-fluoro-pyrrolidin-1-yl]éthyl]-3-pyridyl]-4-méthyl-3,4,6,7,9,9a-hexahydro-1*H*-pyrazino[1,2-a]pyrazin-2-yl]-2-deutério-quinoléine-8-carbonitrile ;
le 5-[(*4S,9aR*)-8-[5-(3-amino-3-méthyl-azétidin-1-yl)-3-méthyl-2-pyridyl]-4-méthyl-3,4,6,7,9,9a-hexahydro-*1H*-pyrazino[1,2-a]pyrazin-2-yl]-2-deutério-quinoléine-8-carbonitrile ;
le 5-[(*4S,9aR*)-4-méthyl-8-(5,6,7,8-tétrahydro-1,6-naphtyridin-2-yl)-3,4,6,7,9,9a-hexahydro-1*H*-pyrazino[1,2-a]pyrazin-2-yl]-2-deutério-quinoléine-8-carbonitrile ;
le 5-[(*4S*,*9aR*)-8-[5-[2-[(*3S*,*4S*)-3-amino-4-méthoxy-pyrrolidin-1-yl]éthyl]-2-pyridyl]-4-méthyl-3,4,6,7,9,9a-hexahydro-1*H*-pyrazino[1,2-a]pyrazin-2-yl]-2-deutério-quinoléine-8-carbonitrile ;
le 5-[(*4S*,*9aR*)-8-[5-[[(*3R*,*4R*)-3-amino-4-méthoxy-pyrrolidin-1-yl]méthyl]-6-méthyl-2-pyridyl]-4-méthyl-3,4,6,7,9,9a-hexahydro-1*H*-pyrazino[1,2-a]pyrazin-2-yl]-2-deutério-quinoléine-8-carbonitrile ;
le 5-[(*4R*,*9aS*)-8-[2-[6-[(*4aR*,*7aR*)-3,4,4a,5,7,7a-hexahydro-2*H*-pyrrolo[3,4-b][1,4]oxazin-6-yl]-3-pyridyl]éthyl]-4-méthyl-3,4,6,7,9,9a-hexahydro-1*H*-pyrazino[1,2-a]pyrazin-2-yl]quinoléine-8-carbonitrile ; et
le 5-[(*4S,9aS*)-4-méthyl-8-[4-[(*2R*)-morpholin-2-yl]phényl]-3,4,6,7,9,9a-hexahydro-*1H*-pyrazino[1,2-a]pyrazin-2-yl]quinoléine-8-carbonitrile ;
ou un sel pharmaceutiquement acceptable de ceux-ci.

**15.** Procédé pour la préparation d'un composé selon l'une quelconque des revendications 1 à 14 comprenant l'une quelconque des étapes suivantes :

a) réaction d'amination de Buchwald-Hartwig ou substitution nucléophile entre un composé de formule (IX),

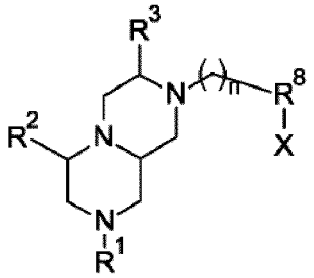

(IX)

et une amine (X),

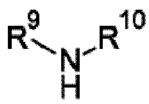

(X) ;

b) réaction d'amination de Buchwald-Hartwig ou substitution nucléophile entre un composé de formule (V),

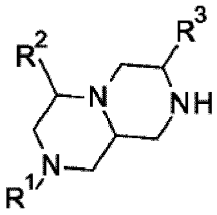

(V)

et un composé de formule (VI),

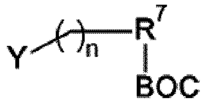 (VI) ;

dans lesquels n représente 0, 1 ou 2 ; X représente un halogène ; Y représente un halogène ou un méthanesulfonate ; $R^7$ et $R^8$ représente un aryle ou un hétéroaryle ; $R^9$ et $R^{10}$ conjointement avec l'atome d'azote ils sont liés pour former un hétérocyclyle.

**16.** Composé ou sel pharmaceutiquement acceptable selon l'une quelconque des revendications 1 à 14 pour une utilisation comme substance thérapeutiquement active.

**17.** Composition pharmaceutique comprenant un composé selon l'une quelconque des revendications 1 à 14 et un véhicule thérapeutiquement inerte.

**18.** Composé ou sel pharmaceutiquement acceptable, énantiomère ou diastéréoisomère selon l'une quelconque des revendications 1 à 14 pour une utilisation dans le traitement ou la prophylaxie du lupus érythémateux disséminé ou de la néphropathie lupique.

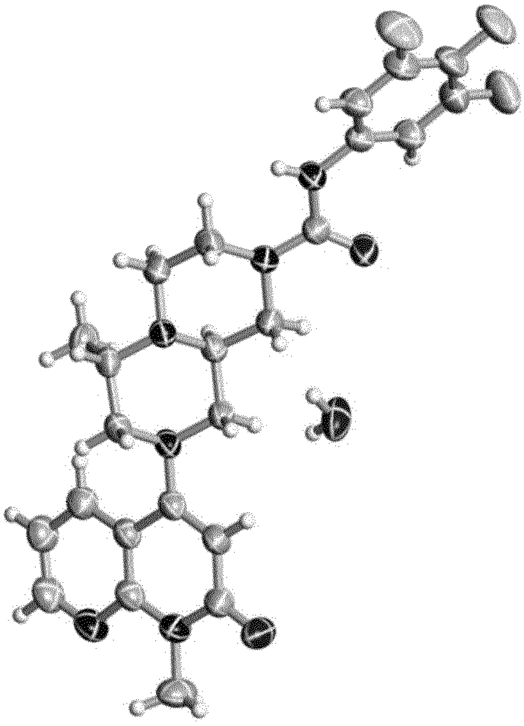

Figure 1

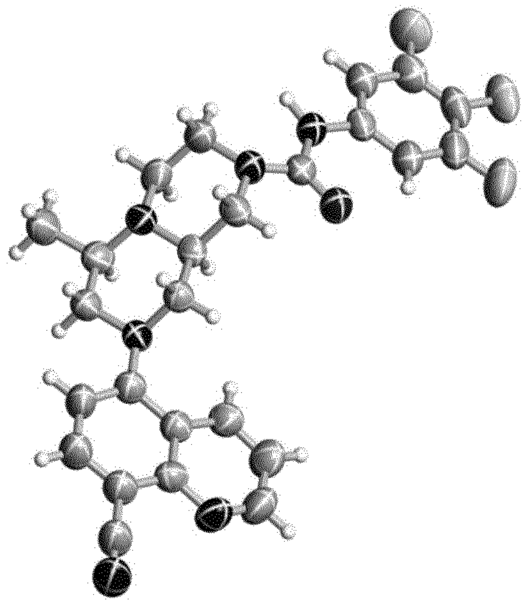

Figure 2

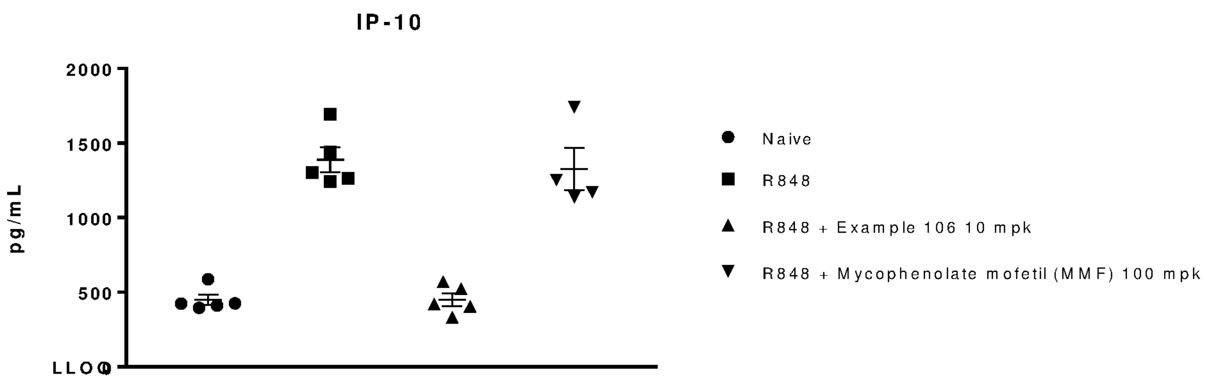
IP-10
2000
1500
1000
500
LLOQ
0
pg/mL
Naive
R848
R848 + Example 106 10 mpk
R848 + Mycophenolate mofetil (MMF) 100 mpk

FIG.3A

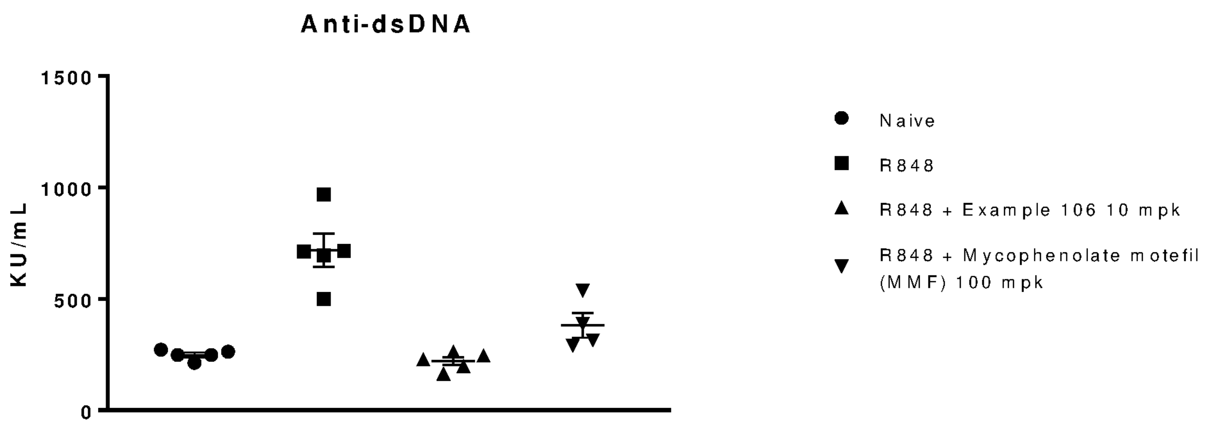
Anti-dsDNA
1500
1000
500
0
KU/mL
Naive
R848
R848 + Example 106 10 mpk
R848 + Mycophenolate motefil (MMF) 100 mpk

FIG.3B

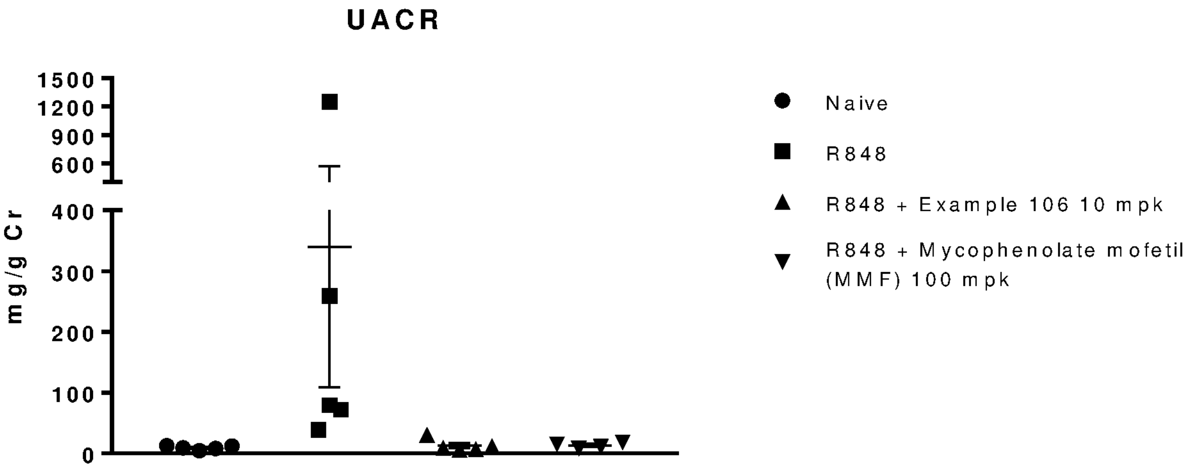
UACR
1500
1200
900
600
400
300
200
100
0
mg/g Cr
Naive
R848
R848 + Example 106 10 mpk
R848 + Mycophenolate mofetil (MMF) 100 mpk

FIG.3C

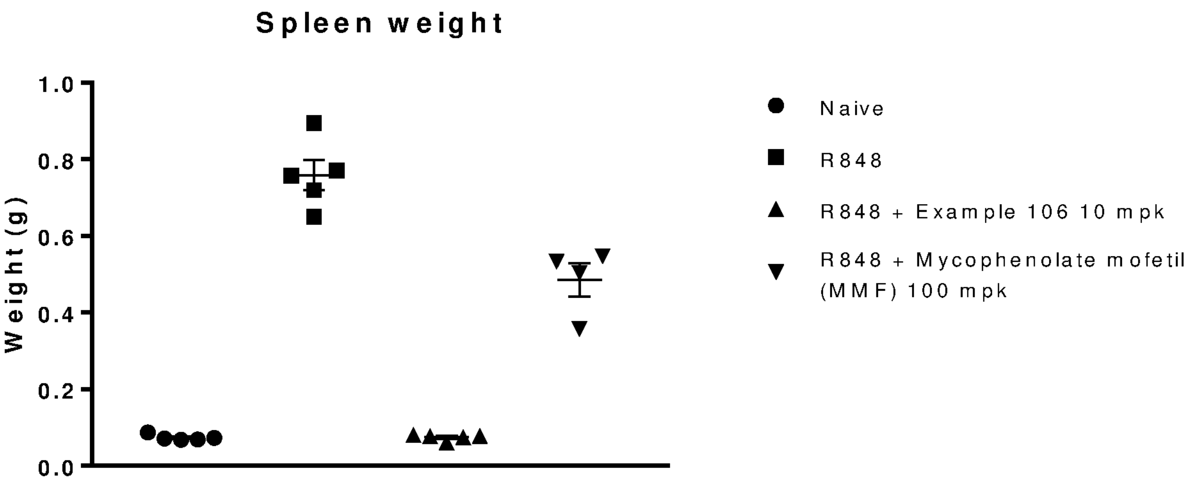
Spleen weight
1.0
0.8
0.6
0.4
0.2
0.0
Weight (g)
Naive
R848
R848 + Example 106 10 mpk
R848 + Mycophenolate mofetil (MMF) 100 mpk

FIG.3D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017106607 A
- WO 2014041007 A1
- WO 20140320 A1


### Non-patent literature cited in the description


- **NAVARRA ; S. V. et al.** *Lancet,* 2011, vol. 377, 721
- **KRIEG ; A. M. et al.** *Immunol. Rev.,* 2007, vol. 220, 251
- **JIMÉNEZ-DALMARONI ; M. J. et al.** *Autoimmun Rev.,* 2016, vol. 15, 1
- **CHEN ; J. Q. et al.** *Clinical Reviews in Allergy & Immunology,* 2016, vol. 50, 1
- *Acc. Chem. Res.,* 1998, vol. 31, 805-818
- *Chem. Rev.,* 2016, vol. 116, 12564-12649
- *Topics in Current Chemistry,* 2002, vol. 219, 131-209
- **TRISTANI-FIROUZI M.** hERG potassium channels and cardiac arrhythmia. *Nature,* 2006, vol. 440, 463-469
- **WEBSTER R ; LEISHMAN D ; WALKER D.** Towards a drug concentration effect relationship for QT prolongation and torsades de pointes. *Curr. Opin. Drug Discov. Devel.,* 2002, vol. 5, 116-26
- **BORENFREUND, E ; PUERNER JA.** Toxicity determined in vitro by morphological alterations and Neutral Red absorption. *Toxicology Lett,* 1985, vol. 24, 119-124
- **LIEBSCH M ; SPIELMANN H ; BALLS M ; BRAND M ; DÖRING B ; DUPUIS J ; HOLZHÜTER HG ; KLECAK G ; L.EPLATTENIER H ; LOVELL W.** First results of the EC/COLIPA Validation Project. In Vitro Phototoxicity Testing. In. *In Vitro Skin Toxicology: Irritation, Phototoxicity, Sensitization,* vol. 10
- Alternative Methods in Toxicology. Mary Ann Liebert Publ, 1994, 243-251
- **SPIELMANN H ; BALLS M ; DUPUIS J ; PAPE WJW ; PECHOVITCH G ; SILVA DEO ; HOLZHÜTTER ; HG ; CLOTHIER R ; DESOLLE P.** The international EU/COLIPA in vitro phototoxicity validation study: Results of phase II. *The 3T3 NRU phototoxicity test. Toxicology in Vitro,* 1998, vol. 12, 305-327